# EUROPEAN PATENT SPECIFICATION

(11) **EP 1 285 002 B1**
(45) Date of publication and mention of the grant of the patent: **18.07.2007**
(21) Application number: 01948240.5
(22) Date of filing: 25.05.2001
(51) Int. Cl.: C07K 14/705, G01N 33/92, A61P 3/06

(54) **REGULATING LIPID LEVELS VIA THE ZMAX1 OR HBM GENE**
REGULATION DES LIPIDNIVEAUS ÜBER DIE GENE ZMAX1 ODER HBM
REGULATION DES TAUX LIPIDIQUES PAR L'INTERMEDIAIRE DU GENE ZMAX1 OU HBM

(30) Priority: 26.05.2000 US 578900
(43) Date of publication of application: 26.02.2003
(73) Proprietor: Oscient Pharmaceuticals Corporation, Waltham MA 02415 (US); CREIGHTON UNIVERSITY, Omaha, NE 68178 (US)
(72) Inventor: CARULLI, John, P., Southboro, MA 01772 (US); LITTLE, Randall, D., Newtonville, MA 02460 (US); RECKER, Robert, R., Omaha, NE 68144 (US); JOHNSON, Mark, L., Omaha, NE 68135 (US)
(74) Representative: Duckworth, Timothy John
(86) International application number: PCT/US2001/016946
(87) International publication number: WO 2001/092891

(56) References cited:
- WO-A-98/46743
- WO-A-02/092000
- HEY, P.J. ET AL.: "Cloning of a novel member of the low-density lipoprotein receptor family" GENE, vol. 216, 1998, pages 103-111, XP002198408
- DONG YU ET AL: "Molecular cloning and characterization of LR3, a novel LDL receptor family protein with mitogenic activity." BIOCHEMICAL AND BIOPHYSICAL RESEARCH COMMUNICATIONS, vol. 25, no. 3, 29 October 1998 (1998-10-29), pages 784-790, XP001076809 ISSN: 0006-291X
- WILSON ET AL.: "Harrison's Princilples of Internal Medicine, 12th Edition" 1991, MCGRAW-HILL, INC. , NEW YORK * page 992 - page 994 *
- Zabaglia et al., Cad Saude Publica, 14:779-785, 1990

### SEQUENCE LISTING

<110> John P. Carulli et al.
<120> REGULATING LIPID LEVELS VIA THE ZMAX1 or HBM GENE
<130> 032796-019
<150> Unassigned
   <151> 2000-05-26
<150> US 09/543,771
   <151> 2000-04-05
<150> US 09/544,398
   <151> 2000-04-05
<160> 62
<210> 1
   <211> 5120
   <212> DNA
   <213 > Homo sapiens
<400> 1
   actaaagcgc cgccgccgcg ccatggagcc cgagtgagcg cggcgcgggc ccgtccggcc 60
   gccggacaac atg gag gca gcg ccg ccc ggg ccg ccg tgg ccg ctg ctg 109
   ctg ctg ctg ctg ctg ctg ctg gcg ctg tgc ggc tgc ccg gcc ccc gcc 157
   gcg gcc tcg ccg ctc ctg cta ttt gcc aac cgc cgg gac gta cgg ctg 205
   gtg gac gcc ggc gga gtc aag ctg gag tcc acc atc gtg gtc agc ggc 253
   ctg gag gat gcg gcc gca gtg gac ttc cag ttt tcc aag gga gcc gtg 301
   tac tgg aca gac gtg agc gag gag gcc atc aag cag acc tac ctg aac 349
   cag acg ggg gcc gcc gtg cag aac gtg gtc atc tcc ggc ctg gtc tct 397
   ccc gac ggc ctc gcc tgc gac tgg gtg ggc aag aag ctg tac tgg acg 445
   gac tca gag acc aac cgc atc gag gtg gcc aac ctc aat ggc aca tcc 493
   cgg aag gtg ctc ttc tgg cag gac ctt gac cag ccg agg gcc atc gcc 541
   ttg gac ccc gct cac ggg tac atg tac tgg aca gac tgg ggt gag acg 589
   ccc cgg att gag cgg gca ggg atg gat ggc agc acc cgg aag atc att 637
   gtg gac tcg gac att tac tgg ccc aat gga ctg acc atc gac ctg gag 685
   gag cag aag ctc tac tgg gct gac gcc aag ctc agc ttc atc cac cgt 733
   gcc aac ctg gac ggc tcg ttc cgg cag aag gtg gtg gag ggc agc ctg 781
   acg cac ccc ttc gcc ctg acg ctc tcc ggg gac act ctg tac tgg aca 829
   gac tgg cag acc cgc tcc atc cat gcc tgc aac aag cgc act ggg ggg 877
   aag agg aag gag atc ctg agt gcc ctc tac tca ccc atg gac atc cag 925
   gtg ctg agc cag gag cgg cag cct ttc ttc cac act cgc tgt gag gag 973
   gac aat ggc ggc tgc tcc cac ctg tgc ctg ctg tcc cca agc gag cct 1021
   ttc tac aca tgc gcc tgc ccc acg ggt gtg cag ctg cag gac aac ggc 1069
   agg acg tgt aag gca gga gcc gag gag gtg ctg ctg ctg gcc cgg cgg 1117
   acg gac cta cgg agg atc tcg ctg gac acg ccg gac ttc acc gac atc 1165
   gtg ctg cag gtg gac gac atc cgg cac gcc att gcc atc gac tac gac 1213
   ccg cta gag ggc tat gtc tac tgg aca gat gac gag gtg cgg gcc atc 1261
   cgc agg gcg tac ctg gac ggg tct ggg gcg cag acg ctg gtc aac acc 1309
   gag atc aac gac ccc gat ggc atc gcg gtc gac tgg gtg gcc cga aac 1357
   ctc tac tgg acc gac acg ggc acg gac cgc atc gag gtg acg cgc ctc 1405
   aac ggc acc tcc cgc aag atc ctg gtg tcg gag gac ctg gac gag ccc 1453
   cga gcc atc gca ctg cac ccc gtg atg ggc ctc atg tac tgg aca gac 1501
   tgg gga gag aac cct aaa atc gag tgt gcc aac ttg gat ggg cag gag 1549
   cgg cgt gtg ctg gtc aat gcc tcc ctc ggg tgg ccc aac ggc ctg gcc 1597
   ctg gac ctg cag gag ggg aag ctc tac tgg gga gac gcc aag aca gac 1645
   aag atc gag gtg atc aat gtt gat ggg acg aag agg cgg acc ctc ctg 1693
   gag gac aag ctc ccg cac att ttc ggg ttc acg ctg ctg ggg gac ttc 1741
   atc tac tgg act gac tgg cag cgc cgc agc atc gag cgg gtg cac aag 1789
   gtc aag gcc agc cgg gac gtc atc att gac cag ctg ccc gac ctg atg 1837
   ggg ctc aaa gct gtg aat gtg gcc aag gtc gtc gga acc aac ccg tgt 1885
   gcg gac agg aac ggg ggg tgc agc cac ctg tgc ttc ttc aca ccc cac 1933
   gca acc cgg tgt ggc tgc ccc atc ggc ctg gag ctg ctg agt gac atg 1981
   aag acc tgc atc gtg cct gag gcc ttc ttg gtc ttc acc agc aga gcc 2029
   gcc atc cac agg atc tcc ctc gag acc aat aac aac gac gtg gcc atc 2077
   ccg ctc acg ggc gtc aag gag gcc tca gcc ctg gac ttt gat gtg tcc 2125
   aac aac cac atc tac tgg aca gac gtc agc ctg aag acc atc agc cgc 2173
   gcc ttc atg aac ggg agc tcg gtg gag cac gtg gtg gag ttt ggc ctt 2221
   gac tac ccc gag ggc atg gcc gtt gac tgg atg ggc aag aac ctc tac 2269
   tgg gcc gac act ggg acc aac aga atc gaa gtg gcg cgg ctg gac ggg 2317
   cag ttc cgg caa gtc ctc gtg tgg agg gac ttg gac aac ccg agg tcg 2365
   ctg gcc ctg gat ccc acc aag ggc tac atc tac tgg acc gag tgg ggc 2413
   ggc aag ccg agg atc gtg cgg gcc ttc atg gac ggg acc aac tgc atg 2461
   acg ctg gtg gac aag gtg ggc cgg gcc aac gac ctc acc att gac tac 2509
   gct gac cag cgc ctc tac tgg acc gac ctg gac acc aac atg atc gag 2557
   tcg tcc aac atg ctg ggt cag gag cgg gtc gtg att gcc gac gat ctc 2605
   ccg cac ccg ttc ggt ctg acg cag tac agc gat tat atc tac tgg aca 2653
   gac tgg aat ctg cac agc att gag cgg gcc gac aag act agc ggc cgg 2701
   aac cgc acc ctc atc cag ggc cac ctg gac ttc gtg atg gac atc ctg 2749
   gtg ttc cac tcc tcc cgc cag gat ggc ctc aat gac tgt atg cac aac 2797
   aac ggg cag tgt ggg cag ctg tgc ctt gcc atc ccc ggc ggc cac cgc 2845
   tgc ggc tgc gcc tca cac tac acc ctg gac ccc agc agc cgc aac tgc 2893
   agc ccg ccc acc acc ttc ttg ctg ttc agc cag aaa tct gcc atc agt 2941
   cgg atg atc ccg gac gac cag cac agc ccg gat ctc atc ctg ccc ctg 2989
   cat gga ctg agg aac gtc aaa gcc atc gac tat gac cca ctg gac aag 3037
   ttc atc tac tgg gtg gat ggg cgc cag aac atc aag cga gcc aag gac 3085
   gac ggg acc cag ccc ttt gtt ttg acc tct ctg agc caa ggc caa aac 3133
   cca gac agg cag ccc cac gac ctc agc atc gac atc tac agc cgg aca 3181
   ctg ttc tgg acg tgc gag gcc acc aat acc atc aac gtc cac agg ctg 3229
   agc ggg gaa gcc atg ggg gtg gtg ctg cgt ggg gac cgc gac aag ccc 3277
   agg gcc atc gtc gtc aac gcg gag cga ggg tac ctg tac ttc acc aac 3325
   atg cag gac cgg gca gcc aag atc gaa cgc gca gcc ctg gac ggc acc 3373
   gag cgc gag gtc ctc ttc acc acc ggc ctc atc cgc cct gtg gcc ctg 3421
   gtg gtg gac aac aca ctg ggc aag ctg ttc tgg gtg gac gcg gac ctg 3469
   aag cgc att gag agc tgt gac ctg tca ggg gcc aac cgc ctg acc ctg 3517
   gag gac gcc aac atc gtg cag cct ctg ggc ctg acc atc ctt ggc aag 3565
   cat ctc tac tgg atc gac cgc cag cag cag atg atc gag cgt gtg gag 3613
   aag acc acc ggg gac aag cgg act cgc atc cag ggc cgt gtc gcc cac 3661
   ctc act ggc atc cat gca gtg gag gaa gtc agc ctg gag gag ttc tca 3709
   gcc cac cca tgt gcc cgt gac aat ggt ggc tgc tcc cac atc tgt att 3757
   gcc aag ggt gat ggg aca cca cgg tgc tca tgc cca gtc cac ctc gtg 3805
   ctc ctg cag aac ctg ctg acc tgt gga gag ccg ccc acc tgc tcc ccg 3853
   gac cag ttt gca tgt gcc aca ggg gag atc gac tgt atc ccc ggg gcc 3901
   tgg cgc tgt gac ggc ttt ccc gag tgc gat gac cag agc gac gag gag 3949
   ggc tgc ccc gtg tgc tcc gcc gcc cag ttc ccc tgc gcg cgg ggt cag 3997
   tgt gtg gac ctg cgc ctg cgc tgc gac ggc gag gca gac tgt cag gac 4045
   cgc tca gac gag gtg gac tgt gac gcc atc tgc ctg ccc aac cag ttc 4093
   cgg tgt gcg agc ggc cag tgt gtc ctc atc aaa cag cag tgc gac tcc 4141
   ttc ccc gac tgt atc gac ggc tcc gac gag ctc atg tgt gaa atc acc 4189
   aag ccg ccc tca gac gac agc ccg gcc cac agc agt gcc atc ggg ccc 4237
   gtc att ggc atc atc ctc tct ctc ttc gtc atg ggt ggt gtc tat ttt 4285
   gtg tgc cag cgc gtg gtg tgc cag cgc tat gcg ggg gcc aac ggg ccc 4333
   ttc ccg cac gag tat gtc agc ggg acc ccg cac gtg ccc ctc aat ttc 4381
   ata gcc ccg ggc ggt tcc cag cat ggc ccc ttc aca ggc atc gca tgc 4429
   gga aag tcc atg atg agc tcc gtg agc ctg atg ggg ggc cgg ggc ggg 4477
   gtg ccc ctc tac gac cgg aac cac gtc aca ggg gcc tcg tcc agc agc 4525
   tcg tcc agc acg aag gcc acg ctg tac ccg ccg atc ctg aac ccg ccg 4573
   ccc tcc ccg gcc acg gac ccc tcc ctg tac aac atg gac atg ttc tac 4621
   tct tca aac att ccg gcc act gcg aga ccg tac agg ccc tac atc att 4669
   cga gga atg gcg ccc ccg acg acg ccc tgc agc acc gac gtg tgt gac 4717
   agc gac tac agc gcc agc cgc tgg aag gcc agc aag tac tac ctg gat 4765
   ttg aac tcg gac tca gac ccc tat cca ccc cca ccc acg ccc cac agc 4813
   cag tac ctg tcg gcg gag gac agc tgc ccg ccc tcg ccc gcc acc gag 4861
   agg agc tac ttc cat ctc ttc ccg ccc cct ccg tcc ccc tgc acg gac 4909
   tca tcc tgacctcggc cgggccactc tggcttctct gtgcccctgt aaatagtttt 4965
   aaatatgaac aaagaaaaaa atatatttta tgatttaaaa aataaatata attgggattt 5025
   taaaaacatg agaaatgtga actgtgatgg ggtgggcagg gctgggagaa ctttgtacag 5085
   tggagaaata tttataaact taattttgta aaaca 5120
<210> 2
   <211> 5120
   <212> DNA
   <213> Homo sapiens
<400> 2
   actaaagcgc cgccgccgcg ccatggagcc cgagtgagcg cggcgcgggc ccgtccggcc 60
   gccggacaac atg gag gca gcg ccg ccc ggg ccg ccg tgg ccg ctg ctg 109
   Met Glu Ala Ala Pro Pro Gly Pro Pro Trp Pro Leu Leu
   1 5 10
   ctg ctg ctg ctg ctg ctg ctg gcg ctg tgc ggc tgc ccg gcc ccc gcc 157
   Leu Leu Leu Leu Leu Leu Leu Ala Leu Cys Gly Cys Pro Ala Pro Ala
   15 20 25
   gcg gcc tcg ccg ctc ctg cta ttt gcc aac cgc cgg gac gta cgg ctg 205
   Ala Ala Ser Pro Leu Leu Leu Phe Ala Asn Arg Arg Asp Val Arg Leu
   30 35 40 45
   gtg gac gcc ggc gga gtc aag ctg gag tcc acc atc gtg gtc agc ggc 253
   Val Asp Ala Gly Gly Val Lys Leu Glu Ser Thr Ile Val Val Ser Gly
   50 55 60
   ctg gag gat gcg gcc gca gtg gac ttc cag ttt tcc aag gga gcc gtg 301
   Leu Glu Asp Ala Ala Ala Val Asp Phe Gln Phe Ser Lys Gly Ala Val
   65 70 75
   tac tgg aca gac gtg agc gag gag gcc atc aag cag acc tac ctg aac 349
   Tyr Trp Thr Asp Val Ser Glu Glu Ala Ile Lys Gln Thr Tyr Leu Asn
   80 85 90
   cag acg ggg gcc gcc gtg cag aac gtg gtc atc tcc ggc ctg gtc tct 397
   Gln Thr Gly Ala Ala Val Gln Asn Val Val Ile Ser Gly Leu Val Ser
   95 100 105
   ccc gac ggc ctc gcc tgc gac tgg gtg ggc aag aag ctg tac tgg acg 445
   Pro Asp Gly Leu Ala Cys Asp Trp Val Gly Lys Lys Leu Tyr Trp Thr
   110 115 120 125
   gac tca gag acc aac cgc atc gag gtg gcc aac ctc aat ggc aca tcc 493
   Asp Ser Glu Thr Asn Arg Ile Glu Val Ala Asn Leu Asn Gly Thr Ser
   130 135 140
   cgg aag gtg ctc ttc tgg cag gac ctt gac cag ccg agg gcc atc gcc 541
   Arg Lys Val Leu Phe Trp Gln Asp Leu Asp Gln Pro Arg Ala Ile Ala
   145 150 155
   ttg gac ccc gct cac ggg tac atg tac tgg aca gac tgg gtt gag acg 589
   Leu Asp Pro Ala His Gly Tyr Met Tyr Trp Thr Asp Trp Val Glu Thr
   160 165 170
   ccc cgg att gag cgg gca ggg atg gat ggc agc acc cgg aag atc att 637
   Pro Arg Ile Glu Arg Ala Gly Met Asp Gly Ser Thr Arg Lys Ile Ile
   175 180 185
   gtg gac tcg gac att tac tgg ccc aat gga ctg acc atc gac ctg gag 685
   Val Asp Ser Asp Ile Tyr Trp Pro Asn Gly Leu Thr Ile Asp Leu Glu
   190 195 200 205
   gag cag aag ctc tac tgg gct gac gcc aag ctc agc ttc atc cac cgt 733
   Glu Gln Lys Leu Tyr Trp Ala Asp Ala Lys Leu Ser Phe Ile His Arg
   210 215 220
   gcc aac ctg gac ggc tcg ttc cgg cag aag gtg gtg gag ggc agc ctg 781
   Ala Asn Leu Asp Gly Ser Phe Arg Gln Lys Val Val Glu Gly Ser Leu
   225 230 235
   acg cac ccc ttc gcc ctg acg ctc tcc ggg gac act ctg tac tgg aca 829
   Thr His Pro Phe Ala Leu Thr Leu Ser Gly Asp Thr Leu Tyr Trp Thr
   240 245 250
   gac tgg cag acc cgc tcc atc cat gcc tgc aac aag cgc act ggg ggg 877
   Asp Trp Gln Thr Arg Ser Ile His Ala Cys Asn Lys Arg Thr Gly Gly
   255 260 265
   aag agg aag gag atc ctg agt gcc ctc tac tca ccc atg gac atc cag 925
   Lys Arg Lys Glu Ile Leu Ser Ala Leu Tyr Ser Pro Met Asp Ile Gln
   270 275 280 285
   gtg ctg agc cag gag cgg cag cct ttc ttc cac act cgc tgt gag gag 973
   Val Leu Ser Gln Glu Arg Gln Pro Phe Phe His Thr Arg Cys Glu Glu
   290 295 300
   gac aat ggc ggc tgc tcc cac ctg tgc ctg ctg tcc cca agc gag cct 1021
   Asp Asn Gly Gly Cys Ser His Leu Cys Leu Leu Ser Pro Ser Glu Pro
   305 310 315
   ttc tac aca tgc gcc tgc ccc acg ggt gtg cag ctg cag gac aac ggc 1069
   Phe Tyr Thr Cys Ala Cys Pro Thr Gly Val Gln Leu Gln Asp Asn Gly
   320 325 330
   agg acg tgt aag gca gga gcc gag gag gtg ctg ctg ctg gcc cgg cgg 1117
   Arg Thr Cys Lys Ala Gly Ala Glu Glu Val Leu Leu Leu Ala Arg Arg
   335 340 345
   acg gac cta cgg agg atc tcg ctg gac acg ccg gac ttc acc gac atc 1165
   Thr Asp Leu Arg Arg Ile Ser Leu Asp Thr Pro Asp Phe Thr Asp Ile
   350 355 360 365
   gtg ctg cag gtg gac gac atc cgg cac gcc att gcc atc gac tac gac 1213
   Val Leu Gln Val Asp Asp Ile Arg His Ala Ile Ala Ile Asp Tyr Asp
   370 375 380
   ccg cta gag ggc tat gtc tac tgg aca gat gac gag gtg cgg gcc atc 1261
   Pro Leu Glu Gly Tyr Val Tyr Trp Thr Asp Asp Glu Val Arg Ala Ile
   385 390 395
   cgc agg gcg tac ctg gac ggg tct ggg gcg cag acg ctg gtc aac acc 1309
   Arg Arg Ala Tyr Leu Asp Gly Ser Gly Ala Gln Thr Leu Val Asn Thr
   400 405 410
   gag atc aac gac ccc gat ggc atc gcg gtc gac tgg gtg gcc cga aac 1357
   Glu Ile Asn Asp Pro Asp Gly Ile Ala Val Asp Trp Val Ala Arg Asn
   415 420 425
   ctc tac tgg acc gac acg ggc acg gac cgc atc gag gtg acg cgc ctc 1405
   Leu Tyr Trp Thr Asp Thr Gly Thr Asp Arg Ile Glu Val Thr Arg Leu
   430 435 440 445
   aac ggc acc tcc cgc aag atc ctg gtg tcg gag gac ctg gac gag ccc 1453
   Asn Gly Thr Ser Arg Lys Ile Leu Val Ser Glu Asp Leu Asp Glu Pro
   450 455 460
   cga gcc atc gca ctg cac ccc gtg atg ggc ctc atg tac tgg aca gac 1501
   Arg Ala Ile Ala Leu His Pro Val Met Gly Leu Met Tyr Trp Thr Asp
   465 470 475
   tgg gga gag aac cct aaa atc gag tgt gcc aac ttg gat ggg cag gag 1549
   Trp Gly Glu Asn Pro Lys Ile Glu Cys Ala Asn Leu Asp Gly Gln Glu
   480 485 490
   cgg cgt gtg ctg gtc aat gcc tcc ctc ggg tgg ccc aac ggc ctg gcc 1597
   Arg Arg Val Leu Val Asn Ala Ser Leu Gly Trp Pro Asn Gly Leu Ala
   495 500 505
   ctg gac ctg cag gag ggg aag ctc tac tgg gga gac gcc aag aca gac 1645
   Leu Asp Leu Gln Glu Gly Lys Leu Tyr Trp Gly Asp Ala Lys Thr Asp
   510 515 520 525
   aag atc gag gtg atc aat gtt gat ggg acg aag agg cgg acc ctc ctg 1693
   Lys Ile Glu Val Ile Asn Val Asp Gly Thr Lys Arg Arg Thr Leu Leu
   530 535 540
   gag gac aag ctc ccg cac att ttc ggg ttc acg ctg ctg ggg gac ttc 1741
   Glu Asp Lys Leu Pro His Ile Phe Gly Phe Thr Leu Leu Gly Asp Phe
   545 550 555
   atc tac tgg act gac tgg cag cgc cgc agc atc gag cgg gtg cac aag 1789
   Ile Tyr Trp Thr Asp Trp Gln Arg Arg Ser Ile Glu Arg Val His Lys
   560 565 570
   gtc aag gcc agc cgg gac gtc atc att gac cag ctg ccc gac ctg atg 1837
   Val Lys Ala Ser Arg Asp Val Ile Ile Asp Gln Leu Pro Asp Leu Met
   575 580 585
   ggg ctc aaa gct gtg aat gtg gcc aag gtc gtc gga acc aac ccg tgt 1885
   Gly Leu Lys Ala Val Asn Val Ala Lys Val Val Gly Thr Asn Pro Cys
   590 595 600 605
   gcg gac agg aac ggg ggg tgc agc cac ctg tgc ttc ttc aca ccc cac 1933
   Ala Asp Arg Asn Gly Gly Cys Ser His Leu Cys Phe Phe Thr Pro His
   610 615 620
   gca acc cgg tgt ggc tgc ccc atc ggc ctg gag ctg ctg agt gac atg 1981
   Ala Thr Arg Cys Gly Cys Pro Ile Gly Leu Glu Leu Leu Ser Asp Met
   625 630 635
   aag acc tgc atc gtg cct gag gcc ttc ttg gtc ttc acc agc aga gcc 2029
   Lys Thr Cys Ile Val Pro Glu Ala Phe Leu Val Phe Thr Ser Arg Ala
   640 645 650
   gcc atc cac agg atc tcc ctc gag acc aat aac aac gac gtg gcc atc 2077
   Ala Ile His Arg Ile Ser Leu Glu Thr Asn Asn Asn Asp Val Ala Ile
   655 660 665
   ccg ctc acg ggc gtc aag gag gcc tca gcc ctg gac ttt gat gtg tcc 2125
   Pro Leu Thr Gly Val Lys Glu Ala Ser Ala Leu Asp Phe Asp Val Ser
   670 675 680 685
   aac aac cac atc tac tgg aca gac gtc agc ctg aag acc atc agc cgc 2173
   Asn Asn His Ile Tyr Trp Thr Asp Val Ser Leu Lys Thr Ile Ser Arg
   690 695 700
   gcc ttc atg aac ggg agc tcg gtg gag cac gtg gtg gag ttt ggc ctt 2221
   Ala Phe Met Asn Gly Ser Ser Val Glu His Val Val Glu Phe Gly Leu
   705 710 715
   gac tac ccc gag ggc atg gcc gtt gac tgg atg ggc aag aac ctc tac 2269
   Asp Tyr Pro Glu Gly Met Ala Val Asp Trp Met Gly Lys Asn Leu Tyr
   720 725 730
   tgg gcc gac act ggg acc aac aga atc gaa gtg gcg cgg ctg gac ggg 2317
   Trp Ala Asp Thr Gly Thr Asn Arg Ile Glu Val Ala Arg Leu Asp Gly
   735 740 745
   cag ttc cgg caa gtc ctc gtg tgg agg gac ttg gac aac ccg agg tcg 2365
   Gln Phe Arg Gln Val Leu Val Trp Arg Asp Leu Asp Asn Pro Arg Ser
   750 755 760 765
   ctg gcc ctg gat ccc acc aag ggc tac atc tac tgg acc gag tgg ggc 2413
   Leu Ala Leu Asp Pro Thr Lys Gly Tyr Ile Tyr Trp Thr Glu Trp Gly
   770 775 780
   ggc aag ccg agg atc gtg cgg gcc ttc atg gac ggg acc aac tgc atg 2461
   Gly Lys Pro Arg Ile Val Arg Ala Phe Met Asp Gly Thr Asn Cys Met
   785 790 795
   acg ctg gtg gac aag gtg ggc cgg gcc aac gac ctc acc att gac tac 2509
   Thr Leu Val Asp Lys Val Gly Arg Ala Asn Asp Leu Thr Ile Asp Tyr
   800 805 810
   gct gac cag cgc ctc tac tgg acc gac ctg gac acc aac atg atc gag 2557
   Ala Asp Gln Arg Leu Tyr Trp Thr Asp Leu Asp Thr Asn Met Ile Glu
   815 820 825
   tcg tcc aac atg ctg ggt cag gag cgg gtc gtg att gcc gac gat ctc 2605
   Ser Ser Asn Met Leu Gly Gln Glu Arg Val Val Ile Ala Asp Asp Leu
   830 835 840 845
   ccg cac ccg ttc ggt ctg acg cag tac agc gat tat atc tac tgg aca 2653
   Pro His Pro Phe Gly Leu Thr Gln Tyr Ser Asp Tyr Ile Tyr Trp Thr
   850 855 860
   gac tgg aat ctg cac agc att gag cgg gcc gac aag act agc ggc cgg 2701
   Asp Trp Asn Leu His Ser Ile Glu Arg Ala Asp Lys Thr Ser Gly Arg
   865 870 875
   aac cgc acc ctc atc cag ggc cac ctg gac ttc gtg atg gac atc ctg 2749
   Asn Arg Thr Leu Ile Gln Gly His Leu Asp Phe Val Met Asp Ile Leu
   880 885 890
   gtg ttc cac tcc tcc cgc cag gat ggc ctc aat gac tgt atg cac aac 2797
   Val Phe His Ser Ser Arg Gln Asp Gly Leu Asn Asp Cys Met His Asn
   895 900 905
   aac ggg cag tgt ggg cag ctg tgc ctt gcc atc ccc ggc ggc cac cgc 2845
   Asn Gly Gln Cys Gly Gln Leu Cys Leu Ala Ile Pro Gly Gly His Arg
   910 915 920 925
   tgc ggc tgc gcc tca cac tac acc ctg gac ccc agc agc cgc aac tgc 2893
   Cys Gly Cys Ala Ser His Tyr Thr Leu Asp Pro Ser Ser Arg Asn Cys
   930 935 940
   agc ccg ccc acc acc ttc ttg ctg ttc agc cag aaa tct gcc atc agt 2941
   Ser Pro Pro Thr Thr Phe Leu Leu Phe Ser Gln Lys Ser Ala Ile Ser
   945 950 955
   cgg atg atc ccg gac gac cag cac agc ccg gat ctc atc ctg ccc ctg 2989
   Arg Met Ile Pro Asp Asp Gln His Ser Pro Asp Leu Ile Leu Pro Leu
   960 965 970
   cat gga ctg agg aac gtc aaa gcc atc gac tat gac cca ctg gac aag 3037
   His Gly Leu Arg Asn Val Lys Ala Ile Asp Tyr Asp Pro Leu Asp Lys
   975 980 985
   ttc atc tac tgg gtg gat ggg cgc cag aac atc aag cga gcc aag gac 3085
   Phe Ile Tyr Trp Val Asp Gly Arg Gln Asn Ile Lys Arg Ala Lys Asp
   990 995 1000 1005
   gac ggg acc cag ccc ttt gtt ttg acc tct ctg agc caa ggc caa aac 3133
   Asp Gly Thr Gln Pro Phe Val Leu Thr Ser Leu Ser Gln Gly Gln Asn
   1010 1015 1020
   cca gac agg cag ccc cac gac ctc agc atc gac atc tac agc cgg aca 3181
   Pro Asp Arg Gln Pro His Asp Leu Ser Ile Asp Ile Tyr Ser Arg Thr
   1025 1030 1035
   ctg ttc tgg acg tgc gag gcc acc aat acc atc aac gtc cac agg ctg 3229
   Leu Phe Trp Thr Cys Glu Ala Thr Asn Thr Ile Asn Val His Arg Leu
   1040 1045 1050
   agc ggg gaa gcc atg ggg gtg gtg ctg cgt ggg gac cgc gac aag ccc 3277
   Ser Gly Glu Ala Met Gly Val Val Leu Arg Gly Asp Arg Asp Lys Pro
   1055 1060 1065
   agg gcc atc gtc gtc aac gcg gag cga ggg tac ctg tac ttc acc aac 3325
   Arg Ala Ile Val Val Asn Ala Glu Arg Gly Tyr Leu Tyr Phe Thr Asn
   1070 1075 1080 1085
   atg cag gac cgg gca gcc aag atc gaa cgc gca gcc ctg gac ggc acc 3373
   Met Gln Asp Arg Ala Ala Lys Ile Glu Arg Ala Ala Leu Asp Gly Thr
   1090 1095 1100
   gag cgc gag gtc ctc ttc acc acc ggc ctc atc cgc cct gtg gcc ctg 3421
   Glu Arg Glu Val Leu Phe Thr Thr Gly Leu Ile Arg Pro Val Ala Leu
   1105 1110 1115
   gtg gtg gac aac aca ctg ggc aag ctg ttc tgg gtg gac gcg gac ctg 3469
   Val Val Asp Asn Thr Leu Gly Lys Leu Phe Trp Val Asp Ala Asp Leu
   1120 1125 1130
   aag cgc att gag agc tgt gac ctg tca ggg gcc aac cgc ctg acc ctg 3517
   Lys Arg Ile Glu Ser Cys Asp Leu Ser Gly Ala Asn Arg Leu Thr Leu
   1135 1140 1145
   gag gac gcc aac atc gtg cag cct ctg ggc ctg acc atc ctt ggc aag 3565
   Glu Asp Ala Asn Ile Val Gln Pro Leu Gly Leu Thr Ile Leu Gly Lys
   1150 1155 1160 1165
   cat ctc tac tgg atc gac cgc cag cag cag atg atc gag cgt gtg gag 3613
   His Leu Tyr Trp Ile Asp Arg Gln Gln Gln Met Ile Glu Arg Val Glu
   1170 1175 1180
   aag acc acc ggg gac aag cgg act cgc atc cag ggc cgt gtc gcc cac 3661
   Lys Thr Thr Gly Asp Lys Arg Thr Arg Ile Gln Gly Arg Val Ala His
   1185 1190 1195
   ctc act ggc atc cat gca gtg gag gaa gtc agc ctg gag gag ttc tca 3709
   Leu Thr Gly Ile His Ala Val Glu Glu Val Ser Leu Glu Glu Phe Ser
   1200 1205 1210
   gcc cac cca tgt gcc cgt gac aat ggt ggc tgc tcc cac atc tgt att 3757
   Ala His Pro Cys Ala Arg Asp Asn Gly Gly Cys Ser His Ile Cys Ile
   1215 1220 1225
   gcc aag ggt gat ggg aca cca cgg tgc tca tgc cca gtc cac ctc gtg 3805
   Ala Lys Gly Asp Gly Thr Pro Arg Cys Ser Cys Pro Val His Leu Val
   1230 1235 1240 1245
   ctc ctg cag aac ctg ctg acc tgt gga gag ccg ccc acc tgc tcc ccg 3853
   Leu Leu Gln Asn Leu Leu Thr Cys Gly Glu Pro Pro Thr Cys Ser Pro
   1250 1255 1260
   gac cag ttt gca tgt gcc aca ggg gag atc gac tgt atc ccc ggg gcc 3901
   Asp Gln Phe Ala Cys Ala Thr Gly Glu Ile Asp Cys Ile Pro Gly Ala
   1265 1270 1275
   tgg cgc tgt gac ggc ttt ccc gag tgc gat gac cag agc gac gag gag 3949
   Trp Arg Cys Asp Gly Phe Pro Glu Cys Asp Asp Gln Ser Asp Glu Glu
   1280 1285 1290
   ggc tgc ccc gtg tgc tcc gcc gcc cag ttc ccc tgc gcg cgg ggt cag 3997
   Gly Cys Pro Val Cys Ser Ala Ala Gln Phe Pro Cys Ala Arg Gly Gln
   1295 1300 1305
   tgt gtg gac ctg cgc ctg cgc tgc gac ggc gag gca gac tgt cag gac 4045
   Cys Val Asp Leu Arg Leu Arg Cys Asp Gly Glu Ala Asp Cys Gln Asp
   1310 1315 1320 1325
   cgc tca gac gag gtg gac tgt gac gcc atc tgc ctg ccc aac cag ttc 4093
   Arg Ser Asp Glu Val Asp Cys Asp Ala Ile Cys Leu Pro Asn Gln Phe
   1330 1335 1340
   cgg tgt gcg agc ggc cag tgt gtc ctc atc aaa cag cag tgc gac tcc 4141
   Arg Cys Ala Ser Gly Gln Cys Val Leu Ile Lys Gln Gln Cys Asp Ser
   1345 1350 1355
   ttc ccc gac tgt atc gac ggc tcc gac gag ctc atg tgt gaa atc acc 4189
   Phe Pro Asp Cys Ile Asp Gly Ser Asp Glu Leu Met Cys Glu Ile Thr
   1360 1365 1370
   aag ccg ccc tca gac gac agc ccg gcc cac agc agt gcc atc ggg ccc 4237
   Lys Pro Pro Ser Asp Asp Ser Pro Ala His Ser Ser Ala Ile Gly Pro
   1375 1380 1385
   gtc att ggc atc atc ctc tct ctc ttc gtc atg ggt ggt gtc tat ttt 4285
   Val Ile Gly Ile Ile Leu Ser Leu Phe Val Met Gly Gly Val Tyr Phe
   1390 1395 1400 1405
   gtg tgc cag cgc gtg gtg tgc cag cgc tat gcg ggg gcc aac ggg ccc 4333
   Val Cys Gln Arg Val Val Cys Gln Arg Tyr Ala Gly Ala Asn Gly Pro
   1410 1415 1420
   ttc ccg cac gag tat gtc agc ggg acc ccg cac gtg ccc ctc aat ttc 4381
   Phe Pro His Glu Tyr Val Ser Gly Thr Pro His Val Pro Leu Asn Phe
   1425 1430 1435
   ata gcc ccg ggc ggt tcc cag cat ggc ccc ttc aca ggc atc gca tgc 4429
   Ile Ala Pro Gly Gly Ser Gln His Gly Pro Phe Thr Gly Ile Ala Cys
   1440 1445 1450
   gga aag tcc atg atg agc tcc gtg agc ctg atg ggg ggc cgg ggc ggg 4477
   Gly Lys Ser Met Met Ser Ser Val Ser Leu Met Gly Gly Arg Gly Gly
   1455 1460 1465
   gtg ccc ctc tac gac cgg aac cac gtc aca ggg gcc tcg tcc agc agc 4525
   Val Pro Leu Tyr Asp Arg Asn His Val Thr Gly Ala Ser Ser Ser Ser
   1470 1475 1480 1485
   tcg tcc agc acg aag gcc acg ctg tac ccg ccg atc ctg aac ccg ccg 4573
   Ser Ser Ser Thr Lys Ala Thr Leu Tyr Pro Pro Ile Leu Asn Pro Pro
   1490 1495 1500
   ccc tcc ccg gcc acg gac ccc tcc ctg tac aac atg gac atg ttc tac 4621
   Pro Ser Pro Ala Thr Asp Pro Ser Leu Tyr Asn Met Asp Met Phe Tyr
   1505 1510 1515
   tct tca aac att ccg gcc act gcg aga ccg tac agg ccc tac atc att 4669
   Ser Ser Asn Ile Pro Ala Thr Ala Arg Pro Tyr Arg Pro Tyr Ile Ile
   1520 1525 1530
   cga gga atg gcg ccc ccg acg acg ccc tgc agc acc gac gtg tgt gac 4717
   Arg Gly Met Ala Pro Pro Thr Thr Pro Cys Ser Thr Asp Val Cys Asp
   1535 1540 1545
   agc gac tac agc gcc agc cgc tgg aag gcc agc aag tac tac ctg gat 4765
   Ser Asp Tyr Ser Ala Ser Arg Trp Lys Ala Ser Lys Tyr Tyr Leu Asp
   1550 1555 1560 1565
   ttg aac tcg gac tca gac ccc tat cca ccc cca ccc acg ccc cac agc 4813
   Leu Asn Ser Asp Ser Asp Pro Tyr Pro Pro Pro Pro Thr Pro His Ser
   1570 1575 1580
   cag tac ctg tcg gcg gag gac agc tgc ccg ccc tcg ccc gcc acc gag 4861
   Gln Tyr Leu Ser Ala Glu Asp Ser Cys Pro Pro Ser Pro Ala Thr Glu
   1585 1590 1595
   agg agc tac ttc cat ctc ttc ccg ccc cct ccg tcc ccc tgc acg gac 4909
   Arg Ser Tyr Phe His Leu Phe Pro Pro Pro Pro Ser Pro Cys Thr Asp
   1600 1605 1610
   tca tcc tgacctcggc cgggccactc tggcttctct gtgcccctgt aaatagtttt 4965
   Ser Ser
   1615
   aaatatgaac aaagaaaaaa atatatttta tgatttaaaa aataaatata attgggattt 5025
   taaaaacatg agaaatgtga actgtgatgg ggtgggcagg gctgggagaa ctttgtacag 5085
   tggagaaata tttataaact taattttgta aaaca 5120
<210> 3
   <211> 1615
   <212> PRT
   <213> Homo sapiens
<400> 3
   Met Glu Ala Ala Pro Pro Gly Pro Pro Trp Pro Leu Leu Leu Leu Leu
   1 5 10 15
   Leu Leu Leu Leu Ala Leu Cys Gly Cys Pro Ala Pro Ala Ala Ala Ser
   20 25 30
   Pro Leu Leu Leu Phe Ala Asn Arg Arg Asp Val Arg Leu Val Asp Ala
   35 40 45
   Gly Gly Val Lys Leu Glu Ser Thr Ile Val Val Ser Gly Leu Glu Asp
   50 55 60
   Ala Ala Ala Val Asp Phe Gln Phe Ser Lys Gly Ala Val Tyr Trp Thr
   65 70 75 80
   Asp Val Ser Glu Glu Ala Ile Lys Gln Thr Tyr Leu Asn Gln Thr Gly
   85 90 95
   Ala Ala Val Gln Asn Val Val Ile Ser Gly Leu Val Ser Pro Asp Gly
   100 105 110
   Leu Ala Cys Asp Trp Val Gly Lys Lys Leu Tyr Trp Thr Asp Ser Glu
   115 120 125
   Thr Asn Arg Ile Glu Val Ala Asn Leu Asn Gly Thr Ser Arg Lys Val
   130 135 140
   Leu Phe Trp Gln Asp Leu Asp Gln Pro Lys Ala Ile Ala Leu Asp Pro
   145 150 155 160
   Ala His Gly Tyr Met Tyr Trp Thr Asp Trp Gly Glu Thr Pro Arg Ile
   165 170 175
   Glu Arg Ala Gly Met Asp Gly Ser Thr Arg Lys Ile Ile Val Asp Ser
   180 185 190
   Asp Ile Tyr Trp Pro Asn Gly Leu Thr Ile Asp Leu Glu Glu Gln Lys
   195 200 205
   Leu Tyr Trp Ala Asp Ala Lys Leu Ser Phe Ile His Arg Ala Asn Leu
   210 215 220
   Asp Gly Ser Phe Arg Gln Lys Val Val Glu Gly Ser Leu Thr His Pro
   225 230 235 240
   Phe Ala Leu Thr Leu Ser Gly Asp Thr Leu Tyr Trp Thr Asp Trp Gln
   245 250 255
   Thr Arg Ser Ile His Ala Cys Asn Lys Arg Thr Gly Gly Lys Arg Lys
   260 265 270
   Glu Ile Leu Ser Ala Leu Tyr Ser Pro Met Asp Ile Gln Val Leu Ser
   275 280 285
   Gln Glu Arg Gln Pro Phe Phe His Thr Arg Cys Glu Glu Asp Asn Gly
   290 295 300
   Gly Trp Ser His Leu Cys Leu Leu Ser Pro Ser Glu Pro Phe Tyr Thr
   305 310 315 320
   Cys Ala Cys Pro Thr Gly Val Gln Met Gln Asp Asn Gly Arg Thr Cys
   325 330 335
   Lys Ala Gly Ala Glu Glu Val Leu Leu Leu Ala Arg Arg Thr Asp Leu
   340 345 350
   Arg Arg Ile Ser Leu Asp Thr Pro Asp Phe Thr Asp Ile Val Leu Gln
   355 360 365
   Val Asp Asp Ile Arg His Ala Ile Ala Ile Asp Tyr Asp Pro Leu Glu
   370 375 380
   Gly Tyr Val Tyr Trp Thr Asp Asp Glu Val Arg Ala Ile Arg Arg Ala
   385 390 395 400
   Tyr Leu Asp Gly Ser Gly Ala Gln Thr Leu Val Asn Thr Glu Ile Asn
   405 410 415
   Asp Pro Asp Gly Ile Ala Val Asp Trp Val Ala Arg Asn Leu Tyr Trp
   420 425 430
   Thr Asp Thr Gly Thr Asp Arg Ile Glu Val Thr Arg Leu Asn Gly Thr
   435 440 445
   Ser Arg Lys Ile Leu Val Ser Glu Asp Leu Asp Glu Pro Arg Ala Ile
   450 455 460
   Ala Leu His Pro Val Met Gly Leu Met Tyr Trp Thr Asp Trp Gly Glu
   465 470 475 480
   Asn Pro Lys Ile Glu Cys Ala Asn Leu Asp Gly Gln Glu Arg Arg Val
   485 490 495
   Leu Val Asn Ala Ser Leu Gly Trp Pro Asn Gly Leu Ala Leu Asp Leu
   500 505 510
   Gln Glu Gly Lys Leu Tyr Trp Gly Asp Ala Lys Thr Asp Lys Ile Glu
   515 520 525
   Val Ile Asn Val Asp Gly Thr Lys Arg Arg Thr Leu Leu Glu Asp Lys
   530 535 540
   Leu Pro His Ile Phe Gly Phe Thr Leu Leu Gly Asp Phe Ile Tyr Trp
   545 550 555 560
   Thr Asp Trp Gln Arg Arg Ser Ile Glu Arg Val His Lys Val Lys Ala
   565 570 575
   Ser Arg Asp Val Ile Ile Asp Gln Leu Pro Asp Leu Met Gly Leu Lys
   580 585 590
   Ala Val Asn Val Ala Lys Val Val Gly Thr Asn Pro Cys Ala Asp Arg
   595 600 605
   Asn Gly Gly Cys Ser His Leu Cys Phe Phe Thr Pro His Ala Thr Arg
   610 615 620
   Cys Gly Cys Pro Ile Gly Leu Glu Leu Leu Ser Asp Met Lys Thr Cys
   625 630 635 640
   Ile Val Pro Glu Ala Phe Leu Val Phe Thr Ser Arg Ala Ala Ile His
   645 650 655
   Arg Ile Ser Leu Glu Thr Asn Asn Asn Asp Val Ala Ile Pro Leu Thr
   660 665 670
   Gly Val Lys Glu Ala Ser Ala Leu Asp Phe Asp Val Ser Asn Asn His
   675 680 685
   Ile Tyr Trp Thr Asp Val Ser Leu Lys Asn Ile Ser Arg Ala Phe Met
   690 695 700
   Asn Gly Ser Ser Val Glu His Val Val Glu Phe Gly Leu Asp Tyr Pro
   705 710 715 720
   Glu Gly Met Ala Val Asp Trp Met Gly Lys Asn Leu Tyr Trp Ala Asp
   725 730 735
   Thr Gly Thr Asn Arg Ile Glu Val Ala Arg Leu Asp Gly Gln Phe Arg
   740 745 750
   Gln Val Leu Val Trp Arg Asp Leu Asp Asn Pro Arg Ser Leu Ala Leu
   755 760 765
   Asp Pro Thr Lys Gly Tyr Ile Tyr Trp Thr Glu Trp Gly Gly Lys Pro
   770 775 780
   Arg Ile Val Arg Ala Phe Met Asp Gly Thr Asn Cys Met Thr Leu Val
   785 790 795 800
   Asp Lys Val Gly Arg Ala Asn Asp Leu Thr Ile Asp Tyr Ala Asp Gln
   805 810 815
   Arg Leu Tyr Trp Thr Asp Leu Asp Thr Asn Met Ile Glu Ser Ser Asn
   820 825 830
   Met Leu Gly Gln Glu Arg Val Val Ile Ala Asp Asp Leu Pro His Pro
   835 840 845
   Phe Gly Leu Thr Gln Tyr Ser Asp Tyr Ile Tyr Trp Thr Asp Trp Asn
   850 855 860
   Leu His Ser Ile Glu Arg Ala Asp Lys Thr Ser Gly Arg Asn Arg Thr
   865 870 875 880
   Leu Ile Gln Gly His Leu Asp Phe Val Met Asp Ile Leu Val Phe His
   885 890 895
   Ser Ser Arg Gln Asp Gly Leu Asn Asp Cys Met His Asn Asn Gly Gln
   900 905 910
   Cys Gly Gln Leu Cys Leu Ala Ile Pro Gly Gly His Arg Cys Gly Cys
   915 920 925
   Ala Ser His Tyr Thr Leu Asp Pro Ser Ser Arg Asn Cys Ser Pro Pro
   930 935 940
   Thr Thr Phe Leu Leu Phe Ser Gln Lys Ser Ala Ile Ser Arg Met Ile
   945 950 955 960
   Pro Asp Asp Gln His Ser Pro Asp Leu Ile Leu Pro Leu His Gly Leu
   965 970 975
   Arg Asn Val Lys Ala Ile Asp Tyr Asp Pro Leu Asp Lys Phe Ile Tyr
   980 985 990
   Trp Val Asp Gly Arg Gln Asn Ile Lys Arg Ala Lys Asp Asp Gly Thr
   995 1000 1005
   Gln Pro Phe Val Leu Thr Ser Leu Ser Gln Gly Gln Asn Pro Asp Arg
   1010 1015 1020
   Gln Pro His Asp Leu Ser Ile Asp Ile Tyr Ser Arg Thr Leu Phe Trp
   1025 1030 1035 1040
   Thr Cys Glu Ala Thr Asn Thr Ile Asn Val His Arg Leu Ser Gly Glu
   1045 1050 1055
   Ala Met Gly Val Val Leu Arg Gly Asp Arg Asp Lys Pro Arg Ala Ile
   1060 1065 1070
   Val Val Asn Ala Glu Arg Gly Tyr Leu Tyr Phe Thr Asn Met Gln Asp
   1075 1080 1085
   Arg Ala Ala Lys Ile Glu Arg Ala Ala Leu Asp Gly Thr Glu Arg Glu
   1090 1095 1100
   Val Leu Phe Thr Thr Gly Leu Ile Arg Pro Val Ala Leu Val Val Asp
   1105 1110 1115 1120
   Asn Thr Leu Gly Lys Leu Phe Trp Val Asp Ala Asp Leu Lys Arg Ile
   1125 1130 1135
   Glu Ser Cys Asp Leu Ser Gly Ala Asn Arg Leu Thr Leu Glu Asp Ala
   1140 1145 1150
   Asn Ile Val Gln Pro Leu Gly Leu Thr Ile Leu Gly Lys His Leu Tyr
   1155 1160 1165
   Trp Ile Asp Arg Gln Gln Gln Met Ile Glu Arg Val Glu Lys Thr Thr
   1170 1175 1180
   Gly Asp Lys Arg Thr Arg Ile Gln Gly Arg Val Ala His Leu Thr Gly
   1185 1190 1195 1200
   Ile His Ala Val Glu Glu Val Ser Leu Glu Glu Phe Ser Ala His Pro
   1205 1210 1215
   Cys Ala Arg Asp Asn Gly Gly Cys Ser His Ile Cys Ile Ala Lys Gly
   1220 1225 1230
   Asp Gly Thr Pro Arg Cys Ser Cys Pro Val His Leu Val Leu Leu Gln
   1235 1240 1245
   Asn Leu Leu Thr Cys Gly Glu Pro Pro Thr Cys Ser Pro Asp Gln Phe
   1250 1255 1260
   Ala Cys Ala Thr Gly Glu Ile Asp Cys Ile Pro Gly Ala Trp Arg Cys
   1265 1270 1275 1280
   Asp Gly Phe Pro Glu Cys Asp Asp Gln Ser Asp Glu Glu Gly Cys Pro
   1285 1290 1295
   Val Cys Ser Ala Ala Gln Phe Pro Cys Ala Arg Gly Gln Cys Val Asp
   1300 1305 1310
   Leu Arg Leu Arg Cys Asp Gly Glu Ala Asp Cys Gln Asp Arg Ser Asp
   1315 1320 1325
   Glu Val Asp Cys Asp Ala Ile Cys Leu Pro Asn Gln Phe Arg Cys Ala
   1330 1335 1340
   Ser Gly Gln Cys Val Leu Ile Lys Gln Gln Cys Asp Ser Phe Pro Asp
   1345 1350 1355 1360
   Cys Ile Asp Gly Ser Asp Glu Leu Met Cys Glu Ile Thr Lys Pro Pro
   1365 1370 1375
   Ser Asp Asp Ser Pro Ala His Ser Ser Ala Ile Gly Pro Val Ile Gly
   1380 1385 1390
   Ile Ile Leu Ser Leu Phe Val Met Gly Gly Val Tyr Phe Val Cys Gln
   1395 1400 1405
   Arg Val Val Cys Gln Arg Tyr Ala Gly Ala Asn Gly Pro Phe Pro His
   1410 1415 1420
   Glu Tyr Val Ser Gly Thr Pro His Val Pro Leu Asn Phe Ile Ala Pro
   1425 1430 1435 1440
   Gly Gly Ser Gln His Gly Pro Phe Thr Gly Ile Ala Cys Gly Lys Ser
   1445 1450 1455
   Met Met Ser Ser Val Ser Leu Met Gly Gly Arg Gly Gly Val Pro Leu
   1460 1465 1470
   Tyr Asp Arg Asn His Val Thr Gly Ala Ser Ser Ser Ser Ser Ser Ser
   1475 1480 1485
   Thr Lys Ala Thr Leu Tyr Pro Pro Ile Leu Asn Pro Pro Pro Ser Pro
   1490 1495 1500
   Ala Thr Asp Pro Ser Leu Tyr Asn Met Asp Met Phe Tyr Ser Ser Asn
   1505 1510 1515 1520
   Ile Pro Ala Thr Ala Arg Pro Tyr Arg Pro Tyr Ile Ile Arg Gly Met
   1525 1530 1535
   Ala Pro Pro Thr Thr Pro Cys Ser Thr Asp Val Cys Asp Ser Asp Tyr
   1540 1545 1550
   Ser Ala Ser Arg Trp Lys Ala Ser Lys Tyr Tyr Leu Asp Leu Asn Ser
   1555 1560 1565
   Asp Ser Asp Pro Tyr Pro Pro Pro Pro Thr Pro His Ser Gln Tyr Leu
   1570 1575 1580
   Ser Ala Glu Asp Ser Cys Pro Pro Ser Pro Ala Thr Glu Arg Ser Tyr
   1585 1590 1595 1600
   Phe His Leu Phe Pro Pro Pro Pro Ser Pro Cys Thr Asp Ser Ser
   1605 1610 1615
<210> 4
   <211> 1615
   <212> PRT
   <213> Homo sapiens
<400> 4
   Met Glu Ala Ala Pro Pro Gly Pro Pro Trp Pro Leu Leu Leu Leu Leu
   1 5 10 15
   Leu Leu Leu Leu Ala Leu Cys Gly Cys Pro Ala Pro Ala Ala Ala Ser
   20 25 30
   Pro Leu Leu Leu Phe Ala Asn Arg Arg Asp Val Arg Leu Val Asp Ala
   35 40 45
   Gly Gly Val Lys Leu Glu Ser Thr Ile Val Val Ser Gly Leu Glu Asp
   50 55 60
   Ala Ala Ala Val Asp Phe Gln Phe Ser Lys Gly Ala Val Tyr Trp Thr
   65 70 75 80
   Asp Val Ser Glu Glu Ala Ile Lys Gln Thr Tyr Leu Asn Gln Thr Gly
   85 90 95
   Ala Ala Val Gln Asn Val Val Ile Ser Gly Leu Val Ser Pro Asp Gly
   100 105 110
   Leu Ala Cys Asp Trp Val Gly Lys Lys Leu Tyr Trp Thr Asp Ser Glu
   115 120 125
   Thr Asn Arg Ile Glu Val Ala Asn Leu Asn Gly Thr Ser Arg Lys Val
   130 135 140
   Leu Phe Trp Gln Asp Leu Asp Gln Pro Lys Ala Ile Ala Leu Asp Pro
   145 150 155 160
   Ala His Gly Tyr Met Tyr Trp Thr Asp Trp Val Glu Thr Pro Arg Ile
   165 170 175
   Glu Arg Ala Gly Met Asp Gly Ser Thr Arg Lys Ile Ile Val Asp Ser
   180 185 190
   Asp Ile Tyr Trp Pro Asn Gly Leu Thr Ile Asp Leu Glu Glu Gln Lys
   195 200 205
   Leu Tyr Trp Ala Asp Ala Lys Leu Ser Phe Ile His Arg Ala Asn Leu
   210 215 220
   Asp Gly Ser Phe Arg Gln Lys Val Val Glu Gly Ser Leu Thr His Pro
   225 230 235 240
   Phe Ala Leu Thr Leu Ser Gly Asp Thr Leu Tyr Trp Thr Asp Trp Gln
   245 250 255
   Thr Arg Ser Ile His Ala Cys Asn Lys Arg Thr Gly Gly Lys Arg Lys
   260 265 270
   Glu Ile Leu Ser Ala Leu Tyr Ser Pro Met Asp Ile Gln Val Leu Ser
   275 280 285
   Gln Glu Arg Gln Pro Phe Phe His Thr Arg Cys Glu Glu Asp Asn Gly
   290 295 300
   Gly Trp Ser His Leu Cys Leu Leu Ser Pro Ser Glu Pro Phe Tyr Thr
   305 310 315 320
   Cys Ala Cys Pro Thr Gly Val Gln Met Gln Asp Asn Gly Arg Thr Cys
   325 330 335
   Lys Ala Gly Ala Glu Glu Val Leu Leu Leu Ala Arg Arg Thr Asp Leu
   340 345 350
   Arg Arg Ile Ser Leu Asp Thr Pro Asp Phe Thr Asp Ile Val Leu Gln
   355 360 365
   Val Asp Asp Ile Arg His Ala Ile Ala Ile Asp Tyr Asp Pro Leu Glu
   370 375 380
   Gly Tyr Val Tyr Trp Thr Asp Asp Glu Val Arg Ala Ile Arg Arg Ala
   385 390 395 400
   Tyr Leu Asp Gly Ser Gly Ala Gln Thr Leu Val Asn Thr Glu Ile Asn
   405 410 415
   Asp Pro Asp Gly Ile Ala Val Asp Trp Val Ala Arg Asn Leu Tyr Trp
   420 425 430
   Thr Asp Thr Gly Thr Asp Arg Ile Glu Val Thr Arg Leu Asn Gly Thr
   435 440 445
   Ser Arg Lys Ile Leu Val Ser Glu Asp Leu Asp Glu Pro Arg Ala Ile
   450 455 460
   Ala Leu His Pro Val Met Gly Leu Met Tyr Trp Thr Asp Trp Gly Glu
   465 470 475 480
   Asn Pro Lys Ile Glu Cys Ala Asn Leu Asp Gly Gln Glu Arg Arg Val
   485 490 495
   Leu Val Asn Ala Ser Leu Gly Trp Pro Asn Gly Leu Ala Leu Asp Leu
   500 505 510
   Gln Glu Gly Lys Leu Tyr Trp Gly Asp Ala Lys Thr Asp Lys Ile Glu
   515 520 525
   Val Ile Asn Val Asp Gly Thr Lys Arg Arg Thr Leu Leu Glu Asp Lys
   530 535 540
   Leu Pro His Ile Phe Gly Phe Thr Leu Leu Gly Asp Phe Ile Tyr Trp
   545 550 555 560
   Thr Asp Trp Gln Arg Arg Ser Ile Glu Arg Val His Lys Val Lys Ala
   565 570 575
   Ser Arg Asp Val Ile Ile Asp Gln Leu Pro Asp Leu Met Gly Leu Lys
   580 585 590
   Ala Val Asn Val Ala Lys Val Val Gly Thr Asn Pro Cys Ala Asp Arg
   595 600 605
   Asn Gly Gly Cys Ser His Leu Cys Phe Phe Thr Pro His Ala Thr Arg
   610 615 620
   Cys Gly Cys Pro Ile Gly Leu Glu Leu Leu Ser Asp Met Lys Thr Cys
   625 630 635 640
   Ile Val Pro Glu Ala Phe Leu Val Phe Thr Ser Arg Ala Ala Ile His
   645 650 655
   Arg Ile Ser Leu Glu Thr Asn Asn Asn Asp Val Ala Ile Pro Leu Thr
   660 665 670
   Gly Val Lys Glu Ala Ser Ala Leu Asp Phe Asp Val Ser Asn Asn His
   675 680 685
   Ile Tyr Trp Thr Asp Val Ser Leu Lys Asn Ile Ser Arg Ala Phe Met
   690 695 700
   Asn Gly Ser Ser Val Glu His Val Val Glu Phe Gly Leu Asp Tyr Pro
   705 710 715 720
   Glu Gly Met Ala Val Asp Trp Met Gly Lys Asn Leu Tyr Trp Ala Asp
   725 730 735
   Thr Gly Thr Asn Arg Ile Glu Val Ala Arg Leu Asp Gly Gln Phe Arg
   740 745 750
   Gln Val Leu Val Trp Arg Asp Leu Asp Asn Pro Arg Ser Leu Ala Leu
   755 760 765
   Asp Pro Thr Lys Gly Tyr Ile Tyr Trp Thr Glu Trp Gly Gly Lys Pro
   770 775 780
   Arg Ile Val Arg Ala Phe Met Asp Gly Thr Asn Cys Met Thr Leu Val
   785 790 795 800
   Asp Lys Val Gly Arg Ala Asn Asp Leu Thr Ile Asp Tyr Ala Asp Gln
   805 810 815
   Arg Leu Tyr Trp Thr Asp Leu Asp Thr Asn Met Ile Glu Ser Ser Asn
   820 825 830
   Met Leu Gly Gln Glu Arg Val Val Ile Ala Asp Asp Leu Pro His Pro
   835 840 845
   Phe Gly Leu Thr Gln Tyr Ser Asp Tyr Ile Tyr Trp Thr Asp Trp Asn
   850 855 860
   Leu His Ser Ile Glu Arg Ala Asp Lys Thr Ser Gly Arg Asn Arg Thr
   865 870 875 880
   Leu Ile Gln Gly His Leu Asp Phe Val Met Asp Ile Leu Val Phe His
   885 890 895
   Ser Ser Arg Gln Asp Gly Leu Asn Asp Cys Met His Asn Asn Gly Gln
   900 905 910
   Cys Gly Gln Leu Cys Leu Ala Ile Pro Gly Gly His Arg Cys Gly Cys
   915 920 925
   Ala Ser His Tyr Thr Leu Asp Pro Ser Ser Arg Asn Cys Ser Pro Pro
   930 935 940
   Thr Thr Phe Leu Leu Phe Ser Gln Lys Ser Ala Ile Ser Arg Met Ile
   945 950 955 960
   Pro Asp Asp Gln His Ser Pro Asp Leu Ile Leu Pro Leu His Gly Leu
   965 970 975
   Arg Asn Val Lys Ala Ile Asp Tyr Asp Pro Leu Asp Lys Phe Ile Tyr
   980 985 990
   Trp Val Asp Gly Arg Gln Asn Ile Lys Arg Ala Lys Asp Asp Gly Thr
   995 1000 1005
   Gln Pro Phe Val Leu Thr Ser Leu Ser Gln Gly Gln Asn Pro Asp Arg
   1010 1015 1020
   Gln Pro His Asp Leu Ser Ile Asp Ile Tyr Ser Arg Thr Leu Phe Trp
   1025 1030 1035 1040
   Thr Cys Glu Ala Thr Asn Thr Ile Asn Val His Arg Leu Ser Gly Glu
   1045 1050 1055
   Ala Met Gly Val Val Leu Arg Gly Asp Arg Asp Lys Pro Arg Ala Ile
   1060 1065 1070
   Val Val Asn Ala Glu Arg Gly Tyr Leu Tyr Phe Thr Asn Met Gln Asp
   1075 1080 1085
   Arg Ala Ala Lys Ile Glu Arg Ala Ala Leu Asp Gly Thr Glu Arg Glu
   1090 1095 1100
   Val Leu Phe Thr Thr Gly Leu Ile Arg Pro Val Ala Leu Val Val Asp
   1105 1110 1115 1120
   Asn Thr Leu Gly Lys Leu Phe Trp Val Asp Ala Asp Leu Lys Arg Ile
   1125 1130 1135
   Glu Ser Cys Asp Leu Ser Gly Ala Asn Arg Leu Thr Leu Glu Asp Ala
   1140 1145 1150
   Asn Ile Val Gln Pro Leu Gly Leu Thr Ile Leu Gly Lys His Leu Tyr
   1155 1160 1165
   Trp Ile Asp Arg Gln Gln Gln Met Ile Glu Arg Val Glu Lys Thr Thr
   1170 1175 1180
   Gly Asp Lys Arg Thr Arg Ile Gln Gly Arg Val Ala His Leu Thr Gly
   1185 1190 1195 1200
   Ile His Ala Val Glu Glu Val Ser Leu Glu Glu Phe Ser Ala His Pro
   1205 1210 1215
   Cys Ala Arg Asp Asn Gly Gly Cys Ser His Ile Cys Ile Ala Lys Gly
   1220 1225 1230
   Asp Gly Thr Pro Arg Cys Ser Cys Pro Val His Leu Val Leu Leu Gln
   1235 1240 1245
   Asn Leu Leu Thr Cys Gly Glu Pro Pro Thr Cys Ser Pro Asp Gln Phe
   1250 1255 1260
   Ala Cys Ala Thr Gly Glu Ile Asp Cys Ile Pro Gly Ala Trp Arg Cys
   1265 1270 1275 1280
   Asp Gly Phe Pro Glu Cys Asp Asp Gln Ser Asp Glu Glu Gly Cys Pro
   1285 1290 1295
   Val Cys Ser Ala Ala Gln Phe Pro Cys Ala Arg Gly Gln Cys Val Asp
   1300 1305 1310
   Leu Arg Leu Arg Cys Asp Gly Glu Ala Asp Cys Gln Asp Arg Ser Asp
   1315 1320 1325
   Glu Val Asp Cys Asp Ala Ile Cys Leu Pro Asn Gln Phe Arg Cys Ala
   1330 1335 1340
   Ser Gly Gln Cys Val Leu Ile Lys Gln Gln Cys Asp Ser Phe Pro Asp
   1345 1350 1355 1360
   Cys Ile Asp Gly Ser Asp Glu Leu Met Cys Glu Ile Thr Lys Pro Pro
   1365 1370 1375
   Ser Asp Asp Ser Pro Ala His Ser Ser Ala Ile Gly Pro Val Ile Gly
   1380 1385 1390
   Ile Ile Leu Ser Leu Phe Val Met Gly Gly Val Tyr Phe Val Cys Gln
   1395 1400 1405
   Arg Val Val Cys Gln Arg Tyr Ala Gly Ala Asn Gly Pro Phe Pro His
   1410 1415 1420
   Glu Tyr Val Ser Gly Thr Pro His Val Pro Leu Asn Phe Ile Ala Pro
   1425 1430 1435 1440
   Gly Gly Ser Gln His Gly Pro Phe Thr Gly Ile Ala Cys Gly Lys Ser
   1445 1450 1455
   Met Met Ser Ser Val Ser Leu Met Gly Gly Arg Gly Gly Val Pro Leu
   1460 1465 1470
   Tyr Asp Arg Asn His Val Thr Gly Ala Ser Ser Ser Ser Ser Ser Ser
   1475 1480 1485
   Thr Lys Ala Thr Leu Tyr Pro Pro Ile Leu Asn Pro Pro Pro Ser Pro
   1490 1495 1500
   Ala Thr Asp Pro Ser Leu Tyr Asn Met Asp Met Phe Tyr Ser Ser Asn
   1505 1510 1515 1520
   Ile Pro Ala Thr Ala Arg Pro Tyr Arg Pro Tyr Ile Ile Arg Gly Met
   1525 1530 1535
   Ala Pro Pro Thr Thr Pro Cys Ser Thr Asp Val Cys Asp Ser Asp Tyr
   1540 1545 1550
   Ser Ala Ser Arg Trp Lys Ala Ser Lys Tyr Tyr Leu Asp Leu Asn Ser
   1555 1560 1565
   Asp Ser Asp Pro Tyr Pro Pro Pro Pro Thr Pro His Ser Gln Tyr Leu
   1570 1575 1580
   Ser Ala Glu Asp Ser Cys Pro Pro Ser Pro Ala Thr Glu Arg Ser Tyr
   1585 1590 1595 1600
   Phe His Leu Phe Pro Pro Pro Pro Ser Pro Cys Thr Asp Ser Ser
   1605 1610 1615
<210> 5
   <211> 3096
   <212> DNA
   <213> Homo sapiens
<400> 5
   catcttctca cacgatctct cgcttcgcac tccttccttt gattggtttt caccatttac 60
   tcagacgacg gtccttcttc gatctttgca cattcttcta tcatctacta ccttcatacc 120
   cagctccgtc ccctaatatt catgcgcgga tggcccattc cgtggtgaaa attcccttct 180
   actctgctaa tctgctgttc tctctccctc ccgtcgggtt ctgctcctgc cacgttctcc 240
   cctctcccca ccaaaggctg ggttttcttt gtcagggctc ctttcccctt tggaagaagg 300
   ggggctgtat ggccttggtg cgaggccctc cagtgacagg atcccccatc acccagagtt 360
   ccacaggccc tggtagggag gagggggagc agaagaggag gtgccatctt tgcctgctgg 420
   ggaagggcag gggccaccca cacagagctc tcccatttgc tgtggaccct ggggccactg 480
   cccagttcct tccaaaggaa agccagctcc ccaggtggtg ggagagtgat atggcttcct 540
   cttaaactta gggaattgag tgtgtggttg cttctaagtg ccttagaagc cgggagcggc 600
   tcctggaaag agcctgcctg ccacagcggg ccttaccctg gctgtgccca cagatgtccc 660
   tggggcctgc cgctcctgcc cggctctcct ggcctccccc ggtgtgggtt gggaaaagca 720
   cagcaaatta aaaaacacct ccatctctgg cctttgaaga atgcatctga acagccgaga 780
   gtgtaaaccg tggtgaaatg tggtctttcc agtttgggga gaagcagggc agagctgggg 840
   cttttgtacc cagggtttcc aagagctcct gcctccctcg gctgggctgg ccagggcccc 900
   ccgctgggac ctccagctgt aatagggaag gttttactgg gttgctggcc actgtggact 960
   gcccctaagg gcaggtatgc ctgcctttac ccgggttccc ctcctgcctg gaagatacag 1020
   cccatgggag gcctgttgtc tgtgggatcc tccagcatca gagacactgg ggccagcgtc 1080
   tgcctggtga ggtgcaggcc tggcaggccc ggtcccccac ctgcttgagc acccacggtg 1140
   gtgggggctc gctgcctccc gagacaatct atgtcattgt tgtccaagga agctaattta 1200
   gagtagaaag ttccgtgtcc agtcccactc tgtgcgtgtg ttagcagggg actctcgggc 1260
   cggagctggg tccaccctgg tagggggact tcatggggcc tgggcgacag cactgtgtat 1320
   ttgtgtgtgt gtgtgtttgt gtgtgtgtgt gtctgaggag gtggaccagt ttctcaaaag 1380
   gcctgtgacc ccaagaacca aggaatttca gcctgggtgg atcacacctt cactggtgag 1440
   tgggacaagc tgggggccct cgccacagga gcagccaggg catggggcac agttggcctc 1500
   attcacaaaa tgggagtata agtgatccct gctctggcgg ccaggacgat gagtgggaac 1560
   acaccgtgtg ggggctgcct ggcctgggtg tgccgcgggt gtccttgttg gtgatggttc 1620
   cacctgcttg tgccaccagt gccctctggg tctcacacac aactctcttc ccagcgaagg 1680
   cccctcctgc cctcaggcct cagtgctgct tccgtctcgg aaggccccag gagctcctgc 1740
   atcctgggcg tgattcctgt gtgcctgcag accccctcgc ggctgccatc tcatcctttg 1800
   gtgcacctgt tggccagacc tcctggtagc gggtgctgca ctcccctgaa tgtgccgggg 1860
   cctgggggca gggacctggg ctcctccctc actgagtgga gggaactcag tgtcttggag 1920
   ttggggtgcc tgcaggctgg gtggtgcagg tgaaatgcag acctctcagc tggtgttcca 1980
   gagcagctgc cttcccccgc ccgagggact tcacccgcag cccagtcagg ggtggcgcct 2040
   gggtgcatcg cccgcaggct gggtaggggt ggagcctggg tggccctgcc tgtgagctgc 2100
   atagttgtcg cctttgaccc tgagttttct tcgttatctg tttggacctg tttggggcag 2160
   gcaggggatg agatctgaag ataaatgcct tagctgtgac catctccttt tgtgagaggt 2220
   caatgtccag ttccgctgca gttataacat cccatttttt gatttctttt tattttttcc 2280
   tttttctttt tgagatggag tctcgctctg tcacccaggc tggagtgcaa tggggtgacc 2340
   tcagctcact gcaacctcca cttctcgggt tcaagtgatt ctcctgcctc agcctcctga 2400
   ctagcagggg ttacaggcgt gagccaccac gcccagctaa tttttgtatt tttagtagag 2460
   gcaaggtttc gtcatgttgg ccaggctggt ctcaaactcc tggccttaag tgatctgccc 2520
   gcctcggcct cccaaagtgc tgagatgaca ggtgtgagcc accgtgcccg gcccagaact 2580
   ctttaattcc cacctgaaac ttgccgcctt aagcaggtcc ccagtctccc tcccctagtc 2640
   cctggtccca ccattctgct ttctgtctca atgaatttgc ctaccgtaag tacctcatat 2700
   aaattgaatc ataaagtatt tgtcttttta tatctggctt atttcactta gcataacatt 2760
   cttaagtttc atccatgttg tagcatgtgt cagaatctct ctcttttttt tttttttttt 2820
   tttttttttt ttttgcagac agagtctcgc tctgtcatct agactggagt tcagtggcac 2880
   gatctcggtt cactgcaaca tctgcctcct gggtccaagc aattctcctg cctcagcctc 2940
   cttagcagct ggaactacag gcgcgtgcca ccatgccttg ctaatttttg tatttttatg 3000
   tggaggcagg gtttcaccat cttggccagg ctggtctcga attcctggtc ttcaccacgg 3060
   gggcccgaag gacccgggca aagcgtggag gggagg 3096
<210> 6
   <211> 26928
   <212> DNA
   <213> Homo sapiens
<220>
   <221> unsure
   <222> (12044),(12489),(26433),(26434),(26435),(26436),(26439),(26441)
   < 223 > Identity of nucleotide sequences at the above locations are unknown.
<400> 6
   gaagaccaag ggcacacagc gaggcagttt cagggcgggc agcctggggc cccacggggc 60
   ggccccggac acttgttctc acctgtggag ggcagagaag ggaacaggga gagaagtggc 120
   cggctgggag tggaggtggg tttgaggttt tactgtaaac taaatgtgta ccctctacct 180
   tagttatgaa ttatgagaca cgaagactgc gaaacagaca cactcctcta aaagtgcctc 240
   taggctgaca gggagaaagt cccgccaggc tcccagacgc cacctttgag tccttcaaca 300
   agcccgccag ggcctcttgc ccaccggtgt cagctcagcc actgaaccct ccaggaagaa 360
   gacgtgctgg taggagaaga atctcaccca ggcacagcct ggaaggggca cagaaggggc 420
   tccggaacca gcaagcccaa gttggaactc ccagtctgct actttctaga acgactgtgc 480
   ccttggcggg tctaagtaga acctctccgc gcactctttc ctcctttgta aagtggggac 540
   agcaatggcc accttgcagg ttcagagagg gcttgcagta cctcacagaa ctgagtgccc 600
   gtgaacgtgt gtgttcctcc agatttgtga cagctttgcc aggctggagt caggctgaac 660
   gcctctgccc tcatggggtt tatattctag gaagaccaac aaaaacaaga agacggaaaa 720
   ttaaaacaac aaaagcccca ttgacaggcc gtgaagaatg ccatgaaaaa tgaatggcgt 780
   tgtgctgcag tctttgggga aacgggctta cggaaagaag gacacttgag ctgctaccaa 840
   tgagcagccg tccggtggga gggcagttca ggaagagcag acatccactg aggaggcgct 900
   ggggcagagg gcagcctggt cgctggattc gggggaggaa ccacatcagg ccatgagctg 960
   gagctggtgg tagaatgtac aggagaggcc agccagggcc agctcatgtc agacctcaag 1020
   cggggaagat gaatcgagaa tgcaccccac gagcaatggg aagccagtct acgatttaag 1080
   cagcaaaaat attttccctt cttccaccct gcatccagct ctaccagcac agcctggggt 1140
   tctattttca agatagaata gacccagact cccagctctt cttacacttc tactactgcc 1200
   acctgtcacc cactcatgcg tccccacttg cagcctcgac ccccttccac ctgatctcat 1260
   ggcagccagg gaagctccag ggctcgtgag ggctgccatc tcaggaaaga agcaaaagcc 1320
   ttcggcacct gcagggcctg ctccaaccac acttcttcct tgacctctca gcttccttag 1380
   ccactccctt cccacatctc accctgctcc agccacagtg gtgtctctgt gggttctcaa 1440
   acacaccagg tgcactcctg cctcagggcc tttgtgcttg ctgttctctg ctgggactct 1500
   tttttttttt tttttttttg agacagggtc tcactctgtg gcccaggctg gagtgtagtg 1560
   gtgtgatcgt agctcattgc aacctcaaac tcctgggctc aagcaatcct cccacctcag 1620
   cctctcaagt agttagcttt tgttgttttg ttttgagatg ggatctcact ctgttgccca 1680
   ggctggagtg cagtggggca atcttggctc accacaacct ctgcctccca ggctcaagca 1740
   attctcctgc ctcagcctcc caagtagctg ggattacagg catgtgccac cacgcccagc 1800
   ttatttttgt atttttagta gagacagggt ttcaccatgt tggtctggct ggtcttgaac 1860
   tcctggcctc agatgatcca cctgcctcgg cctcccaaag tgctgggatg acaggcatga 1920
   gcctgtctct agtagttagg actacagaga ggggccatca tgcctggtga tcctcccacc 1980
   ttttctgctc caactctttc accccactta gcctcgtggc tcactctctt acctcttcag 2040
   ctcctcagtc aggcctgagg acccctgttg aaaattgcaa accacacccc ccaccaccac 2100
   cacccactat tgccagcact ttctactcca tttctctgct ttacttttct cctttgtact 2160
   catcaccacc tgactcatta catgtttacg tatctttctt ctctccacta gcatggaagc 2220
   tccaggagag cagagagtgt agttttattc cctgatgtgt ttcctgtgcc cgtaccaggg 2280
   cctagcacac agtaggtgct cagtaaatgt gtgttggatg aacaaataca gtgaaaggat 2340
   ctgatctaca tttataaaga aggcactctg gctgctgagt ggggatgaga ctgtcaggag 2400
   gaaagaggcc cctgtggggg cctggccagc aggtgggtac aatggtagca gccaggagag 2460
   agggcctctt ggactcaagt ggatggggcc tgctcagggc tccggccaca ggaacaaagg 2520
   gaagggggcc caggatggcc tgtcatagag gacacattac aactggccca aagttcaagt 2580
   caggtttcta aatttgggaa gggatacaga aaaactaaag actctactgg acagtcagtt 2640
   attgaaatga ttacatagaa aatgtaccaa gaattaaaaa aaaaaaaaaa aagcattatg 2700
   aaggggccac cagagactcc cagagaggaa agggactatg ggctggatgc ggtgactcac 2760
   acctataatc ccagcacttt gggaggccga ggagggtgga tcacgaggtc aggagttcaa 2820
   aaccagccta ggcaacatgg taaaaccccc gtttctacta aaaatacaaa aaattagctg 2880
   ggcatggcag catgtgcctg taatcccagc tactcgggag gctgaggcag gagagttgct 2940
   agaacccagg aggcagaggt tgcagtgagc cgagattgag ccactatgct ccagcttggg 3000
   cgacagagca agactccgtc tctaaaaaaa agaaaaaaaa ggccagatga ggtggctcat 3060
   gcctgtaatc ccagcacttt gggaggccga ggtgggtgga tcacgaggtc aggagatcga 3120
   gaccatcctg gctaacatgg tgaaactcca tctctactta aaatacaaaa aattagccgg 3180
   gcgtggtggc gggcacctgt agtcccagct acttgggagg ctgaggcagg agaatggcgt 3240
   gaacctggga ggcggagctt gcagtgagcc gagattgcgc cactgcactc catccagcct 3300
   gggcgacaga gttagactcc gtctcaaaaa aaaaaaaaaa aaaaaaatta gctgattagt 3360
   tgggcttggt ggcgggcgcc tgtaatccca actactcggg aggctgaggc gggagaatca 3420
   cttgaacccg ggaggcagag gttgcaatga gccgatatca cgccactaca ctccagcctg 3480
   ggcgacagag caagactcca tctcaaaaaa gaaaaaaaaa aagaaagggg ctgtgctgtg 3540
   gcctgggacc caaagcacac tactgcaagg tcccagggtg cctgactcca accggagcct 3600
   tgagaacatt catttgcaaa gaatgaatta aaattcagca ctattttatt ctgcaggatt 3660
   ccagcacccc aaggacagtc atttttagac ccttcagtaa cgtaataagt aaccggagga 3720
   tgtgctgagc ttccacttcc ccagacggtt gcctgtcaca gctcatcagg ccaacaaact 3780
   tttcttaggc ctcaaatttg gaaatgttca ctctcagttc gttccttaga tgcaagtcca 3840
   tcccaatgaa gtaacagggg ctcagcacct gtccaatctc attgcttccg gggacagggg 3900
   cccatgagga tgtcgtttca gcccggtgac acttgggcaa agtgcctttt ggtttccctc 3960
   ccaggctgga acgtgctggc tctgtgaagt tacgctgggc acaagagccc cccccaaccc 4020
   ggcaggactg actgctgtgg tcagaggcgc ccctggggct ttgggagcca cagaatcttc 4080
   ctgagggcag cgccggagga ggccccagtg agagtgccca ctgccaggct cattcctcag 4140
   gctgccgcag gcctctcccc aaaacaggca atgcttctca gcaacctgcc ccaggagcag 4200
   gccagggaag gccgccatcg gcctacagtg ctgggctctg gagggcttgg ttggtaacag 4260
   gccatggttt ctatgagcca gctggggtgt gaaggacaca ggctggattc acctctctgg 4320
   gcctcagttt ctgcattcaa aaagtgggaa tcatgatatc tgctctattt cttatctctc 4380
   agtgctgatg tgaacctcca ataagacttt taaaaatact ctttctacct tacttttatt 4440
   tttcatttat tttaagataa tgtctagctg tctcacccag gctggagtgc agtggtgtga 4500
   ttacggctca ctacagcctt aacctcccag gctcaagtga tcctcctacc acagcctccc 4560
   aagtagctgg aactacaggc atgcaccacc gcacctggat aattttttct tttgagacaa 4620
   ggtttcactc tgttgcccag gctggagtgc agtggtgcac tcttggctca ctgcagcctc 4680
   aacctccctg ggcttaggtg atcctcacac ttcagtctcc caagtagctg ggactacagg 4740
   tatgtgccag tacacccagc taatattttt gaaggatggg gtttcactat attgcccagg 4800
   ctggtcttga actccagggt ttaagcaatc taccttcctc agcctgccaa agtgctagga 4860
   ttataggtat gagccacccc ccggcctata atcctaccac tttaaaaaag cctgtaattt 4920
   tagcacttta aaaaattttt ctaaattttt tatagagatg ggggacagct gtggtctcac 4980
   tgtgttgccc aggctggtct tgaactccta ggatcaagcc atcctcctgg cctggcctcc 5040
   caaagtgttg ggattataag cataagcctt accttacctt ttttttttga gttgcagttt 5100
   tgttcttgtt gctcaggctg gagtgcaatg gcaagatctt ggctcactgc aacctccacc 5160
   tcccgggttc aagcaattct cctgcctcag cctcccgagt agctgggatt acaggcatgc    5220
   gccaccacac ccagctaatt ttgtattttt agtagagatg gggtttctct atatacctta    5280
   attttaaagc actgcattca tgtaaattgt gattaacatg gattcaagag agggagtgag 5340
   gatgaatgag ccaggcagtc acctcggctg tcaccctcca cttctctcct ccttctgaca 5400
   gtcatcgtcc atccgtttct gcagctgttt gtttgactct cctgatcatt ttgcttgcca 5460
   cataacttgc ctcctgggaa agaatgccct gggcaggccc acatgagtag tgaaaaataa 5520
   tctgcagtga aaaataaaac taagtagtct ggtccacaga gcagtcttat tttttcactg 5580
   cagatgaagg agttgacatt caggcttcat tctcatttat aagtgtttta aagacacata 5640
   cagtggattg aacagtggcc ttcaaaaaga tgtatctaca tcctaatccc tgggacctgt 5700
   gaatgttaac caagttagga aaagggtctt cccgggtgtc attaagttag agatcttgag 5760
   atgaggagct catcgtggat tatccaggtg gaccctgcat ccaaggacaa atggtcctta 5820
   gaaaagaaaa gcagaggctg ggcacagtgg ctcaagcctg taatcccagc actttgagag 5880
   gccgaggtgg gtggatcacc taaggtcatg agttcgagag cagcctggcc aacatgatga 5940
   aatcccatct ctactaaaaa tacaaaaatt agcaaggcat ggtggcgggt gcctataatc 6000
   ccagctactc aggaagctga ggcaggagaa tggcttgcac ctgggaggcg gaggttgcag 6060
   tgagccaaga tcgcgccact gcactccagc ctgagggaga aaagtgaaac tctgtctcat 6120
   aaaagaaaag aaaagcagac agagatctga gacagaagag gagagtgaag gaaaaaaggc 6180
   catgtgaaga tgaggcagag gttggagcca tgcagccaca agccaaggaa tacctggagc 6240
   cccagaagtt gcaagaggta ggaagaagcc tcccctagag cctccagacg gagcacagcc 6300
   ctgccaacac ctccacctca gacttctggc ctccagcact gtgagataat caactgctgt 6360
   tgttttaagc caccagattt gtggtaattt gttatggcag ccacaggaaa ctaatacagt 6420
   acctaatctt cacaaaccca tcttacagaa aaggaaactg aagtcagaga ggtagtggct 6480
   tgtgcagtgt gttaggccat tcttgtatta ctataaagaa atacctgagg ccgggcatgg 6540
   tggctcacgc ctgtaatccc agcactttgg gaggccaagg tgagtggatc acttgaggtc 6600
   aggagttcaa gaccagcctg gacaacatgg tgaaacccca tttctactga aaatatgaaa 6660
   attagccagg catggtggcg tgcatctgta gtcccagcta ctcaggaggc tgaggcagga 6720
   gaatcacttg cgcccgggag gaggaggttg tagtgagcca agattgtgcc actgcactcc 6780
   agcctgggag acaagagaga aaccctgtct caaaataaat aaaaaacaaa taaacacctg 6840
   agactgggta gtttataaag aaaggggtta actggctccc ggttctgcag gctgtacaag 6900
   catggtgccg gcatctgctt ggttgctggg aaggcttcag ggagttttac tcatcgtgga 6960
   aggcagagcc agagcaggtg catcacacag caaaagcagg agcgagagag agagagagca 7020
   gggaggtgtg cacactttta aatgagcaga tctcacgaga actcaccatt gcaaggacag 7080
   caccaagcca cgaggggtct gcccccatga cccaaacctc ccactaggcc ccacccccaa 7140
   cattgggaat tacagttcaa catgaggttt ggggggacaa atatccaaac tatatcattc 7200
   cacccctggc cccccagatc tcatgttctt ctcacattgc aaaatatagt catgccttcc 7260
   cagtagcccc ccaaagtctt aactcatccc agcattaact caaaaatccc attcccaagt 7320
   ccaacgtctc atctgaagat gagttccttt cacctacaag actgtaaaaa tgaaaacagt 7380
   tatttactgc tgagatacaa tgggggcata ggcattaggt aaacattcct gttccaaaag 7440
   ggagaaatcg gtcaaaagaa aggggctata ggccccaagc aagtccaaaa cccagcagag 7500
   caatcattca atcttaaagc tccaaaataa cctccttaaa ctccatgtcc catagccagg 7560
   gcacactggt gcaaggggca ggctcccaag gccttgggca gctctattcc tgcggctttg 7620
   cagaattcag tccccatggc tgctcttaca gattggagat gagggcctgc ggcttttcca 7680
   ggtgcagggt gcaagctgct ggtgatctac cattctgggg tgtggatggt ggcggccccg 7740
   tcccgcagct ccactaggca ttgtcccagt ggggactcta tgtggggcct ccaaccccac 7800
   atttcccctc caatgggaag gctctgcccc tgcagcagcc ttcttcctgg gctcccaggc 7860
   tttctcatac atcctctgac atctaggtgg atggtgtcaa gcttccttca ctcttgcact 7920
   ctgcacacct acaggcttaa caccacatgg aagctgccaa ggtgtatggc tggaaccctc 7980
   tgaagcagca gcctgagctg tgactatggc cctttgagcc aaggctggag ctggaacagt 8040
   ctagatgcag gcagggagca gtgtcctgag gctgtgcaga gcagcagggc cctgtgcctg 8100
   gacaatgaaa ccattctttc ctcctcatcc tctgggcctg tgatgggagg gttgtggaag 8160
   atctctgaaa tgcctttgag gcctttttgc ctctgaggcc tatttcctat tgtctcagtt 8220
   attggcagtc ggctcctttt tagttatgca aatcctctag caagaggtta ctccactgcc 8280
   ggcttgaact cctctcctga aaaagctttt tctttctttg tcacatggcc aggctgcaaa 8340
   ttttccaaac ttttatgctc tgttttacct ttaaatataa cttctaactt taattcattt 8400
   atttgctcct gcatttgagc atagggaatt caaagaagct gggccacatc ttgaatgctt 8460
   tgctgcttca aaatttatgg ccacgcttgg tggctcacac ctgtaatccc agcactttgg 8520
   gaggcctagg tgggcagatc acgagatcag gagatcgaga ccatcctggt caacatggtg 8580
   aaacccatct ctactaaaaa tacaaaaaaa ttagcttggt gtggtggcgc agacctgtag 8640
   tcccagctac tggagaggct gaggcaggag aattacttga acctgggagg cagaggttgc 8700
   agtgagccca gatcatgcca ctgcactcca gcctggtgac agaataagat ttgatctcga 8760
   aaggaaggaa ggaaggagga agggaagaaa tgtcttcccc ccagatgtcc tgggtcatcc 8820
   ctcttatgtt caaacttcaa cagatcccta gggcatgaaa ataatacagc caaattattt 8880
   gctaaggcat aacgaaagtg acctttgctc cagttcccaa taagttcctc atttccatct 8940
   gagactcatc accctggcct tggcttgtcc atatcactgt cagcattttg gtcacaatca 9000
   tttaaccagc taatcgggag gctgaggcaa gaggatcact tgaacccagg aggttgaggc 9060
   tgcagtgagc tgtgatcaca tcactgcagt ccagcttggg caacagagca agatcctgtc 9120
   tcaataaata aataaataaa tacataaata acttaagttt atttaaagct gcatctttgc 9180
   caccatggag aaaggccagg ccagctcctt ctctctttct gcacgtgttc ctcccacctc 9240
   agctgcctct gctcctcaag gaggaacaga gggagtagga aaggccatcc caggaggccc 9300
   agcaccccat gacctggctc tggggccttg tgggtttatg gattcccagt gctgagtcat 9360
   ccctcacagg ctcttgtggg caccttggac attggtcaga agcatgtggt ccccgggaac 9420
   acaccttttc ctgatcatct gggaagggca gcttgtgcca gcgaggccac ctgttcagcg 9480
   ccacggcccg ccagacagct gcagccacag ccttgccttt gatcagagca aacaccagac 9540
   atgtgtgtca tgcccccaac ccatctccag gggacacatg tcctttcttg ccaggcctga 9600
   gatgaacaag agagggacaa gtccccaagc ctctctctcc ttcctgcctc acccactccg 9660
   ctgttagatt ctcaaggtgg atggtgggct aactagggca accgaccatc ctggtttacc 9720
   tagaactgag ggggcatttt caggaataaa actgcaaaag tctggagcaa acaggagcaa 9780
   gttggtcact ctggggctgg tggagtcagg tttccttctg caggccccct ccccgcaagc 9840
   atgggtggaa cccaggacag gaacacagag caggccccag gaccgggctt gtcacttaca 9900
   agtctttttt tttttttttt ttttgagatg gagtcttgct ctgtcatcag ggctggagta 9960
   cagtggtgcc atcttagctc actgcaacct ctgccttctg ggttcaagtg atccccctgc 10020
   ctcagcctcc tgagtagctg ggactacagg tggcaccacc acgcccagct aattttttgt 10080
   atttctagta gagatgagat ggccaggctg gtcttgaact cctgacctca agtgatctgc 10140
   ccgccttggc ctcccaaagt gctgggatta caggtgtgag ccactgtgcc tggccccact 10200
   cacaagtctt aaaccatgcc tcagcacatc aatgccattt acaaaaaggt agagggattt 10260
   tccaggcaaa aatagatgaa agacatagga tgattgatca tgtcctgctt aaacataggt 10320
   ctgatgctat taagaattga gggctgggag cggtggctca cgcctgtaat cccagcactt 10380
   tgggaggccg aggcgggcgg atcacgaggt caggagatcg agaccatcct ggctaacacg 10440
   gtgaaacccc atctctacta aaaatacaaa aaatggccgc gcgcggtgac tcacgcctgt 10500
   aatcccagca ctttgggagg ccaaggcggg cggatcacga ggtcaggaga tcgagaccat 10560
   cctggctaac acagtgaagc cccgtctcta ctaaaaaata caaaaaaaat tagccaggca 10620
   tggtggcggg cgcctgtagt cccagcaact tgggaggctg aggcaggaga agaatggtgt 10680
   gaacctggga ggtggagctt ccagtgagcc gagatcacac cactgcactc cagcctgggc 10740
   gacagagtga aactccatct caaaaaaaaa ataaataaat aaataagaat tgttagtatt 10800
   ttgcaggtgt gacaaatgat tctgtttctg tggcagaatg ttctcaggag atctcttttg 10860
   aactctcatg gaaagcatca tgctgttggc aacatcacat ttatttttat ttatttatta 10920
   ttttttagag acagggtctt gctctgttgc ccaggctgga gtgcagtggc acaatcacag 10980
   ctcactgcag cctcaacctc ctgggctcaa gcaatcctcc tgcctcagcc tcccaaagta 11040
   gctgggacca caggcgtgag ccactgcact cagcccaatg taccttcaat atttacattt 11100
   ctggcaaagg tagcaaaacc ttaacaaatt ttgaatctag ataataaaat tatgaggctg 11160
   ggtgcagtgg ccctgacagg gatggctcac atctgtaatc tcaacatttt gggaggccaa 11220
   ggtaggcgga tcacctgagg ccaggagttt gagaccagcc tggccaacat ggtgtaaccc 11280
   tgtctctaac aaaaatacaa aaaaattagc cagacgtggt ggtgcacgtc tgtcatccca 11340
   gctactaggg aggctgaggc aggagaattg cttgaacccg agaggcagag gttgtgatga 11400
   gccgagatcg cgtcattgca ctccagcctg ggcaaaagca agagcgaaac tctctctcca 11460
   aaaaataaaa aaaaaataaa ttaatgaatt aattaaaata aaataaaata atggatagtc 11520
   actgtaaaga aaaaataaat gtatatatca gccaacaagt gatggaatag agcaccccat 11580
   ctccctggct ggacagatac atcccacaac acctggaagg cggctccatg tagaactttc 11640
   tggactgctt gaggtgctgt gctggagcac ggtgacagag gagctggacc atggacctcc 11700
   cccggccccc accaagggcg aggtccccct gtggtgggtc tgagggaggc atccgtatgg 11760
   cctctgcggc ttgggcaggg aatttggggt ccaagtactt ggtgcaaagc ctggaaagag 11820
   ggtttgggtg ctgagggcat atcccctggg ccacatgggg gcagaagtgg ggccccctga 11880
   agcttggagt cctgggcagg ggcatctatt ttgctgtctg aggccttcag tacttgaagc 11940
   aaaatggagg cagaatgtcc caccttaatg cccctgattc ctccaaacca attccagaga 12000
   cagcaagggc cagaacaggg atggccctgc ccagggtcat gcancgagga agtggccagg 12060
   ctgggatctg aacccaggct aatcccctcc cttgtcctcc tccaggccct cacccctgca 12120
   tagagccctc cagctcactc atcctcggcc agctccatct cctcagcttg taaacccccc 12180
   cgggattttc ctttcttaaa aaacaaaggc ttggccaggc acggtggctc acgcctgtac 12240
   tttgggggtg gctcccagca ctttgggagg ccaaggtggg cggatcatga ggtcaagaga 12300
   ttgagaccat tctggccagc atggtgaaac cctgtattta ctaaaaaaaa aaaaattaac 12360
   tgggcatggt ggctagctac ttaggaggct gaggcaggag aatcgcttga acctgggaga 12420
   aagaggttgc agtgagccaa gatcgcgcca ctccacttta acctggcaac agaacaagat 12480
   tccgtttcna aaaacaaaca aacaaacaaa taaacaaaaa aaggcggagc gcgatggctc 12540
   gcgcctgcaa tcccagcact ttgggaggct gaggcgggcg gatcacttga ggttaggagt 12600
   ttgagaccag cttggccaac atggtgaaac cccatttcca ctaaaagtac aaaaatcagc 12660
   caggtgtggt ggtgggtgcc tgtaatccca gctactcagg aggctgaggc aggagaatcg 12720
   cttgaaccca tgacctggag gctacagtga gctgagattg cgccactgta ctccagcttg 12780
   ggcaacaaga tttgtttctc taaaaaaaaa aaaaaaaaga ctggcccttc cccttcagct 12840
   cttcctcagg gtccctgagc actctacacc cccgtctaca ctgagcactc caccctgctg 12900
   tctacactga gcactccacc ctgccatcta cactgaggac tccaccccac tgtctacact 12960
   ggctgcctcc cgccctcacc tcctgctaag gccattcccc gctgcatctg tcttctagat 13020
   tctgcagcct tcagcacgct gggcccctcc tttgtcccct tgagccacct ccagcctccc 13080
   cctgagctgc tactcctctc ccagcagcct ccacccaagc ccctccagtc cccaagctgt 13140
   cccttgcatc cagcactgcc cttccacgtg ccccttccct ccagcttcac agcagggtgg 13200
   ggcctccagg ccctgcccac tgtgcccatc cacaagttgt ggtgggagct ccgaggggag 13260
   gcaggggtgt gcatggactt gggacgtcca agtctgggac caggggcagc tggttggtgg 13320
   agtgtggagg gggataggga ctttcaggta gagaggctgt aggggcaaga tcgggacggc 13380
   ggatgtccct aaggagggct ctgacctggg aaatattgtg cagcttcctc tttgccattc 13440
   ctggagctca gacactggcc ggctctcacc ccgcccttcc tgcaggacac agctccatcc 13500
   cagtgagttc ctagtgtaga catctccagc agcacggatg ggaaaggaag tcatcaaagg 13560
   tgcccaggac cggaggcttt ttctggaggt ggcagaggag ggtgtgggtc tcagggctct 13620
   ggctgagggc aagcgtggga ggtcttaggt ctgcaccagc cccgtgaagg cccctcctgc 13680
   tccctggtgg agtcctagag ggaacagcag cccctaggct ctagcaggag tgggtagggg 13740
   cttttctggc ttcctactgt gccagcagga tagctgggcc tggcactgag cccaaagatc 13800
   acatgccggg gcattggcgc agtgaggaac agacccttgc caaagctggc aaagaagacc 13860
   ccatggggtg cagctggtga agctgagagc tcaatgtttg ggggagcctg gcaaaagggg 13920
   tcctcccctc cctctgcagg ccaggatcgc aggttttccc tacatgttgg taattctcaa 13980
   acaatcccat ggccactgga gcaaagatca cagtgggcgg cggcctcggg agcagtggac 14040
   agggcacgca gtgcctttga tgccagagcc ctcgccccaa agtcaacaaa ctctgcagcg 14100
   gactttgcac ccggactttg ttttcaccat acaaggaaag ggacagatca caggccctct 14160
   cgctgccctc gctgagccgg aagctgcagc gtgagctctc tcaagcccca tttctaggtt 14220
   ccccaggcgc acccctgagc ccctactcgc ctattaagtt ctcctaatag cccttcaagg 14280
   tcttaatgta tgtccattag acagagggga aaactgaggc gagggcaagt gacttgaccg 14340
   aggttcctcg gcgagcaggg cgtggagctg agaacctcgt tattactgct ccccacacaa 14400
   ccctctggcc gttcttggaa gaaggctgag ccccgggggg gccagagtga cccaaacacc 14460
   atgggccgcc tgcggtaaca cgtgcggcca cgaaggggca gcagtttccc gcccggccgg 14520
   gctctctccg gcgctcagta tccgtcccag gccaagaaga agaaactcgg ggaggagggc 14580
   ggagggggct gcgtgggagg gcgtggaaga tggacgtggc caggggagtg gcagctgcac 14640
   acagtggatg ctgttaagat gaagggaaag aacgtgggct ccgagatcac tggacacggt 14700
   tccacctttc ttcccgctca ctgcatggcc ctgggcgggt tgttgaaccc ttggaaacct 14760
   gtttttcctt ttttcctttt tttttgagac agggtcttgc tctgtggccc agactggagt 14820
   gccgtggcac gatcttggct cactgctgcc tcccaggttc aagtgatcct cccagctcag 14880
   cctcctgcgt agctgggacc ccaggtatgt gtcaccacag ccggctaatt tttgtatttt 14940
   tttgtagaga cgggatttcg ccgtattgcc caggctggtc tcaaactcct gagttcaccg 15000
   gatcttcctg cctcagcctc ccaaagtgct gggattactg gcatgagcca ccgcacccag 15060
   cagagacctc agttttctaa cctgtgccag caggaataat gatagctgcc tagcttggct 15120
   gtgctgggaa ttaagtaaga tgaccgggta gcaaatatga agtattactg gacacagagg 15180
   gccccaggct gggttagcag cggtggtcag ggctgctgct tcctggcctg agctcgaagg 15240
   agggccctca ttaccacctg ggtgagtcct cgtccaagcc tggcactgct gcgtgggaat 15300
   aacttctgcc acccaagttg gcagattgtg tgcaaagtta agtcctgact ctgtggggtg 15360
   gacttcgagg cctcttcatc ggacctgctt ccggtgactg cattcgcacc tcctcctgtt 15420
   cctggtttaa cacagcccag ctttcctcct gctgagccct ccctgggcct gctgtcaccc 15480
   tcgtgccgct gtgcctcgca gtgccactcc ctgtaccctg aatactttgc cctgcctctc 15540
   cacccagctg agagtcaggg cccctgtgag gctctgccca gcccgtcctc cgggtttctg 15600
   cctctgctga gcacttccct gcatgattgc ttctgagagt ccccccagcc tgtgagcttc 15660
   tcaggactgg gacagcttct caggaccgag gcttcctggt ctgcttgcaa ttttacaggc 15720
   gggcacattt tcccttggcc aacatcagag actggacatc tgcagatctg tgctagccac 15780
   tgagcaccca ggcaccccag caggtagctc tgtaaccaac ccattctgta aagctgaggc 15840
   tcagagaggt gaagcgcctg gcctggggcc acagcctgcg tcagctgcag agccaggagc 15900
   tgagatatgc acctgcggct ctgctcacag ggtcctgcac agactgctgc tggagccacc 15960
   tatgtagagt caagagagtt catgttaact ccctctcaca tccctcagcc agggtggggg 16020
   ctgacgatag acactcaggg atggcctacc ctccccaaca acccccgtca ggtttgccgg 16080
   atctccttgg aagaaaagtt ctgggcagaa ttccaccgtt ggcctggcct acactctcct 16140
   tagtggctta ggaccctcag cggtggataa gttgtgggca gaagagatgc aatcaggatt 16200
   ctcacccact caccccttgc cagccccaat aagctcaata agctgggctc ggtctgagga 16260
   agtgtccagg aaatgtgcaa atggcctggg acagccctgt gttcctttca gtaaggttgc 16320
   tgaaggtgag gctgaaagtt ggagaaacag aagccagtgc ttatggtttt aattaagata 16380
   atggaatgta tgtatgtatg tatgtatgta tgtatgtatt tatgtattta tctttagaga 16440
   tagagtctca ctctgttgcc caggctggaa tgcggtgaca caatcatagc tccttgcagc 16500
   ctcgacttcc tatgcccaaa tgatcctcct acctcagcct cctgagtagc tgggactaca 16560
   gacacacgcc aactatgcct agctaatttt tatttctatt ttttgtggag actgggttct 16620
   cactttgttg cccaggctgg tcttgaaccc ctagcttcaa gcaatcctcc tgcctcagcc 16680
   tcccaaagtg gagggattac aggtgtgagc caccacacct ggcctggaat ttatttgtat 16740
   tctgcttata aaattaatac attcttattg cagaaaagtt tgaaaataaa agaaaggaca 16800
   aagaacaaaa agcgtatata atttcacagc tcagatctca ctgctattaa catttttatt 16860
   tactttcagg cttttttctt tctaggtaca tatgcagaga ttattttatt ttatttattt 16920
   tattttatat tttattttat attttttatt tcattatttt attttatttt attttattat 16980
   ttttagagac agggcctcac tctgtcaccc aggctggagt acaatggagt gatcatagct 17040
   cactgcagcc tcaaacacct gggctcaagc aatcccccca ctcagccttc tgagtagttg 17100
   ggactaaagt gtgagtctgg ctaatttttt ttactttttg tattgacaga ggtctcacta 17160
   tgttgcccag gctgatctca aactcctggg ttcaagcgat cctcccacct tggactccca 17220
   aagtgctggg attacaggca tgagccacca tgcctggcct aaaatgccac tttttgtcat 17280
   ttactaaaat cccatggaca ctttgacatg tctgtattct atgctattga tctgactgtt 17340
   ggcatctaca tcattatggc catctatcat ctatcataat ccattttaac attaaaattg 17400
   tgctgctgct tagatttttc tggcctgtct cctatttgta ttcttccaga taaattttag 17460
   aatcatttta tcaaattccc cttgcagaaa aagccctatt ggattttggt tgaaaaatac 17520
   tgaattttta cattaactta ggaaagggct gggcacggtg gctcacgcct gtaatcccta 17580
   cacttttcga ggccaaggca ggtggatcac ttgaggttgg gagtttgaga ccagcctggc 17640
   caacatggtg aaactcggtc tttactaaaa atacaaaaat tgccaggcgc attggctcac 17700
   ctgtaatccc agcactttgg gaggccgagg tgggtggatc acgaggtcag gagatagaga 17760
   ccatcctggc taacacggtg caaccccgtc tctcctaaaa atacaaaaaa ttagccaggc 17820
   gtggtggtgg gcgcctgtgg tctcagctac ttaggaggct gaggcaggag aatggtgtga 17880
   acccaggagg cggagcttgc agtgagccaa gatcgcgcca ctgcactcca gcctgggcga 17940
   cagagtgaga ctccatctca aaaaaaaata ataataataa tacaaaaatt agccgggggt 18000
   cgtggcgtgc acctataatc ccagttactt gggaggctga ggcaggagaa tcgcttgaat 18060
   ccaggaggtg gaggttgcaa tgagcagaga tcgtgccact gtactccagc ctgggtgaca 18120
   gagtgacact ctgtgaaaaa aaaaaaaaaa ttctgaagga ttgagactct tagactctta 18180
   ggtcttccta tccaagagca caatatagct tttcatgtat tcaagccttt ttcaatgcat 18240
   caacagaatt ttacagtttt tttcatgata tcctgctatt tcttataaaa tgtattccta 18300
   gatattctgc atgttttccg gttgtttgtt aataaatatt tttcatttgt cattatttcc 18360
   taattggctg ttatttgtat atatgacatc tgttgaattt tttgattact ttgaaaatgg 18420
   ccattctttt gtgttttttt ttaactttct attttgagat aattttgact tacagaagat 18480
   ttgcaaaaat agtacagaga gttcctgttt cccccttatg ttaacccagt ttctccttat 18540
   gttaacatct tacataacta cagaacaatt gtcaaatcta agaatcaacc tgggcacaat 18600
   gctattaact aaactgcaga agctgttcag atctcaccag ttcttctact gctccccttt 18660
   tctcttccag tgttcaatcc ggaatcctac attatattta gttgtcattt ctctttggtg 18720
   tcttccaatc tgtgacagtt cctcagtctt tctttgtctt tcatgacttt cattttttta 18780
   tacttttgaa aaatactggc cggttgtttt gtagaacgcc ctcagtttgg gtttgcctga 18840
   agttttttgt gattagatcg aggtcatgca ttattggaga gggtgccacc gcctcgatgt 18900
   gcaagctcaa tgcatcatat cagagggttt gtaatgtcag tttataccgc cggagaccct 18960
   aacctggagc atttcgtgaa ggtgctgtct gccaggattc tccactagaa agttactatt 19020
   tttccctttt taattactga atgtctgagg ggaaatactt tgagactatg caaatatcct 19080
   gtttctgctt taacttcggc tcactaagtt tagcattcat ctatggatct cgcttatagc 19140
   aagtattact gtggagttct aatggtaatt ttctgtttct ctcattcctt caacctttat 19200
   taatatgctt cttcctcact tattcatttt gtttcagttg tttataccaa catggatttg 19260
   tggatattgg ttttattctt tgggttgcaa ttgaatccta tcattatttt gttagtcagt 19320
   tgttccatcc gaccttggtc attaggagcc cttgaaattt ggctcccatg cctttttttt 19380
   tttttttgag accgagtctc actctgtcac ccaggtttga gtgcagtggc atgatcttgg 19440
   cttcctgcaa cctccgcctc ccaggttcaa gcaattctcc tgcctcagcc tcctgagtag 19500
   ctggtattat aggcgctcca ccaccttgcc cggctaattt tttgtatttt tagtagagat 19560
   ggggttttat tatgttggcc aggctggtct caaactcctg acctcaggtg atctgcccgc 19620
   ctcggcctcc caaagtgctg ggactacagg cgtgagccac cacacctggc ctcctatgcc 19680
   attttaacat gcccgtcttt tctttttctt tcctactttc tgtgactgta agaagctcca 19740
   ggatacattt ttgctgccct agacttagcc tcaatcagtt ctcagaaaag ctctggttct 19800
   ttttatggga tacttagaaa actagctctg tatggcctgg cgcggtggct cacgcctgta 19860
   atcccagtac tttgggaggc cgaggtgggc agatcacaga tcacgaagtc aggagatcaa 19920
   gaccatcctg gctaacatgg tgaaactctg tctctactaa acatacaaaa aattagtcca 19980
   ggcgcggtgg cgggcgcctg tagtcccagc tactcaggag gctgaggcag gagaacggca 20040
   tgaacccggg aggcggagct tgcagtgagc cgagatcggc agccactgca ctccagcctg 20100
   ggccacagag cgagactccg tctcaaaaaa aaaaaaagga aaaagaaaaa agaaaactag 20160
   ctctgtatgc tagttttttt tttaagacag ggtctctctt gccccagctg gagtgtagca 20220
   gcacgatcac agctcactgt agcctcaacc ttctgggctc aagcaatcct cctgcctcag 20280
   tctcctaagt agctgggtct acaggcatgc accaccgtac gtggcaattt ttaaaaactg 20340
   tttgtagaga tggagtctcc ctatgttgcc tggtctggaa ctcctggcct caagtgatcc 20400
   tcctgcctcg gcctcccaaa gtgctgagat tacaggcatg agccactgta cctggcctgg 20460
   ccaaggtctg tcttttttta aaagaagttg ttgtatagtt gttttttttt ttattttttt 20520
   ttctgagacg gagtctcgct ctgtcgccca ggctggagtg cagtggtgcg atctcggctc 20580
   actgcaagct ccgcctccca ggttcacgcc attctcctgc ctcagcctcc cgagtagctg 20640
   ggcctacagg cgcccgctac cacgcccggc taattttttg catttttagt agagacgggg 20700
   tttcaccgtg ttagccagga tggtctcgat ctcctgacct cgtgatccgc ccgcctcggc 20760
   ctcccaaagt gctgggatta caggcgtgag ccaccgcgcc cggcctgttg tatagttttt 20820
   atctcgagtt ttctagcgat ttaatcatat tggttacaaa aaaggatgat tttactacct 20880
   cctttccaat gtttctacat attttttcat tttatctaac tgcattttaa aataaacttt 20940
   taattttaga atggtttcat atttacagaa aatgtgcaaa gatagtacag agagttcctg 21000
   tgtactccac acccggtttc cttattatta tcttaacgtg atacacaatt aataaaccag 21060
   taacattatt attcactgaa gtccacactt tctttttttt tttttctgag acggagtcta 21120
   cttctgtcac ccaggctgga gtgcagtggc gcaatctcgg ctcactgcaa cctccacctc 21180
   ctgggttcag gcaattctgt ggctcagcat cccaagtagc tgggaataca ggtgcccgcc 21240
   accacgcccg gctaattttt tgtattttta gtagagatgg ggtttcacca tgttagccag 21300
   gatggtcttg aactcctgac ctcgtgatct gcctgcctca gcctcccaaa gtgctgggat 21360
   tacaggcgtg agccaccgcg cccggcgtcc atactttctt tagatatcct tcctttttac 21420
   ctaacgtcct tcttctggtt caggatccca tccagaaagc aacattaccc ctcgccatca 21480
   cgtcttcaca ggctcccctt gacgggaaga gttcctcaga ctttccttgt ttttgttgac 21540
   cttgacagtt ttgaggagga ctggtatctt agtctgtttt gtgctgctat cacagactag 21600
   ctgagaccga tacatgatac atgaaaaaaa atgtattctt acagttgtgg aggctgggaa 21660
   gttcaagacg aagttgctgg ttggtttggt ctctggtttc aagatggcgc cttgctgctg 21720
   catcctctgg agaagaagaa tgcggtgtcc tctcactgca gaagatggaa gcgctaaaag 21780
   gaatgaactc cctttgccaa gccattttat aatgggcatt aatccacaaa ggatgaaacc 21840
   ctgagaaaca tcaagcttta aagcactggt tctcaacctt tttggtctca ggagcccttt 21900
   atactcttaa aacgttttga ggatcccaaa aaaaggcttc tacaggttcc atcttttaat 21960
   atttaccata tcaaaaatta aactgaaaaa attttaaatt atttattcat ttaaaataac 22020
   aaggataaac ccattacatg ctaacataaa tcatgtattt tatgaaaaat agctatattt 22080
   atcaaaacaa aaattagtga gaagagtggc atgtataatt ttttttgttt attttttgtt 22140
   tttagatgga atcttattct gtcgcccagg ctggagtgca gtggtgtgat ctcggctcac 22200
   tgcaagctct gcctcccagg ttcacaccat tctcctgcct cagcctcctg agtagctggg 22260
   actgcaggtg cctgccacca cgcccggcta attttttgta tttttagtag agatggagtt 22320
   tcaccgtgtt agccaggatg gtcttgatct cctgaccttg tgatccaccc gcctcagcct 22380
   cccaaagtgc tgggattaca ggcttgagcc actgcgtctg gcctaaattt ttgtgaatgt 22440
   ctttaatgcc tgccttctca tatttgtttc tgcattcaag ttattgcaaa atgttgtgtt 22500
   ggttgaagtt tgtaaagaaa atgtggcctc atacagttgt gtagttggaa aggcaagagt 22560
   attttgattc tctcttcaaa caactatgga caacctgctg ttacaaaacc agaatgcaaa 22620
   aagttgtagt aaatacaggt taggtgtagt gtggaatctg aaagcatgtg aatgaacttt 22680
   ctgagttttg taacattaaa gtccagttgc gttaagctac tgtgatagca tatagcattg 22740
   tcctaatact ggaattagta tcagaagtgg ggtgctactg ttaataaata aaaagaaata 22800
   aataaatcat gtgatactgg ctcagaagtc aggcagtagg ctgtgtggaa cctgacatca 22860
   cgccatgtaa tacattggca accatttgat ccagctgtct gtcatgatga cttggaaagt 22920
   caaccacata cttacagagc ctgtagacat aggggaaaat agtataaaac agaatactaa 22980
   cagtggacct tggttcttgc cagttgcatt tagccaaata ttaaacaaaa gagatattct 23040
   tgggcagcaa ctggaccatc ttcaagtaaa agtgaaaggt aataaacaga gtccagacat 23100
   ttgtgcccat gcgggttaag aaaaatccag ttgcttctag acaccgtata tgaaaacaac 23160
   gctgaaaaca agcctttgag tggtaaaggc cgattaacac tcagcgcggt aacaaagacc 23220
   aggtgggcta acccgaaatg aaatgagaag cctgtggtga tgaggaggca gagaagtaaa 23280
   atcaagtttg agcatttcgt ttaggagagt ttgggctctg attacttgca catgcaaacg 23340
   aactggaaac aaacagatca gatgtctacc acttcttcga gggaattgca ttgccaaaga 23400
   agtcatgaaa gcagactcta tactgattag gcattaaaac aaaaacaatc tttaggcccc 23460
   taaacttgca tgggcaggaa gtgggctgtc aaagctgttc atcctctaag gtggacctag 23520
   ttcctagtcc ccagtataca cttcagatgt ggccctggag gacactggac atggaggacc 23580
   tcccagagga tgaggctagg gcttcatttc tccaatgacc tcagctgcct ctatttcccc 23640
   ttcttcctct ggaagtccta tcatcgttat tattattatt atcatcattt ttattttgag 23700
   ataaggtctc gctctgttgc ccaggctgga gtgcagtgac atgatcatgg ctcactgcag 23760
   ccctcccagg ctcaagtgat cctcctgcct cagcctcctg agtagctggg agtacaggca 23820
   catgccacca tgcttggcta tttttttttt cagtagagat agggctctca ctatgttgcc 23880
   agggctgatc tcaacctcct gggttcaaga gatcctccta cctcagctcc tgagtagctg 23940
   ggattcgggt gcacaccacc atgccaacta atttttaatt tttttttgta tggacaggat 24000
   gtacagtgtt agaaatggat tgcttgcaga ggcaggagga tcacttgagc ccaggagttt 24060
   gatcacactg tgaaccatga tcgcacccct gcactccaat ctgggcaaca gagtgagacc 24120
   ttgtctcaaa aaaaaaaaaa aagagagaga gagagagact caaagatagg caaaaaagtg 24180
   ggaaagcttt atagtggaca aaaaggaacg ctctaagtct gccctattgg catggtgctg 24240
   aaggtgggct aactagagat agggggtact atgtggttga ctatgggtgc atctttggct 24300
   ttccctgggt gatcctaagt tggaagcagg gacaaaaatt agggaagctg ttagttattc 24360
   atcacgttct ggcagtagtg gactggttgt gatagaagtt attgttttgg ccaggtgcgg 24420
   tggctcatgc ctgtaatcct agccctttca gagttcaacg tgggtggatc aggaaggagg 24480
   gaggatttgg gaggtcagga gttagcctgg ctaacctggc gaaatcccat ctctactaaa 24540
   aatacaaaaa ttagctgggc gtggtggtgc atgcctataa tcccagctac tcgggacgct 24600
   gaggcaggag aatcagttga acctggggag gcggaggttg cagtgagcca agatcgtgcc 24660
   caatttcatc tcaaaaaaaa aaaaaaagtt atcgtttagc ttcctcgatt gttactggac 24720
   gtagtaatct ggcttcctgc aagtctaact ttcagcagac tggctacatg ggctgtgtac 24780
   tgtagataag gcagtaagta aagcaaaaat tgatagagca tcaaggataa atagaaaatc 24840
   cgtaatcaag cagaagattt gaacacttca ctttcagtaa ctgataaaac aagtagacaa 24900
   aaaaaatcag taaggatgta gaagatttga acaacgtaat taacaaactt gacttgattt 24960
   acacgtctag aaccctgcag aacacacact ttttcaagca tactcagaac atttatataa 25020
   agtgaccata tggtggacca taaagcagtt tcaacaaatc tcacaggagt aaaataacag 25080
   accgtgtttt ctgaccgtaa gtacagttaa cctagaaatt gaaaacaaaa agctagaaaa 25140
   accccatgta tctggaaatt ttaatataca ctttgaaata acaaatggat cagagattaa 25200
   ttcaaatagg aatttagaaa taccttgaac tgaaaaataa tgagaatact ataccccaaa 25260
   actgtggggt gcagctgaac agtatataga cgaaaagtat actcatatgt gcatacctta 25320
   aggagcgggg aggattgaaa gttaatggga ggcaaaagca ggtggatcac ttgaggttag 25380
   gagttcaaga tcagcctggc taacagggtg aaaccccatc tctactaaaa atacaaaaaa 25440
   ttatccaggc gtagtgaggc tgaggcaaga gaatcgttgg aacccaggag gcagaggttg 25500
   cagtgagccg cgattgcgcc actgcacccc agcctgggag acagagcgag actccatctc 25560
   aagaaagaaa aaaaaaaaag aaaaggccag gcgcggtggc tcatgcctgt aatcccagca 25620
   ttttgggagg ccgaggtggg cggatcacga ggtcaggaga tcgagactat cctggctagc 25680
   acggtgaaac cccgcctcta ctaaaaatac aaaaaaatta gccaggcgtg gtggcgggtg 25740
   cctgtagtcc cagctactca ggaggctgag gcaggagaat gtcatgaacc caggaggcag 25800
   agcttgcagt gagccgagat cgcgccactg tactccagcc tgggcaacag agagagactc 25860
   tgtctcaaaa aaaaaaaaaa gttaatggga taaacatcca tctcaagaag ttagaaagga 25920
   atgacaaata aaccaaaaaa aaaaaaatca aaagaagaaa atcataaggt caagactata 25980
   aagagagtgg ctgggtgcag tggctcaggc ctgtaatctc agcattttgg gaagcagagg 26040
   tgggcagatc acttgagccc aggagttcaa gaccagcctg agtaacatag agagacctca 26100
   tctttgctga aaataaaaat aaaaaattag ccaggcatgg tggtactgag gtgggaggat 26160
   cacttgagcc taggaggttg aggctgcagt aagccatgat tgtgccactg cacttcagcc 26220
   tgggtgacag agtgggaccc tgtctctaaa aaactaaaat aaggctgggc gcggtggctc 26280
   aaatctgtaa tcccaccact ttgggaggcc aaggctgagg tcagcagttt gagaacagct 26340
   tggccaacaa gatgaaacct catctctact aaaaatacaa aaaattagtt gggtgtggtg 26400
   gcatgtgcct gtaatcccag ctacttagga ggnnnnctnt ngattatatt ttctccttcc 26460
   tacgtcgtta ttggactgaa ttcagaatga tgactctcat tggagctctt cctgtctcct 26520
   aactacagtg gcttccgacc ccactctggt tttcacttca cccctctgct gctcatacga 26580
   gtagatactt ccttccttct ttctcacttg ttgctcttcc tcaacccccc ccgttggtgt 26640
   cccctcctct ttatcttttt ctcgcgacac ctgcgttctc ttgccctctt atcatccctt 26700
   tctcgaggcg gtcctttcct ttatccagct taaatacctt ctcctctgtt tatttggggg 26760
   ttgggttttt atctctcacc ctccctctaa tttctttcct ctttccgcac ccatcaagcc 26820
   tctcgtggtt tctcttcctc tactctcggg tcccccccct ctccccttct ttttttcttc 26880
   acccccccaa gcgctttgcc ttttttttct ttgcccttta ttcccccc 26928
<210> 7
   <211> 29430
   <212> DNA
   <213> Homo sapiens
<220>
   <221> unsure
   <222 > (4336),(4345),(4349),(4392),(4447),(4490)
   <223> Identity of nucleotide sequences at the above locations are unknown.
<400> 7
<210> 8
   <211> 33769
   <212> DNA
   <213> Homo sapiens
<220>
   <221> unsure
   <222> (33739),(33749),(33758)
   <223> Identity of nucleotide sequences at the above locations are unknown.
<400> 8
   cttcccctta cactggtcct tcgacccgcc tcggatgaaa actgaatggg tttagcctta 60
   gaggctctcg gtctctaagg gaggtgggtc aggatgccgg ggacagggtc ctcttcctgg 120
   ggcaacgtgg gggaacgagc cacctacccc tccactgaat tgccctgggg tgtgggtacc 180
   gacggctcat tcggtgtcca gggtctgaga tgtgttgaca ggaagaatga aaggggatgg 240
   gagggatggg gcgaaagaag ccacctgcag ccccaggaac tatctggcca gcacaccgtc 300
   acccagcggc ctgagccacc cctgccagag ccaggaggag accctgccaa tgggtcacca 360
   gtgtgcagga actcagaagg tcatcacagt taataccctc catgccccaa tgtgggaaaa 420
   caggtttttt cacaacaaac aagataattt ttgttatttt ggcaaaagga ggcagggcag 480
   ccccggacac ctccatccca cctcatcacc cagccgcagg gccccggcca tccctgcaga 540
   cagagtggat gtcacaacct ccctgcaccg aaccaagtgc agctcccagg ccacaggcca 600
   cccaggaaag gtccagtggc ccccggaggc tcccaccgca ggcctcccac cacagccggc 660
   accaacccag gatagctgtg ttctcctggc ttcttttcac acgggtagca gaaagctgag 720
   atccggggaa agctgagatc cagggaaagc tgagaatcgg cctctgctgc ccggacgccc 780
   acccccagct ctgctcccag ctccagggcc tccttctcag gtgcccttac aggaggcaga 840
   gggcttgagc cacctcctgg gcctggggca cgcaggatga acggggtcac ggtgcaggcc 900
   actgtccact gcgcagatcc caaggccata aacagcctgg ccacagtggc ttcccagctg 960
   gcaggcggcc agattatttt tgttgtttag caattgatta agtttctccg ctgcccccag 1020
   gggtaagtgg tggggcaaat gccgcaaccg cagcatttga cccgggatcc tgtgccaagt 1080
   gaccataggg tcacaaagca caagggaagt ggctgggccc gatgctggct ctgctggaac 1140
   ctgaggccgg ccactgtcac ctgcacggtg cctgggacct tccagcaagc acagagaagc 1200
   tatggccctc caggagcagc tggcaggcac cttggcctgc agtcaggggc tctgtctgct 1260
   cagctctaaa acaggaaagt cgctgctctg cctggggtca gggcagccag agagtgacca 1320
   agtcagtgcc ggcctcagga agggacctgc aggcgggtcc cttcctctcc catccctcgg 1380
   tgccagccag cccctcctgt ggccccccac tgcctgcctc tgcccccatg ccccaccaca 1440
   acctcaggcc catggctgca tggccactcc ccaggcaggc agtggggatg ggatttcacc 1500
   atgttggcca ggctggtctc gaactcctga cctcaggtga ggagttccta aagtgctggg 1560
   attacaggcg tgagccaccg cgccagccct ccctgtggta ctaaacactc acaccccctt 1620
   gctggggacc ctggtgaggg aacacagcct cacaagtgaa gtgtggtttt gttgagcaaa 1680
   tgacgcctgg gcagccctct catctttgcc taaaactgaa gaatttaggg gcgtggatgt 1740
   ataaaacagt tggtgactta aatgaaaaag aaggccacac tccccccttt aggcaggcgg 1800
   cctaattctt taaaagccag cacagggtgc ctttctgaac ccaggcacac agtaggtgtt 1860
   caatggacag cagcggttac ttgtactgct catgacaccc tgtctgtggc ctctgcagct 1920
   ggctccagcc tgacgcatgg ctgcgcccct ccgcaaggcc accccggtat acatggaaac 1980
   tctgtggaga aggccttggg ggccggccag gacgccaggc ccagatccca tctgcgccct 2040
   tcctccatag acctcagcga gctctcggca ccatgtgcct caggcccatt taagaagtag 2100
   ggccggccag gcatggtggc tcatgcctgt aatcccagca ctttgggagg cccaaggtgg 2160
   gtggatcacg agatggtcag gagatcgaga ccatcctggc taacacggtg aaaccccatc 2220
   tctactaaaa atacaaaaaa taagccgagt gtggtggcgg gtgcctatag tccaagctac 2280
   tcgggaggct gaggcaggat aatcgcttga gctcagcagg cagaggttgc agttagcgga 2340
   gatcgcgcca ttgcactcca gcctaggtga cagagagaga ctctgtctca attaaaaaaa 2400
   aaaaaaataa aaaaaagaag cagggccagc cacggacgac ccctcacaca gctcccagga 2460
   cgcgtgcctg ggtatagggc tcaggaccat gaccgctgca gtggccccca agaaacgtta 2520
   cttttgtcac ccaccccgcc tcagtggcag tagccaaaat aacggattag aatggaacca 2580
   tgtgacaatg ccactgcccc aactgacaga agatggctat cagcagttca cgcggcccca 2640
   cctatcacaa gtgcagggca ctctacaact tatgcatcct tccccagaca ccgtcctttc 2700
   gaccctccca ggtcagcaag gcacacaggg cctacatttc acagccacac agcagagggc 2760
   tgaggctgga actcggatgc tctgatttcc gttcaatcac atccccagag gtggcacaga 2820
   gacggggggc ttctcttgac aaagtcaaga aagtcactgc cagctccact gaagaccaaa 2880
   gaacctcagc tctcaaaccc tcttgaaggt gttaccgaac tctcccagcc tgtttcctgg 2940
   gtcccgatgt tggtcccgtg ggacacagga agaggaagaa gctccctaga gcagagcctg 3000
   gtgcacctgc cacactctca gagggctgcg cacgggcgga ggagccgtgt gcaggagtgg 3060
   ggtctggatg gaggggcgct gtggccgggg gcagggggca ggggaagggt gctccaggtg 3120
   gtgggcacag cacgagcagg ggcagggagg tccacactca gatgtgcaca gggagaaaca 3180
   aatcgtgcat ttccattgga ataggcggta aaaggtagaa aaacagagtg ggggccagga 3240
   agggagtcgg agccttctag tgtctctctg caggtgagcg gcagcccgag gtgtcagctc 3300
   agcagacttg gggtccaggg gccgtgtctt ctatcactga ccccagggca cacggaactg 3360
   gggagggaga gcagaggcac agggcacggt cagtgaaacg aaacaaggag tcatcaccaa 3420
   atgcggaaag ggcaaggagt gcccgcagcc gcacaagggt tctgtctggg caacgtgggc 3480
   gtcccaccag gccccgcacc ctgcaagcgc aaagctcgcc actgaagata aagggaagct 3540
   gttggagctg cggagctggt ctggggtccg catggagctg ggcttatgct gcagtcacaa 3600
   gggggacatg gaagaggctg caggggacaa aaccagtgac cacagtctaa ctctgagcct 3660
   gtggaaaggc gcccacagca ttcacccatc ccagagatgc cattccccct gtgcccccgc 3720
   tccacggtga cagcgttctc caggaatatg atgcgcccct ctcctcttgc atcagccctg 3780
   acagtgagta ttcaggccaa aaagcagaag agcacagctg cgtggttcca tttccatgta 3840
   gttctggaac aggcaacgct aatccaaggt gatagaagtc aggagagtgg tggagggggc 3900
   gggggttgag gatggcaaag gggcaccggg aactttccca gtggtagaaa tgttctctgt 3960
   ctggaccgtg tggtagttat gcagacatat gcagctgtca aagttaatcc aaatgtacac 4020
   gttaaaatgt gtgcgtttta ttgcctgcaa gttatacctc aattaaaaaa ataaagttag 4080
   cactcaggct tcttccacaa cttcctgaac cgtgtgagct gattttcttg ctattaaaaa 4140
   ttcacggtcc atggctgaga acagcagctg ccttctgttt gcaaagtcaa cgccaatcac 4200
   tgcccggccg cggcagactc ggccccacag gacctccttt cttttttccc tttgacctac 4260
   ttccctgata agtgacaaga cagccagact ctgggaacaa acgcccgtta ttcggccccg 4320
   agctgagcgg gccctgcttc ctgagctaat ccgcccggac agacggaggg acgtgagggg 4380
   ctttgccgtc ggctccagct gtcagtctgc ccgtcagact cgacagtggc cccctctgtt 4440
   cctcccgctg cccccactcc atccccgact tctttttgtt tcctgtccct gacagacgaa 4500
   catctgttaa aactctgtct gggtgagctg tggccagcgg cccacaaatc cccaagccgc 4560
   accccagcct catctgggcg ctgccgggag cactgcctgg ccaccctctg gacatagctc 4620
   tgagagccac cggccagggc acgtgtggcc cgagtggcat ggtgcacgcc gctaagccca 4680
   ctgcccaaag gcccccaagc aggagggatg tgcaggagac aaaagtcaaa agaacagggg 4740
   cacgttccac agaggatggg gctggagggg tggcagtgag gaacagcagc ttccgaggat 4800
   ggcggtggca actcccaaat aaggcctcac tcctgctgtt tttagctcat tccacataat 4860
   tggaaaaaca tggcagaaac cgaagccagc tgctgccttg gtcctggggc tgtgtggagg 4920
   gggtggggag gccggaggcc caggctctgc actcgactgc tggggatgag agtgactctg 4980
   agctgcagag agcagcatcg cagccgccat ggtcccattg agccccggcc acgctgggcg 5040
   gcagaggctc gtgggatata cctgccctgt ctcatggggg tcacttcagg aggggcgggg 5100
   gagccaggac acagcccagg gctagcggtc accctgcagc tcaggggcca cgtaaatagt 5160
   gccaccttga aggcacacag cagtgcgggg ccccccccgc caccaacgca tccctacctc 5220
   taggaggccg cctgtgtgcc cctgggaacg ctgctccctg tcccttgggg tcctggtgtg 5280
   accaccctct cagccccttc cttggggaag gcacctgact ccctacaccc agctggcttt 5340
   catttgctca aaatcaggaa aaagcagaat tcaagacatc acagaaatgt cttcgcctgt 5400
   aactccatga aagataaacg gtcagacacc caggagggag tcccagggac ccttgagtct 5460
   cacctgaggc tctggcttca aacctcgaga tgtttccagc catgctagcg ccgcccccca 5520
   caacctgccc cacacagtcc tcccttggga actcacagat ttggccccca cctgccccgt 5580
   ttcttctggt ggagtgggtg cgttgggttg gggtggggct ggggactctg gatgtgtctt 5640
   aagagtctga gtgattctga cacagccagg ccctgccccc ctcctgacct tcgccccaca 5700
   ggaaagggag ccacacgcct gaagcgccca gcacaccccc ctccgtcctc cccaggtcac 5760
   ccgctggccg tgtgagccgt gctccccact gccccttcac ccaccccagc tcctcctggc 5820
   agcacccagc cttggaagct acttctgatt acaaccgccg aaggaagact cgctccctcg 5880
   gcactgaccc agacagcctg caccatcacg ctgctcagca caacccacac agccttcctc 5940
   caaaccccat ggagcgggga gtataatcac cccctttcta ccaacggaca aactgaagca 6000
   cagagaggtt aagtcacttt cctaagctcc caacacgatg acaaaaaata gaaggtcagc 6060
   ccgcaagtgg aactaggtgc tccaagtccc cggtctgcct gacactgcac ctcctcgccg 6120
   ccacggtccc gggtccgcct gacactgcac ctcctcgccg ccacggtccc gggtccgcct 6180
   gacactgcac ctcctcgccg ccacggtccc gggtccgcct gacactgcac ctcctcgccg 6240
   ccacggtccc gggtccgcct gacactgcac ctcctcgccg ccacggtccc gggtccgcct 6300
   gacactgcac ctcctcgccg ccacggtccc gggtccgcct gacactgcac ctcctcgccg 6360
   ccacggtccc gggtctgcct gacactgcac ctcctcaaca ccaccacggt cccgggtctg 6420
   cctgacactg cacctcctca ccaccaccac agtcccgggt ctgcctgaca ctgcatttcc 6480
   tcatcaccac agtcccgggt ctgcctgaca ctgcatttcc tcatcaccac ggtcccgggt 6540
   ctgcctgaca ctgcacctcc tcaccgccac ggtcccgggt ctgcctgaca ctgcactttc 6600
   tcaacaccac tccttggccg gctcccaact acaaaccaag ccatgtcttc catcctgaat 6660
   cctcttggcc taaacatcac tcacaatgcc tccctcggga acaggcacaa gtcccaccag 6720
   cacagcctcc ttcgttacct gcgtttccgc tagcccaggg ccagctccag agccctcacc 6780
   acagagcctc tatccttcac ccccggacac tggacctcac caacccatag cctggaggag 6840
   atccctgtgt gaccccaggg cctcctctgc ccgactctga atttcactgc ccaacgtgac 6900
   acctcggaag gctctctggg cactggcagc cctccatggg caccgctcct tctggccagc 6960
   tctgacatcc cggctggtga ggtgccctgc acgaggcctc tgcccactgg gacctcacag 7020
   ccgtgctgtc agctgcaaca agcgacagaa tttcacgttt tcttcacgtt gcccctgggt 7080
   gagcagctcc aggtagtttt cagtcgaggc gaggcgtccc gtcagcagcc aggcggcaca 7140
   gctaattcat gcccgccggg cgcacggccg caataccaat gggcacctgc agcctggaaa 7200
   gccacagagg aaccgagaac agcgactgtg ctcaggtgac aggactgtgg tcttttaaca 7260
   aaacattttc ctttaacgtg atattttacg gcaaggaatg aaacctggag ggcaggacat 7320
   ttggatacta aagccccagg ctgccgcgtg gtctgctttg tgaagtctga agcccgcgcc 7380
   ccattctggc cccgctcaca ggtccggctc tgactcacca gcttcaatgc taggccgtgc 7440
   ctgtcctcca accagaacat gacttcctta aggacaaagc cgtttctcgc ccatccccat 7500
   ctccctctgg attaagaaat atgggaagat cttctagaac cacctcaaat ttgcagagag 7560
   ccatcctggt gacaaaccct tgaaatgctt ctaagaagag tttaggtttc ttctcaactc 7620
   taaaacctct agaaaactct atttccacac cagctgcccc tggaacactt cagcttcaaa 7680
   agggcccagg gcagggagac ggaggagcca gcatccacac cgagcaccag cctgttaatt 7740
   aacgggaagc gggtggggcc catctccagg cagctctgag gtcagactgg ggaaccatgc 7800
   ttacaaaaaa aagtgaactg aaacgctcac gtcctcatgc aaaaccagac tcccagttgc 7860
   atctttctgt ctcattgagg agctttttcc tccctttgac agaacaccct acacacggca 7920
   tctggaacca aagcagaaag attcaggctc agagtaaaac agtccccaca ctggctgcat 7980
   gtggacgttc ccggcccaga gtctcgccca agcagggcct ataaatgaca caaaatgttt 8040
   ttctcctgcg tgccagtcat gctccaactg agttatgtgt aaaagtgcct ctcacggctg 8100
   agggcaaaaa cagttcccac aagactagag aaaggtgacc cctgacggct gagtctctag 8160
   ggagcgtgga gctgcgtgct cagccctgcg gccctgacgg ctctggaatg gaaaagctat 8220
   ccaactggaa gggcagggct cgctgctagt ccagcggtcc aaccccacag gtgtctgtgg 8280
   tgtcagctcc atgccacaga gcccagggct ggggccagag ccaccaggcc ccctgccagc 8340
   ctgcaggggc ctcctcctct gggtagccta accaccccct gtgagcgcag gcagcctcct 8400
   ctaatcacca cagggcctgt ccccccctct cccccgcttg caggaaaatg agccctgagg 8460
   actccccagg gctgctctgg gcctggacat ggagactggg aattacattt gcagaaggag 8520
   cgcaatgccc ttgaagggct cagccacgag cagccagtcc ccagggctca gaaggcccag 8580
   ctgttagaac cctgggagcc agcaaagagc caggggctcc acctaagtct atagcccctg 8640
   cctcttctgg ttgggaaaga aatcaacgcc cctttactgg ctcccactga cagcccactc 8700
   ccccaggtat gggaggattc tgggacgatg caggcaaacc tggaccctga gtgaacctgc 8760
   cccagctctc acgggcctgg caccagccac agcacctaag gcgccggtca tggtgacaac 8820
   atgaaggtga taagggcatg gacagtggac atggcagctg gacactgggc acccactgga 8880
   tgccaggcac ccagcacggc tccgtcaccc ctggatgagc agtggccctt tgcaagccag 8940
   ggtagcctgg gcaagttatt tgggggtctc caagcttgtc cagctgtgcg acttcactga 9000
   gccatgagtc tgggatttta tcagggccca cacccgttcc tggaactctg atacgtgagg 9060
   gagccacaca gggaccctta acaaaagctc ccagggcaac atgttctctt gcctcagtct 9120
   cccaaatagc tgggattaca ggcgcacgac taccgcccgg ctaatttttg tatttttagt 9180
   agagacaggg tttcaccatg ttggccaggc tggtcttgaa cccctgacct caaatgatcc 9240
   ttccactgtt agggcaaggc acctgacagg cacgactgca cgatctgctt gttgggggct 9300
   gtgtccattc cccactcctt cgacaaatgt ccacacccag ccttgctttg acaccccaag 9360
   aacagagatg gtgacacctg cttcctacat gcccattgct ctcccaaggc agacatcccc 9420
   agcagatgca acacagtgtt taggcagaca tcaccaatcg atggtggcaa cagacaccag 9480
   gccctgctcc ctctaactcc agtggccagg ccccaagcca gctctcacct gcccactccc 9540
   aacccacagc agcaagactc agaaatggca aaaacacaaa gagaacagaa acgccccata 9600
   gcgggaggat gactaaaaga catgtcttga taagatattg ttcaggcata ggccaggcac 9660
   agtggctcat gcctgtgatc ctagaacttt aggaggctga ggtaggtgga tcacctgagg 9720
   ttaggagttc aagaccagcc tagccaacat ggtgaaaccc catctctact aaacatacaa 9780
   aaattagcca gacatagtag cgggcgcctg taatcccagc tgcttgggag gctgaggcag 9840
   gagaattgct tgaacctggg aggtggaagc tgctgtgagc cactgtactc caacctggac 9900
   aacagagcaa gactctgtct caaaaaaaaa aaaaaaaaaa gatatccttc actaaaactc 9960
   atgtctttga tacatattta cctcctgcaa tcgcaaatgc ttctgcagtg cataaagtga 10020
   aataaatagc aggaagcctt acggttcgat cacccacaca gacacacagt cacatacagg 10080
   aaaaacgcag ggagggctgg ggaacaaaaa aacagaagat aaaatgtgga gacagacaca 10140
   ccaagagagt aagagaccac ctccagacct cccttcagct tctcaaacac acgagccggg 10200
   cccgttacag aatttgcggg gaccgctgca aaatggaagt gcagacagcc ccttactcaa 10260
   aaggtaggaa tttcaggtca acaacagagc tcacctcata tgactacaca ggtcacacag 10320
   cccgtgaagt cggtcccaac accagcatgc tcctgcctca aagccgctgc acgtgctgtt 10380
   ccttctcgcc tttccctctt ttagtccttc agatctcagg cctcctgaga gagacctctg 10440
   acctgccggc tcaggcggcc acacccccag tacaggagtc tccggctcag cccctgctgt 10500
   gttccgtacc cgatccaggt ctgtcctatg tccatctgtg tgccggcttg cttcctgaca 10560
   tggcccccac cacacgtgtg cctcggggca ggggaacagg cccgtctcat taactgcttt 10620
   cttctcagat attttctgga atatttgtgg atattgggca acatatatgc tccacctttt 10680
   tcagactagc caggacgagc tgcatttttt tttttttttt tttgagacag ggtctcactc 10740
   tgttgcccag gctggagtat agcggcatga tcttggctca gtgcaacctc cgcctcctag 10800
   gctcaagcaa ttctcctgcc tcagtctccc aagtagctgg gattacaggc ccgtgccact 10860
   actgcccagc taatttttat atttttagta gagatggagt ttcaccatgt tggccaggct 10920
   ggtcttgaac tcctgacctc aaatgatcca cctgccttgg actcccaaat tgttgggatt 10980
   acaggcgtga gccactgcgc ccggcccgag ctgcctgttt tacacctttg ccatattccg 11040
   gtgattctct ctcccctccg tcccccggcc ctgactgtgg tggccactcc ctgccgtcat 11100
   gagcccgtat gtcctcactc tttccctttc cgccaggact tcaaccaaca ctgcagagcg 11160
   cagggtccag ctccagcact gagttcagcc tcttctcacc aacagacagg caggaaagaa 11220
   aacaaactct gagaaggcca aggttcccgg gcagccagca agccaagcat ccttctccgc 11280
   tgaggcttgt gcagccgagg caccccctcc tccagggagc aggcagcgtc ctggggcagt 11340
   ctgcgaggga gaccagggcc cttgctccac cagggcccca ggtatggggg cagcagcaaa 11400
   ctcatggctc tgggagccag accccacctg ctagaaccta ctatgccacc tgctgtgggc 11460
   aaccccaggc tggtgacttg ccctggcctc ctctgtaaac aaagggctca tccaacctgg 11520
   tcaaaccact cctccccttc aagggtctat aatcctccct taacctgctt ggtccaaacc 11580
   cctggtgtcg ccaggtcact caggaggcag ctcatctgga ctccttccct gggtccagtt 11640
   tctctctcaa cattgccttt gaggccgagg tgaacggtca acagcgaagg gccccagagg 11700
   tgatggagga gcgggtgtcc aagacactca ccctttctaa tgcactgact ccctcgtgga 11760
   ctcacttgtg ccgtctcccc cacccaccca gccccagagc ccagagtgcg agcgccagag 11820
   gcccgggatt ctgtctgcac cgcggggtcc ccagtgcctc ggagcaatgc cagcacccgg 11880
   caagtgttcg acaaatgcct gctgaatgag caaatggatg gatgaacgaa tgaatgagca 11940
   agcagatgaa tgaatggggt gctgtccaga gccgtgagga ctaggccgcc caagtcccca 12000
   tttctcaaat tctccttctc ccgacttggg aaacaagatg cttggtcggg gaggctctcc 12060
   aaccatcccc tgcagcagcc ggcacagcgg acagaccctt tgatgtaaca gccatgtctt 12120
   cattaaagat gccctgctct cagaaagaga aagacaaata caaacctgga aaatcctcac 12180
   caaacgcagg acccctgcca gggagcagag aaaagaccca cacgccacgg gcgccacgac 12240
   cacacacaca ccccagccgc tgcacacaaa cacagaccct agccagcaag aacaggggga 12300
   ccaggaaact gttcctaaag tcaggacccc catgtgctca gacagcagtg agagcaagga 12360
   cacttctcca tccaccggat gccaggagag tccttttagg gggccccaca ccgagactct 12420
   gcccttagga ctgttcctga gtgtggaagc cagcccactt ggaagccccc tgccctcccg 12480
   agtgggacac cggcacagga agcaggccct gtcccccacc actttctgca agctgggccc 12540
   catcacgcta cagaaacggg gaggactggt cccagggatg gcgctttcct gacacctctc 12600
   gttaccccct cgcttgccag gccccagggt cagccccaga ggccagactg gctatcccag 12660
   gcccgggagc atccccgaag gcgagctgca tcctgaacgt gtgtgatttc ccgaagggcc 12720
   cgccccgaac cgacacctgg aaagaaagat cctcagccgg tgccccagag gagaagagcc 12780
   atgcctcact gcaacacagt cccaggaagc accaagtgcc tgaggaccaa ggcggagagt 12840
   aaaaaagtgg aaaatatctg gggcaaaaat aaaacaaaac aaaacaggat tgacctcctg 12900
   ggctcaagca atcctcccaa ctcagcttcc cgagtagctg ggaccacaga cttgaatcac 12960
   cacacccgcc aagtggatca tttcgaacgg gtttgccgag gttccttctg gggcaccccc 13020
   ggcggccgca acccattccc gccaggcccc gccccgcccg cccgccccgt cccgtcccac 13080
   cgcctcacct gccttacacg tcctgccgtt gtcctgcagc tgcacacccg tggggcaggc 13140
   gcatgtgtag aaaggctcgc ttggggacag caggcacagg tgggagcagc cgccattgtc 13200
   ctcctcacag cgagtgtgga ctgagaaaac caggacagac tgagagaagg ttccagaaga 13260
   ggaccgtcac ttgtttctga atgagtcaca tcctgcctcg tcccccgtga cagcctccag 13320
   tgtgtccctc tgcccaaaca tcggcctcaa gtggcatcag ggacctcccc gcgggcacca 13380
   ttccacctgc ctcatcgctg gccccgtcca catggggccc tcagcctggc cagacggcct 13440
   gcaatttccc caaaaccagc cgtgaccttc ctggccaccc tcacacccag atgtgacctg 13500
   cccatggagt gacatcctcc ccatctgctt cctcccacca agctcctatg actagaacac 13560
   cctccccagc tcctcggagc ccccaaagga cacccctctg caaaggctgc cccccacgct 13620
   ccaatggccg gggtcaggac ctgcctgtgt ggtagtgacg ggaaccccag agacaatggg 13680
   ctcctgggca aaaggcttgt cttgtctttg tgctatgtgt ggacccagca gcttccatag 13740
   gaacactgtc cttcttgctg ggatggccaa gcttgtcact ctcccaagcc ctcctatgac 13800
   caacagcaat tgaacggaac tcgataaatg cttccagcac ctcattcaaa ccaggggaaa 13860
   gctgggtgta gcagccccaa aatacggata taactggaac aacaaactca tcaaaatgaa 13920
   cctctccctc cctcatgctg ccccaagtgt agatgggttt tgtgaccacg actttctcac 13980
   caggaaacag ctccagagag ccccaccctc ctgtgtcctg ctctgggaac agctggcacc 14040
   cctaggcccc acatttcaat tcaaagtcca aaccttccat aatggcctgg ccagaaatct 14100
   ccatccctgg tccctgtggg agtgggccac tgtccccaga gccgcagccc cactgtcaca 14160
   gaagctggtg catttcccca tcagggacct ctgtcacaac ccagcgtggc ccccaggctg 14220
   agaactgctg attctgggca gattattcat tgataaatac gcgacttgca gggccaagca 14280
   tggtggctca tacctgtgac cccagcactt tgggaagtca aggtgtgagg atcactggag 14340
   cccacgagtt tgagacaagc ctgggcaacg tggcaaaatc tctcatctct attaaaaata 14400
   catacacaca cacacacaca cacacacaca cacatatata tgtatatata aataaccata 14460
   tatatatata cacacatacg tgtatgtgta tataaataca tatacacaca cacacagaca 14520
   acttcttctg ggccttgaaa acgaggcaac cttccttgga aatccccttg ccactgctga 14580
   gcctgaaata gcccccatga gctctgcaga ggggtcctct gcaggcccgt gtcccccagc 14640
   cagccacaca cctccctcca ttgcagcagg taccccttta gagagggggc cccccagagc 14700
   atgggcttct gcagggaggg gtcacctgcc cccccacccc acccacgccc gcgcaccccc 14760
   acgcccccgc atcctcccac tcccctgccc cgcgcccccg ctccccccag ccccctcacc 14820
   ctctcccccg tgccccaacc ggcactcaca aaaaggctgc cgctcctggc tcagcacctg 14880
   gatgtccatg ggtgagtata gggcactcag gatctccttc ctcttccccc cagtgcgctt 14940
   gttgcaggca tggatggagc gggtctgcca gtctgtccag tacagagtgt ccccggagag 15000
   cgtcagggcg aaggggtgcg tcaggctgcc ctccaccacc ttctgcctgc agtcagggaa 15060
   gcggggtgga ggagccatca ggagggtccc ccgacagtca ttgctgctga cccaattaat 15120
   ttcttttttt ttttttgaga tggagtctcg gtctgtcgcc caggctggag tgcagtgatg 15180
   taatctcagc tcactgcaac ctccgcctcc cgggttcaag caattatcct gcctcagcct 15240
   cccgagtagc tgggatcact gatgcccacc actacgccca gatgattttt gtatttttag 15300
   tagagacagg gtttcatcat gttggcaagg ctggtctcga actcctgacc tcaggtgatc 15360
   cacccacctc agcctctcaa agcgctggga ttacaggcgt gcgccaccat gccaggcttc 15420
   ccatttgctt tcaaccagac aagtgaggcc aggtcaagag ccccaggagc tggcgccctc 15480
   gtacatttct cccggcgtgc acagggcacc tcccaaacac agcctgtgat ggtgacacac 15540
   gggctccccc aggtcaagtg gcaaagtctc ccccagggaa gaaaggagga agccatgcct 15600
   ggcaaaaagc acacctctcc tgcccaacgc tttaacctct gtatacaaat caggccatgt 15660
   gcactcgctc cttcttacaa tgctcataat ttatactttc agagtaaatg aaacttggca 15720
   tcaacccgag aaacagctat tcttttctag atgcttacag tgcccagcaa atgaggactc 15780
   gggtgtaatg agattatgga cactggaaac aggatcataa tgtgacgtgg tcggtaatgt 15840
   gcagttttat ttgcttaatg accctcgccc cgtgacaggc tccctgaggg tgggcctggg 15900
   ggcagaggtc cccgccacgt ccccagccct cagcacagtt gccaggagag ggtgacactc 15960
   atgaagtggc acagggaaga tgggagctgt gggctctgca gatccaccac ctcttctgtt 16020
   catttttgtt gatgctgttt tttaagaaaa ttattgaagt aaaattcaca ggacatacgt 16080
   ttactttttt tttttttttt ggagatgggg tctcactctg tcacccaggt tggagtgcag 16140
   tggtgtgatc tcagctcact gcaacctctg cctcccaggt tcaagcgatt ctcccacctc 16200
   cgcctccaga gtagctggga ccacaggcgt gcaccaccac acccagctaa tttttggggg 16260
   gtatcttttt ggtagagaca gggtttcgcc atgttgccca aggctggtct tgaagccctg 16320
   agctcaggcg atccacccgc cttggcctct caaagtgctg ggattacagg cataagccac 16380
   tgcacccagc ctaaatttac cactttaaag tgaatagtgt tacctagtgc attcgcaagg 16440
   cggtgcagcc tccacttctg tctagttcca aagcacttcc attgccccac aggcaaaccc 16500
   cacacccggc agcagtcatg ccccagtccc cgcccccagc cccggcaaac acttttgatg 16560
   gacttaacta cacacattct caacatctca tataaacgga atcacaatat acagcctctg 16620
   atgtctgtct tctttgactt ggcaccatgt tttcgaggtt catccaggct gtagcatgtc 16680
   agtgcttcat cccgttttag gggtgaacca tattccagtg tgcagacaga aaccaatctg 16740
   tgcatccatt cacccactgg gggacctttg tgtcatttcc accctcggct gttgtgcaca 16800
   gtgctgctac ggacattact gtccattcac attttgtgtg aagacctgtt ttcgattctt 16860
   aagagtatac agctaggagc ggaattgctg ggtcatacgt aaatcaatgt ttacgtctca 16920
   aggaatcaac aaactgtttt ccacaatgtt gtcttttttg tttgttttct gagacagggt 16980
   cttgctctgt cacccaggct ggagtgcggt ggtgtgatca tggctcactg cagcctcaat 17040
   ctcctaagct caatccatcc tcctgcctca gcctcctgag tagctgggaa cacaggtatg 17100
   taccaccatg gccagctaat tttctaattt tatttttttt tgtttttgtt tttttgagac 17160
   agagtctcgc tctgtcgccc aggctggagt gcagtggtgc catctcagct cactgcaagc 17220
   tctgcctccc gggttcacac cattctcctg cctcagcctc ccgagtggct gggactatag 17280
   tcaccggcca ccacgcctgg ctaatttttt tgtattttta gtagagatgg ggtttcaccg 17340
   tgttacccag gatggtctcg atctcctaac ttcatgatcc acctgccttg gcctcccaaa 17400
   gttctgggat tacaggcgtg agccaccacg cccgacctta cttttaattt tttaatttta 17460
   ttattttatt ttattttttt tttttttgag acagagtctc gctctgtagc ccaggctgga 17520
   gtgcagtggc gggatctcag ctcactgcaa gctccacctc ccaggttcac gccattctcc 17580
   tgcctcagcc tcccgagtag ctgggactac aggtgcccac cacgatgccc ggctaatttt 17640
   ttgtattttt agtagagaca gggtttcact gtgttagcca ggatgatctc aatctcctga 17700
   cctcgtgatc cgcccgtctc agcctcccaa agtgctggga ttacaggcgt gagccaccgc 17760
   gcccagcctt tttttttttt tttttttttt ttttgagata gagtcttgct ctgtcgccca 17820
   ggctggagtg cagtggcggg atctcagctc actgcaagct ccgcctccca ggttcacgcc 17880
   attctcctgc ctcagcctcc cgagtagctg ggactacagg cacccaccac cacacctggc 17940
   taatgttttg tatttttagt agagacgagg tttcaccgtg ttagccagga tggtctcgat 18000
   ctcctgacct cgtaatccgc ccgcctcggc ctcccaaagt gctgggatta cacgcgtaag 18060
   ccatggcgcc cagcccatgt ggccattttt cagtgagaga agccagaggc ccatcactct 18120
   cggttgctcc ctgggccatg ctctgcctca gccagaagca ctgagggaag gtcagcctcg 18180
   gcccttgccc cagccacagt cacagataaa ggggcctgca caggtctgtg tggctccaga 18240
   gctcgtcacc caacacacga cgcttccatg tgaatagccc caggtgcatc atgaagagcg 18300
   atggccgctg cagaggcaga agaatcccgc ggggaagcag gtgggagaga ggctgagaac 18360
   agaccagacc ctggagctac agaccctatg ttccaaccct ggctgggact agctgtgtgg 18420
   ctctgggcaa attcacatgc ttctctgtgc acaggggatc aaaatagcaa acacaggcta 18480
   ggcacagtgg ttcacaccta taatcccagt gctttgagag gccgaggtgg acacatggct 18540
   taagctcagg agtttgagac cagcctgggc aacatggtga aacctcgtct ctacaaaaaa 18600
   aataccaaat aaattagcca ggcgtggtgg tacgtgcctg tggtctcagc tacttggaag 18660
   gctgaggcgg gaggaacact tgagcccaag aagtcaaggc tgtggccgcg tgtggtggct 18720
   cacgcctgta atcccagcac tttgagaggc tcaggtgggt ggatcacttg tgatcaggag 18780
   ttcaagacca gcctggccaa catggtgaaa ccccgtccct actaaaaaaa tacaacaatt 18840
   tgccaggcgt ggtggcgggc acctgtaatc ccagctactt gggaggctga ggcaggagaa 18900
   tagttagaac ttgggaggtg gaggttgtag ttagccaaga tggtgccgct gcactccagc 18960
   cagggggaca gagcaagact ccatcccaaa aaaaaaaaaa acaaacaaac aaacaaaaaa 19020
   agaggtcaag gctgcagtga accatgattg tgccaatgca ctccagcctg ggtgacaaag 19080
   tgagaccctg cctcaaaaca ataaaaatat aaataaaaat aaaacataat agcaaacgtt 19140
   tcatagaggt ggtatgagca ttaaatgaac tgataaacgt ccctggaaaa cagtaagtgc 19200
   tatggaagga ttcgctgccg ccaccgccac caccattagc atgtttcaac ctccatcacc 19260
   ctcactgtcc cctgtcacca tcctttgacc agggcactcc cagctgcagc ctttctatcc 19320
   tcttgtccac ccttcataac tgtaagatca ctcagctccc aagaaccaca gtctacaggg 19380
   taaccacatt tccaaatctc aaaccagacc cgctggtctg cacttccagg gacaacagga 19440
   tattttcaaa ccagcccaaa agagatgtgt ggctcagcat aagaggaaca ggagaaactg 19500
   aggcctcttg ccctgagaat gagcttggaa gtggatgtcc cggcctcact caaaccttca 19560
   gatgactgag gcccagccag gagcttgagt gtaccctcag gtcataccct gagccagaag 19620
   cacccagcta atccactcct catcactgac tccctcccca taaaaaacct gtttgctgtt 19680
   tcaggctgtt aagttgtggg ctgttttgtt acacagcaat ggataactaa cacacgaggc 19740
   ctggcaagtg tggagcaaag ctgcccaagc cctcaagtct gttcatgtgg gtgttggcct 19800
   gtgtttgcag aaatccagcc actgagtcct cccatgcagt cactactgcc ctctgcacag 19860
   acacctgcca catccctgcc tgggccagga gctccactag tgcaggaatg gggtctgccg 19920
   tcccaggagg atccctgaca cctagcacag ggctagcagc aggcagcact tggttagtga 19980
   ataaactgcc cttcacctgt acacagaagg gatgtttcta taaggggtaa ttaagtacag 20040
   agctgggaag ctatgctgac cagaaggctc taaaagcaat taaccaacga ggggaaaacc 20100
   cttcctactc attctcggcc cattttattg agcactgacc atgtggaagg ccccctggtg 20160
   agactgggga atgcaccaat aactgagaca gcttccggct gttgccctca ggatgcctga 20220
   gctgggatag ggccagggtg ggggtggtgc gtgtgacagg gttactgttc acaaccctgc 20280
   cgggccataa gccctcccca acaattccaa aatccaaaac gctctgaaga tggaaagctt 20340
   ttgttgctca tctggtgaca aaacctcatt tggtgcatgg gccgggtgcg gtggctcacg 20400
   cctgtaatcc cagcactctg ggagccgagg ggaaggatcc cttgagctta ggagtttgag 20460
   accagcctga gcaacatgtg agaccccgtc tctaccaaaa atacaaaaat tagccaggtg 20520
   tggtggcgca ctcctgtagt cccagctact cgggaggctg aggcgggagg atcgcttgag 20580
   cctgggaggt gggggctgca gtgagctgag attatgacat tgcactccag cctgggtgaa 20640
   agagtgagac tctgtctcaa aaaaacaaag ttaaaaaaaa aaaaactgtg catgggtgtg 20700
   ggctacagat agtcttttct gccctactta gaatgaacgt gccacatttg ctatagaaat 20760
   attcaagggc tggtggcaaa tgccacacag accctgacgc tgttccaagt tctgagaagt 20820
   cctgcattcc tcagggcccc agagtttcag agaagagtct gtaggcctga gttaagaagg 20880
   aacgccttca aaagccctgg ggacaaaggg gaaaggggtg ccccaggact gcgtgggtac 20940
   ctaccggaac gagccgtcca ggttggcacg gtggatgaag ctgagcttgg cgtcagccca 21000
   gtagagcttc tgctcctcca ggtcgatggt cagtccattg ggccagtaaa tgtccgagtc 21060
   cacaatgatc ttccgggtgc tgccatccat ccctgcccgc tcaatccggg gcgtctcacc 21120
   ccagtctgtc cagtacatgt acctgtgacg ggggcagggc aagagaagca gctaacacag 21180
   atctgttttt tgtttttgtc tgcatagatg cagacatgaa acaacagaca gtgaacttgc 21240
   cctaaaatct cacccatcgg aaataaccaa caggtatggt ttcaggtatt cctgccttaa 21300
   gctgggcaat caaaatatac tatttccaac ttgttctcag ttaacagtaa attctgggca 21360
   ccttcccttc ttgtggatag aaagattcct tgttcttttg atgattgcct agtgtactct 21420
   gctgtaagtt ttttaaagaa cttcaggtta tttctgattt ttttgctacc atgaaaatgc 21480
   tgtaaatgaa cctctaaaag gcaattcaaa acactcagga tggaatatta tttagtggta 21540
   taaagaaatg agctatcggc tgggcccagt ggctcacacc tctaatccca gcactttggg 21600
   aggccaaggc gggtggatca cgaggtcggg agatcaagac catcctggct aacacagtga 21660
   aaccccgtcc ctactaaaaa tacaaaacat tagccaggcg tggtagtgag cacctgtagt 21720
   cccagctact taggaggctg aggcaggaga atcatttgaa cccgggaggg ggaggttgca 21780
   gtgagcagaa atcgcaccat tgcactccat cctgggcgac agagcgagac tccatctcaa 21840
   aaaaaaaaaa aagaaaagaa aagaaatgat ctatcaagcc atgaaaagac atggaggaaa 21900
   cttaaatgca tgttagtagg tgaaagagcc aatctgtatg agtccagttc taaacactct 21960
   ggaaaaagca aatacacaga gacagtaaag catcagtggt tgccaggagt tggagaggag 22020
   agggatgaat gagtggagca cagaaaatca gggcagtgga actatcctgt atgacatgga 22080
   atggtgggtg catgtcctta ctcatctgtc taaaccaaga atgtacaaat caagggcgaa 22140
   ccctcgtgta aacgtggatt ttgggtgatg gtgcgtcagc cagctttcat cagttgtaac 22200
   aaatgtacca ccctgcacag gatgctgaca gttgggaagg ctgtgtgggt gtgaggacag 22260
   ggatgtatag gaactcagta cctgctgctc atcaattttg ctgtgaacct acaactgttt 22320
   gaaaaaatta agtctattta aaaacaacaa aacatggcca ggcacgatgg cttgcacctg 22380
   taattccagt acttcgggag gctgaggtgg gtgggtcact tgagccaccc tgggcaacat 22440
   ggcaaaatcc cacctctaca aaaaataaaa attaaaaaaa agttagctgg gcatggtggc 22500
   acactcttgt agtcccagct acttgggagg ctgacgtggg aggatcccttcagccctggg 22560
   aggtcgaggc tgcagtgagc tgtgactgta ccactgcact ccagcctgga tgacagagtg 22620
   agaccctgcc taaaaaaaaa aaaaaaaagg ctgggtgcgg tggctcatgc ctgtaattcc 22680
   agcgctttgg gaggccgaga tgggcggatc acgaggtcag gagatcgaga ccatcctggc 22740
   taacacggtg aaaccccgtc tctactaaaa gtacaaaaaa aaaaattagc cgggcatggt 22800
   ggcggacacc tgtagtcaca gctactcggg aggctgaggc aggagaatgg cgtgaacccg 22860
   ggaggcggag cttgcagtga gccaagatca caccactgca ctctcagcct gggagacagc 22920
   aacactccgt ctcaaaaaaa aaagaataaa acccatggct gggatggacc ctgaacctgc 22980
   agctgcagct gttcctgggt aggtctgtgg gcgacgtggc tttgcttctc catgttccca 23040
   agagacaagc atcacccatc catgagaaac aagcacatcc tcagggcgcc cttacgtgat 23100
   ctctggccaa tgaaccaaga caaagtgagc agacaccagg tctgggatgg caggtcccac 23160
   ccccaccagt gcccagtgtg ccctgtttgg aggtgaccac agggtgtgtg cccagaggct 23220
   gggcgtgact ctcagcggag accagagggg aaccacacca gcttggagga ctcagttccc 23280
   atcccagcca gctgggatga gccacaggac acaagggctg gcagacctat tgtgttttgt 23340
   ccacccttca cagcagagaa aggggacagt gcccagaatg tcctctgagg agcctcctcc 23400
   cactcttggt ccttgtaaaa tggtgctgac tcccttgctc ccttcttcct ggggtgggcg 23460
   gcaaacccca ttcccctcag ccttagcaag tgatttagaa acaggcagct cgcccaagcc 23520
   aggcatgaga gtgatcccgg gacacaggga gaacaagccc cgctttgccc tctgggggtc 23580
   tccattcagc agaagaggca aatgacagac acacagccgc ctcctccccc accatggtgc 23640
   tctgcagcct caggagcctc aggtgcacca agggccaccc catccagggg gccatgcttc 23700
   cttgagtggt atcgttcctg agcgagtacc atctccacct tccagagggg ctgtgacaag 23760
   atcaacaaga atgagggcat aggagcctcg aaccaaacat gccctcttcc ctgcagaggc 23820
   tgactgcgcc cagctgctat caccaagccc ctgctcctcc ggccccgtgg ggacagggta 23880
   agaggggtgt cacatggaac agctctccaa acagtccctc tcaagctgct gtctcctgtg 23940
   catctagtga gaacccaacc aacaaaggga aggtgggaat tgctattccc attaggcaga 24000
   tgagaaaact gaggccccga aaggctggcc tgttccaggt tacaggcgct gagcggctgc 24060
   tctgggaaca cacttggtgt ctgctgaggg cccgagcccg gccatcatat gactcaccct 24120
   tcgccagcaa agcccgggtg tgggtgaact tttcctggca gcctgggact ccaaggtgct 24180
   ggcagccagc ccagggaagg ctcccgcgtg cctgcggcag acgccttgct ttacctgcac 24240
   gtccccaccc ctaggagcct ggacagagcc cagaccctcc gccacctcct gagaaggtat 24300
   caggggcatc agtctggact tgggggggaa tccacacagg ccttccccaa atgctccacc 24360
   gtggcccatg gaaaaggctg gaaaacgtgc aggagcagga gcctccgcat ggagcataat 24420
   tcacattcct tccccgagtt tcataacaga ggcctgctgg tttccttaaa tggggaattt 24480
   gcgagccagt cggtgaccag agactggttg gcgtggacgt gctcttgcag agtctcaaac 24540
   gctaccacaa gcccagccaa attccacgga ggaaaatcga cttccgaaga aaagagctgc 24600
   agcatggcct tcgtgcagag ccagctgcgg ttgtggttgt gtgttatttt agggaagggc 24660
   cattttgcat tttaaagagg gggttgggtt tcaccctggc tttaatttga gacccggggg 24720
   ccactgcagc cccttgtcag gctggtacag gccggggact cctcccatgc taagccagtg 24780
   tctttctggc cccagatcct caggggccag agggtcatcc ccagagcccg ctctgccacc 24840
   cacatgggta ccctgggcct gggagggatg tgccttccct caaccctgcc tggatgtccg 24900
   cacggggcca cctgcattgc tgaaactgca acgaagtcga gtctcaggag gggcccccct 24960
   ggctgcaggg ctcttgatcc ttttggccac gtgcacactg aggtggacgc tcggacccag 25020
   agaccccctt catgatgatg gccggggcag gaaccccctc ctctgaggaa ggaccctggt 25080
   gggggacagc actgcaggag ggcacaggag atgacggggg ctctagcagg gccgggagga 25140
   aggccaagat gctcctcgca accgtgtgcc tgtggccagg acagaggaca aacccaccct 25200
   ccactgtccc cactctcagg acagcagtcc tgccccagga ctcagcgccc acacttatgc 25260
   ctgaggacca ctattcaagt cagtatttgg cgagcagggg ttgctgccgc gggcgctgtg 25320
   acaggctgga atcctctccc tctccctctc cctctccgga gacatggagc ctacagggac 25380
   agagtcagca cctcagggta ggaccatggc tggcgtcatc agcatcactg gatctgatga 25440
   gtgggagccg gcatctcact gttttcactc tctcattcaa atgactggag caaagggaag 25500
   gtgtggggag aggcccagga atcaacacta aggtcaactt tgcccccagg ggcaggggtg 25560
   ggagtgaaca gccacaggtg tgatcctggg gagggcttct gggagagaat tcagaggcaa 25620
   gcatgtagag gaaccatttc aaatagttaa gaaaagccag agccaaacag ggacagttgg 25680
   ctcgcagaga tgatgcaggc aaagccagct cagatctgag catgggaaag actactccca 25740
   accaagggcc cagcatctcc caaccaagca ccaagtacct cccaaccaaa tgccaagcac 25800
   ctcccaatca aatacctccc aaccaagcac ctagcacctc tcaactggac accaactact 25860
   cccaaccagg caccaagtac ctcccaacca agtgccaagc acctcccaac caagtaccaa 25920
   ttacctccca accaagcgcc tagcacctcc caactgagca tcatgcacct cccaacagag 25980
   catctagcac ctcccaactg atcacctccc aacctagcac cgagcacctc ccaaccaagt 26040
   gcagagcacc tcccaaccaa gtgccaagca cctcccaatc aaatacctcc caaccaagca 26100
   cctagcacct ctcaactgga caccaacaac tcccaaccaa gcgccaagca cctcctaaca 26160
   aagtaccaat caccttccaa ccgagcacct agcacctccc aactgagcat catgcacctc 26220
   ccaacaaatc acctagcacc tcccgactga tcacctccca acctagcact gagcacttcc 26280
   caaccaacat agcaaaagcc ataaagaagt aaaaagacaa aaccacgtag gcatggagac 26340
   tggacttctg gtggcgagga aagggcattt ttattataac gacagctaac atttgttgaa 26400
   ctcacaaact gttcttggtg ttttcctcat gacatgcagc atggtcacgc ctctgtacag 26460
   acaaggatac tgaggcacag agtggcaccg tgccaacctt gtctcatctt tttatcgaac 26520
   ctacatgcag agtgccagca aatccagctg tcttttctct tcagaacaga tcccaaatct 26580
   cgccactcct tacccccaca agtgaggtgt ccccgctgct gctttctgtc gccaggatcc 26640
   cggtaataac cgtggagagg gctcctgccc ccacgccacc caccccacag ctcactctcg 26700
   ctccagccac caggggatgc cttccagcac gagtcagagc tggcacctcc tctgctcgag 26760
   acctcatgtg tcctctcctc acaccttggg ccctgtttcc ctacattctg ctacagcccc 26820
   tcaaacaggc cccgccccaa accagcccag ggcctttgca ctggctgatc cctctgcctg 26880
   gaccgcgctg cccccagaca gccacacggt tctcagcctc atctgcttcc agtctcgact 26940
   caaaagtcac caagaggcct tcccagcacc tgagctccga cggaagcccc tcgccacagc 27000
   acccaagcac tgctttatcc ccctacgcac acgtcccttt caaatactat tcatttacca 27060
   tctcctccca ctcactgaaa gggccagaga ctgggctata cccgctgcgt ggggagcagg 27120
   accaggcgca agggctcaca aatgcagtgg atgcctggtt gggaggtgag ggagctgcag 27180
   cgacccacgc tgggagggaa cgcaatgaca ggaggagcgc aggtcctggc gacacgatgg 27240
   ccatggcagc cgctggtgag caaccgcagg ccggccctgg gagagggctt ctagcaagct 27300
   gctatcttca gcctctccga ctactgcaga tgccccctcc tagccagaga cactgctaca 27360
   ccagccgacc cttccaaaaa gaaggtcagt aaccccgcga ctcctggagc cacagtgcag 27420
   ggggagaggg ctgagagggc aacagttcac caagcggaac agaggctgcc ccggaggtca 27480
   gctggctccc cggcagctgc aggggtggct agcccactcg gagggcagcg agggcatacg 27540
   aggggctcca gggatgagtg gttgcccagc acagcacccc tgggaggccg ggggcacttc 27600
   tcaggtagtg ggggcacgag gctgctctgg cctgacctca gggactcaaa atactttggc 27660
   gataaattcc accgtgtccc acccctgctg gtaccccata cttacacaca gactggttca 27720
   gatgcagaca ctctcgcgca catactcgct cacacgggca catacacgtg cacacacagt 27780
   cacatgcgca cactcataca cacacaaata tccactcaca cgcatgcatg cacacacacg 27840
   gacacacaca ggctcacacg tatgcacgca tatgcgtgca cacgcacaca cacacacaca 27900
   cgctcacatc ctcccactcc cacactcagt tgctcagaca cacacacgcc tggctctcac 27960
   acaaacctgt tgggctctga aaggctccag cccttcccat gctcgtcaga agccagtcaa 28020
   tggcttccta agtcaccaca cagatcaaag aggtgaactt ggccacatgg cactctgctt 28080
   cctgagctcc caaacaccag ccttggtgag gacagaccct caccccacac cctcattccc 28140
   actaccctgg gcaggcccag aggaggggca tctgcaggat ctggcaacca gcccctcccg 28200
   cccggctcct gcagccggca ccatgggagt cagggggagg tcactgcaaa gggcaacagc   28260
   aagttggtgg ccccaggact agagcccagg ggtcttcagt cctactccag agcttggaca 28320
   ctgtcccaca gggcatggcc aagggaaggg cttccagagc cctgacttca gggaggaggg 28380
   caggcgggct cctgtggcag gcctggatgc atggccgccc actcctggga ctttctaacc 28440
   tagaatatct aggtcaggct gggtgcagtg gctcacgcct gcaatcccaa cactttggga 28500
   ggccgaggag ggtggatcac ttgaggttag gagtttgaga ccagcctggc caacatggcg 28560
   aaaccctgtg tctactaaaa atacaaaacc tagccaggtg tggtagtgca cgcctgtaat 28620
   cacagctact caggaggctg aggcaggaga atcacttgaa ctcgggaggt ggaggttgca 28680
   gtgagctgag atcgtgccat tgcgcaaaga agatctaggc cggcccctca accggtgagg 28740
   tccaggctgg gagtgctgag agactgtggt gacactgaat gaactaacag gcaaagggct 28800
   tccaactgag cctgggggtg gtgggaaatg gctcttgtgt tctagtcaag acctctgcca 28860
   accagttctg acactgaccc agcacagaac ctgacaggtc agcaagggcc agggcttagc 28920
   acagcccagg taagggtgtg tgtacggccc ccagagtcac tcccaggctg caagaaaagg 28980
   gacaaaggag ggacaagggg tggccaagca aactgttccc tctgctcggg agtctggggt 29040
   gacctggcct agctggccag tggagctggg ccacctcccc ttaaactctc caccccggac 29100
   ttcgactcca aagctttcct gccacccacg ctctccccac ctgggatcac ggccaggccc 29160
   tgagccttca agggcccagg tgaactcagc cagactagga gctgaggagg acacagggca 29220
   gcttccagaa cggacccgag aaccactccc agcaggttct gcttccagac aaggagctgc 29280
   actttttcag ccaatgcaat tagaaagcca ggagaaggtg caaattccac ctgcctgagc 29340
   gtccgcactt cccaggccgc ccaccataca cacagcaaag atgtgtttaa ccattcaaac 29400
   ccatggccaa ccacatcggt tgcctcagac atgcaagttt taaaaaggaa cataactatg 29460
   ggccaggcac ggtggttcac gtctgtaatc ccagcacttt gggaggccga ggtgggtgga 29520
   tcacctgagg tcaggagttc gagaccagcc tagacaccat ggtgaaaccc catctgtacc 29580
   aaaactacaa aaattagctg ggcgtggtgg tgggcgcctg taatcccagc tacttgggaa 29640
   gctgaggcag gagaatcact tgaacccggg aggcgaaggt tgcagtgagc cgagattgtg 29700
   ccactgcact ccagcctggg caacaaggga gactccatct caattaaaaa aaaaaaaaaa 29760
   aaaaaggaac ataactatgg agtctcaagg ggaagtaatt ccttcaacaa taacaaatct 29820
   tgaaagctga gctctttttt ttttttgaga caggatctcc tcactttgtc gcccaggctg 29880
   gagtgcagtg gtgggatcac agctcactgc agcctcgatc tcccaggctc aaatgatcct 29940
   cctacctcag cctcccaaga agctgggatt acaggtgcat accatcacac ccgattcatt 30000
   tttgtatact ttgaagagat ggggtctcac catgttgccc agtgtggtct tgaattcctg 30060
   gactcaggtg atctgcccgc cttggcctcc cagagtgctg ggattacagg cctgagccaa 30120
   cacccccacg ggttcatttt cagagtcgca ccgagtgctg gggttacagg cctgagccaa 30180
   cccccccacg ggttcatttt aagagtgaca ccgagtgctg gggttacagg cctgaaccaa 30240
   cccccccacg agttcatttt cagagtcgca ccgagtgctg gggttacagg cctgagccaa 30300
   cccccccacg ggttcatttt aagagtgaca ccgagtgctg gggttacagg cctgagccaa 30360
   cacccccacg ggttcatttt cagagtcaca ccgagtgctg gggttacagg cctgagccaa 30420
   cccccccacg ggttcatttt cagagtcaca ccctttttct gaaaaacaac ttgggctcat 30480
   gcaaattcga gagagagatg gtgacactcc ccgccccctg gacccaggtg gagtcgcagc 30540
   agggtttacc cgtgagcggg gtccaaggcg atggccctcg gctggtcaag gtcctgccag 30600
   aagagcacct tccgggatgt gccattgagg ttggccacct cgatgcggtt ggtctctgag 30660
   tccgtccagt acagcttctt gcccacccag tcgcaggcga ggccgtcggg agagaccagg 30720
   ccggagatga ccacgttctg cacggcggcc cccgtctggt tcaggtaggt ctgcttgatg 30780
   gcctcctcgc tcacgtctgt ccagtacacg gctcccttgg aaaactggaa gtccactgcg 30840
   gccgcatcct ccaggccgct gaccacgatg gtggactcca gcttgactcc gccggcgtcc 30900
   accagccgta cgtcccggcg gttggcaaat agcaggagcg gcgaggctgt ggggcagaag 30960
   caaaccgtga gggccactgg ctaagccagc aagatacaca gccctgggat ggagcactat 31020
   gcccagagca ctcctggtac tgccctgccc atgcccaaga cctccagttc cttcctccca 31080
   cccctaaggc gttgtcagga agttgcctgg gcagccccgg cccgcatcat tcagaggctc 31140
   ctgcagcgca gcaaacagcc ttcttcccac attcggtgac agcacctgtt tgtttaccaa 31200
   ctgttacgtc tgttccccca gatatgggtg acccttcctg ccatgcccaa aacctcccac 31260
   atcgtcctcc agaggctaca ggggccctgt cctgttctgc agagaagcca catccccttt 31320
   gttggcctga cacaggggat ggggacatgc aggcacagca ctggccatgc tgctcgctac 31380
   agacccagcc acagggccac attttttgag gggttcagag cccaggccag acagagcctc 31440
   aagattccct tacaagtctt tgaccactgt ccaagctcag gcccgtttcc ttggccgtgg 31500
   catcagcttc ccatccaccc ctgtattcca tgtttctccc accctgcttc tggacattcc 31560
   tacatttaaa gggtcactct ggaatgccac cccttggctc agacaccttc cacagctccc 31620
   tgtgccagtg ccatgcagaa caaggtcaga ccccctagcc tggcctccaa ggccttggcc 31680
   tctggcctca cctacacttc tctccaccac cccaccccaa gcattcctga tctgcctgcg 31740
   gccaggctgg ctccctcacc tccctgtgca ccgcagccct cagccccttc tgcctgtgca 31800
   agaagcctca tctcacagac aacggtctca ttcccacaac gggctcaatg agaaatcagg 31860
   agaggccttc agaccatcac cccaccagac acctcagacg tcggaccagg agggtccagc 31920
   aacccccaac acagactcag agggactaag aagccacatg aggagtgaac acaagatgtg 31980
   gacaggagga ggttaagggc ctccagggag ctccatcagt ccgtgttctg ctgtcagcag 32040
   ggttaggctg ggctggccac aaacaccccc aaaaaacatc tgaagccttg gcttgaaaca 32100
   gctgacattc ctcatgaaaa ctgcagaccc ctgggtcctc ctgcgcagat gggggagccc 32160
   agccaacccc acactcccac cttcaccaag aaagagaaag ccaaaacaaa ctcaactcag 32220
   ccaatgacaa tcacagaact gaatcctgta gttagttcag ttggtttcat ttcagcaggg 32280
   gaaagatttg cagcctctat gagggtagct gggaacacaa agggccagag catggcccag 32340
   gagaccccag cgcagtgggg tagatggttc cgagcacagg cctccctgcc aagacaagca 32400
   ctggctcaaa tcctggcccc tcccattccc aggagacatg ctccacagga tgggaggaca 32460
   cacagaggac ctgaggccag gaaaatgaca gcggcgcctc cgccgcccca cccgtgctgt 32520
   catcatctta ggtctacagt tctttgtggc aacgagggac actgtgaaag tcaaacaaca 32580
   ggaaggcata ggccacaaat aaagacaaac gggacttcat gggaagctaa agattttgtg 32640
   catcaaaaga cactatcgag agagtaaaaa ggcaacccac agaatgagag aaaatatttc 32700
   caaatcatag atctactaag agattaatat ccatgaaata cagagaactc ctaaaactca 32760
   acaatgagaa aacaactaag ccaactcaaa aatgggcaaa caacttgaac agacatttct 32820
   ccaaagatga catataaatg gccaataaac acatcaaaac aggcttaata tatccctaat 32880
   catcagggaa atgcaaatca aaactacaat aagataccat cttgcaccaa ttaggacggc 32940
   tactatcaaa aaaacaaaat agcaagtgtt ggtgaggatc tggagcaact ggaacccttg 33000
   tgcaccactg gcaaaaatgt gaaatggtgc agctactatg gaaaacagca tggcagttcc 33060
   ccaaaaactt aaacacagaa ttaccatatg acccagcaat ttcgctttgg gttatatacc 33120
   caaaagaact gaaaacaggg acacaatcag atatgcatac accttggatc acagcagcat 33180
   ccttcccaac agctaaaaca tggaggcagc caggcatggt ggctcacgcc tgtaatccca 33240
   gcactttggg aggctgaggc gggtggatca cctgaggtca ggagttcgag accagcctgg 33300
   ccaacatggt gaaaccccgt ctctactaaa atacaaaaat tagctgggcg tagtgacggg 33360
   cacctgtaat cccagctact cacaagtctg aggcaggaga atcacttgaa ccctggaagt 33420
   ggacgttgca gtgagccaag attgcgccac tgcattccag cctgggtgac acagcgagac 33480
   tctgtctcaa aaaacagcaa aacaaaaaca aaaaaacaaa caaacatgga agcaacccaa 33540
   gcgtccctct actgagggat gaatagcggg gcaaaatctg ctccatccac acaatggagt 33600
   actattcagt ctcaaaaagg aaaaagattc tggtcaggca cggtggctca tgcctgtaat 33660
   cccagcactt ggggaggctg aggcgggtgg atcacctgaa gtcaggaatt caaggcccgc 33720
   ctggccaaga ctggcaccna gctacacana aagtatangg ccccggaaa 33769
<210> 9
   <211> 72049
   <212> DNA
   <213> Homo sapiens
<220>
   <221> unsure
   <222> (8356),(8385),(38585)
   <223 > Identity of nucleotide sequences at the above locations are unknown.
<400> 9
<210> 10
   <211> 8705
   <212> DNA
   <213> Homo sapiens
<400> 10
<210> 11
   <211> 66933
   <212> DNA
   <213> Homo sapiens
<400> 11
<210> 12
   <211> 21
   <212> DNA
   <213> Artificial Sequence
<220>
   <223 > Artificial sequence is a primer.
<400> 12
   ctgagcggaa ttcgtgagac c 21
<210> 13
   <211> 23
   <212> DNA
   <213> Artificial Sequence
<220>
   <223> Artificial sequence is a primer.
<400> 13
   ttggtctcac gtattccgct cga 23
<210> 14
   <211> 20
   <212> DNA
   <213> Artificial Sequence
<220>
   <223 > Artificial sequence is a primer.
<400> 14
   ctcgagaatt ctggatcctc 20
<210> 15
   <211>22
   <212> DNA
   <213> Artificial Sequence
<220>
   <223> Artificial sequence is a primer.
<400> 15
   ttgaggatcc agaattctcg ag 22
<210> 16
   <211> 21
   <212> DNA
   <213> Artificial Sequence
<220>
   <223> Artificial sequence is a primer.
<400> 16
   tgtatgcgaa ttcgctgcgc g 21
<210> 17
   <211> 23
   <212> DNA
   <213> Artificial Sequence
<220>
   <223> Artificial sequence is a primer.
<400> 17
   ttcgcgcagc gaattcgcat aca 23
<210> 18
   <211> 21
   <212> DNA
   <213> Artificial Sequence
<220>
   <223> Artificial sequence is a primer.
<400> 18
   gtccactgaa ttctcagtga g 21
<210> 19
   <211> 23
   <212> DNA
   <213> Artificial Sequence
<220>
   <223> Artificial sequence is a primer.
<400> 19
   ttgtcactga gaattcagtg gac 23
<210> 20
   <211> 21
   <212> DNA
   <213> Artificial Sequence
<220>
   <223 > Artificial sequence is a primer.
<400> 20
   gaatccgaat tcctggtcag c 21
<210> 21
   <211> 23
   <212> DNA
   <213> Artificial Sequence
<220>
   <223> Artificial sequence is a primer.
<400> 21
   ttgctgacca ggaattcgga ttc 23
<210> 22
   <211> 33
   <212> DNA
   <213> Artificial Sequence
<220>
   < 223 > Artificial sequence is a primer.
<400> 22
   cuacuacuac uactgagcgg aattcgtgag arc 33
<210> 23
   <211> 32
   <212> DNA
   <213> Artificial Sequence
<220>
   <223> Artificial sequence is a primer.
<400> 23
   cuacuacuac uactcgagaa ttctggatcc tc 32
<210> 24
   <211> 33
   <212> DNA
   < 213 > Artificial Sequence
<220>
   <223> Artificial sequence is a primer.
<400> 24
   cuacuacuac uatgtatgcg aattcgctgc gcg 33
<210> 25
   <211 > 33
   <212> DNA
   <213> Artificial Sequence
<220>
   <223 > Artificial sequence is a primer.
<400> 25
   cuacuacuac uagtccactg aattctcagt gag 33
<210> 26
   <211> 33
   <212> DNA
   <213> Artificial Sequence
<220>
   <223> Artificial sequence is a primer.
<400> 26
   cuacuacuac uagaatccga attcctggtc age 33
<210> 27
   <211> 45
   <212> DNA
   <213> Artificial Sequence
<220>
   <223> Artificial sequence is a primer.
<400> 27
   aactggaaga attcgcggcc gcaggaattt tttttttttt ttttt 45
<210> 28
   <211> 13
   <212> DNA
   <213> Artificial Sequence
<220>
   <223> Artificial sequence is a primer.
<400> 28
   aattcggcac gag 13
<210> 29
   <211> 9
   <212> DNA
   <213> Artificial Sequence
<220>
   <223> Artificial sequence is a primer.
<400> 29
   ctcgtgccg 9
<210> 30
   <211> 14
   <212> DNA
   <213> Artificial Sequence
<220>
   <223> Artificial sequence is a primer.
<400> 30
   gtacgacggc cagt 14
<210> 31
   <211> 16
   <212> DNA
   <213> Artificial Sequence
<220>
   <223 > Artificial sequence is a primer.
<400> 31
   aacagctatg accatg 16
<210> 32
   <211> 18
   <212> DNA
   <213> Artificial Sequence
<220>
   <223 > Artificial sequence is a primer.
<400> 32
   ccaagttctg agaagtcc 18
<210> 33
   <211> 20
   <212> DNA
   <213> Artificial Sequence
<220>
   < 223> Artificial sequence is a primer.
<400> 33
   aatacctgaa accatacctg 20
<210> 34
   <211> 57
   <212> DNA
   < 213 > Artificial Sequence
<220>
   <223> Artificial sequence is a primer.
<400> 34
   agctgctcgt agctgtctct ccctggatca cgggtacatg tactggacag actgggt 57
<210> 35
   <211> 56
   <212> DNA
   <213> Artificial Sequence
<220>
   <223> Artificial sequence is a primer.
<400> 35
   tgagacgccc ggattgagcg ggcagggata gcttattccc tgtgccgcat tacggc 56
<210> 36
   <211> 27
   <212> DNA
   <213> Artificial Sequence
<220>
   <223> Artificial sequence is a primer.
<400> 36
   agctgctcgt agctgtctct ccctgga 27
<210> 37
   <211> 27
   <212> DNA
   <213> Artificial Sequence
<220>
   <223> Artificial sequence is a primer.
<400> 37
   gccgtaatgc ggcacaggga ataagct 27
<210> 38
   <211> 20
   <212> DNA
   <213> Artificial Sequence
<220>
   <223> Artificial sequence is a primer.
<400> 38
   gagaggctat atccctgggc 20
<210> 39
   <211> 20
   <212> DNA
   <213> Artificial Sequence
<220>
   <223> Artificial sequence is a primer.
<400> 39
   acagcacgtg tttaaagggg 20
<210> 40
   <211> 163
   <212> DNA
   <213> Homo sapiens
<400> 40
   actaaagcgc cgccgccgcg ccatggagcc cgagtgagct cggcgcgggc ccgtccggcc 60
   gccggacaac atggaggcag ctccgcccgg gccgccgtgg ccgctgctgc tgctgctgct 120
   gctgctgctg gcgctgtgcg gctgcccggc ccccgccgcg gcc 163
<210> 41
   <211> 419
   <212> DNA
   <213> Homo sapiens
<400> 41
   gccccacagc ctcgccgctc ctgctatttg ccaaccgccg ggacgtacgg ctggtggacg 60
   ccggcggagt caagctggag tccaccatcg tggtcagcgg cctggaggat gcggccgcag 120
   tggacttcca gttttccaag ggagccgtgt actggacaga cgtgagcgag gaggccatca 180
   agcagaccta cctgaaccag acgggggccg ccgtgcagaa cgtggtcatc tccggcctgg 240
   tctctcccga cggcctcgcc tgcgactggg tgggcaagaa gctgtactgg acggactcag 300
   agaccaaccg catcgaggtg gccaacctca atggcacatc ccggaaggtg ctcttctggc 360
   aggaccttga ccagccgagg gccatcgcct tggaccccgc tcacgggtaa accctgctg 419
<210> 42
   <211> 221
   <212> DNA
   <213> Homo sapiens
<400> 42
   ccccgtcaca ggtacatgta ctggacagac tggggtgaga cgccccggat tgagcgggca 60
   gggatggatg gcagcacccg gaagatcatt gtggactcgg acatttactg gcccaatgga 120
   ctgaccatcg acctggagga gcagaagctc tactgggctg acgccaagct cagcttcatc 180
   caccgtgcca acctggacgg ctcgttccgg taggtaccca c 221
<210> 43
   <211> 221
   <212> DNA
   <213> Homo sapiens
<400> 43
   tccctgactg caggcagaag gtggtggagg gcagcctgac gcaccccttc gccctgacgc    60
   tctccgggga cactctgtac tggacagact ggcagacccg ctccatccat gcctgcaaca 120
   agcgcactgg ggggaagagg aaggagatcc tgagtgccct atactcaccc atggacatcc 180
   aggtgctgag ccaggagcgg cagccttttt gtgagtgccg g 221
<210> 44
   <211> 156
   <212> DNA
   <213> Homo sapiens
<400> 44
   tttctcagtc cacactcgct gtgaggagga caatggcggc tggtcccacc tgtgcctgct 60
   gtccccaagc gagccttttt acacatgcgc ctgccccacg ggtgtgcaga tgcaggacaa 120
   cggcaggacg tgtaaggcag gtgaggcggt gggacg 156
<210> 45
   <211> 416
   <212> DNA
   <213> Homo sapiens
<400> 45
   ctccacagga gccgaggagg tgctgctgct ggcccggcgg acggacctac ggaggatctc 60
   gctggacacg ccggacttca ccgacatcgt gctgcaggtg gacgacatcc ggcacgccat 120
   tgccatcgac tacgacccgc tagagggcta tgtctactgg acagatgacg aggtgcgggc 180
   catccgcagg gcgtacctgg acgggtctgg ggcgcagacg ctggtcaaca ccgagatcaa 240
   cgaccccgat ggcatcgcgg tcgactgggt ggcccgaaac ctctactgga ccgacacggg 300
   cacggaccgc atcgaggtga cgcgcctcaa cggcacctcc cgcaagatcc tggtgtcgga 360
   ggacctggac gagccccgag ccatcgcact gcaccccgtg atggggtaag acgggc 416
<210> 46
   <211> 198
   <212> DNA
   <213 > Homo sapiens
<400> 46
   ttcttctcca gcctcatgta ctggacagac tggggagaga accctaaaat cgagtgtgcc 60
   aacttggatg ggcaggagcg gcgtgtgctg gtcaatgcct ccctcgggtg gcccaacggc 120
   ctggccctgg acctgcagga ggggaagctc tactggggag acgccaagac agacaagatc 180
   gaggtgaggc tcctgtgg 198
<210> 47
   <211> 244
   <212> DNA
   < 213 > Homo sapiens
<400> 47
   ccgtcctgca ggtgatcaat gttgatggga cgaagaggcg gaccctcctg gaggacaagc 60
   tcccgcacat tttcgggttc acgctgctgg gggacttcat ctactggact gactggcagc 120
   gccgcagcat cgagcgggtg cacaaggtca aggccagccg ggacgtcatc attgaccagc 180
   tgcccgacct gatggggctc aaagctgtga atgtggccaa ggtcgtcggt gagtccgggg 240
   ggtc 244
<210> 48
   <211> 313
   <212> DNA
   <213> Homo sapiens
<400> 48
   gttcgcttcc aggaaccaac ccgtgtgcgg acaggaacgg ggggtgcagc cacctgtgct 60
   tctgcacacc ccacgcaacc cggtgtggct gccccatcgg cctggagctg ctgagtgaca 120
   tgaagacctg catcgtgcct gaggcctttt tggtcttcac cagcagagcc gccatccaca 180
   ggatctccct cgagaccaat aacaacgacg tggccatccc gctcacgggc gtcaaggagg 240
   cctcagccct ggactttgat gtgtccaaca accacatcta ctggacagac gtcagcctga 300
   aggtagcgtg ggc 313
<210> 49
   <211> 255
   <212> DNA
   <213> Homo sapiens
<400> 49
   cctgctgcca gaccatcagc cgcgccttca tgaacgggag ctcggtggag cacgtggtgg 60
   agtttggcct tgactacccc gagggcatgg ccgttgactg gatgggcaag aacctctact 120
   gggccgacac tgggaccaac agaatcgaag tggcgcggct ggacgggcag ttccggcaag 180
   tcctcgtgtg gagggacttg gacaacccga ggtcgctggc cctggatccc accaaggggt 240
   aagtgtttgc ctgtc 255
<210> 50
   <211> 210
   <212> DNA
   <213> Homo sapiens
<400> 50
   gtgccttcca gctacatcta ctggaccgag tggggcggca agccgaggat cgtgcgggcc 60
   ttcatggacg ggaccaactg catgacgctg gtggacaagg tgggccgggc caacgacctc 120
   accattgact acgctgacca gcgcctctac tggaccgacc tggacaccaa catgatcgag 180
   tcgtccaaca tgctgggtga gggccgggct 210
<210> 51
   <211> 352
   <212> DNA
   <213> Homo sapiens
<400> 51
   gtgttcatgc aggtcaggag cgggtcgtga ttgccgacga tctcccgcac ccgttcggtc 60
   tgacgcagta cagcgattat atctactgga cagactggaa tctgcacagc attgagcggg 120
   ccgacaagac tagcggccgg aaccgcaccc tcatccaggg ccacctggac ttcgtgatgg 180
   acatcctggt gttccactcc tcccgccagg atggcctcaa tgactgtatg cacaacaacg 240
   ggcagtgtgg gcagctgtgc cttgccatcc ccggcggcca ccgctgcggc tgcgcctcac 300
   actacaccct ggaccccagc agccgcaact gcagccgtaa gtgcctcatg gt 352
<210> 52
   <211> 225
   <212> DNA
   <213> Homo sapiens
<400> 52
   gcctcctcta cgcccaccac cttcttgctg ttcagccaga aatctgccat cagtcggatg 60
   atcccggacg accagcacag cccggatctc atcctgcccc tgcatggact gaggaacgtc 120
   aaagccatcg actatgaccc actggacaag ttcatctact gggtggatgg gcgccagaac 180
   atcaagcgag ccaaggacga cgggacccag gcaggtgccc tgtgg 225
<210> 53
   <211> 235
   <212> DNA
   <213> Homo sapiens
<400> 53
   ctttgtctta cagccctttg ttttgacctc tctgagccaa ggccaaaacc cagacaggca 60
   gccccacgac ctcagcatcg acatctacag ccggacactg ttctggacgt gcgaggccac 120
   caataccatc aacgtccaca ggctgagcgg ggaagccatg ggggtggtgc tgcgtgggga 180
   ccgcgacaag cccagggcca tcgtcgtcaa cgcggagcga gggtaggagg ccaac 235
<210> 54
   <211> 218
   <212> DNA
   <213> Homo sapiens
<400> 54
   ccaccctccc gcaggtacct gtacttcacc aacatgcagg accgggcagc caagatcgaa 60
   cgcgcagccc tggacggcac cgagcgcgag gtcctcttca ccaccggcct catccgccct 120
   gtggccctgg tggtggacaa cacactgggc aagctgttct gggtggacgc ggacctgaag 180
   cgcattgaga gctgtgacct gtcaggtacg cgccccgg 218
<210> 55
   <211> 234
   <212> DNA
   <213> Homo sapiens
<400> 55
   ggctgcttgc aggggccaac cgcctgaccc tggaggacgc caacatcgtg cagcctctgg 60
   gcctgaccat ccttggcaag catctctact ggatcgaccg ccagcagcag atgatcgagc 120
   gtgtggagaa gaccaccggg gacaagcgga ctcgcatcca gggccgtgtc gcccacctca 180
   ctggcatcca tgcagtggag gaagtcagcc tggaggagtt ctgtacgtgg gggc 234
<210> 56
   <211> 157
   <212> DNA
   <213> Homo sapiens
<400> 56
   ttgtctttgc agcagcccac ccatgtgccc gtgacaatgg tggctgctcc cacatctgta 60
   ttgccaaggg tgatgggaca ccacggtgct catgcccagt ccacctcgtg ctcctgcaga 120
   acctgctgac ctgtggaggt aggtgtgacc taggtgc 157
<210> 57
   <211> 272
   <212> DNA
   <213> Homo sapiens
<400> 57
   gttctcctct gtccctcccc cagagccgcc cacctgctcc ccggaccagt ttgcatgtgc 60
   cacaggggag atcgactgta tccccggggc ctggcgctgt gacggctttc ccgagtgcga 120
   tgaccagagc gacgaggagg gctgccccgt gtgctccgcc gcccagttcc cctgcgcgcg 180
   gggtcagtgt gtggacctgc gcctgcgctg cgacggcgag gcagactgtc aggaccgctc 240
   agacgaggtg gactgtgacg gtgaggccct cc 272
<210> 58
   <211> 134
   <212> DNA
   < 213 > Homo sapiens
<400> 58
   tctccttgca gccatctgcc tgcccaacca gttccggtgt gcgagcggcc agtgtgtcct 60
   catcaaacag cagtgcgact ccttccccga ctgtatcgac ggctccgacg agctcatgtg 120
   tggtgagcca gctt 134
<210> 59
   <211> 274
   <212> DNA
   <213> Homo sapiens
<400> 59
   gtttgtctct ggcagaaatc accaagccgc cctcagacga cagcccggcc cacagcagtg 60
   ccatcgggcc cgtcattggc atcatcctct ctctcttcgt catgggtggt gtctattttg 120
   tgtgccagcg cgtggtgtgc cagcgctatg cgggggccaa cgggcccttc ccgcacgagt 180
   atgtcagcgg gaccccgcac gtgcccctca atttcatagc cccgggcggt tcccagcatg 240
   gccccttcac aggtaaggag cctgagatat ggaa 274
<210> 60
   <211> 164
   <212> DNA
   <213> Homo sapiens
<400> 60
   cttccctgcc aggcatcgca tgcggaaagt ccatgatgag ctccgtgagc ctgatggggg 60
   gccggggcgg ggtgcccctc tacgaccgga accacgtcac aggggcctcg tccagcagct 120
   cgtccagcac gaaggccacg ctgtacccgc cggtgagggg cggg 164
<210> 61
   <211> 130
   <212> DNA
   <213> Homo sapiens
<400> 61
   ttggctctcc tcagatcctg aacccgccgc cctccccggc cacggacccc tccctgtaca 60
   acatggacat gttctactct tcaaacattc cggccactgc gagaccgtac aggtaggaca 120
   tcccctgcag 130
<210> 62
   <211> 496
   <212> DNA
   <213> Homo sapiens
<400> 62
   tcaaacattc cggccactgc gagaccgtac aggccctaca tcattcgagg aatggcgccc 60
   ccgacgacgc cctgcagcac cgacgtgtgt gacagcgact acagcgccag ccgctggaag 120
   gccagcaagt actacctgga tttgaactcg gactcagacc cctatccacc cccacccacg 180
   ccccacagcc agtacctgtc ggcggaggac agctgcccgc cctcgcccgc caccgagagg 240
   agctacttcc atctcttccc gccccctccg tccccctgca cggactcatc ctgacctcgg 300
   ccgggccact ctggcttctc tgtgcccctg taaatagttt taaatatgaa caaagaaaaa 360
   aatatatttt atgatttaaa aaataaatat aattgggatt ttaaaaacat gagaaatgtg 420
   aactgtgatg gggtgggcag ggctgggaga actttgtaca gtggagaaat atttataaac 480
   ttaattttgt aaaaca 496

## Description

### FIELD OF THE INVENTION

The present invention relates generally to the field of genetics, genomics and molecular biology. The present disclosure relates to methods and materials used to isolate, detect and sequence a high bone mass gene and corresponding wild-type gene, and mutants thereof that may be involved with modulating lipid levels. The present disclosure also relates to the high bone mass gene, the corresponding wild-type gene, and mutants thereof. The genes identified in the present disclosure are implicated in the ontology and physiology of atherosclerosis, arteriosclerosis and associated diseases and conditions related thereto. Also disclosed are nucleic acids, proteins, cloning vectors, expression vectors, transformed hosts, methods of developing pharmaceutical compositions, methods of identifying molecules involved in arteriosclerosis and associated conditions, and methods of treating or preventing diseases associated with abnormal lipid levels. The present disclosure is directed to methods for treating, diagnosing, preventing and screening for normal and abnormal lipid-associated conditions, including arteriosclerosis, cardiovascular disease and stroke.

### BACKGROUND OF THE INVENTION

Cardiovascular disease is the number one killer in the United States, and atherosclerosis is the major cause of heart disease and stroke. It is widely appreciated that cholesterol plays an important role in atherogenesis. Normally, most cholesterol serves as a structural element in the walls of cells, whereas much of the rest is in transit through the blood or functions as the starting material for the synthesis of bile acids in the liver, steroid hormones in endocrine cells and vitamin D in skin. The transport of cholesterol and other lipids through the circulatory system is facilitated by their packaging into lipoprotein carriers. These spherical particles comprise protein and phospholipid shells surrounding a core of neutral lipid, including unesterified ("free") or esterified cholesterol and triglycerides. Risk for atherosclerosis increases with increasing concentrations of low density lipoprotein (LDL) cholesterol, whereas risk is inversely proportional to the levels of high density lipoprotein (HDL) cholesterol. The receptor-mediated control of plasma LDL levels has been well-defined, and recent studies have now provided new insights into HDL metabolism.

The elucidation of LDL metabolism began in 1974 by Michael Brown and Joseph Goldstein. In brief, the liver synthesizes a precursor lipoprotein (very low density lipoprotein, VLDL) that is converted during circulation to intermediate density lipoprotein (IDL) and then to LDL. The majority of the LDL receptors expressed in the body are on the surfaces of liver cells, although virtually all other tissues ("peripheral tissues") express some LDL receptors. After binding, the receptor-lipoprotein complex is internalized by the cells via coated pits and vesicles, and the entire LDL particle is delivered to lysosomes, wherein it is disassembled by enzymatic hydrolysis, releasing cholesterol for subsequent cellular metabolism. This whole-particle uptake pathway is called "receptor-mediated endocytosis." Cholesterol-mediated feedback regulation of both the levels of LDL receptors and cellular cholesterol biosynthesis help ensure cellular cholesterol homeostasis. Genetic defects in the LDL receptor in humans results in familial hypercholesterolemia, a disease characterized by elevated plasma LDL cholesterol and premature atherosclerosis and heart attacks. One hypothesis for the deleterious effects of excess plasma LDL cholesterol is that LDL enters the artery wall, is chemically modified, and then is recognized by a special class of receptors called macrophage scavenger receptors, that mediate the cellular accumulation of the LDL cholesterol in the artery, eventually leading to the formation of an atherosclerotic lesion.

The major lipoprotein classes include intestinally derived chylomicrons that transport dietary fats and cholesterol, hepatic-derived VLDL, IDL and LDL that can be atherogenic, and hepatic- and intestinally-derived HDL that are antiatherogenic. Apoprotein B (ApoB) is necessary for the secretion of chylomicrons (Apo B48) and VLDL, IDL and LDL (Apo B100). Plasma levels of VLDL triglycerides are determined mainly by rates of secretion in LPL lipolytic activity. Plasma levels of LDL cholesterol are determined mainly by the secretion of Apo B100 into plasma, the efficacy with which VLDL are converted to LDL and by LDL receptor-mediated clearance. Regulation of HDL cholesterol levels is complex and is affected by rates of synthesis of its Apo proteins, rates of esterfication of free cholesterol to cholesterol ester by LCAT, levels of triglyceride-rich lipoproteins and CETP-mediated transfer of cholesterol esters from HDL, and clearance from plasma of HDL lipids and Apo proteins.

Normal lipoprotein transport is associated with low levels of triglycerides and LDL cholesterol and high levels of HDL cholesterol. When lipoprotein transport is abnormal, lipoprotein levels can change in ways that predispose individuals to atherosclerosis and arteriosclerosis (see Ginsberg, Endocrinol. Metab. Clin. North Am., 27: 503-19 (1998)).

Several lipoprotein receptors may be involved in cellular lipid uptake. These receptors include: scavenger receptors; LDL receptor-related protein/α2-macroglobulin receptor (LRP); LDL receptor; and VLDL receptor. With the exception of the LDL receptor, all of these receptors are expressed in atherosclerotic lesions while scavenger receptors are mostly expressed in macrophages, the LRP and VLDL receptors may play an important role in mediating lipid uptake in smooth muscle cells (Hiltunen et al., Atherosclerosis, 137 suppl.: S81-8 (1998)).

A major breakthrough in the pharmacologic treatment of hypercholesterolemia has been the development of the "statin" class of 3-hydroxy-3-methylglutaryl-CoA reductase (HMG CoA reductase) inhibitory drugs. 3-Hydroxy-3-methylglutaryl-CoA reductase is the rate controlling enzyme in cholesterol biosynthesis, and its inhibition in the liver stimulates LDL receptor expression. As a consequence, both plasma LDL cholesterol levels and the risk for atherosclerosis decrease. The discovery and analysis of the LDL receptor system has had a profound impact on cell biology, physiology, and medicine.

HDL is thought to remove unesterified, or "free" cholesterol (FC) from peripheral tissues, after which most of the cholesterol is converted to cholesteryl ester (CE) by enzymes in the plasma. Subsequently, HDL cholesterol is efficiently delivered directly to the liver and steroidogenic tissues via a selective uptake pathway and the HDL receptor, SR-BI (class B type I scavenger receptor) or, in some species, transferred to other lypoproteins for additional transport in metabolism. For additional discussion on HDL and LDL metabolism see Krieger, Proc. Natl. Acad Sci. USA, 95:4077-4080, 1998.

PCT/GB98/01102 (WO-A-98/46743) discloses the LDL-receptor related protein-3 (LRP-3) gene. The gene is associated with type I diabetes (insulin dependent diabetes mellitus) and experimental evidence suggests that it is the IDDM susceptibility gene *IDDM4.*

Recently, a strong interest in the genetic control of peak bone mass has developed in the field of osteoporosis. The interest has focused mainly on candidate genes with suitable polymorphisms to test for association with variation in bone mass within the normal range, or has focused on examination of genes and gene loci associated with low bone mass in the range found in patients with osteoporosis. The vitamin D receptor locus (VDR) (Morrison et al., Nature, 367:284-287 (1994)), PTH gene (Howard et al., J Clin. Endocrinol. Metab., 80:2800-2805 (1995); Johnson et al., J. Bone Miner. Res., 8:11-17 (1995); Gong et al., J. Bone Miner. Res., 10:S462 (1995)) and the estrogen receptor gene (Hosoi et al., J. Bone Miner. Res., 10:S170 (1995); Morrison et al., Nature, 367:284-287 (1994)) have figured most prominently in this work. These studies are difficult because bone mass (the phenotype) is a continuous, quantitative, polygenic trait, and is confounded by environmental factors such as nutrition, co-morbid disease, age, physical activity, and other factors. Also, this type of study design requires large numbers of subjects. In particular, the results of VDR studies to date have been confusing and contradictory (Garnero et al., J. Bone Miner. Res., 10:1283-1288 (1995); Eisman et al., J. Bone. Miner. Res., 10:1289-1293 (1995); Peacock, J. Bone Miner. Res., 10:1294-1297 (1995)). Furthermore, the work thus far has not shed much light on the mechanism(s) whereby the genetic influences might exert their effect on bone mass.

While it is well known that peak bone mass is largely determined by genetic rather than environmental factors, studies to determine the gene loci (and ultimately the genes) linked to variation in bone mass are difficult and expensive. Study designs which utilize the power of linkage analysis, e.g., sib-pair or extended family, are generally more informative than simple association studies, although the latter do have value. However, genetic linkage studies involving bone mass are hampered by two major problems. The first problem is the phenotype, as discussed briefly above. Bone mass is a continuous, quantitative trait, and establishing a discrete phenotype is difficult. Each anatomical site for measurement may be influenced by several genes, many of which may be different from site to site. The second problem is the age component of the phenotype. By the time an individual can be identified as having low bone mass, there is a high probability that their parents or other members of prior generations will be deceased and therefore unavailable for study, and younger generations may not have even reached peak bone mass, making their phenotyping uncertain for genetic analysis.

Regardless, linkage analysis can be used to find the location of a gene causing a hereditary "disorder" and does not require any knowledge of the biochemical nature of the disorder, i.e., a mutated protein that is believed to cause the disorder does not need to be known. Traditional approaches depend on assumptions concerning the disease process that might implicate a known protein as a candidate to be evaluated. The genetic localization approach using linkage analysis can be used to first find the general chromosomal region in which the defective gene is located and then to gradually reduce the size of the region in order to determine the location of the specific mutated gene as precisely as possible. After the gene itself is discovered within the candidate region, the messenger RNA and the protein are identified and, along with the DNA, are checked for mutations.

The genetic localization approach has practical implications since the location of the disease can be used for prenatal diagnosis even before the altered gene that causes the disease is found. Linkage analysis can enable families, even many of those that do not have a sick child, to know whether they are carriers of a disease gene and to evaluate the condition of an unborn child through molecular diagnosis. The transmission of a disease within families, then, can be used to find the defective gene. As used herein, reference to "high bone mass" (HBM) is analogous to reference to a disease state, although from a practical standpoint high bone mass can actually help a subject avoid the disease known as osteoporosis.

Linkage analysis is possible because of the nature of inheritance of chromosomes from parents to offspring. During meiosis, the two parental homologues pair to guide their proper separation to daughter cells. While they are lined up and paired, the two homologues exchange pieces of the chromosomes, in an event called "crossing over" or "recombination." The resulting chromosomes are chimeric, that is, they contain parts that originate from both parental homologues. The closer together two sequences are on the chromosome, the less likely that a recombination event will occur between them, and the more closely linked they are. In a linkage analysis experiment, two positions on the chromosomes are followed from one generation to the next to determine the frequency of recombination between them. In a study of an inherited disease, one of the chromosomal positions is marked by the disease gene or its normal counterpart, i.e., the inheritance of the chromosomal region can be determined by examining whether the individual displays symptoms of the disorder or not. The other position is marked by a DNA sequence that shows natural variation in the population such that the two homologues can be distinguished based on the copy of the "marker" sequence that they possess. In every family, the inheritance of the genetic marker sequence is compared to the inheritance of the disease state. If, within a family carrying an autosomal dominant disorder such as high bone mass, every affected individual carries the same form of the marker and all the unaffected individuals carry at least one different form of the marker, there is a great probability that the disease gene and the marker are located close to each other. In this way, chromosomes may be systematically checked with known markers and compared to the disease state. The data obtained from the different families is combined, and analyzed together by a computer using statistical-methods. The result is information indicating the probability of linkage between the genetic marker and the disease allowing different distances between them. A positive result can mean that the disease is very close to the marker, while a negative result indicates that it is far away on that chromosome, or on an entirely different chromosome.

Linkage analysis is performed by typing all members of the affected family at a given marker locus and evaluating the co-inheritance of a particular disease state with the marker probe, thereby determining how often the two of them are co-inherited. The recombination frequency can be used as a measure of the genetic distance between two gene loci. A recombination frequency of 1% is equivalent to 1 map unit, or 1 centiMorgan (cM), which is roughly equivalent to 1,000 kb of DNA. This relationship holds up to frequencies of about 20% or 20 cM.

The entire human genome is 3,300 cM long. In order to find an unknown disease gene within 5-10 cM of a marker locus, the whole human genome can be searched with roughly 330 informative marker loci spaced at approximately 10 cM intervals (Botstein et al., Am. J. Hum. Genet., 32:314-331 (1980)). The reliability of linkage results is established by using a number of statistical methods. The method most commonly used for the analysis of linkage in humans is the LOD score method (Morton, Prog, Clin. Biol. Res., 147:245-265 (1984), Morton et al., Am. J. Hum. Genet., 38:868-883 (1986)) which was incorporated into the computer program LIPED by Ott, Am. J. Hum. Genet., 28:528-529 (1976). LOD scores are the logarithm of the ratio of the likelihood that two loci are linked at a given distance to that they are not linked (>50 cM apart). The advantage of using logarithmic values is that they can be summed among families with the same disease. This becomes necessary given the relatively small size of human families.

By convention, a total LOD score greater than + 3.0 (that is, odds of linkage at the specified recombination frequency being 1000 times greater than odds of no linkage) is considered to be significant evidence for linkage at that particular recombination frequency. A total LOD score of less than - 2.0 (that is, odds of no linkage being 100 times greater than odds of linkage at the specified frequency) is considered to be strong evidence that the two loci under consideration are not linked at that particular recombination frequency. Until recently, most linkage analyses have been performed on the basis of two-point data, which is the relationship between the disorder under consideration and a particular genetic marker. However, as a result of the rapid advances in mapping the human genome over the last few years, and concomitant improvements in computer methodology, it has become feasible to carry out linkage analyses using multi-point data. Multi-point analysis provide a simultaneous analysis of linkage between the disease and several linked genetic markers, when the recombination distance among the markers is known.

Multi-point analysis is advantageous for two reasons. First, the informativeness of the pedigree is usually increased. Each pedigree has a certain amount of potential information, dependent on the number of parents heterozygous for the marker loci and the number of affected individuals in the family. However, few markers are sufficiently polymorphic as to be informative in all those individuals. If multiple markers are considered simultaneously, then the probability of an individual being heterozygous for at least one of the markers is greatly increased. Second, an indication of the position of the disease gene among the markers may be determined. This allows identification of flanking markers, and thus eventually allows isolation of a small region in which the disease gene resides. Lathrop et al., Proc. Natl. Acad. Sci. USA, 81:3443-3446 (1984) have written the most widely used computer package, LINKAGE, for multi-point analysis.

There is a need in the art for identifying the gene associated with a high bone mass phenotype. The present invention is directed to this, as well as other, important ends.

### SUMMARY OF THE INVENTION

The present invention is based on the identification of the *Zmax1* gene and the *HBM* gene on chromosome 11q13.3 by genetic linkage and mutation analysis. The use of additional genetic markers linked to the genes has aided this discovery. By using linkage analysis and mutation analysis, persons predisposed to lipid associated disorders may be readily identified. Cloning methods using Bacterial Artificial Chromosomes have enabled the inventors to focus on the chromosome region of 11q13.3 and to accelerate the sequencing of the autosomal dominant gene. The *Zmax1* gene and the *HBM* gene have also been identified, as has a guanine-to-thymine polymorphism mutation at position 582 in the *Zmax1* gene that produces the *HBM* gene and the HBM phenotype as well as altered lipid levels.

The *Zmax1* gene and the *HBM* gene described herein can be used to determine if people are predisposed to abnormal lipid levels and, therefore, susceptible to diseases mediated by lipids, including, for example, atherosclerosis, arteriosclerosis and associated conditions. Individuals with the *HBM* gene have lower LDL, triglyceride and VLDL levels and higher HDL levels. In other words, the *HBM* gene is a suppressor of atherosclerosis, arteriosclerosis and associated conditions. This *in vivo* observation is strong evidence that treatment of normal individuals with the *HBM* gene or protein, or fragments thereof, will ameliorate atherosclerosis, arteriosclerosis and conditions related thereto.

Moreover, such treatment will be indicated in the treatment of lipid-mediated diseases, particularly arteriosclerosis and conditions related thereto. For example, persons predisposed to elevated lipid levels (i.e., diabetes, hypercholesteremia and other genetic diseases, obesity, male gender, and individuals who smoke) may be identified and/or treated. Moreover, such treatments will be of use in the treatment or prevention of diabetic atherosclerotic disease, neurovascular conditions caused by plaque build-up (e.g., stroke), cardiovascular disease, poor circulation due to plaque build-up and associated poor wound healing.

Additionally, the Zmax1 gene and the HBM gene described herein may be used in, for example, gene therapy, pharmaceutical development, and diagnostic assays for bone development disorders. In preferred embodiments, the present invention is directed to methods for treating, diagnosing, preventing and screening for osteoporosis.

According to the invention, there is thus provided a method for identifying a molecule involved in lipid regulation comprising:
(A) constructing a first host that contains a gene having the sequence set out in SEQ ID NO.: 1 or a polypeptide encoded thereby;
(B) constructing a second host that contains a gene having the sequence set out in SEQ ID NO: 2 or a polypeptide encoded thereby;
(C) analyzing a difference between the first host and the second host; and
(D) identifying a molecule that, when added to the first host, causes the first host to exhibit lipid levels characteristic of the second host.

The invention also provides:
- a method for identifying a patient who has an increased disposition to arteriosclerosis, an arteriosclerosis associated condition, hypercholesteremia, hypolipidemia or atherosclerosis, which method comprises screening a sample from the patient to determine the presence or absence of a polypeptide having the sequence set out in SEQ ID NO: 3 or a nucleic acid encoding a said polypeptide in the said sample, thereby to determine if the patient has a genetic predisposition to a said condition; and
- a method of expressing the polypeptide having,the sequence of SEQ ID NO: 3 in tissue comprising constructing an expression vector comprising a promoter that directs expression in tissue operably linked to SEQ ID NO:1 and the tissue in which the said polypeptide is expressed is a lipid regulating cell or a cell involved in lipid metabolism.

### BRIEF DESCRIPTION OF THE FIGURES

**Figs**. **1A** and **1B** show the pedigree of the individuals used in the genetic linkage studies. Under each individual is an ID number, the z-score for spinal BMD, and the allele calls for the critical markers on chromosome 11. Solid symbols represent "affected" individuals. Symbols containing "N" are "unaffected" individuals. DNA from 37 individuals was genotyped. Question marks denote unknown genotypes or individuals who were not genotyped.

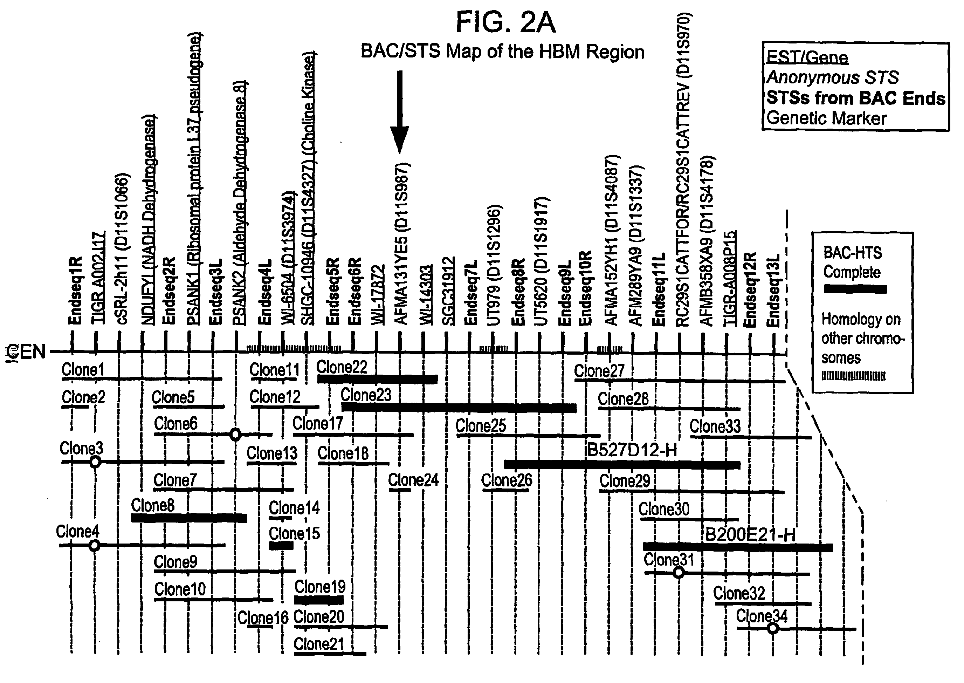

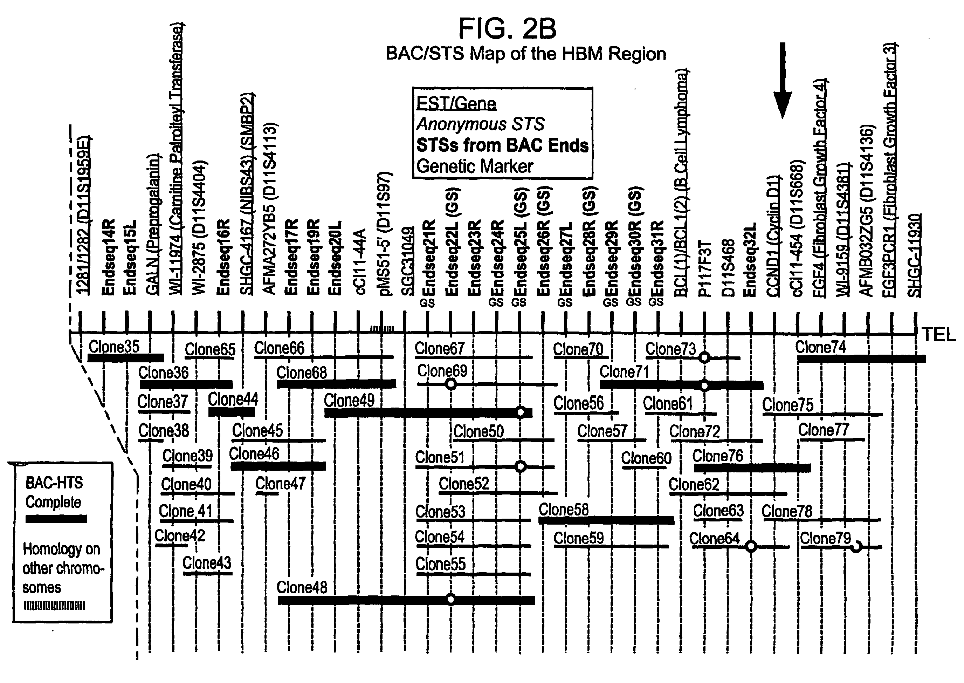

**Figs. 2A** and **2** depict the BAC/STS content physical map of the HBM region in 11q13.3. STS markers derived from genes, ESTs, microsatellites, random sequences, and BAC endsequences are denoted above the long horizontal line. For markers that are present in GDB the same nomenclature has been used. Locus names (D11S####) are listed in parentheses after the primary name if available. STSs derived from BAC endsequences are listed with the BAC name first followed by L or R for the left and right end of the clone, respectively. The two large arrows indicate the genetic markers that define the HBM critical region. The horizontal lines below the STSs indicate BAC clones identified by PCR-based screening of a nine-fold coverage BAC library. Open circles indicate that the marker did not amplify the corresponding BAC library address during library screening. Clone names use the following convention: B for BAC, the plate, row and column address, followed by -H indicating the HBM project (i.e., B36F16-H).

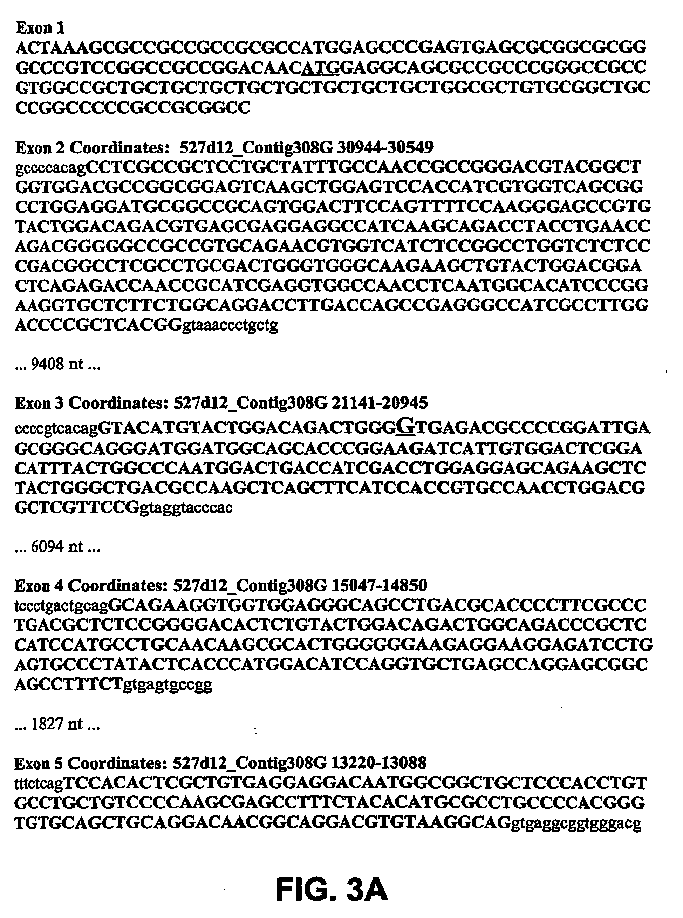

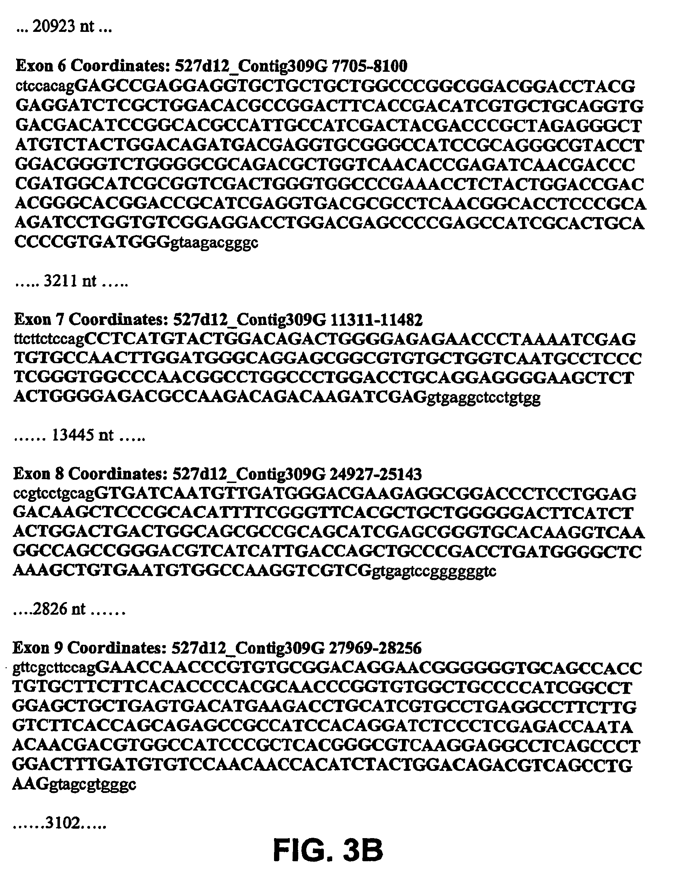

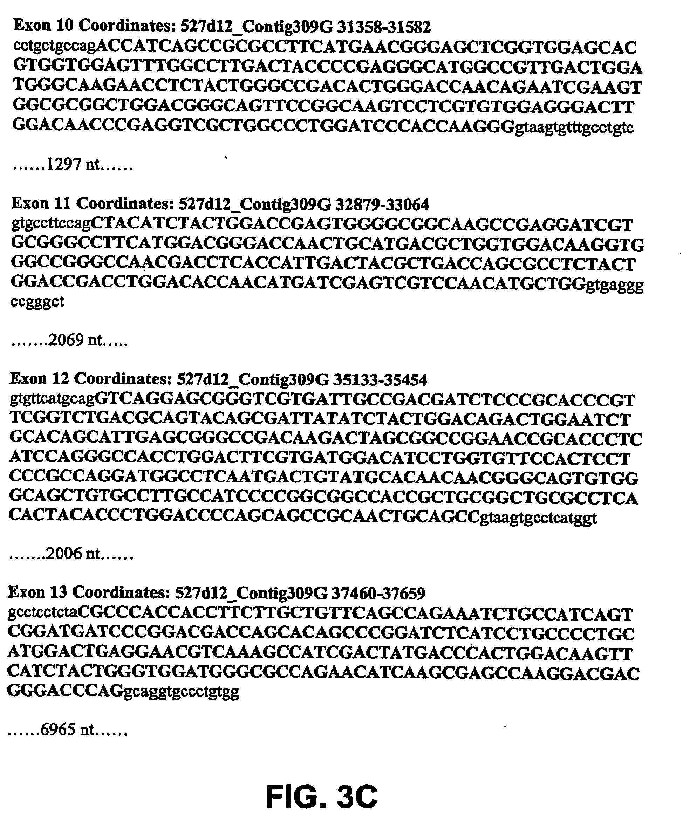

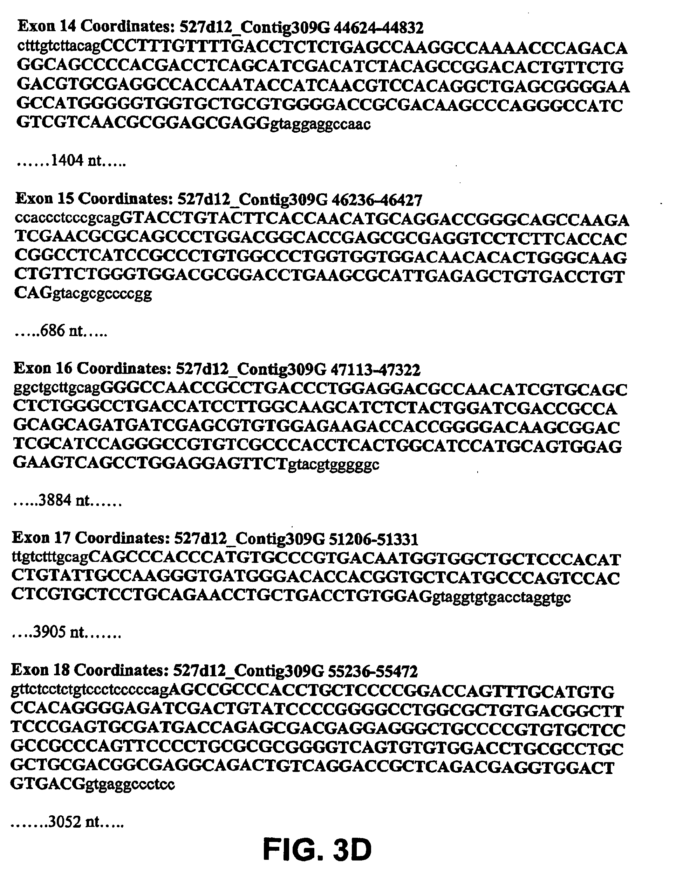

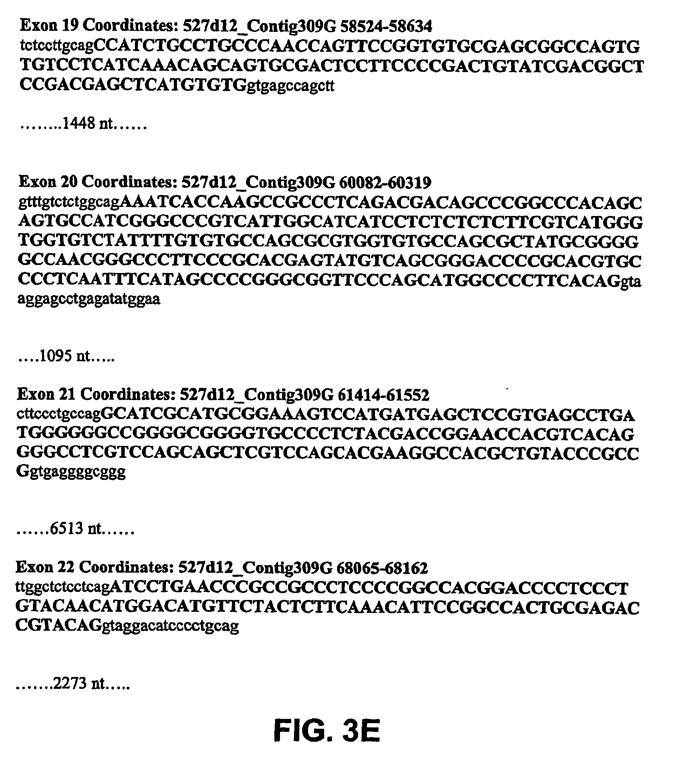

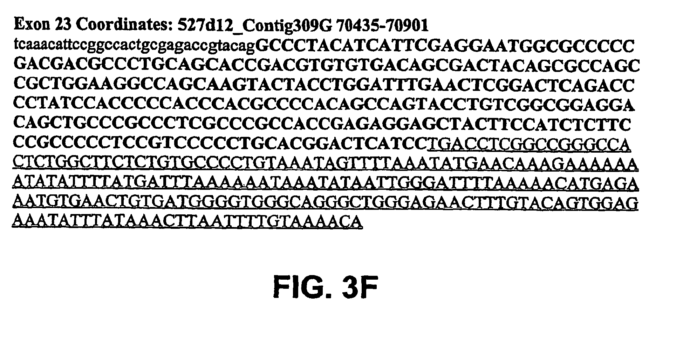

**Figs. 3A-3F** show the genomic structure of Zmax1 with flanking intron sequences. Translation is initiated by the underlined "ATG" in exon 1. The site of the polymorphism in the *HBM* gene is in exon 3 and is represented by the underlined "G," whereby this nucleotide is a "T" in the *HBM* gene. The 3' untranslated region of the mRNA is underlined within exon 23 (exon 1, SEQ ID NO:40; exon 2, SEQ ID NO:41; exon 3, SEQ ID NO:42; exon 4, SEQ ID NO:43; exon 5, SEQ ID NO:44; exon 6, SEQ ID NO:45; exon 7, SEQ ID NO:46; exon 8, SEQ ID NO:47; exon 9, SEQ ID NO:48; exon 10, SEQ ID NO:49; exon 11, SEQ ID NO:50; exon 12, SEQ ID NO:51; exon 13, SEQ ID NO:52; exon 14, SEQ ID NO:53; exon 15, SEQ ID NO:54; exon 16, SEQ ID NO:55; exon 17, SEQ ID NO:56; exon 18, SEQ ID NO:57; exon 19, SEQ ID NO:58; exon 20, SEQ ID NO:59; exon 21, SEQ ID NO:60; exon 22, SEQ ID NO:61; and exon 23; SEQ ID NO:62).

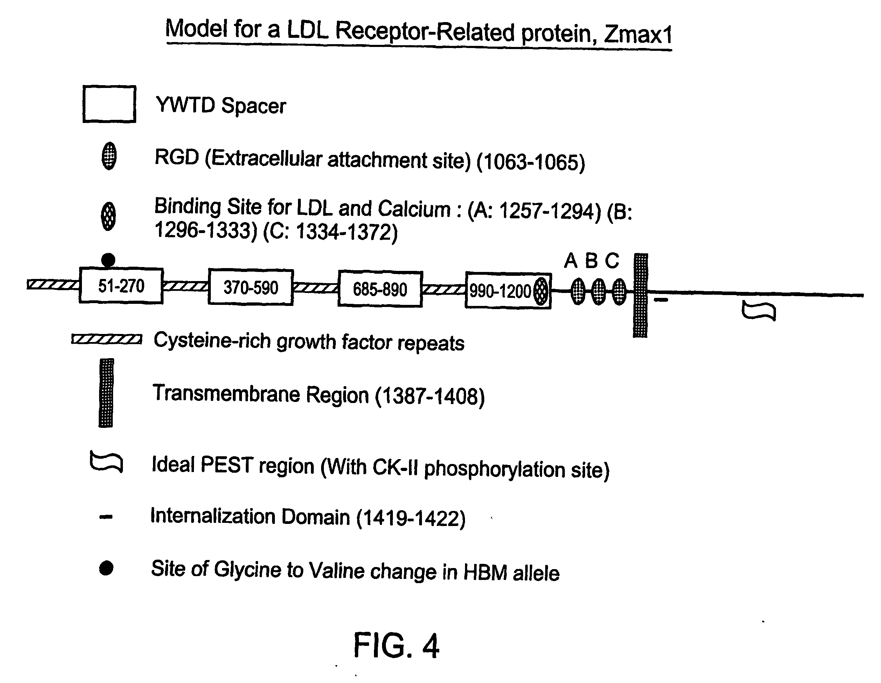

**Fig. 4** shows the domain organization of Zmax1, including the YWTD spacers, the extracellular attachment site, the binding site for LDL and calcium, the cysteine-rich growth factor repeats, the transmembrane region, the ideal PEST region with the CK-II phosphorylation site and the internalization domain. **Fig. 4** also shows the site of the glycine to valine change that occurs in the HBM protein. The signal peptide is located at amino acids 1-22, the extracellular domain is located at amino acids 23-1385, the transmembrane segment is located at amino acids 1386-1413, and the cytoplasmic domain is located at amino acids 1414-1615.
**Fig. 5** shows a schematic illustration of the BAC contigs B527D12 and B200E21 in relation to the *HBM* gene.
**Figs. 6A-6J** show the nucleotide and amino acid sequences of the wild-type gene, Zmax1. The location for the base pair substitution at nucleotide 582, a guanine to thymine, is underlined. This allelic variant is the *HBM* gene. The *HBM* gene encodes for a protein with an amino acid substitution of glycine to valine at position 171. The 5' untranslated region (UTR) consists of bases 1 to 70, and the 3' UTR consists of bases 4916-5120.
**Figs. 7A** and **7B** show northern blot analyses showing the expression of Zmax1 in various tissues.

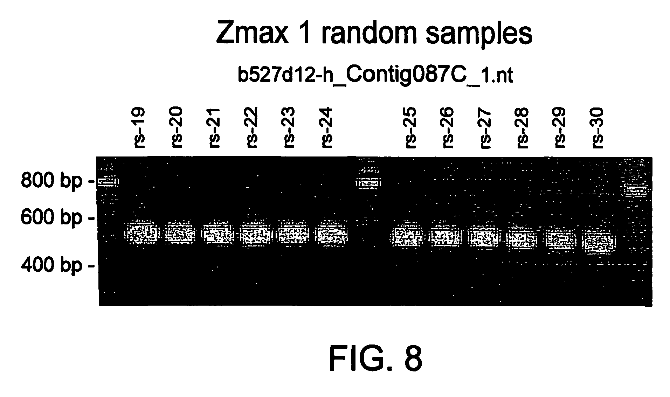

**Fig. 8** shows a PCR product analysis.
**Fig. 9** shows allele specific oligonucleotide detection of the Zmax1 exon 3 mutation.
**Figs. 10A** and **10B** show the cellular localization of mouse Zmax1 by *in situ* hybridization at 100X magnification using sense and antisense probes.
**Figs. 11A** and **11B** show the cellular localization of mouse Zmax1 by *in situ* hybridization at 400X magnification using sense and antisense probes.
**Figs. 12A** and **12B** show the cellular localization of mouse Zmax1 by *in situ* hybridization of osteoblasts in the endosteum at 400X magnification using sense and antisense probes.
**Fig. 13** shows antisense inhibition of Zmax1 expression in MC-3T3 cells.

### DETAILED DESCRIPTION OF THE INVENTION

To aid in the understanding of the specification and claims, the following definitions are provided.

"Gene" refers to a DNA sequence that encodes through its template or messenger RNA a sequence of amino acids characteristic of a specific peptide. The term "gene" includes intervening, non-coding regions, as well as regulatory regions, and can include 5' and 3' ends.

"Gene sequence'' refers to a DNA molecule, including both a DNA molecule which contains a non-transcribed or non-translated sequence. The term is also intended to include any combination of gene(s), gene fragment(s), non transcribed sequence(s) or non-translated sequence(s) which are present on the same DNA molecule.

Sequences for use in the present invention may be derived from a variety of sources including DNA, cDNA, synthetic DNA, synthetic RNA or combinations thereof. Such sequences may comprise genomic DNA which may or may not include naturally occurring introns. Moreover, such genomic DNA may be obtained in association with promoter regions or poly (A) sequences. The sequences, genomic DNA or cDNA may be obtained in any of several ways. Genomic DNA can be extracted and purified from suitable cells by means well known in the art. Alternatively, mRNA can be isolated from a cell and used to produce cDNA by reverse transcription or other means.

"cDNA" refers to complementary or copy DNA produced from an RNA template by the action ofRNA-dependent DNA polymerase (reverse transcriptase). Thus, a "cDNA clone" means a duplex DNA sequence complementary to an RNA molecule of interest, carried in a cloning vector or PCR amplified. This term includes genes from which the intervening sequences have been removed.

"Recombinant DNA" means a molecule that has been recombined by *in vitro* splicing cDNA or a genomic DNA sequence.

"Cloning" refers to the use of *in vitro* recombination techniques to insert a particular gene or other DNA sequence into a vector molecule. In order to successfully clone a desired gene, it is necessary to use methods for generating DNA fragments, for joining the fragments to vector molecules, for introducing the composite DNA molecule into a host cell in which it can replicate, and for selecting the clone having the target gene from amongst the recipient host cells.

"cDNA library" refers to a collection of recombinant DNA molecules containing cDNA inserts which together comprise the entire genome of an organism. Such a cDNA library can be prepared by methods known to one skilled in the art and described by, for example, Cowell and Austin, "cDNA Library Protocols," Methods in Molecular Biology (1997). Generally, RNA is first isolated from the cells of an organism from whose genome it is desired to clone a particular gene.

"Cloning vehicle" refers to a plasmid or phage DNA or other DNA sequence which is able to replicate in a host cell. The cloning vehicle is characterized by one or more endonuclease recognition sites at which such DNA sequences may be cut in a determinable fashion without loss of an essential biological function of the DNA, which may contain a marker suitable for use in the identification of transformed cells.

"Expression control sequence" refers to a sequence of nucleotides that control or regulate expression of structural genes when operably linked to those genes. These include, for example, the lac systems, the trp system, major operator and promoter regions of the phage lambda, the control region of fd coat protein and other-sequences known to control the expression of genes in prokaryotic or eukaryotic cells. Expression control sequences will vary depending on whether the vector is designed to express the operably linked gene in a prokaryotic or eukaryotic host, and may contain transcriptional elements such as enhancer elements, termination sequences, tissue-specificity elements and/or translational initiation and termination sites.

"Expression vehicle" refers to a vehicle or vector similar to a cloning vehicle but which is capable of expressing a gene which has been cloned into it, after transformation into a host. The cloned gene is usually placed under the control of (i.e., operably linked to) an expression control sequence.

"Operator" refers to a DNA sequence capable of interacting with the specific repressor, thereby controlling the transcription of adjacent gene(s).

"Promoter" refers to a DNA sequence that can be recognized by an RNA polymerase. The presence of such a sequence permits the RNA polymerase to bind and initiate transcription of operably linked gene sequences.

"Promoter region" is intended to include the promoter as well as other gene sequences which may be necessary for the initiation of transcription. The presence of a promoter region is sufficient to cause the expression of an operably linked gene sequence.

"Operably linked" means that the promoter controls the initiation of expression of the gene. A promoter is operably linked to a sequence of proximal DNA if upon introduction into a host cell the promoter determines the transcription of the proximal DNA sequence(s) into one or more species of RNA. A promoter is operably linked to a DNA sequence if the promoter is capable of initiating transcription of that DNA sequence.

"Prokaryote" refers to all organisms without a true nucleus, including bacteria.

"Eukaryote" refers to organisms and cells that have a true nucleus, including mammalian cells.

"Host" includes prokaryotes and eukaryotes, such as yeast and filamentous fungi, as well as plant and animal cells. The term includes an organism or cell that is the recipient of a replicable expression vehicle.

By "animal" is meant to include vertebrates. Preferred vertebrates include mammals and birds, but also include fish, reptiles and amphibians. Preferred mammals include: humans, primates, rodents, canines, felines and livestock.

"Fragment" of a gene refers to any variant of the gene that possesses the biological activity of that gene.

"Variant" refers to a gene that is substantially similar in structure and biological activity or immunological characteristics to either the entire gene or to a fragment of the gene. Provided that the two genes possess a similar activity, they are considered variant as that term is used herein even if the sequence of amino acid residues is not identical.

"Amplification of nucleic acids" refers to methods such as polymerase chain reaction (PCR), ligation amplification (or ligase chain reaction, LCR) and amplification methods based on the use of Q-beta replicase. These methods are well known in the art and described, for example, in U.S. Patent Nos. 4,683,195 and 4,683,202. Reagents and hardware for conducting PCR are commercially available. Primers useful for amplifying sequences from the HBM region are preferably complementary to, and hybridize specifically to sequences in the HBM region or in regions that flank a target region therein. HBM sequences generated by amplification may be sequenced directly. Alternatively, the amplified sequence(s) may be cloned prior to sequence analysis.

"Antibodies" may refer to polyclonal and/or monoclonal antibodies and fragments thereof, and immunologic binding equivalents thereof, that can bind to the HBM and Zmax1 proteins and fragments thereof or to nucleic acid sequences from the HBM or Zmax1 region, particularly from the HBM locus or a portion thereof. The term antibody is used both to refer to a homogeneous molecular entity, or a mixture such as a serum product made up of a plurality of different molecular entities. Proteins may be prepared synthetically in a protein synthesizer and coupled to a carrier molecule and injected over several months into rabbits. Rabbit sera is tested for immunoreactivity to the HBM protein or fragment. Monoclonal antibodies may be made by injecting mice with the proteins, or fragments thereof. Monoclonal antibodies will be screened by ELISA and tested for specific immunoreactivity with HBM protein or fragments thereof. Harlow et al., Antibodies: A Laboratory Manual, Cold Spring Harbor Laboratory, Cold Spring Harbor, NY (1988). These antibodies will be useful in assays as well as pharmaceuticals. Antibodies can include antibody fragments (*e.g.,* scFv, Fab, F(ab')₂, *etc.)* as well as human antibodies, humanized antibodies and primatized antibodies.

"HBM" refers to high bone mass, but polymorphisms associated with *HBM* gene, which can also be involved in lipid modulation.

"HBM protein" refers to a protein that is identical to a Zmax1 protein except that it contains an alteration of glycine 171 to valine. An HBM protein is defined for any organism that encodes a Zmax1 true homologue. For example, a mouse HBM protein refers to the mouse Zmax1 protein having the glycine 170 to valine substitution.

*"HBM* gene" refers to the genomic DNA sequence found in individuals showing the HBM characteristic or phenotype, where the sequence encodes the protein indicated by SEQ ID NO: 4. The *HBM* gene and the *Zmax1* gene are allelic. The protein encoded by the *HBM* gene has the property of causing elevated bone mass and also altering physiologic lipid levels, while the protein encoded by the *Zmax1* gene does not. The *HBM* gene and the *Zmax1* gene differ in that the *HBM* gene has a thymine at position 582, while the *Zmax1* gene has a guanine at position 582. The *HBM* gene comprises the nucleic acid sequence shown as SEQ ID NO: 2. The *HBM* gene may also be referred to as an "HBM polymorphism."

"Normal," "wild-type," "unaffected" and "Zmax1" all refer to the genomic DNA sequence that encodes the protein indicated by SEQ ID NO: 3. The *Zmax1* gene has a guanine at position 582. The *Zmax1* gene comprises the nucleic acid sequence shown as SEQ ID NO: 1. "Normal," "wild-type," "unaffected" and "Zmax1" also refer to allelic variants of the genomic sequence that encodes proteins that do not contribute to elevated bone mass. The *Zmax1* gene is common in the human population, while the *HBM* gene is rare.

"5YWT+EGF" refers to a repeat unit found in the Zmax1 protein, consisting of five YWT repeats followed by an EGF repeat.

"Bone development" generally refers to any process involved in the change of bone over time, including, for example, normal development, changes that occur during disease states, and changes that occur during aging. "Bone development disorder" particularly refers to any disorders in bone development including, for example, changes that occur during disease states and changes that occur during aging. Bone development may be progressive or cyclical in nature. Aspects of bone that may change during development include, for example, mineralization, formation of specific anatomical features, and relative or absolute numbers of various cell types.

"Bone modulation" or "modulation of bone formation" refers to the ability to affect any of the physiological processes involved in bone remodeling, as will be appreciated by one skilled in the art, including, for example, bone resorption and appositional bone growth, by, inter alia, osteoclastic and osteoblastic activity, and may comprise some or all of bone formation and development as used herein.

"Normal bone density" refers to a bone density within two standard deviations or a Z score of 0.

By "lipid regulation" or "lipid modulation" is meant the ability to alter by modulating the *HBM* or *Zmax1* genes, mRNA or protein encoded thereby the levels of a lipid. Altered levels of lipid include very low density lipoproteins (VLDL), low density lipoproteins (LDL), high density lipoprotein (HDL) and triglycerides. The regulation or modulation can be an increase or decrease in the lipid level by an agent, which when administered to a subject modulates HBM or Zmax1 activity. By "lipid metabolism" is meant the physiological cycle through which the various triglycerides and lipoproteins proceed. Agents of the invention can also be said to modulate the metabolism of various lipids.

"Lipid" preferably includes very low density lipoproteins (VLDL), low density lipoproteins (LDL), intermediate density lipoprotein (IDL), high density lipoprotein (HDL) and triglycerides. Lipids can also include apolipoproteins, such as apolipoprotein A-1 (APO A-1), apolipoprotein B (APO B), apoliprotein E (APO E) and lipoproteins such as lipoprotein a (LIPOa).

By "lipid-mediated disease or condition" is meant to include arteriosclerosis and related conditions, hypercholesteremia, hyperlipidemia, atherosclerosis, and conditions or lifestyles associated with elevated lipid levels (*e.g.,* diabetes mellitus, smoking and obesity) such as those discussed herein.

By "arteriosclerosis" is meant to include hypertrophy of the media and subintimal fibrosis with hyaline degeneration which can result in ectasia, aneurysm, increased systolic pressure, thrombus formation and embolism. Disorders associated with arteriosclerosis include, but are not limited to, nonatheromatous arteriosclerosis conditions such as: diabetes mellitus, chronic renal insufficiency, chronic vitamin D intoxication, pseudoxanthoma elasticum, idiopathic arterial calcification in infancy, aortic valvular calcification in the elderly, and Werner's syndrome. Additional disorders associated with arteriosclerosis and atherosclerosis include: diabetes mellitus, hypertension, familial hypercholesterolemia, familial combined hyperlipidemia, familial dysbetalipoproteinemia, familial hypoalphalipoproteinemia, hypothyroidism, cholesterol ester storage disease, systemic lupus erythematosus and homocysteinemia.

By "atherosclerosis" is meant patchy intramural thickening of the subintima that encroaches on the arterial lumen and can cause obstruction. Atherosclerotic plaque consists of the accumulation of lipids, cells, annective tissue and glycosaminoglycans. It can cause the following conditions: stenosis, thrombosis, aneurysm, or embolus supervenes, as well as angina as well as the conditions listed above.

A "Zmax1 system" refers to a purified protein, cell extract, cell, animal, human or any other composition of matter in which Zmax1 is present in a normal or mutant form.

A "surrogate marker" refers to a diagnostic indication, symptom, sign or other feature that can be observed in a cell, tissue, human or animal that is correlated with the *HBM* gene or elevated bone mass or both, but that is easier to measure than bone density. The general concept of a surrogate marker is well accepted in diagnostic, medicine.

Described herein are the *Zmax1* gene and Zmax1 protein in the forms indicated by SEQ ID NOS: 1 and 3, respectively, and other closely related variants, as well as the adjacent chromosomal regions of Zmax1 necessary for its accurate expression. Also described herein is a sequence having at least 15 contiguous nucleotides of the nucleic acid sequence of SEQ ID NO: 1.

Also described herein are the *HBM* gene and HBM protein in the forms indicated by SEQ ID NO: 2 and 4, respectively, and other closely related variants, as well as the adjacent chromosomal regions of the *HBM* gene necessary for its accurate expression. Also described is a sequence having at least 15 contiguous nucleotides of the nucleic acid sequence of SEQ ID NO: 2, for example, a sequence having at least 15 contiguous nucleotides of the nucleic acid sequence of SEQ ID NO: 2, wherein one of the 15 contiguous nucleotides is the thymine at nucleotide 582.

The nucleotide sequence of the *Zmax1* gene region, as well as the nucleotide sequence of the *HBM* gene region are described herefore. More particularly, BAC clones containing segments of the *Zmax1* gene region B200E21-H and B527D12-H are described such as the nucleotide sequence of the BAC clones consisting of SEQ ID NOS: 5-12.

The nucleotide sequence may be used to identify DNA probes for the *Zmax1* gene and the *HBM* gene, PCR primers to amplify the *Zmax1* gene and the *HBM* gene, nucleotide polymorphisms in the *Zmax1* gene and the *HBM* gene, and regulatory elements of the *Zmax1* gene and the *HBM* gene.

The chromosomal location of the *Zmax1* gene and *HBM* gene on chromosome 11q13.3 is between genetic markers D11S987 and SNP_CONTIG033-6, as well as the DNA sequences of the *Zmax1* gene and the *HBM* gene. The chromosomal location was refined by the addition of more genetic markers to the mapping panel used to map the gene, and by the extension of the pedigree to include more individuals. The pedigree extension was critical because the new individuals that have been genotyped harbor critical recombination events that narrow the region. To identify genes in the region on 11q13.3, a set of BAC clones containing this chromosomal region was identified. The BAC clones served as a template for genomic DNA sequencing, and also as a reagent for identifying coding sequences by direct cDNA selection. Genomic sequencing and direct cDNA selection were used to characterize more than 1.5 million base pairs of DNA from 11q13.3. The Zmax1 gene was identified within this region and the *HBM* gene was then discovered after mutational analysis of affected and unaffected individuals.

When a gene has been genetically localized to a specific chromosomal region, the genes in this region can be characterized at the molecular level by a series of steps that include: cloning of the entire region of DNA in a set of overlapping clones (physical mapping), characterization of genes encoded by these clones by a combination of direct cDNA selection, exon trapping and DNA sequencing (gene identification), and identification of mutations in these genes by comparative DNA sequencing of affected and unaffected members of the HBM kindred (mutation analysis).

Physical mapping is accomplished by screening libraries of human DNA cloned in vectors that are propagated in *E. coli* or *S. cereviseae* using PCR assays designed to amplify unique molecular landmarks in the chromosomal region of interest. To generate a physical map of the HBM candidate region, a library of human DNA cloned in Bacterial Artificial Chromosomes (BACs) was screened with a set of Sequence Tagged Site (STS) markers that had been previously mapped to chromosome 1 1q12-q13 by the efforts of the Human Genome Project.

STSs are unique molecular landmarks in the human genome that can be assayed by PCR Through the combined efforts of the Human Genome Project, the location of thousands of STSs on the twenty-two autosomes and two sex chromosomes has been determined. For a positional cloning effort, the physical map is tied to the genetic map because the markers used for genetic mapping can also be used as STSs for physical mapping. By screening a BAC library with a combination of STSs derived from genetic markers, genes, and random DNA fragments, a physical map comprised of overlapping clones representing all of the DNA in a chromosomal region of interest can be assembled.

BACs are cloning vectors for large (80 kilobase to 200 kilobase) segments of human or other DNA that are propagated in *E. coli.* To construct a physical map using BACs, a library of BAC clones is screened so that individual clones harboring the DNA sequence corresponding to a given STS or set of STSs are identified. Throughout most of the human genome, the STS markers are spaced approximately 20 to 50 kilobases apart, so that an individual BAC clone typically contains at least two STS markers. In addition, the BAC libraries that were screened contain enough cloned DNA to cover the human genome six times over. Therefore, an individual STS typically identifies more than one BAC clone. By screening a six-fold coverage BAC library with a series of STS markers spaced approximately 50 kilobases apart, a physical map consisting of a series of overlapping BAC clones, i.e. BAC contigs, can be assembled for any region of the human genome. This map is closely tied to the genetic map because many of the STS markers used to prepare the physical map are also genetic markers.

When constructing a physical map, it often happens that there are gaps in the STS map of the genome that result in the inability to identify BAC clones that are overlapping in a given location. Typically, the physical map is first constructed from a set of STSs that have been identified through the publicly available literature and World Wide Web resources. The initial map consists of several separate BAC contigs that are separated by gaps of unknown molecular distance. To identify BAC clones that fill these gaps, it is necessary to develop new STS markers from the ends of the clones on either side of the gap. This is done by sequencing the terminal 200 to 300 base pairs of the BACs flanking the gap, and developing a PCR assay to amplify a sequence of 100 or more base pairs. If the terminal sequences are demonstrated to be unique within the human genome, then the new STS can be used to screen the BAC library to identify additional BACs that contain the DNA from the gap in the physical map. To assemble a BAC contig that covers a region the size of the HBM candidate region (2,000,000 or more base pairs), it is often necessary to develop new STS markers from the ends of several clones.

After building a BAC contig, this set of overlapping clones serves as a template for identifying the genes encoded in the chromosomal region. Gene identification can be accomplished by many methods. Three methods are commonly used: (1) a set of BACs selected from the BAC contig to represent the entire chromosomal region can be sequenced, and computational methods can be used to identify all of the genes, (2) the BACs from the BAC contig can be used as a reagent to clone cDNAs corresponding to the genes encoded in the region by a method termed direct cDNA selection, or (3) the BACs from the BAC contig can be used to identify coding sequences by selecting for specific DNA sequence motifs in a procedure called exon trapping.

To sequence the entire BAC contig representing the HBM candidate region, a set of BACs was chosen for subcloning into plasmid vectors and subsequent DNA sequencing of these subclones. Since the DNA cloned in the BACs represents genomic DNA, this sequencing is referred to as genomic sequencing to distinguish it from cDNA sequencing. To initiate the genomic sequencing for a chromosomal region of interest, several nonoverlapping BAC clones are chosen. DNA for each BAC clone is prepared, and the clones are sheared into random small fragments which are subsequently cloned into standard plasmid vectors such as pUC18. The plasmid clones are then grown to propagate the smaller fragments, and these are the templates for sequencing. To ensure adequate coverage and sequence quality for the BAC DNA sequence, sufficient plasmid clones are sequenced to yield six-fold coverage of the BAC clone. For example, if the BAC is 100 kilobases long, then phagemids are sequenced to yield 600 kilobases of sequence. Since the BAC DNA was randomly sheared prior to cloning in the phagemid vector, the 600 kilobases of raw DNA sequence can be assembled by computational methods into overlapping DNA sequences termed sequence contigs. For the purposes of initial gene identification by computational methods, six-fold coverage of each BAC is sufficient to yield ten to twenty sequence contigs of 1000 base pairs to 20,000 base pairs.

The sequencing strategy employed was to initially sequence "seed" BACs from the BAC contig in the HBM candidate region. The sequence of the "seed" BACs was then used to identify minimally overlapping BACs from the contig, and these were subsequently sequenced. In this manner, the entire candidate region was sequenced, with several small sequence gaps left in each BAC. This sequence served as the template for computational gene identification. One method for computational gene identification is to compare the sequence of BAC contig to publicly available databases of cDNA and genomic sequences, e.g. unigene, dbEST, genbank. These comparisons are typically done using the BLAST family of computer algorithms and programs (Altschul et al., J. Mol. Biol., 215:403-410 (1990)). The BAC sequence can also be translated into protein sequence, and the protein sequence can be used to search publicly available protein databases, using aversion of BLAST designed to analyze protein sequences (Altschul et al., Nucl. Acids Res., 25:3339-3402 (1997)). Another method is to use computer algorithms such as MZEF (Zhang, Proc. Natl. Acad. Sci., 94:565-568 (1997)) and GRAIL (Uberbacher et al., Methods Enzymol., 266:259-281 (1996)), which predict the location of exons in the sequence based on the presence of specific DNA sequence motifs that are common to all exons, as well as the presence of codon usage typical of human protein encoding sequences.

In addition to identifying genes by computational methods, genes were also identified by direct cDNA selection (Del Mastro et al., Genome Res. 5(2):185-194 (1995)). In direct cDNA selection, cDNA pools from tissues of interest are prepared, and the BACs from the candidate region are used in a liquid hybridization assay to capture the cDNAs which base pair to coding regions in the BAC. In the methods described herein, the cDNA pools were created from several different tissues by random priming the first strand cDNA from polyA RNA, synthesizing the second strand cDNA by standard methods, and adding linkers to the ends of the cDNA fragments. The linkers are used to amplify the cDNA pools. The BAC clones are used as a template for *in vitro* DNA synthesis to create a biotin labelled copy of the BAC DNA. The biotin labelled copy of the BAC DNA is then denatured and incubated with an excess of the PCR amplified, linkered cDNA pools which have also been denatured. The BAC DNA and cDNA are allowed to anneal in solution, and heteroduplexes between the BAC and the cDNA are isolated using streptavidin coated magnetic beads. The cDNAs that are captured by the BAC are then amplified using primers complimentary to the linker sequences, and the hybridization/selection process is repeated for a second round. After two rounds of direct cDNA selection, the cDNA fragments are cloned, and a library of these direct selected fragments is created.

The cDNA clones isolated by direct selection are analyzed by two methods. Since a pool of BACs from the HBM candidate region is used to provide the genomic DNA sequence, the cDNAs must be mapped to individual BACs. This is accomplished by arraying the BACs in microtiter dishes, and replicating their DNA in high density grids. Individual cDNA clones are then hybridized to the grid to confirm that they have sequence identity to an individual BAC from the set used for direct selection, and to determine the specific identity of that BAC. cDNA clones that are confirmed to correspond to individual BACs are sequenced. To determine whether the cDNA clones isolated by direct selection share sequence identity or similarity to previously identified genes, the DNA and protein coding sequences are compared to publicly available databases using the BLAST family of programs.

The combination of genomic DNA sequence and cDNA sequence provided by BAC sequencing and by direct cDNA selection yields an initial list of putative genes in the region. The genes in the region were all candidates for the HBM locus. To further characterize each gene, Northern blots were performed to determine the size of the transcript corresponding to each gene, and to determine which putative exons were transcribed together to make an individual gene. For Northern blot analysis of each gene, probes were prepared from direct selected cDNA clones or by PCR amplifying specific fragments from genomic DNA or from the BAC encoding the putative gene of interest. The Northern blots gave information on the size of the transcript and the tissues in which it was expressed. For transcripts which were not highly expressed, it was sometimes necessary to perform a reverse transcription PCR assay using RNA from the tissues of interest as a template for the reaction.

Gene identification by computational methods and by direct cDNA selection provides unique information about the genes in a region of a chromosome. When genes are identified, then it is possible to examine different individuals for mutations in each gene.

### I. Phenotyping using DXA Measurements

Spinal bone mineral content (BMC) and bone mineral density (BMD) measurements performed at Creighton University (Omaha, Nebraska) were made by DXA using a Norland Instruments densitometer (Norland XR2600 Densitometer, Dual Energy X-ray Absorptiometry, DXA). Spinal BMC and BMD at other locations used the machinery available. There are estimated to be 800 DXA machines currently operating in the U.S. Most larger cities have offices or imaging centers which have DXA capabilities, usually a Lunar or Hologic machine. Each location that provided spine BMC and BMD data included copies of the printouts from their machines to provide verification that the regions of interest for measurement of BMD have been chosen appropriately. Complete clinical histories and skeletal radiographs were obtained.

The HBM phenotype is defined by the following criteria: very high spinal BMD; a clinical history devoid of any known high bone mass syndrome; and skeletal radiographs showing a normal shape of the appendicular skeleton.

### II. Genotyping of Microsatellite Markers

To narrow the genetic interval to a region smaller than that originally reported by Johnson et al., Am. J. Hum. Genet., 60:1326-1332 (1997), additional microsatellite markers on chromosome 11q12-13 were typed. The new markers included: D11S4191, D11S1883, D11S1785, D11S4113, D11S4136, D11S4139, (Dib, et al., Nature, 380:152-154 (1996), FGF3 (Polymeropolous, et al., Nucl. Acid Res., 18:7468 (1990)), as well as GTC_HBM_Marker_1, GTC_HBM_Marker_2, GTC_HBM_Marker_3, GTC_HBM_Marker_4, GTC_HBM_Marker_5, GTC_HBM_Marker_6, and GTC_HBM_Marker_7 (*See* **Fig. 2**).

Blood (20 ml) was drawn into lavender cap (EDTA containing) tubes by a certified phlebotomist. The blood was stored refrigerated until DNA extraction. DNA has been extracted from blood stored for up to 7 days in the refrigerator without reduction in the quality or quantity of yield. For those subjects that have blood drawn at distant sites, a shipping protocol was successfully used on more than a dozen occasions. Blood samples were shipped by overnight express in a styrofoam container with freezer packs to provide cooling. Lavender cap tubes were placed on individual plastic shipping tubes and then into "zip-lock" biohazard bags. When the samples arrived the next day, they were immediately processed to extract DNA.

The DNA extraction procedure used a kit purchased from Gentra Systems, Inc. (Minneapolis, Minnesota). Briefly, the procedure involved adding 3 volumes of a red blood cell lysis buffer to the whole blood. After incubations for 10 minutes at room temperature, the solution was centrifuged in a Beckman tabletop centrifuge at 2,000 X g for 10 minutes. The white blood cell pellet was resuspended in Cell Lysis Buffer. Once the pellet was completely resuspended and free of cell clumps, the solution was digested with RNase A for 15 minutes at 37°C. Proteins were precipitated by addition of the provided Protein Precipitation Solution and removed by centrifugation. The DNA was precipitated out of the supernatant by addition of isopropanol. This method was simple and fast, requiring only 1-2 hours, and allowed for the processing of dozens of samples simultaneously. The yield of DNA was routinely >8 mg for a 20 ml sample of whole blood and had a MW of>50 kb. DNA was archived by storing coded 50 *µ*g aliquots at -80°C as an ethanol precipitate.

DNA was genotyped using one fluorescently labeled oligonucleotide primer and one unlabeled oligonucleotide primer. Labeled and unlabeled oligonucleotides were obtained from Integrated DNA Technologies, Inc. (Coralville, Iowa). All other reagents for microsatellite genotyping were purchased from Perkin Elmer-Applied Biosystems, Inc. ("PE-ABI") (Norwalk, Connecticut). Individual PCR reactions were performed for each marker, as described by PE-ABI using AmpliTag DNA Polymerase. The reactions were added to 3.5 µl of loading buffer containing deionized formamide, blue dextran and TAMRA 350 size standards (PE-ABI). After heating at 95 ° C for 5 minutes to denature the DNA, the samples were loaded and electrophoresed as described in the operator's manual for the Model 377 DNA Sequencer (PE-ABI, Foster City, California). After gel electrophoresis, the data was analyzed using PE-ABI GENESCAN^{™} and GENOTYPER^{™} software. First, within the GENESCAN^{™} software, the lane tracking was manually optimized prior to the first step of analysis. After the gel lane data was extracted, the standard curve profiles of each lane were examined and verified for linearity and size calling. Lanes, which had problems with either of these parameters, were re-tracked and verified. Once all lanes were tracked and the size standards were correctly identified, the data were imported into GENOTYPER^{™} for allele identification To expedite allele calling (binning), the program Linkage Designer from the Internet web-site of Dr. Guy Van Camp (http://alt.www.uia.ac.be/u/dnalab/1d.htm1) was used. This program greatly facilitates the importing of data generated by GENOTYPER^{™} into the pedigree drawing program Cyrillic (Version 2.0, Cherwell Scientific Publishing Limited, Oxford, Great Britain) and subsequent linkage analysis using the program LINKAGE (Lathrop et al., Am. J. Hum. Genet., 37:482-498 (1985)).

### III. Linkage Analysis

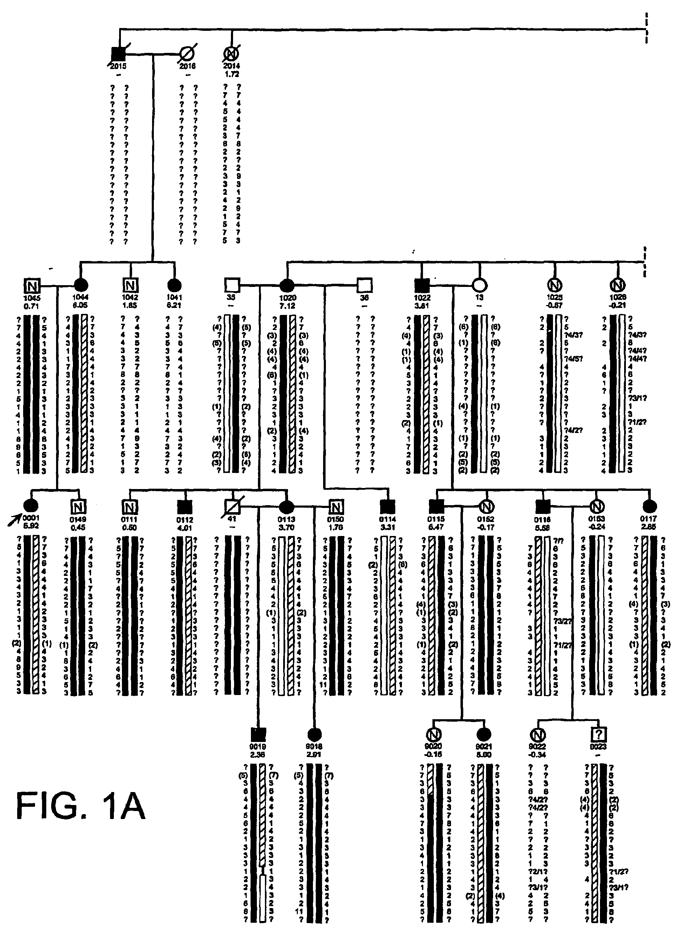

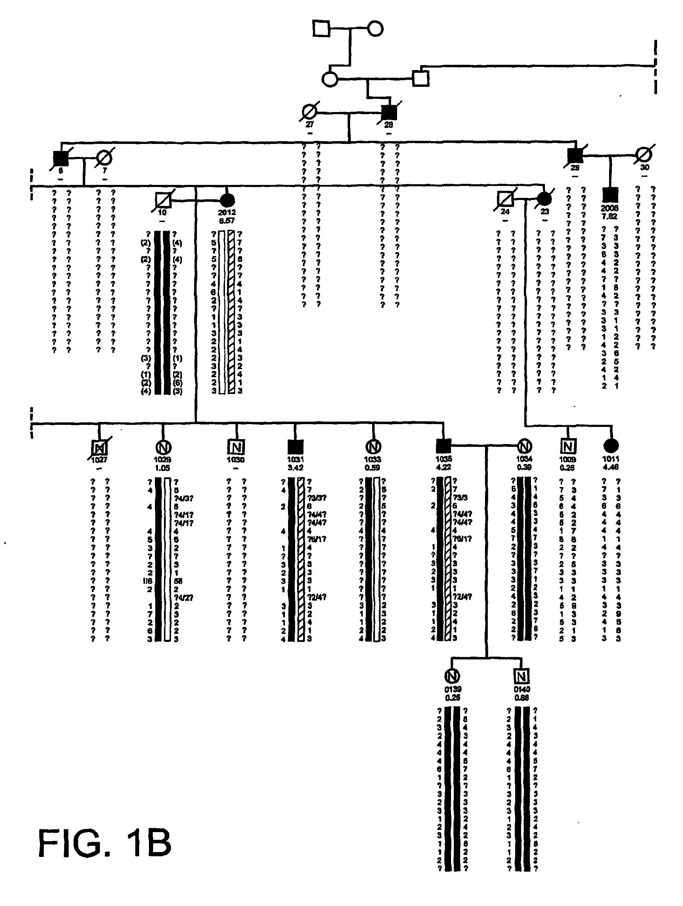

**Fig. 1** demonstrates the pedigree of the individuals used in the genetic linkage studies for this invention. Specifically, two-point linkage analysis was performed using the MLINK and LINKMAP components of the program LINKAGE (Lathrop et al., Am. J. Hum. Genet., 37:482-498 (1985)). Pedigree/marker data was exported from Cyrillic as a pre-file into the Makeped program and converted into a suitable ped-file for linkage analysis.

The original linkage analysis was performed using three models: (i) an autosomal dominant, fully penetrant model, (ii) an autosomal dominant model with reduced penetrance, and (iii) a quantitative trait model. The HBM locus was mapped to chromosome 11q12-13 by analyzing DNA for linked markers from 22 members of a large, extended kindred. A highly automated technology was used with a panel of 345 fluorescent markers which spanned the 22 autosomes at a spacing interval ranging from 6-22 cM. Only markers from this region of chromosome 11 showed evidence of linkage (LOD score ~3.0). The highest LOD score (5.74) obtained by two-point and multipoint analysis was D11S987 (map position 55 in **Fig. 2).** The 95% confidence interval placed the HBM locus between markers D11S905 and D11S937 (map position 41-71 in **Fig. 2).** Haplotype analysis also places the *Zmax1* gene in this same region. Further descriptions of the markers D11S987, D11S905, and D11S937 can be found in Gyapay et al., Nature Genetics, Vol. 7, (1994).

In this invention, the inventors report the narrowing of the HBM interval to the region between markers D11S987 and GTC_HBM_Marker_5. These two markers lie between the delimiting markers from the original analysis (D11S11S905 and D11S937) and are approximately 3 cM from one another. The narrowing of the interval was accomplished using genotypic data from the markers D11S4191, D11S1883, D11S1785, D11S4113, D11S4136, D11S4139, (Dib et al., Nature, 380:152-154 (1996)), FGF3 (Polymeropolous et al., Nucl. Acid Res., 18:7468 (1990)) (information about the genetic markers can be found at the internet site of the Genome Database, http://gdbwww.gdb.org/), as well as the markers GTC_HBM_Maiker_1, GTC_HBM_Marker_2, GTC_HBM_Marker_3, GTC_HBM_Marker_4, GTC_HBM_Marker_5, GTC_HBM_Marker_6, and GTC_HBM_Marker_7.

As shown in **Fig. 1**, haplotype analysis with the above genetic markers identifies recombination events (crossovers) in individuals 9019 and 9020 that significantly refine the interval of chromosome 11 to which the *Zmax1* gene is localized. Individual 9019 is an HBM-affected individual that inherits a portion of chromosome 11 from the maternal chromosome with the *HBM* gene, and a portion from the chromosome 11 homologue. The portion inherited from the *HBM* gene-carrying chromosome includes markers D11S935, D11S1313, GTC_HBM_Marker_4, D11S987, D11S1296, GTC_HBM_Marker_6, GTC_HBM_Marker_2, D11S970, GTC_HBM_Marker_3, D11S4113, GTC_HBM_Marker_1, GTC_HBM_Marker_7 and GTC_HBM_Marker_5. The portion from D11S4136 and continuing in the telomeric direction is derived from the non-HBM chromosome. This data places the *Zmax1* gene in a location centromeric to the marker GTC_HBM_Marker_5. Individual 9020 is an unaffected individual who also exhibits a critical recombination event. This individual inherits a recombinant paternal chromosome 11 that includes markers D11S935, D11S1313, GTC_HBM_Marker_4, D11S987, D11S1296 and GTC_HBM_Marker_6 from her father's (individual 0115) chromosome 11 homologue that carries the *HBM* gene, and markers GTC_HBM_Marker_2, D11S970, GTC_HBM_Marker_3, GTC_HBM_Marker_1, GTC_HBM_Marker_7, GTC_HBM_Marker_5, D11S4136, D11S4139, D11S1314, and D11S937 from her father's chromosome 11 that does not carry the *HBM* gene. Marker D11S4113 is uninformative due to its homozygous nature in individual 0115. This recombination event places the centromeric boundary of the HBM region between markers D11S1296 and D11S987.

Two-point linkage analysis was also used to confirm the location of the *Zmax1* gene on chromosome 11. The linkage results for two point linkage analysis under a model of full penetrance are presented in Table 1 below. This table lists the genetic markers in the first column and the recombination fractions across the top of the table. Each cell of the column shows the LOD score for an individual marker tested for linkage to the *Zmax1* gene at the recombination fraction shown in the first row. For example, the peak LOD score of 7.66 occurs at marker D11S970, which is within the interval defined by haplotype analysis.

**TABLE 1**

| | | | | | | | | | |
|---|---|---|---|---|---|---|---|---|---|
| Marker | 0.0 | 0.05 | 0.1 | 0.15 | 0.2 | 0.25 | 0.3 | 0.35 | 0.4 |
| D11S935 | - infinity | 0.39 | 0.49 | 0.47 | 0.41 | 0.33 | 0.25 | 0.17 | 0.10 |
| D11S1313 | - infinity | 2.64 | 2.86 | 2.80 | 2.59 | 2.30 | 1.93 | 1.49 | 1.00 |
| D11S987 | - infinity | 5.49 | 5.18 | 4.70 | 4.13 | 3.49 | 2.79 | 2.03 | 1.26 |
| D11S4113 | 4.35 | 3.99 | 3.62 | 3.24 | 2.83 | 2.40 | 1.94 | 1.46 | 0.97 |
| D11S1337 | 2.29 | 2.06 | 1.81 | 1.55 | 1.27 | 0.99 | 0.70 | 0.42 | 0.18 |
| D11S970 | 7.66 | 6.99 | 6.29 | 5.56 | 4.79 | 3.99 | 3.15 | 2.30 | 1.44 |
| D11S4136 | 6.34 | 5.79 | 5.22 | 4.61 | 3.98 | 3.30 | 2.59 | 1.85 | 1.11 |
| D11S4139 | 6.80 | 6.28 | 5.73 | 5.13 | 4.50 | 3.84 | 3.13 | 2.38 | 1.59 |
| FGF3 | 0.59 | 3.23 | 3.15 | 2.91 | 2.61 | 2.25 | 1.84 | 1.40 | 0.92 |
| D11S1314 | 6.96 | 6.49 | 5.94 | 5.34 | 4.69 | 4.01 | 3.27 | 2.49 | 1.67 |
| D11S937 | -infinity | 4.98 | 4.86 | 4.52 | 4.06 | 3.51 | 2.88 | 2.20 | 1.47 |

A single nucleotide polymorphism (SNP) further defines the HBM region. This SNP is termed SNP_Contig033-6 and is located 25 kb centromeric to the genetic marker GTC_HBM_Marker_5. This SNP is telomeric to the genetic marker GTC_HBM_Marker_7. SNP_Contig033-6 is present in HBM-affected individual 0113.. However, the HBM-affected individual 9019, who is the son of 0113, does not carry this SNP. Therefore, this indicates that the'crossover is centromeric to this SNP. The primer sequence for the genetic markers GTC_HBM_Marker_5 and GTC_HBM_Marker_7 is shown in Table 2 below.

**TABLE 2**

| **Marker** | **Primer (Forward)** | **Primer (Reverse)** |
|---|---|---|
| GTC_HBM_Marker_5 | TTTTGGGTACACAATTCAGTCG | AAAACTGTGGGTGCTTCTGG |
| GTC_HBM_Marker_7 | GTGATTGAGCCAATCCTGAGA | TGAGCCAAATAAACCCCTTCT |

The kindred described have several features of great interest, the most important being that their bones, while very dense, have an absolutely normal shape. The outer dimensions of the skeletons of the HBM-affected individuals are normal, and, while medullary cavities are present, there is no interference with hematopoiesis. The HBM-affected members seem to be resistant to fracture, and there are no neurologic symptoms, and no symptoms of impairment of any organ or system function in the members examined. HBM-affected members of the kindred live to advanced age without undue illness or disability. Furthermore, the HBM phenotype matches no other bone disorders such as osteoporosis, osteoporosis pseudoglioma, Engelmann's disease, Ribbing's disease, hyperphosphatasemia, Van Buchem's disease, melorheostosis, osteopetrosis, pycnodysostosis, sclerostenosis, osteopoikilosis, acromegaly, Paget's disease, fibrous dysplasia, tubular stenosis, osteogenesis imperfecta,- hypoparathyroidism, pseudohypoparathyroidism, pseudopseudohypoparathyroidism, primary and secondary hyperparathyroidism and associated syndromes, hypercalciuria, medullary carcinoma of the thyroid gland, osteomalacia and other diseases. Clearly, the HBM locus in this family has a very powerful and substantial role in regulating bone density, and its identification is an important step in understanding the pathway(s) that regulate bone density and the pathogenesis of diseases such as osteoporosis.

In addition, older individuals carrying the *HBM* gene, and therefore expression of the HBM protein, do not show loss of bone mass characteristic of normal individuals. Moreover, individuals carrying the *HBM* gene have lower triglycerides, VLDLs, and LDLs and/or increased HDLs. In other words, the *HBM* gene is a suppressor of osteoporosis and may lessen cardiovascular risk arteriosclerotic and/or atherosclerotic associated conditions. In essence, individuals carrying the *HBM* gene are dosed with the HBM protein, and, as a result, lower levels of detrimental lipids (e.g., VLDL, LDL and triglycerides). This *in vivo* observation is strong evidence that treatment of normal individuals with the *HBM* gene or protein, or a fragment thereof, will ameliorate osteoporosis and arterio- or atherosclerotic conditions or diseases.

### IV. Physical Mapping

To provide reagents for the cloning and characterization of the HBM locus, the genetic mapping data described above were used to construct a physical map of the region containing Zmax1 on chromosome 11q13.3. The physical map consists of an ordered set of molecular landmarks, and a set of BAC clones that contain the Z*max1* gene region from chromosome 11q13.3.

Various publicly available mapping resources were utilized to identify existing STS markers (Olson et al., Science, 245:1434-1435 (1989)) in the HBM region. Resources included the GDB, the Whitehead Institute Genome Center, dbSTS and dbEST (NCBI), 11db, the University of Texas Southwestern GESTEC, the Stanford Human Genome Center, and several, literature references (Courseaux et al., Genomics, 40:13-23 (1997), Courseaux et al., Genomics, 37:354-365 (1996), Guru et al., Genomics, 42:436-445 (1997), Hosoda et al., Genes Cells, 2:345-357 (1997), James et al., Nat. Genet., 8:70-76 (1994), Kitamura et al., DNA Research, 4:281-289 (1997), Lemmens et al., Genomics, 44:94-100 (1997), Smith et al., Genome Res., 7:835-842 (1997)). Maps were integrated manually to identify markers mapping to the region containing Zmax1.

Primers for existing STSs were obtained from the GDB or literature references are listed in **Table 3** below. Thus, **Table 3** shows the STS markers used to prepare the physical map of the *Zmax1* gene region.

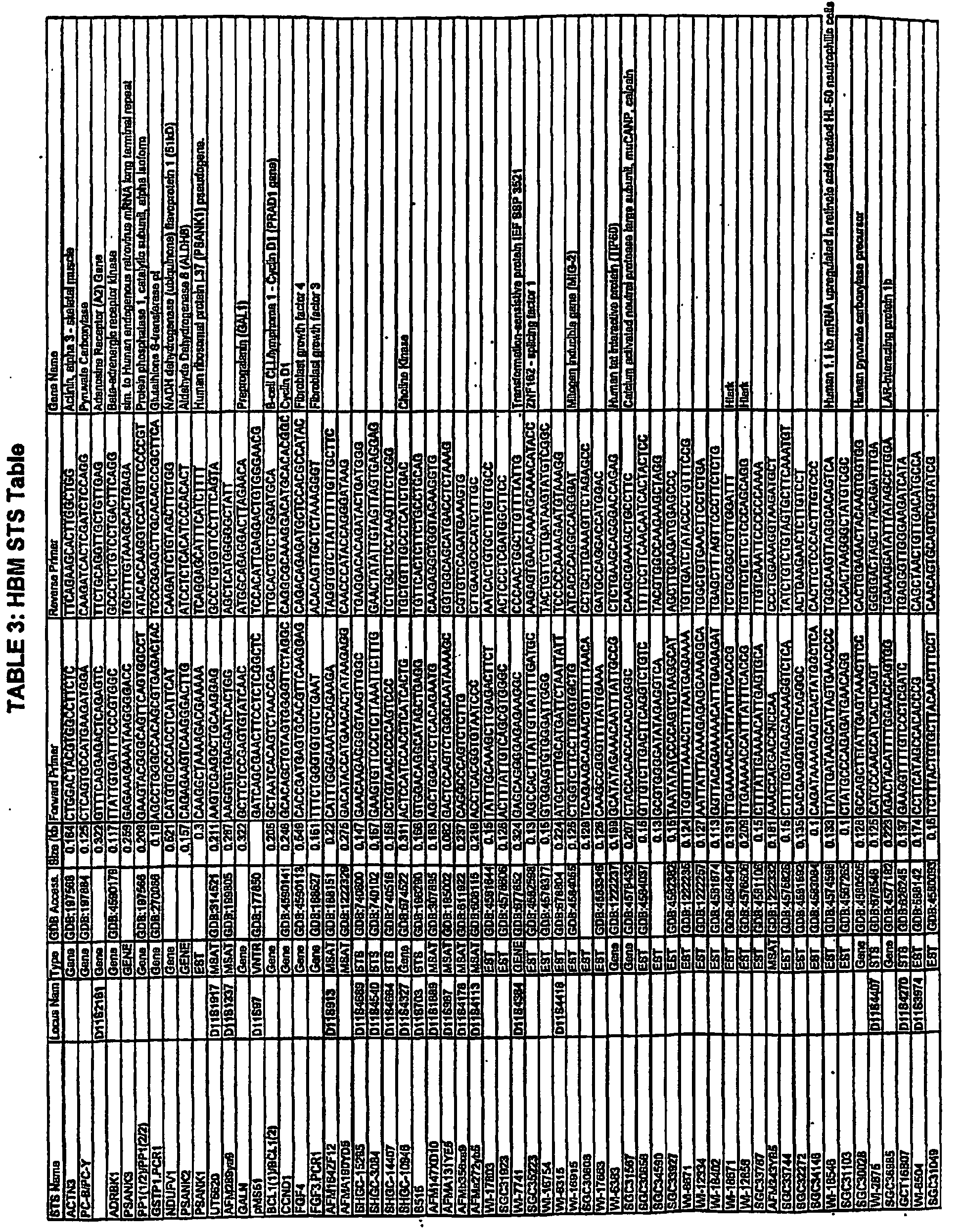

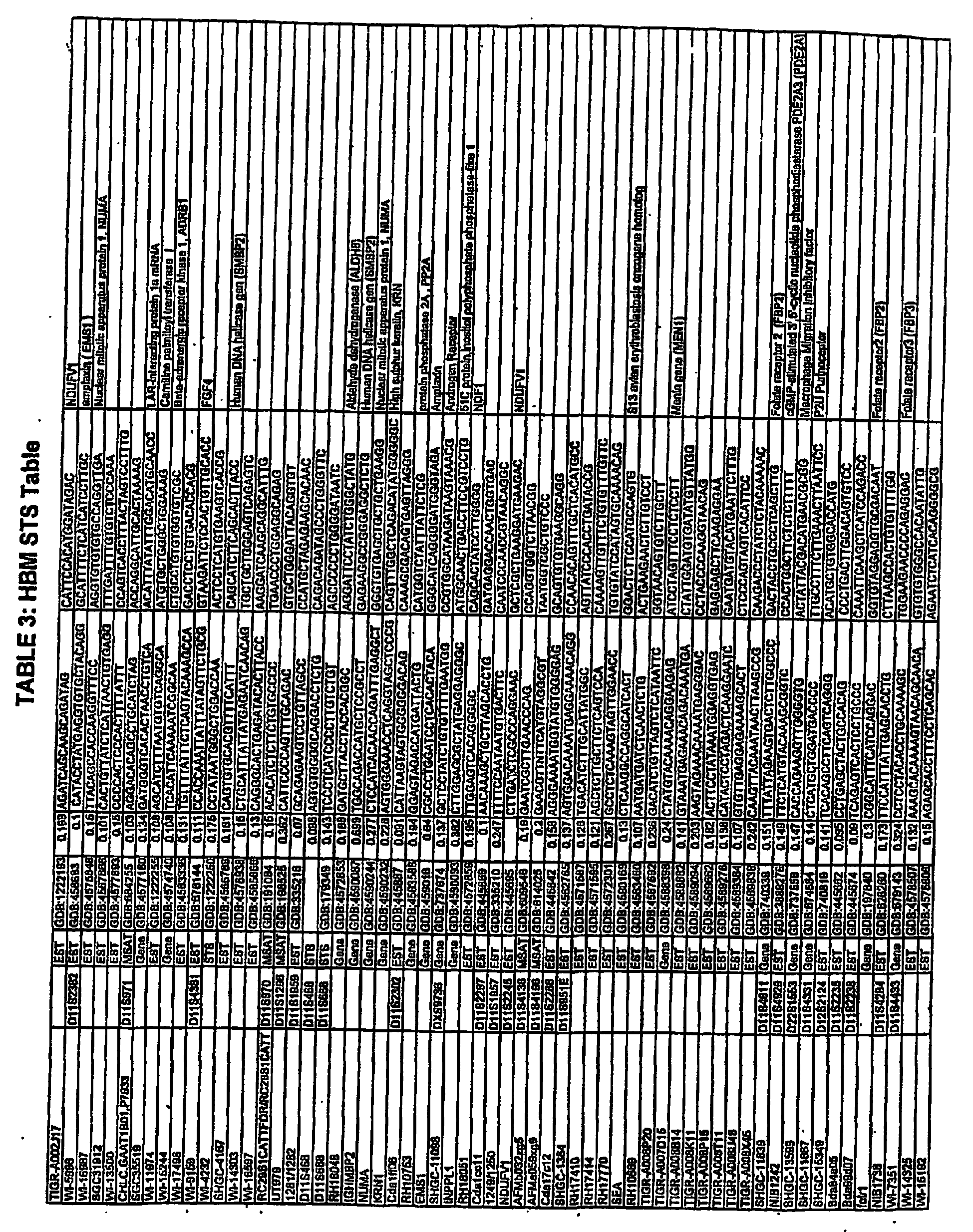

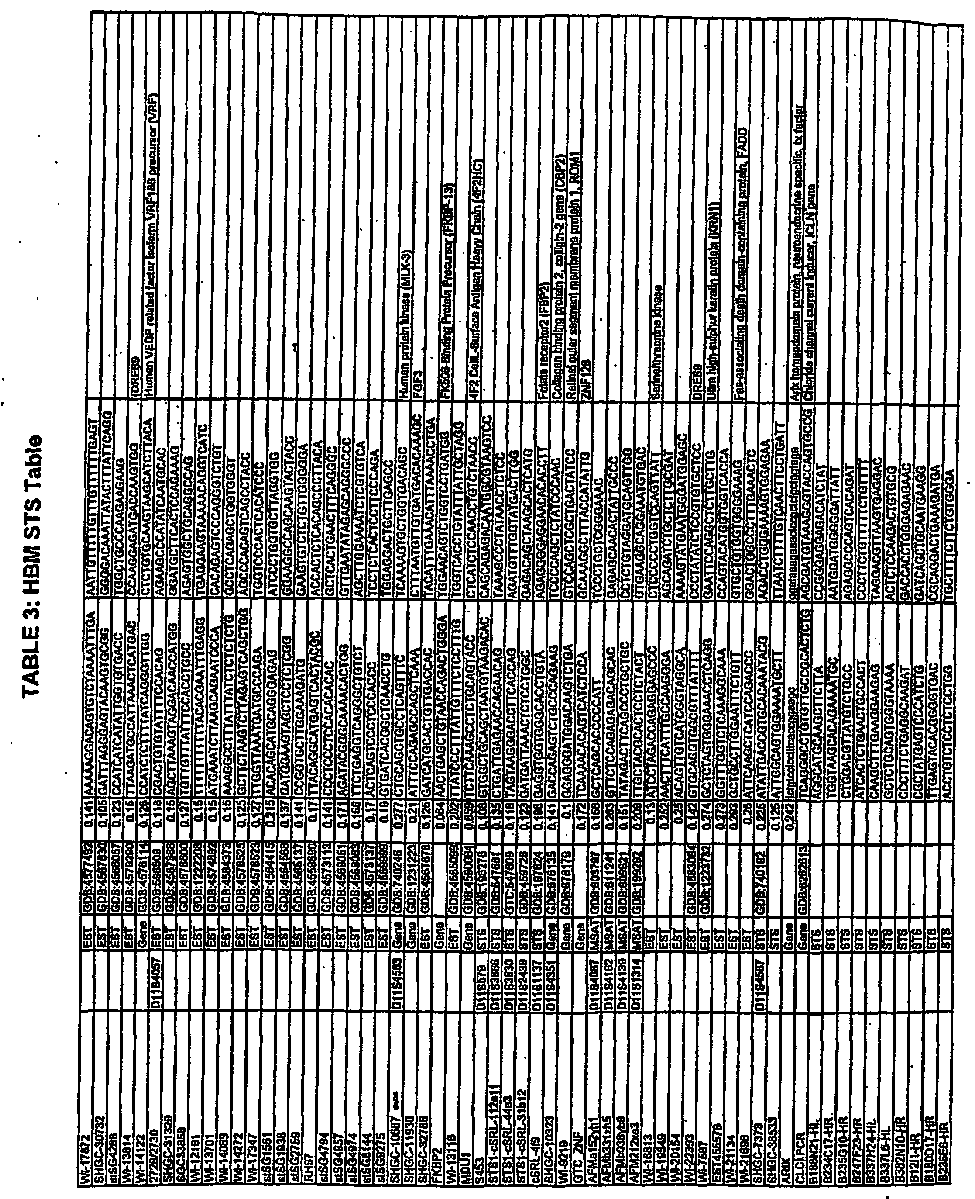

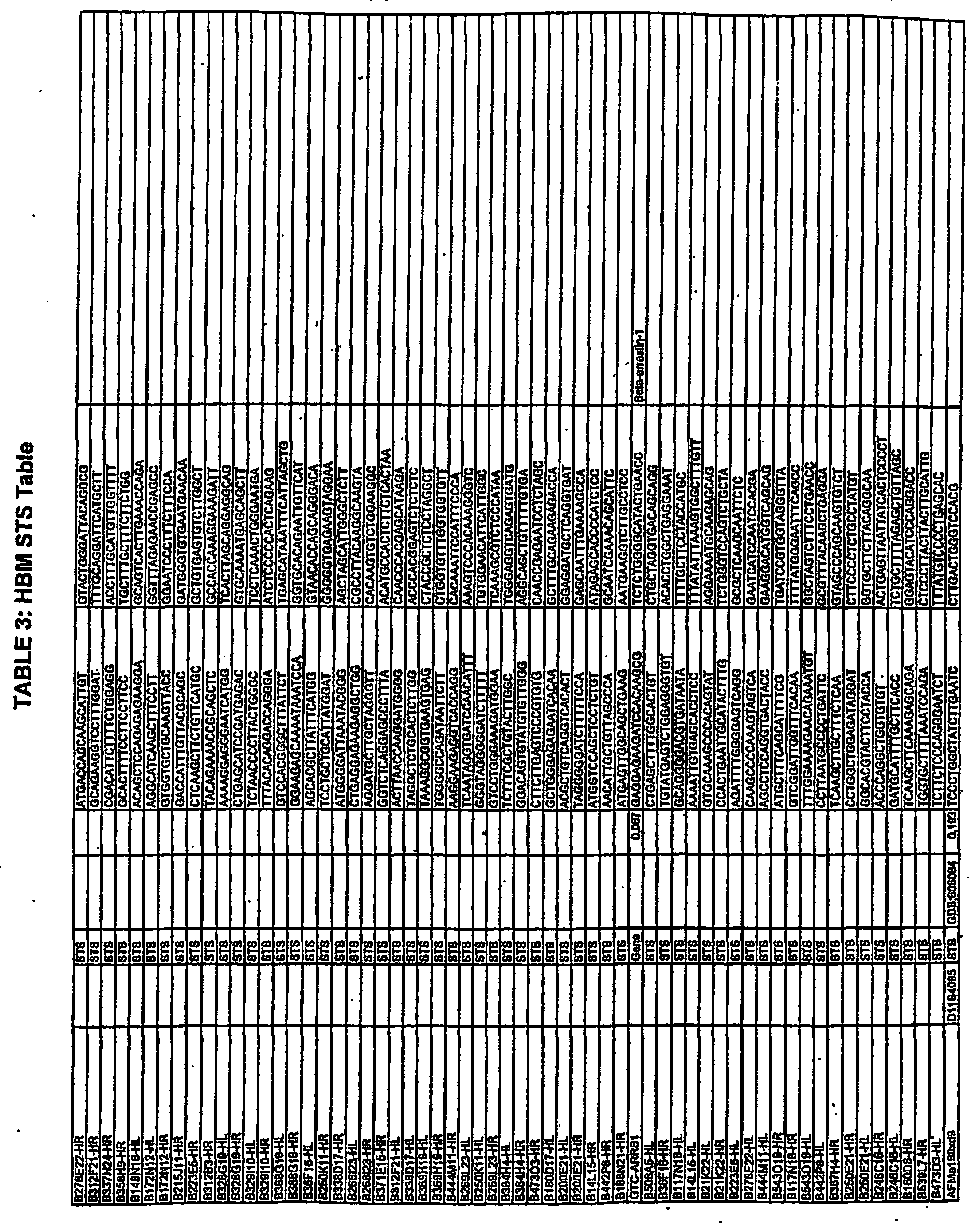

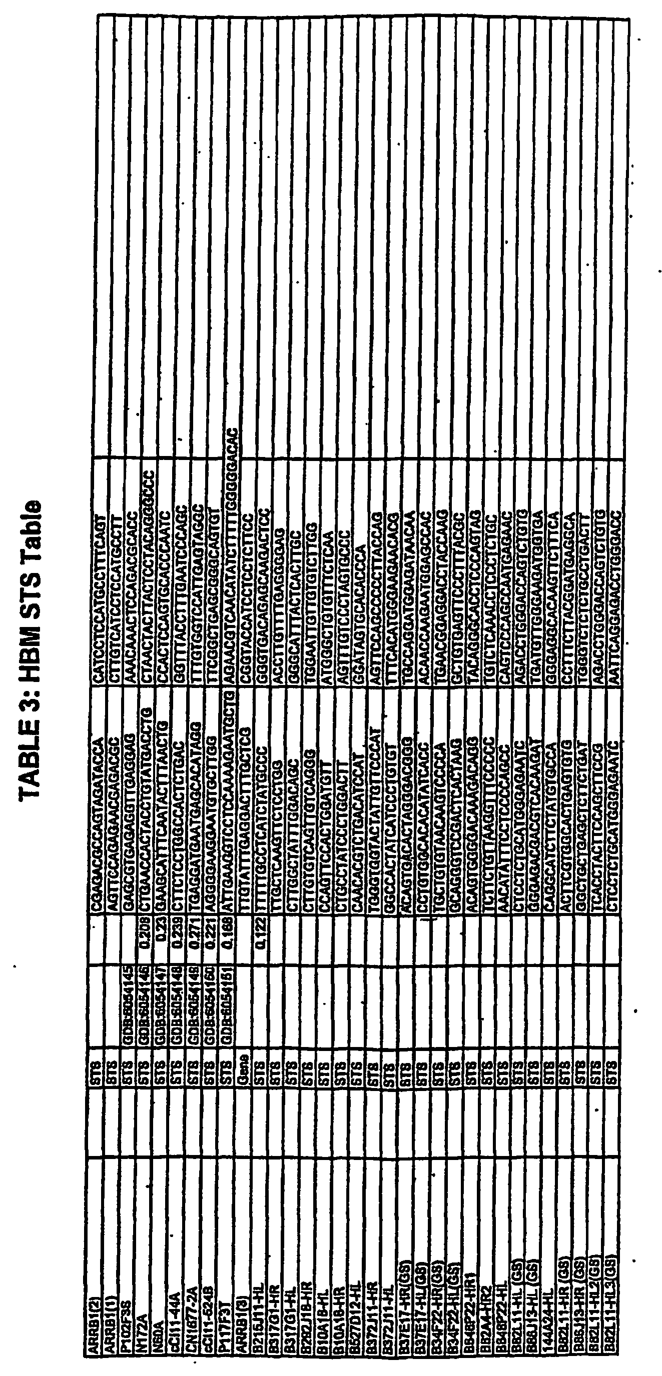

Novel STSs were developed either from publicly available genomic sequence or from sequence-derived BAC insert ends. Primers were chosen using a script which automatically performs vector and repetitive sequence masking using Cross_match (P. Green, U. of Washington) and subsequent primer picking using Primer3 (Rozen, Skaletsky (1996, 1997). Primer3 is available at www.genome.wi.mit. edu/genome_software/other/primer3.html.

Polymerase chain reaction (PCR) conditions for each primer pair were initially optimized with respect to MgCl₂ concentration. The standard buffer was 10 mM-Tris-HCl (pH 8.3), 50 mM KCl, MgCl₂, 0.2 mM each dNTP, 0.2 µM each primer, 2.7 ng/µl human DNA, 0.25 U of AmpliTaq (Perkin Elmer) and MgCl₂ concentrations of 1.0 mM, 1.5 mM, 2.0 mM or 2.4 mM. Cycling conditions included an initial denaturation at 94°C for 2 minutes followed by 40 cycles at 94°C for 15 seconds, 55°C for 25 seconds, and 72°C for 25 seconds followed by a final extension at 72°C for 3 minutes. Depending on the results from the initial round of optimization the conditions were further optimized if necessary. Variables included increasing the annealing temperature to 58°C or 60°C, increasing the cycle number to 42 and the annealing and extension times to 30 seconds, and using AmpliTaqGold (Perkin Elmer).

BAC clones (Kim et al., Genomics, 32:213-218 (1996), Shizuya et al., Proc. Natl. Acad. Sci. USA, 89:8794-8797 (1992)) containing STS markers of interest were obtained by PCR-based screening of DNA pools from a total human BAC library purchased from Research Genetics. DNA pools derived from library plates 1-596 were used corresponding to nine genomic equivalents of human DNA. The initial screening process involved PCR reactions of individual markers against superpools, i.e., a mixture of DNA derived from all BAC clones from eight 384-well library plates. For each positive superpool, plate (8), row (16) and column (24) pools were screened to identify a unique library address. PCR products were electrophoresed m 2% agarose gels (Sigma) containing 0.5 µg/ml ethidium bromide m 1X TBE at 150 volts for 45 min. The electrophoresis units used were the Model A3-1 systems from Owl Scientific Products. Typically, gels contained 10 tiers of lanes with 50 wells/tier. Molecular weight markers (100 bp ladder, Life Technologies, Bethesda, MD) were loaded at both ends of the gel. Images of the gels were captured with a Kodak DC40 CCD camera and processed with Kodak 1D software. The gel data were exported as tab delimited text files; names of the files included information about the library screened, the gel image files and the marker screened. These data were automatically imported using a customized Per1 script into Filemaker^{™} PRO (Claris Corp.) databases for data storage and analysis. In cases where incomplete or ambiguous clone address information was obtained, additional experiments were performed to recover a unique, complete library address.

Recovery of clonal BAC cultures from the library involved streaking out a sample from the library well onto LB agar (Maniatis et al., Molecular Cloning: A Laboratory Manual, Cold Spring Harbor Laboratory, Cold Spring Harbor, NY (1982)) containing 12.5 µg/ml chloramphenicol (Sigma). Two individual colonies and a portion of the initial streak quadrant were tested with appropriate STS markers by colony PCR for verification. Positive clones were stored in LB broth containing 12.5 µg/ml chloramphenicol and 15% glycerol at - 70°C.

Several different types of DNA preparation methods were used for isolation of BAC DNA. The manual alkaline lysis miniprep protocol listed below (Maniatis et al., Molecular Cloning: A Laboratory Manual, Cold Spring Harbor Laboratory, Cold Spring Harbor, NY (1982)) was successfully used for most applications, i.e., restriction mapping, CHEF gel analysis, FISH mapping, but was not successfully reproducible in endsequencing. The Autogen and Qiagen protocols were used specifically for BAC DNA preparation tor endsequencing purposes.

Bacteria were grown in 15 ml Terrific Broth containing 12.5 µg/ml chloramphenicol in a 50 ml conical tube at 37°C for 20 hrs with shaking at 300 rpm. The cultures were centrifuged in a Sorvall RT 6000 D at 3000 rpm (~1800 g) at 4°C for 15 min. The supernatant was then aspirated as completely as possible. In some cases cell pellets were frozen at -20°C at this step for up to 2 weeks. The pellet was then vortexed to homogenize the cells and minimize clumping. 250 µl of P1 solution (50 mM glucose, 15 mM Tris-HCl, pH 8,10 mM EDTA, and 100 µg/ml RNase A) was added and the mixture pipetted up and down to mix. The mixture was then transferred to a 2 ml Eppendorf tube. 350 µl of P2 solution (0.2 N NaOH, 1% SDS) was then added, the mixture mixed gently and incubated for 5 min. at room temperature. 350 µl ofP3 solution (3M KOAc, pH 5.5) was added and the mixture mixed gently until a white precipitate formed. The solution was incubated on ice for 5 min. and then centrifuged at 4 ° C in a microfuge for 10 min. The supernatant was transferred carefully (avoiding the white precipitate) to a fresh 2 ml Eppendorf tube, and 0.9 ml of isopropanol was added, the solution mixed and left on ice for 5 min. The samples were centrifuged for 10 min., and the supernatant removed carefully. Pellets were washed in 70% ethanol and air dried for 5 min, Pellets were resuspended in 200 µl of TE8 (10 mM Tris-HCl, pH 8.0,1.0 mM EDTA), and RNase A (Boehringer Mannheim) added to 100 µg/ml. Samples were incubated at 37°C for 30 min., then precipitated by addition of C₂H₃O₂Na·3H₂O to 0.5 M and 2 volumes of ethanol. Samples were centrifuged for 10 min., and the pellets washed with 70% ethanol followed by air drying and dissolving in 50 µl TE8. Typical yields for this DNA prep were 3-5 µg/15 ml bacterial culture. Ten to 15 µl were used for HindIII restriction analysis; 5 µl was used for NotI digestion and clone insert sizing by CHEF gel electrophoresis.

BACs were inoculated into 15 ml of 2X LB Broth containing 12.5 µg/ml chloramphenicol in a 50 ml conical tube. 4 tubes were inoculated for each clone. Cultures were grown overnight (~16 hr) at 37°C with vigorous shaking (>300 rpm). Standard conditions for BAC DNA isolation were followed as recommended by the Autogen 740 manufacturer. 3 ml samples of culture were placed into Autogen tubes for a total of 60 ml or 20 tubes per clone. Samples were dissolved finally in 100 µl TE8 with 15 seconds of shaking as part of the Autogen protocol. After the Autogen protocol was finished DNA solutions were transferred from each individual tube and pooled into a 2 ml Eppendorf tube. Tubes with large amounts of debris (carry over from the pelleting debris step) were avoided. The tubes were then rinsed with 0.5 ml of TE8 successively and this solution added to the pooled material. DNA solutions were stored at 4°C; clumping tended to occur upon freezing at - 20°C. This DNA was either used directly for restriction mapping, CHEF gel analysis or FISH mapping or was further purified as described below for use in endsequencing reactions.

The volume of DNA solutions was adjusted to 2 ml with TE8, samples were then mixed gently and heated at 65°C for 10 min. The DNA solutions were then centrifuged at 4°C for 5 min. and the supernatants transferred to a 15 ml conical tube. The NaCl concentration was then adjusted to 0.75 M (~0.3 ml of 5 M NaCl to the 2 ml sample). The total volume was then adjusted to 6 ml with Qiagen column equilibration buffer (Buffer QBT). The supernatant containing the DNA was then applied to the column and allowed to enter by gravity flow. Columns were washed twice with 10 ml of Qiagen Buffer QC. Bound DNA was then eluted with four separate 1 ml aliquots of Buffer QF kept at 65°C. DNA was precipitated with 0.7 volumes of isopropanol (~2.8 ml). Each sample was then transferred to 4 individual 2.2 ml Eppendorf tubes and incubated at room temperature for 2 hr or overnight. Samples were centrifuged in a microfuge for 10 min. at 4°C. The supernatant was removed carefully and 1 ml of 70% ethanol was added. Samples were centrifuged again and because the DNA pellets were often loose at this stage, the supernatant removed carefully. Samples were centrifuged again to concentrate remaining liquid which was removed with a micropipet tip. DNA pellets were then dried in a desiccator for 10 min. 20 *µ*l of sterile distilled and deionized H₂O was added to each tube which was then placed at 4°C overnight The four 20 *µ*l samples for each clone were pooled and the tubes rinsed with another 20 *µ*l of sterile distilled and deionized H₂O for a final volume of 100 *µ*l. Samples were then heated at 65 ° C for 5 min. and then mixed gently. Typical yields were 2-5 *µ*g/60 ml culture as assessed by NotI digestion and comparison with uncut lambda DNA.

3 ml ofLB Broth containing 12.5 *µ*g/ml of chloramphenicol was dispensed into autoclaved Autogen tubes. A single tube was used for each clone. For inoculation, glycerol stocks were removed from -70°C storage and placed on dry ice. A small portion of the glycerol stock was removed from the original tube with a sterile toothpick and transferred into the Autogen tube; the toothpick was left in the Autogen tube for at least two minutes before discarding. After inoculation the tubes were covered with tape making sure the seal was tight. When all samples were inoculated, the tube units were transferred into an Autogen rack holder and placed into a rotary shaker at 37 °C for 16-17 hours at 250 rpm. Following growth, standard conditions for BAC DNA preparation, as defined by the manufacturer, were used to program the Autogen. Samples were not dissolved in TE8 as part of the program and DNA pellets were left dry. When the program was complete, the tubes were removed from the output tray and 30 µl of sterile distilled and deionized H₂O was added directly to the bottom of the tube. The tubes were then gently shaken for 2-5 seconds and then covered with parafilm and incubated at room temperature for 1-3 hours. DNA samples were then transferred to an Eppendorf tube and used either directly for sequencing or stored at 4°C for later use.

### V. BAC Clone Characterization for Physical Mapping

DNA samples prepared either by manual alkaline lysis or the Autogen protocol were digested with HindIII for analysis of restriction fragment sizes. This data were used to compare the extent of overlap among clones. Typically 1-2 µg were used for each reaction. Reaction mixtures included: 1X Buffer 2 (New England Biolabs), 0.1 mg/ml bovine serum albumin (New England Biolabs), 50 µg/ml RNase A (Boehringer Mannheim), and 20 units of HindIII (New England Biolabs) in a final volume of 25 µl. Digestions were incubated at 37°C for 4-6 hours. BAC DNA was also digested with NotI for estimation of insert size by CHEF gel analysis (see below). Reaction conditions were identical to those for HindIII except that 20 units of NotI were used. Six µl of 6X Ficoll loading buffer containing bromphenol blue and xylene cyanol was added prior to electrophoresis.

HindIII digests were analyzed on 0.6% agarose (Seakem, FMC Bioproducts) in 1X TBE containing 0.5 µg/ml ethidium bromide. Gels (20 cm X 25 cm) were electrophoresed in a Model A4 electrophoresis unit (Owl Scientific) at 50 volts for 20-24 hrs. Molecular weight size markers included undigested lambda DNA, HindIII digested lambda DNA, and HaeIII digested _X174 DNA. Molecular weight markers were heated at 65 °C for 2 min. prior to loading the gel. Images were captured with a Kodak DC40 CCD camera and analyzed with Kodak 1D software.

NotI digests were analyzed on a CHEF DRII (BioRad) electrophoresis unit according to the manufacturer's recommendations. Briefly, 1% agarose gels (BioRad pulsed field grade) were prepared in 0.5X TBE, equilibrated for 30 minutes in the electrophoresis unit at 14°C, and electrophoresed at 6 volts/cm for 14 hrs with circulation. Switching times were ramped from 10 sec to 20 sec. Gels were stained after electrophoresis in 0.5 µg/ml ethidium bromide. Molecular weight markers included undigested lambda DNA, HindIII digested lambda DNA, lambda ladder PFG ladder, and low range PFG marker (all from New England Biolabs).

BAC DNA prepared either by the manual alkaline lysis or Autogen protocols were labeled for FISH analysis using a Bioprime labeling kit (BioRad) according to the manufacturer's recommendation with minor modifications. Approximately 200 ng of DNA was used for each 50 µl reaction. 3 µl were analyzed on a 2% agarose gel to determine the extent of labeling. Reactions were purified using a Sephadex G50 spin column prior to in *situ* hybridization. Metaphase FISH was performed as described (Ma et al., Cytogenet. Cell Genet., 74:266-271 (1996)).

### VI. BAC Endsequencing

The sequencing of BAC insert ends utilized DNA prepared by either of the two methods described above. The DYEnamic energy transfer primers and Dynamic Direct cycle sequencing kits from Amersham were used for sequencing reactions. Ready made sequencing mix including the M13 -40 forward sequencing primer was used (Catalog # US79730) for the T7 BAC vector terminus; ready made sequencing mix (Catalog # US79530) was mixed with the M13 -28 reverse sequencing primer (Catalog # US79339) for the SP6 BAC vector terminus. The sequencing reaction mixes included one of the four fluorescently labeled dye-primers, one of the four dideoxy termination mixes, dNTPs, reaction buffer, and Thermosequenase. For each BAC DNA sample, 3 µl of the BAC DNA sample was aliquoted to 4 PCR strip tubes. 2 µl of one of the four dye primer/termination mix combinations was then added to each of the four tubes. The tubes were then sealed and centrifuged briefly prior to PCR. Thermocycling conditions involved a 1 minute denaturation at 95°C,15 second annealing at 45 °C, and extension for 1 minute at 70°C for 35 total cycles. After cycling the plates were centrifuged briefly to collect all the liquid to the bottom of the tubes. 5*µ*l of sterile distilled and deionized H₂O was then added into each tube, the plates sealed and centrifuged briefly again. The four samples for each BAC were then pooled together. DNA was then precipitated by adding 1.5 *µ*l of 7.5 M NH₄OAc and 100 *µ*l of - 20°C 100% ethanol to each tube. Samples were mixed by pipetting up and down once. The plates were then sealed and incubated on ice for 10 minutes. Plates were centrifuged in a table top Haraeus centrifuge at 4000 rpm (3,290 xg) for 30 minutes at 4°C to recover the DNA. The supernatant was removed and excess liquid blotted onto paper towels. Pellets were washed by adding 100 µl of -20°C 70% ethanol into each tube and recentrifuging at 4000 rpm (3,290 xg) for 10 minutes at 4°C. The supernatant was removed and excess liquid again removed by blotting on a paper towel. Remaining traces of liquid were removed by placing the plates upside down over a paper towel and centrifuging only until the centrifuge reached 800 rpm. Samples were then air dried at room temperature for 30 min. Tubes were capped and stored dry at -20°C until electrophoresis. Immediately prior to electrophoresis the DNA was dissolved in 1.5 µl of Amersham loading dye. Plates were then sealed and centrifuged at 2000 rpm (825 xg). The plates were then vortexed on a plate shaker for 1-2 minutes. Samples were then recentrifuged at 2000 rpm (825 xg) briefly. Samples were then heated at 65 °C for 2 min. and immediately placed on ice. Standard gel electrophoresis was performed on ABI 377 fluorescent sequencers according to the manufacturer's recommendation.

### VII. Sub-cloning and Sequencing of HBM BAC DNA

The physical map of the *Zmax1* gene region provides a set of BAC clones that contain within them the *Zmax1* gene and the *HBM* gene. DNA sequencing of several of the BACs from the region has been completed. The DNA sequence data is a unique reagent that includes data that one skilled in the art can use to identify the *Zmax1* gene and the *HBM* gene, or to prepare probes to identify the gene(s), or to identify DNA sequence polymorphisms that identify the gene(s).

BAC DNA was isolated according to one of two protocols, either a Qiagen purification of BAC DNA (Qiagen, Inc. as described in the product literature) or a manual purification which is a modification of the standard alkaline lysis/Cesium Chloride preparation of plasmid DNA (see e.g., Ausubel et al., Current Protocols in Molecular Biology, John Wiley & Sons (1997)). Briefly for the manual protocol, cells were pelleted, resuspended in GTE (50 mM glucose, 25 mM Tris-Cl (pH 8), 10 mM EDTA) and lysozyme (50 mg/ml solution), followed by NaOH/SDS (1% SDS/0.2N NaOH) and then an ice-cold solution of 3 M KOAc (pH 4.5-4.8). RnaseA was added to the filtered supernatant, followed by Proteinase K and 20% SDS. The DNA was then precipitated with isopropanol, dried and resuspended in TE (10 mM Tris, 1 mM EDTA (pH 8.0)). The BAC DNA was further purified by Cesium Chloride density gradient centrifugation (Ausubel et al., Current Protocols in Molecular Biology, John Wiley & Sons (1997)).

Following isolation, the BAC DNA was sheared hydrodynamically using an HPLC (Hengen, Trends in Biochem. Sci., 22:273-274 (1997)) to an insert size of 2000-3000 bp. After shearing, the DNA was concentrated and separated on a standard 1% agarose gel. A single fraction, corresponding to the approximate size, was excised from the gel and purified by electroelution (Sambrook et al., Molecular Cloning: A Laboratory Manual, Cold Spring Harbor Laboratory, Cold Spring, NY (1989)).

The purified DNA fragments were then blunt-ended using T4 DNA polymerase. The blunt-ended DNA was then ligated to unique BstXI-linker adapters (5'-GTCTTCACCACGGGG and 5' GTGGTGAAGAC in 100-1000 fold molar excess). These linkers were complimentary to the BstXI-cut pMPX vectors (constructed by the inventors), while the overhang was not self-complimentary. Therefore, the linkers would not concatemerize nor would the cut-vector religate itself easily. The linker-adapted inserts were separated from the unincorporated linkers on a 1% agarose gel and purified using GeneClean (BIO 101, Inc.). The linker-adapted insert was then ligated to a modified pBlueScript vector to construct a "shotgun" subclone library. The vector contained an out-of-frame lacZ gene at the cloning site which became in-frame in the event that an adapter-dimer is cloned, allowing these to be avoided by their blue-color.

All subsequent steps were based on sequencing by ABI377 automated DNA sequencing methods. Only major modifications to the protocols are highlighted. Briefly, the library was then transformed into DH5α competent cells (Life Technologies, Bethesda, MD, DH5α transformation protocol). It was assessed by plating onto antibiotic plates containing ampicillin and IPTG/Xgal. The plates were incubated overnight at 37°C. Successful transformants were then used for plating of clones and picking for sequencing. The cultures were grown overnight at 37°C. DNA was purified using a silica bead DNA preparation (Ng et al., Nucl. Acids Res., 24:5045-5047 (1996)) method. In this manner, 25 µg of DNA was obtained per clone.

These purified DNA samples were then sequenced using ABI dye-terminator chemistry. The ABI dye terminator sequence reads were run on ABI377 machines and the data was directly transferred to UNIX machines following lane tracking of the gels. All reads were assembled using PHRAP (P. Green, Abstracts of DOE Human Genome Program Contractor-Grantee Workshop V, Jan. 1996, p.157) with default parameters and quality scores. The initial assembly was done at 6-fold coverage and yielded an average of 8-15 contigs. Following the initial assembly, missing mates (sequences from clones that only gave one strand reads) were identified and sequenced with ABI technology to allow the identification of additional overlapping contigs. Primers for walking were selected using a Genome Therapeutics program Pick_primer near the ends of the clones to facilitate gap closure. These walks were sequenced using the selected clones and primers. Data were reassembled with PHRAP into sequence contigs.

### VIII. Gene Identification by Computational Methods

Following assembly of the BAC sequences into contigs, the contigs were subjected to computational analyses to identify coding regions and regions bearing DNA sequence similarity to known genes. This protocol included the following steps.
1. Degap the contigs: the sequence contigs often contain symbols (denoted by a period symbol) that represent locations where the individual ABI sequence reads have insertions or deletions. Prior to automated computational analysis of the contigs, the periods were removed. The original data was maintained for future reference.
2. BAC vector sequences were "masked" within the sequence by using the program cross match (Phil Green, http:\\chimera.biotech.washington.edu\UWGC). Since the shotgun libraries construction detailed above leaves some BAC vector in the shotgun libraries, this program was used to compare the sequence of the BAC contigs to the BAC vector and to mask any vector sequence prior to subsequent steps. Masked sequences were marked by an "X" in the sequence files, and remained inert during subsequent analyses.
3. *E. coli* sequences contaminating the BAC sequences were masked by comparing the BAC contigs to the entire *E. coli* DNA sequence.
4. Repetitive elements known to be common in the human genome were masked using cross match. In this implementation of crossmatch, the BAC sequence was compared to a database of human repetitive elements (Jerzy Jerka, Genetic Information Research Institute, Palo Alto, CA). The masked repeats were marked by X and remained inert during subsequent analyses.
5. The location of exons within the sequence was predicted using the MZEF computer program (Zhang, Proc. Natl. Acad. Sci., 94:565-568 (1997)).
6. The sequence was compared to the publicly available unigene database (National Center for Biotechnology Information, National Library of Medicine, 38A, 8N905, 8600 Rockville Pike, Bethesda, MD 20894; www.ncbi.nlm.nih.gov) using the blastn2 algorithm (Altschul et al., Nucl. Acids Res., 25:3389-3402 (1997)). The parameters for this search were: E=0.05, v=50, B=50 (where E is the expected probability score cutoff, V is the number of database entries returned in the reporting of the results, and B is the number of sequence alignments returned in the reporting of the results (Altschul et al., J. Mol. Biol., 215:403-410 (1990)).
7. The sequence was translated into protein for all six reading frames, and the protein sequences were compared to a non-redundant protein database compiled from Genpept Swissprot PIR (National Center for Biotechnology Information, National Library of Medicine, 38A, 8N905, 8600 Rockville Pike, Bethesda, MD 20894; www.ncbi.nlm:nih.gov). The parameters for this search were E=0.05, V=50, B= 50, where E, V, and B are defined as above.
8. The BAC DNA sequence was compared to the database of the cDNA clones derived from direct selection experiments (described below) using blastn2 (Altschul et al., Nucl. Acids. Res., 25:3389-3402 (1997)). The parameters for this search were E=0.05, V=250, B=250, where E, V, and B are defined as above.
9. The BAC sequence was compared to the sequences of all other BACs from the HBM region on chromosome 11q12-13 using blastn2 (Altschul et al., Nucl. Acids. Res., 25:3389-3402 (1997)). The parameters for this search were E=0.05, V=50, B=50, where E, V, and B are defined as above.
10. The BAC sequence was compared to the sequences derived from the ends of BACs from the HBM region on chromosome 1 1q12-13 using blastn2 (Altschul et al., Nucl. Acids. Res., 25:3389-3402 (1997)). The parameters for this search were E=0.05, V=50, B=50, where E, V, and B are defined as above.
11. The BAC sequence was compared to the Genbank database (National Center for Biotechnology Information, National Library of Medicine, 38A, BN905, 8600 Rockville Pike, Bethesda, MD 20894; www.ncbi.nlm.nih.gov) using blastn2 (Altschul et al., Nucl. Acids. Res., 25:3389-3402 (1997)). The parameters for this search were E=0.05, V=50, B=50, where E, V, and B are defined as above.
12. The BAC sequence was compared to the STS division of Genbank database (National Center for Biotechnology Information, National Library of Medicine, 38A, 8N905, 8600 Rockville Pike, Bethesda, MD 20894; www.ncbi.nlm.nih.gov) using blastn2 (Altschul *et al.,* 1997). The parameters for this search were E=0.05, V=50, B= 50, where E, V, and B are defined as above.
13. The BAC sequence was compared to the Expressed Sequence (EST) Tag Genbank database (National Center for Biotechnology Information, National Library of Medicine, 38A, 8N905, 8600 Rockville Pike, Bethesda, MD 20894; www.ncbi.nlm.nih.gov) using blastn2 (Altschul et al., Nucl. Acids. Res., 25:3389-3402 (1997)). The parameters for this search were E=0.05, V=250, B=250, where E, V, and B are defined as above.

### IX. Gene Identification by Direct cDNA Selection

Primary linkered cDNA pools were prepared from bone marrow, calvarial bone, femoral bone, kidney, skeletal muscle, testis and total brain. Poly (A) + RNA was prepared from calvarial and femoral bone tissue (Chomczynski et al., Anal. Biochem., 162:156-159 (1987); D'Alessio et al., Focus, 9:1-4 (1987)) and the remainder of the mRNA was purchased from Clontech (Palo Alto, California). In order to generate oligo(dT) and random primed cDNA pools from the same tissue, 2.5 µg mRNA was mixed with oligo(dT) primer in one reaction and 2.5 µg mRNA was mixed with random hexamers in another reaction, and both were converted to first and second strand cDNA according to manufacturers recommendations (Life Technologies, Bethesda, MD). Paired phosphorylated cDNA linkers (see sequence below) were annealed together by mixing in a 1:1 ratio (10 µg each) incubated at 65°C for five minutes and allowed to cool to room temperature.

### Paired linkers oligo 1/2

OLIGO 1: 5'CTG AGC GGA ATT CGT GAG ACC3' (SEQ ID NO:12)
OLIGO 2: 5'TTG GTC TCA CGT ATT CCG CTC GA3' (SEQ ID NO:13)

### Paired linkers oligo3/4

OLIGO 3: 5'CTC GAG AAT TCT GGA TCC TC3' (SEQ ID NO: 14)
OLIGO 4: 5'TTG AGG ATC CAG AAT TCT CGA G3' (SEQ ID NO:15)

### Paired linkers oligo5/6

OLIGO 5: 5'TGT ATG CGA ATT CGC TGC GCG3' (SEQ ID NO:16)
OLIGO 6: 5'TTC GCG CAG CGA ATT CGC ATA CA3' (SEQ ID NO:17)

### Paired linkers oligo7/8

OLIGO 7: 5'GTC CAC TGA ATT CTC AGT GAG3' (SEQ ID NO:18)
OLIGO 8: 5'TTG TCA CTG AGA ATT CAG TGG AC3' (SEQ ID NO:19)

### Paired linkers oligol 1/12

OLIGO 11; 5'GAA TCC GAA TTC CTG GTC AGC3' (SEQ ID NO:20)
OLIGO 12: 5"TTG CTG ACC AGG AAT TCG GAT TC3' (SEQ ID NO:21)
Linkers were ligated to all oligo(dT) and random primed cDNA pools (see below) according to manufacturers instructions (Life Technologies, Bethesda, MD).

Oligo 1/2 was ligated to oligo(dT) and random primed cDNA pools prepared from bone marrow. Oligo 3/4 was ligated to oligo(dT) and random primed cDNA pools prepared from calvarial bone. Oligo 5/6 was ligated to oligo(dT) and random primed cDNA pools prepared from brain and skeletal muscle. Oligo 7/8 was ligated to oligo(dT) and random primed cDNA pools prepared from kidney. Oligo 11/12 was ligated to oligo(dT) and random primed cDNA pools prepared from femoral bone.

The cDNA pools were evaluated for length distribution by PCR amplification using 1 µl of a 1:1, 1:10, and 1:100 dilution of the ligation reaction, respectively. PCR reactions were performed in a Perkin Elmer 9600, each 25 µl volume reaction contained 1 µl of DNA, 10 mM Tris-HCl (pH 83), 50 mM KCl, 1.5 mM MgC12,0.001% gelatin, 200 mM each dNTPs, 10 *µ*M primer and 1 unit Taq DNA polymerase (Perkin Elmer) and was amplified under the following conditions: 30 seconds at 94°C, 30 seconds at 60°C and 2 minutes at 72°C for 30 cycles. The length distribution of the amplified cDNA pools were evaluated by electrophoresis on a 1% agarose geL The PCR reaction that gave the best representation of the random primed and oligo(dT) primed cDNA pools was scaled up so that ~2-3 µg of each cDNA pool was produced. The starting cDNA for the direct selection reaction comprised of 0.5 µg of random primed cDNAs mixed with 0.5 µg of oligo(dT) primed cDNAs.

The DNA from the 54 BACs that were used in the direct cDNA selection procedure was isolated using Nucleobond AX columns as described by the manufacturer (The Nest Group, Inc.).

The BACs were pooled in equimolar amounts and 1 µg of the isolated genomic DNA was labelled with biotin 16-UTP by nick translation in accordance with the manufacturers instructions (Boehringer Mannheim). The incorporation of the biotin was monitored by methods that could be practiced by one skilled in the art (Del Mastro and Lovett, Methods in Molecular Biology, Humana Press Inc., NJ (1996)).

Direct cDNA selection was performed using methods that could be practiced by one skilled in the art (Del Mastro and Lovett, Methods in Molecular Biology, Humana Press Inc., NJ (1996)). Briefly, the cDNA pools were multiplexed in two separate reactions: In one reaction cDNA pools from bone marrow, calvarial bone, brain and testis were mixed, and in the other cDNA pools from skeletal muscle, kidney and femoral bone were mixed. Suppression of the repeats, yeast sequences and plasmid in the cDNA pools was performed to a Cot of 20. 100 ng of biotinylated BAC DNA was mixed with the suppressed cDNAs and hybridized in solution to a Cot of 200. The biotinylated DNA and the cognate cDNAs was captured on streptavidin-coated paramagnetic beads. The beads were washed and the primary selected cDNAs were eluted. These cDNAs were PCR amplified and a second round of direct selection was performed. The product of the second round of direct selection is referred to as the secondary selected material. A Galanin cDNA clone, previously shown to map to 11q12-13 (Evans, Genomics, 18:473-477 (1993)), was used to monitor enrichment during the two rounds of selection.

The secondary selected material from bone marrow, calvarial bone, femoral bone, kidney, skeletal muscle, testis and total brain was PCR amplified using modified primers of oligos 1, 3, 5, 7 and 11, shown below, and cloned into the UDG vector pAMP10 (Life Technologies, Bethesda, MD), in accordance with the manufacturer's recommendations. Modified primer sequences:
Oligol-CUA : 5'CUA CUA CUA CUA CTG AGC GGA ATT CGT GAG ACC3' (SEQ ID NO:22)
Oligo3-CUA: 5'CUA CUA CUA CUA CTC GAG AAT TCT GGA TCC TC3' (SEQ ID NO:23)
Oligo5-CUA: 5'CUA CUA CUA CUA TGT ATG CGA ATT CGC TGC GCG3' (SEQ ID NO:24)
Oligo7-CUA: 5'CUA CUA CUA CUA GTC CAC TGA ATT CTC AGT GAG3' (SEQ ID NO:25)
Oligol 1-CUA: 5'CUA CUA CUA CUA GAA TCC GAA TTC CTG GTC AGC3' (SEQ ID No:26)

The cloned secondary selected material, from each tissue source, was transformed into MAX Efficiency DH5a Competent Cells (Life Technologies, Bethesda, MD) as recommended by the manufacturer. 384 colonies were picked from each transformed source and arrayed into four 96 well microtiter plates.

All secondarily selected cDNA clones were sequenced using M13 dye primer terminator cycle sequencing kit (Applied Biosystems), and the data collected by the ABI 377 automated fluorescence sequencer (Applied Biosystems).

All sequences were analyzed using the BLASTN, BLASTX and FASTA programs (Altschul et al., J. Mol. Biol., 215:403-410 (1990), Altschul et al., Nucl. Acids. Res., 25:3389-3402 (1997)). The cDNA sequences were compared to a database containing sequences derived from human repeats, mitochondrial DNA, ribosomal RNA, *E. coli* DNA to remove background clones from the dataset using the program cross_match. A further round of comparison was also performed using the program BLASTN2 against known genes (Genbank) and the BAC sequences from the HBM region. Those cDNAs that were >90% homologous to these sequences were filed according to the result and the data stored in a database for further analysis. cDNA sequences that were identified but did not have significant similarity to the BAC sequences from the HBM region or were eliminated by cross_match were hybridized to nylon membranes which contained the BACs from the HBM region, to ascertain whether they hybridized to the target.

Hybridization analysis was used to map the cDNA-clones to the BAC target that selected them. The BACs that were identified from the HBM region were arrayed and grown into a 96 well microtiter plate. LB agar containing 25 µg/ml kanamycin was poured into 96 well microtiter plate lids. Once the agar had solidified, pre-cut Hybond N+ nylon membranes (Amersham) were laid on top of the agar and the BACs were stamped onto the membranes in duplicate using a hand held 96 well replica plater (V&P Scientific, Inc.). The plates were incubated overnight at 37°C. The membranes were processed according to the manufacturers , recommendations.

The cDNAs that needed to be mapped by hybridization were PCR amplified using the relevant primer (oligos 1, 3, 5, 7 and 11) that would amplify that clone. For this PCR amplification, the primers were modified to contain a linkered digoxigenin molecule at the 5' of the oligonucleotide. The PCR amplification was performed under the same conditions as described in Preparation of cDNA Pools (above). The PCR products were evaluated for quality and quantity by electrophoresis on a 1% agarose gel by loading 5 µl of the PCR reaction. The nylon membranes containing the stamped BACs were individually prehybridized in 50 ml conical tubes containing 10 ml of hybridization solution (5x SSPE, 0.5x Blotto, 2.5% SDS and 1 mM EDTA (pH 8.0)). The 50 ml conical tubes were placed in a rotisserie oven (Robbins Scientific) for 2 hours at 65°C. 25 ng of each cDNA probe was denatured and added into individual 50 ml conical tubes containing the nylon membrane and hybridization solution. The hybridization was performed overnight at 65°C. The filters were washed for 20 minutes at 65°C in each of the following solutions: 3x SSPE, 0.1% SDS; 1x SSPE, 0.1% SDS and 0.1x SSPE, 0.1% SDS.

The membranes were removed from the 50 ml conical tubes and placed in a dish. Acetate sheets were placed between each membrane to prevent them from sticking to each other. The incubation of the membranes with the Anti-DIG-AP and CDP-Star was performed according to manufacturers recommendations (Boehringer Mannheim). The membranes were wrapped in Saran wrap and exposed to Kodak Bio-Max X-ray film for 1 hour.

### X. cDNA Cloning and Expression Analysis

To characterize the expression of the genes identified by direct cDNA selection and genomic DNA sequencing in comparison to the publicly available databases, a series of experiments were performed to further characterize the genes in the HBM region. First, oligonucleotide primers were designed for use in the polymerase chain reaction (PCR) so that portions of a cDNA, EST, or genomic DNA could be amplified from a pool of DNA molecules (a cDNA library) or RNA population (RT-PCR and RACE). The PCR primers were used in a reaction containing genomic DNA to verify that they generated a product of the size predicted based on the genomic (BAC) sequence. A number of cDNA libraries were then examined for the presence of the specific cDNA or EST. The presence of a fragment of a transcription unit in a particular cDNA library indicates a high probability that additional portions of the same transcription unit will be present as well.

A critical piece of data that is required when characterizing novel genes is the length, in nucleotides, of the processed transcript or messenger RNA (mRNA). One skilled in the art primarily determines the length of an mRNA by Northern blot hybridization (Sambrook et al., Molecular Cloning: A Laboratory Manual, Cold Spring Harbor Laboratory, Cold Spring Harbor NY (1989)). Groups of ESTs and direct-selected cDNA clones that displayed significant sequence similarity to sequenced BACs in the critical region were grouped for convenience into approximately 30 kilobase units. Within each 30 kilobase unit there were from one up to fifty ESTs and direct-selected cDNA clones which comprised one or more independent transcription units. One or more ESTs or direct-selected cDNAs were used as hybridization probes to determine the length of the mRNA in a variety of tissues, using commercially available reagents (Multiple Tissue Northern blot; Clontech, Palo Alto, California) under conditions recommended by the manufacturer.

Directionally cloned cDNA libraries from femoral bone, and calvarial bone tissue were constructed by methods familiar to one skilled in the art (for example, Soares in Automated DNA Sequencing and Analysis, Adams, Fields and Venter, Eds., Academic Press, NY, pages 110-114 (1994)). Bones were initially broken into fragments with a hammer, and the small pieces were frozen in liquid nitrogen and reduced to a powder in a tissue pulverizer (Spectrum Laboratory Products). RNA was extracted from the powdered bone by homogenizing the powdered bone with a standard Acid Guanidinium Thiocyanate-Phenol-Chloroform extraction buffer (e.g. Chomczynski and Sacchi, Anal. Biochem., 162:156-159 (1987)) using a polytron homogenizer (Brinkman Instruments). Additionally, human brain and lung total RNA was purchased from Clontech. PolyA RNA was isolated from total RNA using dynabeads-dT according to the manufacturer's recommendations (Dynal, Inc.).

First strand cDNA synthesis was initiated using an oligonucleotide primer with the sequence: 5'-AACTGGAAGAATTCGCGGCCGCAGGAATTTTTTTTTTTTTTTTTT-3' (SEQ ID NO:27). This primer introduces a NotI restriction site (underlined) at the 3' end of the cDNA. First and second strand synthesis were performed using the "one-tube" cDNA synthesis kit as described by the anufacturer (Life Technologies, Bethesda, MD). Double stranded cDNAs were treated with T4 polynucleotide kinase to ensure that the ends of the molecules were blunt (Soares, in Automated DNA Sequencing and Analysis, Adams, Fields and Venter, Eds., Academic Press, NY, pages 110-114 (1994)), and the blunt ended cDNAs were then size selected by a Biogel column (Huynh et al.. in DNA Cloning, Vol. 1, Glover, Ed., IRL Press, Oxford, pages 49-78 (1985)) or with a size-sep 400 sepharose column (Pharmacia, catalog # 27-5105-01). Only cDNAs of 400 base pairs or longer were used in subsequent steps. EcoRI adapters (sequence: 5' OH-AATTCGGCACGAG-OH 3' (SEQ ID NO:28), and 5' p-CTCGTGCCG-OH 3' (SEQ ID NO:29)) were then ligated to the double stranded cDNAs by methods familiar to one skilled in the art (Soares, 1994). The EcoRI adapters were then removed from the 3' end of the cDNA by digestion with NotI (Soares, 1994). The cDNA was then ligated into the plasmid vector pBluescript II KS+ (Stratagene, La Jolla, California), and the ligated material was transformed into *E. coli* host DH10B or DH12S by electroporation methods familiar to one skilled in the art (Soares, 1994). After growth overnight at 37°C, DNA was recovered from the *E. coli* colonies after scraping the plates by processing as directed for the Mega-prep kit (Qiagen, Chatsworth, California). The quality of the cDNA libraries was estimated by counting a portion of the total numbers of primary transformants and determining the average insert size and the percentage of plasmids with no cDNA insert. Additional cDNA libraries (human total brain, heart, kidney, leukocyte, and fetal brain) were purchased from Life Technologies, Bethesda, MD.

cDNA libraries, both oligo (dT) and random hexamer (N₆) primed, were used for isolating cDNA clones transcribed within the HBM region: human bone, human brain, human kidney and human skeletal muscle (all cDNA libraries were made by the inventors, except for skeletal muscle (dT) and kidney (dT) cDNA libraries). Four 10 x 10 arrays of each of the cDNA libraries were prepared as follows: the cDNA libraries were titered to 2.5 x 10⁶ using primary transformants. The appropriate volume of frozen stock was used to inoculate 2 L of LB/ampicillin (100 mg/ml). This inoculated liquid culture was aliquotted into 400 tubes of 4 ml each. Each tube contained approximately 5000 cfu. The tubes were incubated at 30°C overnight with gentle agitation. The cultures were grown to an OD of 0.7-0.9. Frozen stocks were prepared for each of the cultures by aliquotting 100 µl of culture and 300 µl of 80% glycerol. Stocks were frozen in a dry ice/ethanol bath and stored at -70°C. The remaining culture was DNA prepared using the Qiagen (Chatsworth, CA) spin miniprep kit according to the manufacturer's instructions. The DNAs from the 400 cultures were pooled to make 80 column and row pools. The cDNA libraries were determined to contain HBM cDNA clones of interest by PCR. Markers were designed to amplify putative exons. Once a standard PCR optimization was performed and specific cDNA libraries were determined to contain cDNA clones of interest, the markers were used to screen the arrayed library. Positive addresses indicating the presence of cDNA clones were confirmed by a second PCR using the same markers.

Once a cDNA library was identified as likely to contain cDNA clones corresponding to a specific transcript of interest from the HBM region, it was manipulated to isolate the clone or clones containing cDNA inserts identical to the EST or direct-selected cDNA of interest. This was accomplished by a modification of the standard "colony screening" method (Sambrook et al., Molecular Cloning : A Laboratory Manual, Cold Spring Harbor Laboratory, Cold Spring Harbor NY (1989)). Specifically, twenty 150 mm LB+ampicillin agar plates were spread with 20,000 colony forming units (cfu) of cDNA library and the colonies allowed to grow overnight at 37°C. Colonies were transferred to nylon filters (Hybond from Amersham, or equivalent) and duplicates prepared by pressing two filters together essentially as described (Sambrook et al., Molecular Cloning: A Laboratory Manual, Cold Spring Harbor Laboratory, Cold Spring Harbor NY (1989)). The "master" plate was then incubated an additional 6-8 hours to allow the colonies to grow back. The DNA from the bacterial colonies was then affixed to the nylon filters by treating the filters sequentially with denaturing solution (0.5 N NaOH, 1.5 M NaCl) for two minutes, neutralization solution (0.5 M Tris-Cl pH 8.0,1.5 M NaCl) for two minutes (twice). The bacterial colonies were removed from the filters by washing in a solution of 2X SSC/0.1% SDS for one minute while rubbing with tissue paper. The filters were air dried and baked under vacuum at 80°C for 1-2 hours.

A cDNA hybridization probe was prepared by random hexamer labeling (Fineberg and Vogelstein, Anal. Biochem., 132:6-13 (1983)) or by including gene-specific primers and no random hexamers in the reaction (for small fragments). Specific activity was calculated and was >5X10⁸ cpm/10⁸ µg of cDNA. The colony membranes were then prewashed in 10 mM Tris-Cl pH 8.0, 1 M NaCl, 1 mM EDTA, 0.1% SDS for 30 minutes at 55°C. Following the prewash, the filters were prehybridized in > 2 ml/filter of 6X SSC, 50 % deionized formamide, 2% SDS, 5X Denhardt's solution, and 100 mg/ml denatured salmon sperm DNA, at 42 °C for 30 minutes. The filters were then transferred to hybridization solution (6X SSC, 2% SDS, 5X Denhardt's, 100 mg/ml denatured salmon sperm DNA) containing denatured α³²P-dCTP-labelled cDNA probe and incubated at 42°C for 16-18 hours.

After the 16-18 hour incubation, the filters were washed under constant agitation in 2X SSC, 2% SDS at room temperature for 20 minutes, followed by two washes at 65°C for 15 minutes each. A second wash was performed in 0.5 X SSC, 0.5% SDS for 15 minutes at 65°C. Filters were then wrapped in plastic wrap and exposed to radiographic film for several hours to overnight. After film development, individual colonies on plates were aligned with the autoradiograph so that they could be picked into a 1 ml solution ofLB Broth containing ampicillin. After shaking at 37°C for 1-2 hours, aliquots of the solution were plated on 150 mm plates for secondary screening. Secondary screening was identical to primary screening (above) except that it was performed on plates containing ~250 colonies so that individual colonies could be clearly identified for picking.

After colony screening with radiolabeled probes yielded cDNA clones, the clones were characterized by restriction endonuclease cleavage, PCR, and direct sequencing to confirm the sequence identity between the original probe and the isolated clone. To obtain the full-length cDNA, the novel sequence from the end of the clone identified was used to probe the library again. This process was repeated until the length of the cDNA cloned matches that estimated to be full-length by the northern blot analysis.

RT-PCR was used as another method to isolate full length clones. The cDNA was synthesized and amplified using a "Superscript One Step RT-PCR" kit (Life Technologies, Gaithersburg, MD). The procedure involved adding 1.5 µg of RNA to the following: 25 µl of reaction mix provided which is a proprietary buffer mix with MgSO₄ and dNTP's, 1 µl sense primer (10 µM) and 1 µl anti-sense primer (10 µM), 1 µl reverse transcriptase and Taq DNA polymerase mix provided and autoclaved water to a total reaction mix of 50 µl. The reaction was then placed in a thermocycler for cycle at 50°C for 15 to 30 minutes, then 94°C for 15 seconds, 55-60°C for 30 seconds and 68-72°C for 1 minute per kilobase of anticipated product and finally 1 cycle of 72°C for 5-10 minutes. The sample was analyzed on an agarose gel. The product was excised from the gel and purified from the gel (GeneClean, Bio 101). The purified product was cloned in pCTNR (General Contractor DNA Cloning System, 5 Prime - 3 Prime, Inc.) and sequenced to verify that the clone was specific to the gene of interest.

Rapid Amplification of cDNA ends (RACE) was performed following the manufacturer's instructions using a Marathon cDNA Amplification Kit (Clontech, Palo Alto, CA) as a method for cloning the 5' and 3' ends of candidate genes. cDNA pools were prepared from total RNA by performing first strand synthesis, where a sample of total RNA sample was mixed with a modified oligo (dT) primer, heated to 70°C, cooled on ice and followed by the addition of: 5x first strand buffer, 10 mM dNTP mix, and AMV Reverse Transcriptase (20 U/*µ*l). The tube was incubated at 42°C for one hour and then the reaction tube was placed on ice. For second strand synthesis, the following components were added directly to the reaction tube: 5x second strand buffer, 10 mM dNTP mix, sterile water, 20x second strand enzyme cocktail and the reaction tube was incubated at 16°C for 1.5 hours. T4 DNA Polymerase was added to the reaction tube and incubated at 16°C for 45 minutes. The second-strand synthesis was terminated with the addition of an EDTA/Glycogen mix. The sample was subjected to a phenol/chloroform extraction and an ammonium acetate precipitation. The cDNA pools were checked for quality by analyzing on an agarose gel for size distribution. Marathon cDNA adapters (Clontech) were then ligated onto the cDNA ends. The specific adapters contained priming sites that allowed for amplification of either 5' or 3' ends, depending on the orientation of the gene specific primer (GSP) that was chosen. An aliquot of the double stranded cDNA was added to the following reagents: 10 µM Marathon cDNA adapter, 5x DNA ligation buffer, T4 DNA ligase. The reaction was incubated at 16°C overnight. The reaction was heat inactivated to terminate the reaction. PCR was performed by the addition of the following to the diluted double stranded cDNA pool: 10x cDNA PCR reaction buffer, 10 µM dNTP mix, 10 µM GSP, 10 µM AP1 primer (kit), 50x Advantage cDNA Polymerase Mix. Thermal Cycling conditions were 94°C for 30 seconds, 5 cycles of 94°C for 5 seconds, 72°C for 4 minutes, 5 cycles of 94°C for 5 seconds, 70°C for 4 minutes, 23 cycles of 94°C for 5 seconds, 68°C for 4 minutes. After the first round of PCR was performed using the GSP to extend to the end of the adapter to create the adapter primer binding site, exponential amplification of the specific cDNA of interest was observed. Usually a second nested PCR is performed to confirm the specific cDNA. The RACE product was analyzed on an agarose gel and then excised and purified from the gel (GeneClean, BIO 101). The RACE product was then cloned into pCTNR (General Contractor DNA Cloning System, 5' - 3', Inc.) and the DNA sequence determined to verify that the clone is specific to the gene of interest.

### XI. Mutation Analysis

Comparative genes were identified using the above procedures and the exons from each gene were subjected to mutation detection analysis. Comparative DNA sequencing was used to identify polymorphisms in HBM candidate genes from chromosome 11q12-13. DNA sequences for candidate genes were amplified from patient lymphoblastoid cell lines.

The inventors developed a method based on analysis of direct DNA sequencing of PCR products amplified from candidate regions to search for the causative polymorphism. The procedure consisted of three stages that used different subsets ofHBM family to find segregating polymorphisms and a population panel to assess the frequency of the polymorphisms. The family resources result from a single founder leading to the assumption that all affected individuals will share the same causative polymorphism.

Candidate regions were first screened in a subset of the HBM family consisting of the proband, daughter, and her mother, father and brother. Monochromosomal reference sequences were produced concurrently and used for comparison. The mother and daughter carried the HBM polymorphism in this nuclear family, providing the ability to monitor polymorphism transmission. The net result is that two HBM chromosomes and six non-HBM chromosomes were screened. This allowed exclusion of numerous frequent alleles. Only alleles exclusively present in the affected individuals passed to the next level of analysis.

Polymorphisms that segregated exclusively with the HBM phenotype in this original family were then re-examined in an extended portion of the HBM pedigree consisting of two additional nuclear families. These families consisted of five HBM and three unaffected individuals. The HBM individuals in this group included the two critical crossover individuals, providing the centromeric and telomeric boundaries of the critical region. Tracking the heredity of polymorphisms between these individuals and their affected parents allowed for further refining of the critical region. This group brought the total of HBM chromosomes screened to seven and the total of non-HBM chromosomes to seventeen.

When a given polymorphism continued to segregate exclusively with the HBM phenotype in the extended group, a population panel was then examined. This panel of 84 persons consisted of 42 individuals known to have normal bone mineral density and 42 individuals known to be unrelated but with untyped bone mineral density. Normal bone mineral density is within two standard deviations of BMD Z score 0. The second group was from the widely used CEPH panel of individuals. Any segregating polymorphisms found to be rare in this population were subsequently examined on the entire HBM pedigree and a larger population.

Polymerase chain reaction (PCR) was used to generate sequencing templates from the HBM family's DNA and monochromosomal controls. Enzymatic amplification of genes within the HBM region on 1 q12-13 was accomplished using the PCR with oligonucleotides flanking each exon as well as the putative 5' regulatory elements of each gene. The primers were chosen to amplify each exon as well as 15 or more base pairs within each intron on either side of the splice. All PCR primers were made as chimeras to facilitate dye primer sequencing. The M13-21F (5'- GTA A CGA CGG CCA GT -3') (SEQ m NO:30) and-28REV (5'- AAC AGC TAT GAC CAT G -3') (SEQ ID NO:31) primer binding sites were built on to the 5' end of each forward and reverse PCR primer, respectively, during synthesis. 150 ng of genomic DNA was used in a 50 µl PCR with 2UAmpliTaq, 500 nM primer and 125 µM dNTP. Buffer and cycling conditions were specific to each primer set. TaqStart antibody (Clontech) was used for hot start PCR to minimize primer dimer formation. 10% of the product was examined on an agarose gel. The appropriate samples were diluted 1:25 with deionized water before sequencing.

Each PCR product was sequenced according to the standard Energy Transfer primer (Amersham) protocol. All reactions took place in 96 well trays. 4 separate reactions, one each for A, C, G and T were performed for each template. Each reaction included 2 µl of the sequencing reaction mix and 3 µl of diluted template. The plates were then heat sealed with foil tape and placed in a thermal cycler and cycled according to the manufacturer's recommendation. After cycling, the 4 reactions were pooled. 3 µl of the pooled product was transferred to a new 96 well plate and 1 µl of the manufacturer's loading dye was added to each well. All 96 well pipetting procedures occurred on a Hydra 96 pipetting station (Robbins Scientific, USA). 1 µl of pooled material was directly loaded onto a 48 lane gel running on an ABI 377 DNA sequencer for a 10 hour, 2.4 kV run.

*Polyphred* (University of Washington) was used to assemble sequence sets for viewing with *Consed* (University of Washington). Sequences were assembled in groups representing all relevant family members and controls for a specified target region. This was done separately for each of the three stages. Forward and reverse reads were included for each individual along with reads from the monochromosomal templates and a color annotated reference sequence. *Polyphred* indicated potential polymorphic sites with a purple flag. Two readers independently viewed each assembly and assessed the validity of the purple-flagged sites.

A total of 23 exons present in the mature mRNA and several other portions of the primary transcript were evaluated for heterozygosity in the nuclear family of two HBM-affected and two unaffected individuals. Twenty-five single nucleotide polymorphisms (SNPs) were identified, as shown in the table below.

**TABLE 4: Single Nucleotide Polymorphisms in the Zmax1 gene and Environs**

| **Exon Name** | **Location** | **Base Change** |
|---|---|---|
| b200e21-h_Contig1_1.nt | 69169 (309G) | C/A |
| b200e21-h_Contig4_12.nt | 27402 (309G) | A/G |
| b200e21-h_Contig4_13.nt | 27841 (309G) | T/C |
| b200e21-h_Contig4_16.nt | 35600 (309G) | A/G |
| b200e21-h_Contig4_21.nt | 45619 (309G) | G/A |
| b200e21-h_Contig4_22.nt-a | 46018 (309G) | T/G |
| b200e21-h_Contig4_22.nt-b | 46093 (309G) | T/G |
| b200e21-h_Contig4_22.nt-c | 46190 (309G) | A/G |
| b200e21-h_Contig4_24.nt-a | 50993 (309G) | T/C |
| b200e21-h_Contig4_24.nt-b | 51124 (309G) | C/T |
| b200e21-h_Contig4_25.nt | 55461 (309G) | C/T |
| b200e21-h_Contig4_33.nt-a | 63645 (309G) | C/A |
| b200e21-h_Contig4_33.nt-b | 63646 (309G) | A/C |
| b200e21-h_Contig4_61.nt | 24809 (309G) | T/G |
| b200e21-h_Contig4_62.nt | 27837 (309G) | T/C |
| b200e21-h_Contig4_63.nt-a | 31485 (309G) | C/T |
| b200e21-h_Contig4_63.nt-b | 31683 (309G) | A/G |
| b200e21-h_Contig4_9.nt | 24808 (309G) | T/G |
| b527d12-h_Contig030g_1.nt-a | 31340 (308G) | T/C |
| b527d12-h_Contig030g_1.nt-b | 32538 (308G) | A/G |
| b527d12-h_Contig080C_2.nt | 13224 (308G) | A/G |
| b527d12-h_Contig087C_1.nt | 21119 (308G) | C/A |
| b527d12-h_Config087C_4.nt | 30497 (308G) | G/A |
| b527d12-h_Contig088C_4.nt | 24811 (309G) | A/C |
| b527d12-h_Contig089_1HP.nt | 68280 (309G) | G/A |

In addition to the polymorphisms presented m Table 4, two additional polymorphisms can also be present in SEQ ID NO:2. These is a change at position 2002 of SEQ ID NO:2. Either a guanine or an adenine can appear at this position. This polymorphism is silent and is not associated with any change in the amino acid sequence. The second change is at position 4059 of SEQ ID NO:2 corresponding in a cytosine (C) to thymine (T) change. This polymorphism results in a corresponding amino acid change from a valine (V) to an alanine (A). Other polymorphisms were found in the candidate gene exons and adjacent intron sequences. Any one or combination of the polymorphisms listed in Table 4 or the two discussed above could also have a minor effect on bone mass or lipid levels when present in SEQ ID NO:2.

Accordingly, herein are described nucleic acid sequences having the nucleic acid sequence of SEQ ID NO: 1 with the above-identified point mutations.

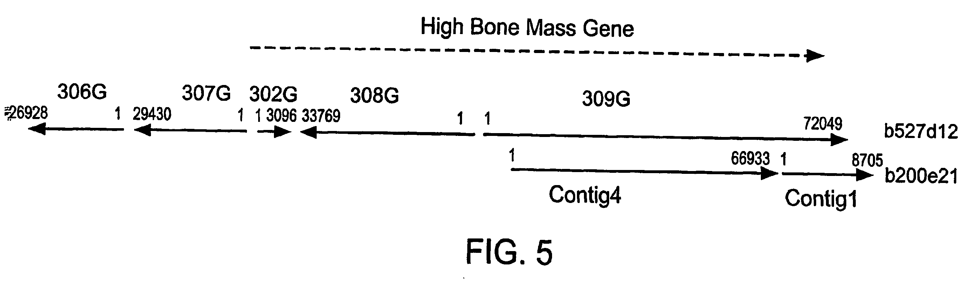

Also described is a sequence encompassing the nucleic acid of SEQ ID NO: 2. Specifically, a base-pair substitution changing G to T at position 582 in the coding sequence of Zmax1 (the *HBM* gene) was identified as heterozygous in all HBM individuals, and not found in the unaffected individuals (i.e., b527d12-h_Contig087C_1.nt). Fig. 5 shows the order of the contigs in B527D12. The direction of transcription for the *HBM* gene is from left to right. The sequence of contig308G of B527D 12 is the reverse complement of the coding region to the *HBM* gene. Therefore, the relative polymorphism in contig 308G shown in Table 4 as a base change substitution of C to A is the complement to the G to T substitution in the *HBM* gene. This mutation causes a substitution of glycine 171 with valine (G171V).

The HBM polymorphism was confirmed by examining the DNA sequence of different groups of individuals. In all members of the HBM pedigree (38 individuals), the HBM polymorphism was observed in the heterozygous form in affected (i.e., elevated bone mass) individuals only (N=18). In unaffected relatives (N=20) (BMDZ<2.0) the HBM polymorphism was never observed. To determine whether this gene was ever observed in individuals outside of the HBM pedigree, 297 phenotyped individuals were characterized at the site of the *HBM* gene. None were heterozygous at the site of the HBM polymorphism. In an imphenotyped control group, 1 of 42 individuals was observed to be heterozygous at position 582. Since this individual is deceased, their bone mineral density could not be obtained. Taken together, these data prove that the polymorphism observed in the kindred displaying the high bone mass phenotype is strongly correlated with the G→T polymorphism at position 582 of Zmax1. Taken together, these results establish that the HBM polymorphism genetically segregates with the HBM phenotype, and that both the HBM polymorphism and phenotype are rare in the general population.

### XII. Allele Specific Oligonucleotide (ASO) Analysis

The amplicon containing the HBM1 polymorphism was PCR amplified using primers specific for the exon of interest. The appropriate population of individuals was PCR amplified in 96 well microtiter plates as follows. PCR reactions (20 µl) containing 1X Promega PCR buffer (Cat. # M1883 containing 1.5 mM MgCl₂), 100mM dNTP, 200 nM PCR primers (1863F: CCAAGTTCTGAGAAGTCC and 1864R: AATACCTGAAACCAT ACCTG), 1 U Amplitaq, and 20 ng of genomic DNA were prepared and amplified under the following PCR conditions: 94°C, 1 minute, (94°C, 30 sec.; 58°C, 30 sec.; 72°C, 1 min.) X35 cycles), 72°C, 5', 4°C, hold. Loading dye was then added and 10 µl of the products was electrophoresed on 1.5% agarose gels containing 1 µg/ml ethidium bromide at 100-150 V for 5-10 minutes. Gels were treated 20 minutes in denaturing solution (1.5 M NACl, 0.5 N NaOH), and rinsed briefly with water. Gels were then neutralized in 1 M Tris-HCl, pH 7.5, 1.5 M NaCl, for 20 minutes and rinsed with water. Gels were soaked m 10 X SSC tor 20 minutes and blotted onto nylon transfer membrane (Hybond N+- Amersham) in 10X SSC overnight. Filters were the rinsed in 6X SSC for 10 minutes and UV crosslinked.

The allele specific oligonucleotides (ASO) were designed with the polymorphism approximately in the middle. Oligonucleotides were phosphate free at the 5'end and were purchased from Gibco BRL. Sequences of the oligonucleotides are:
2326 Zmax1.ASO.g: AGACTGGGGTGAGACGC
2327 Zmax1.ASO.t: CAGACTGGGTTGAGACGCC
The polymorphic nucleotides are underlined. To label the oligos, 1.5 µl of 1 µg/µl ASO oligo (2326.Zmax1.ASO.g or 2327.Zmax1.ASO.t), 11 µl ddH₂O, 2 µl 10X kinase forward buffer, 5 µl γ-³²P-ATP (6000 Ci/mMole), and 1 µl T4 polynucleotide kinase (10 U/µl) were mixed, and the reaction incubated at 37°C for 30-60 minutes. Reactions were then placed at 95°C for 2 minutes and 30 ml H₂O was added. The probes were purified using a G25 microspin column (Pharmacia).

Blots were prehybridized in 10 ml 5X SSPE, 5X Denhardt's, 2% SDS, and 100 µg/ml, denatured, sonicated salmon sperm DNA at 40°C for 2 hr. The entire reaction mix of kinased oligo was then added to 10 ml fresh hybridization buffer (5X SSPE, 5X Denhardts, 2% SDS) and hybridized at 40°C for at least 4 hours to overnight

All washes done in 5X SSPE, 0.1 % SDS. The first wash was at 45°C for 15 minutes; the solution was then changed and the filters washed 50°C for 15 minutes. Filters were then exposed to Kodak biomax film with 2 intensifying screens at -70°C for 15 minutes to 1 hr. If necessary the filters were washed at 55°C for 15 minutes and exposed to film again. Filters were stripped by washing in boiling 0.1X SSC, 0.1% SDS for 10 minutes at least 3 times.

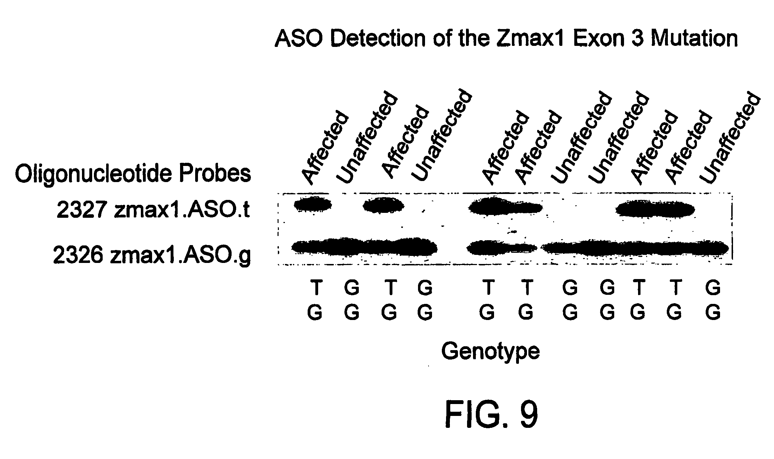

The two films that best captured the allele specific assay with the 2 ASOs were converted into digital images by scanning them into Adobe PhotoShop. These images were overlaid against each other in Graphic Converter and then scored and stored in FileMaker Pro 4.0 (see **Fig. 9**).

### XIII. Cellular Localization of Zmax1

### A. Gene Expression in Rat tibia by non isotopic In Situ Hybridization

*In situ* hybridization was conducted by Pathology Associates International (PAI), Frederick, MD. This study was undertaken to determine the specific cell types that express the *Zmax1* gene in rat bone with particular emphasis on areas of bone growth and remodeling. Zmax1 probes used in this study were generated from both human (HuZmax1) and mouse (MsZmax1) cDNAs, which share an 87% sequence identity. The homology of human and mouse Zmax1 with rat Zmax1 is unknown.

For example, gene expression by non-isotopic *in situ* hybridization was performed as follows, but other methods would be known to the skilled artisan. Tibias were collected from two 6 to 8 week old female Sprague Dawley rats euthanized by carbon dioxide asphyxiation. Distal ends were removed and proximal tibias were snap frozen in OCT embedding medium with liquid nitrogen immediately following death. Tissues were stored in a -80°C freezer.

Probes for amplifying PCR products from cDNA were prepared as follows. The primers to amplify PCR products from a cDNA clone were chosen using published sequences of both human LRP5 (Genbank Accession No. ABO17498) and mouse LRP5 (Genbank Accession No. AFO64984). In order to minimize cross reactivity with other genes in the LDL receptor family, the PCR products were derived from an intracellular portion of the protein coding region. PCR was performed in a 50 µl reaction volume using cDNA clone as template. PCR reactions contained 1.5 mM MgCl₂, 1 unit Amplitaq, 200 µM dNTPs and 2 µM each primer. PCR cycling conditions were 94°C for 1 min., followed by 35 cycles of 94°C for 30 seconds, 55°C for 30 seconds, 72°C for 30 seconds; followed by a 5 minute extension at 72°C. The reactions were then run on a 1.5% agarose Tris-Acetate gel. DNA was eluted from the agarose, ethanol precipitated and resuspended in 10 mM Tris, pH 8.0. Gel purified PCR products were prepared for both mouse and human cDNAs and supplied to Pathology Associates International for *in situ* hybridizations.

The sequence of the human and mouse PCR primers and products were as follows:
Human Zmax1 sense primer (HBM12531)
   CCCGTGTGCTCCGCCGCCCAGTTC
Human Zmax1 antisense primer (HBM1465)
   GGCTCACGGAGCTCATCATGGACTT
Human Zmax1 PCR product
Mouse Zmax1 Sense primer (HBM1655)
   AGCGAGGCCACCATCCACAGG
Mouse Zmax1 antisense primer (HBM1656)
   TCGCTGGTCGGCATAATCAAT
Mouse Zmax1 PCR product

Riboprobes were synthesized as follows. The PCR products were reamplified with chimeric primers designed to incorporate either a T3 promoter upstream, or a T7 promoter downstream of the reamplification products. The resulting PCR products were used as template to synthesize digoxigenin-labeled riboprobes by *in vitro* transcription (IVT). Antisense and sense riboprobes were synthesized using T7 and T3 RNA polymerases, respectively, in the presence of digoxigenin-11-UTP (Boehringer-Mannheim) using a MAXIscript IVT kit (Ambion) according to the manufacturer. The DNA was then degraded with Dnase-1, and unincorporated digoxigenin was removed by ultrafiltration. Riboprobe integrity was assessed by electrophoresis through a denaturing polyacrylamide gel. Molecular size was compared with the electrophoretic mobility of a 100-1000 base pair (bp) RNA ladder (Ambion). Probe yield and labeling was evaluated by blot immunochemistry. Riboprobes were stored in 5 µl aliquots at -80°C.

The *in situ* hybridization was performed as follows. Frozen rat bone was cut into 5 µM sections on a Jung CM3000 cryostat (Leica) and mounted on adhesive slides (Instrumedics). Sections were kept in the cryostat at -20°C until all the slides were prepared in order to prevent mRNA degradation prior to post-fixation for 15 minutes in 4% paraformaldehyde. Following post-fixation, sections were incubated with 1 ng/µl of either antisense or sense riboprobe in Pathology Associates International (PAI) customized hybridization buffer for approximately 40 hours at 58°C. Following hybridization, slides were subjected to a series of post-hybridization stringency washes to reduce nonspecific probe binding. Hybridization was visualized by immunohistochemistry with an anti-digoxigenin antibody (FAB fragment) conjugated to alkaline phosphatase. Nitroblue tetrazolium chloride/bromochloroindolyl phosphate (Boehringer-Mannheim), a precipitating alkaline phosphatase substrate, was used as the chromogen to stain hybridizing cells purple to nearly black, depending on the degree of staining. Tissue sections were counter-stained with nuclear fast red. Assay controls included omission of the probe, omission of probe and anti-digoxigenin antibody.

Specific cell types were assessed for demonstration of hybridization with antisense probes by visualizing a purple to black cytoplasmic and/or peri-nuclear staining indicating a positive hybridization signal for mRNA. Each cell type was compared to the replicate sections, which were hybridized with the respective sense probe. Results were considered positive if staining was observed with the antisense probe and no staining or weak background with the sense probe.

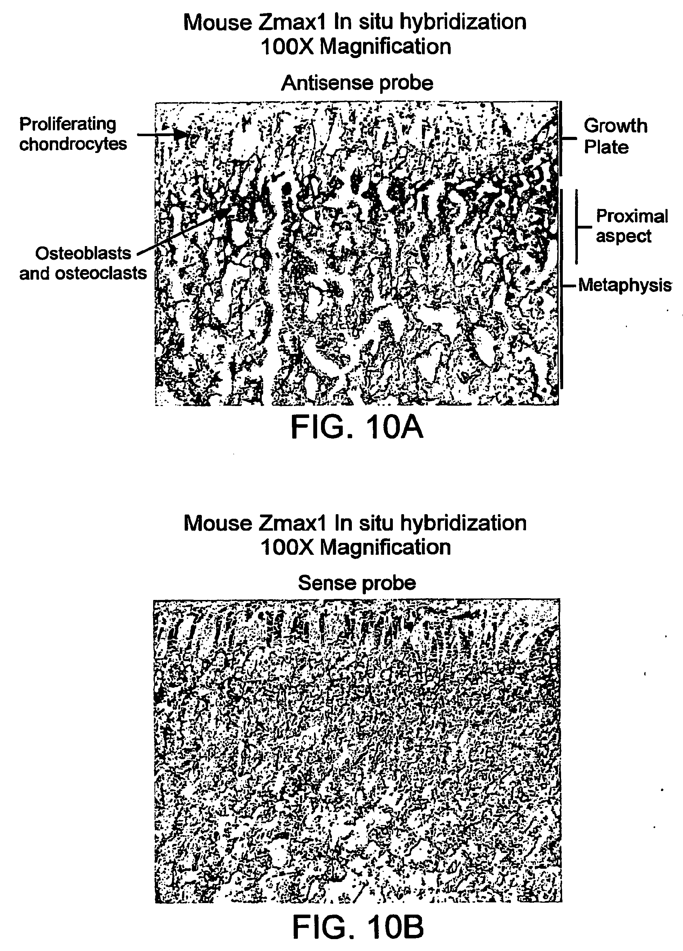

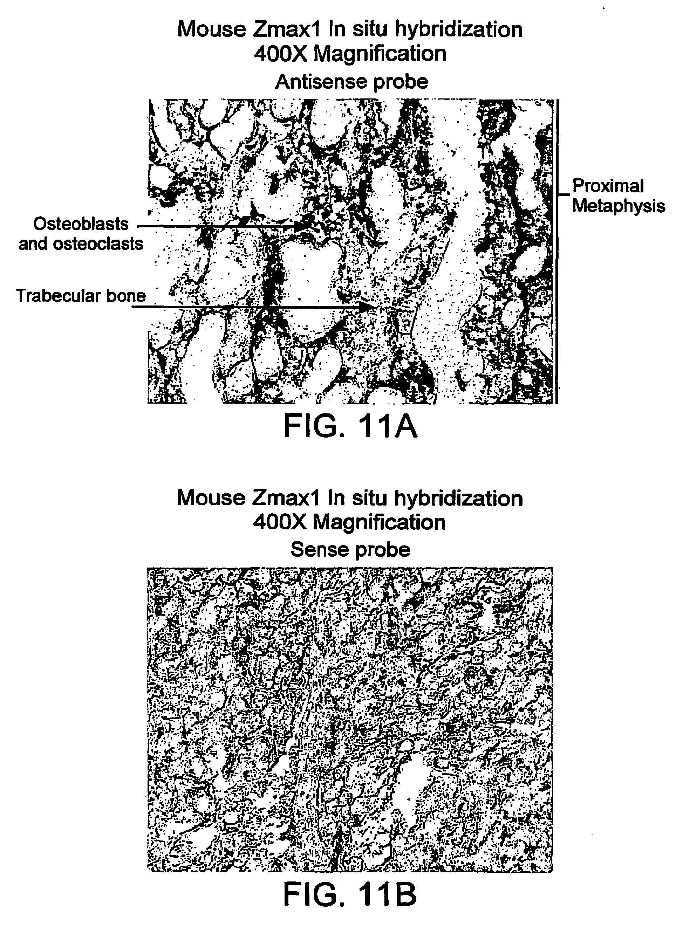

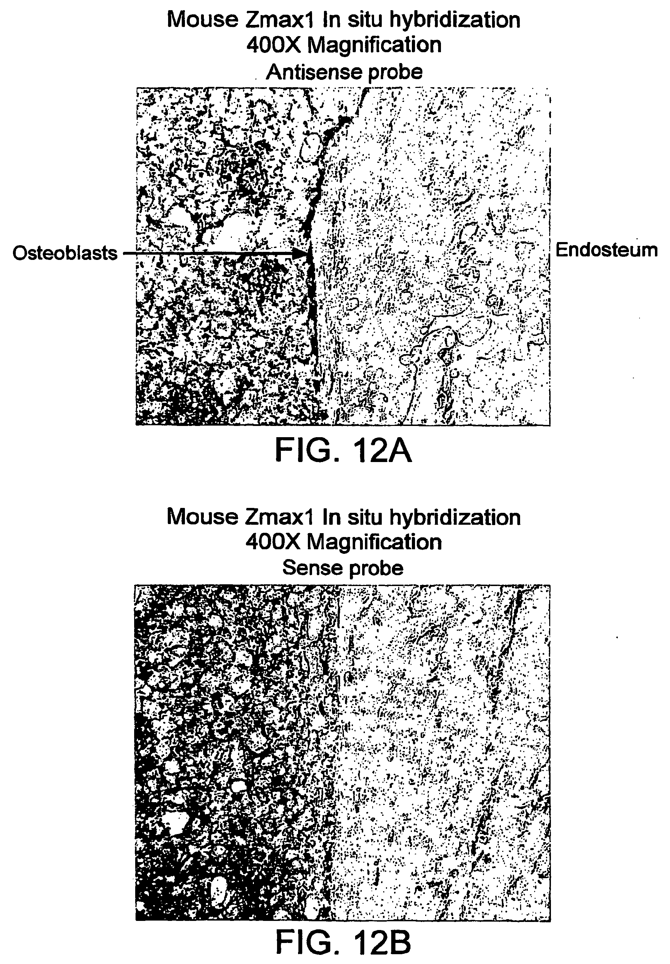

The cellular localization of the hybridization signal for each of the study probes is summarized in **Table 5.** Hybridization for Zmax1 was primarily detected in areas of bone involved in remodeling, including the endosteum and trabecular bone within the metaphysis. Hybridization in selected bone lining cells of the periosteum and epiphysis were also observed. Positive signal was also noted in chondrocytes within the growth plate, particularly in the proliferating chondrocytes. See **Figs. 10, 11** and **12** for representative photomicrographs of in *situ* hybridization results.

**TABLE 5**

| **Summary of Zmax1 *in situ* hybridization in rat tibia** | | |
|---|---|---|
| **PROBE** | **SITE** | **ISH SIGNAL** |
| Hu Zmax1 | Epiphysis | |
| | Osteoblasts | + |
| | Osteoclasts | - |
| | Growth Plate | |
| | resting chondrocytes | - |
| | proliferating chondrocytes | + |
| | hypertrophic chondrocytes | - |
| | Metaphysis | |
| | osteoblasts | + |
| | osteoclasts | + |
| | Diaphysis | - |
| | Endosteum | |
| | osteoblasts | + |
| | osteoclasts | + |
| | Periosteum | - |
| | | |
| | | |

| MsZmax1 | Epiphysis | |
|---|---|---|
| | Osteoblasts | + |
| | Osteoclasts | - |
| | Growth Plate | |
| | resting chondrocytes | - |
| | proliferating chondrocytes | + |
| | hypertrophic chondrocytes | + |
| | Metaphysis | |
| | osteoblasts | + |
| | osteoclasts | + |
| | Diaphysis | - |
| | Endosteum | |
| | osteoblasts | + |
| | osteoclasts | + |
| | Periosteum | + |
| | | |

| | | |
|---|---|---|
| Legend: "+" = hybridization signal detected "-" = no hybridization signal detected "ISH"- *In situ* hybridization | | |

These studies confirm the positional expression of Zmax1 in cells involved in bone remodeling and bone formation. Zmax1 expression in the zone of proliferation and in the osteoblasts and osteoclasts of the proximal metaphysis, suggests that the *Zmax1* gene is involved in the process of bone growth and mineralization. The activity and differentiation of osteoblasts and osteoclasts are closely coordinated during development as bone is formed and during growth as well as in adult life as bone undergoes continuous remodeling. The formation of internal bone structures and bone remodeling result from the coupling of bone resorption by activated osteoclasts with subsequent deposition of new material by osteoblasts. Zmax1 is related to the LDL receptor gene, and thus may be a receptor involved in mechanosensation and subsequent signaling in the process of bone remodeling. Therefore, changes in the level of expression of this gene could impact on the rate of remodeling and degree of mineralization of bone. Similar studies can be designed for *in situ* analysis of HBM or Zmax1 in other cells or tissues.

### XIV. Antisense

Antisense oligonucleotides are short synthetic nucleic acids that contain complementary base sequences to a targeted RNA. Hybridization of the RNA in living cells with the antisense oligonucleotide interferes with RNA function and ultimately blocks protein expression Therefore, any gene for which the partial sequence is known can be targeted by an antisense oligonucleotide.

Antisense technology is becoming a widely used research tool and will play an increasingly important role in the validation and elucidation of therapeutic targets identified by genomic sequencing efforts.

Antisense technology was developed to inhibit gene expression by utilizing an oligonucleotide complementary to the mRNA that encodes the target gene. There are several possible mechanisms for the inhibitory effects of antisense oligonucleotides. Among them, degradation of mRNA by RNase H is considered to be the major mechanism of inhibition of protein function. This technique was originally used to elucidate the function of a target gene, but may also have therapeutic applications, provided it is designed carefully and properly.

An example of materials and methods for preparing antisense oligonucleotides can be performed as follows. Preliminary studies have been undertaken in collaboration with Sequiter (Natick, MA) using the antisense technology in the osteoblast-like murine cell line, MC3T3. These cells can be triggered to develop along the bone differentiation sequence. An initial proliferation period is characterized by minimal expression of differentiation markers and initial synthesis of collagenous extracellular matrix. Collagen matrix synthesis is required for subsequent induction of differentiation markers. Once the matrix synthesis begins, osteoblast marker genes are activated in a clear temporal sequence: alkaline phosphatase is induced at early times while bone sialoprotien and osteocalcin appear later in the differentiation process. This temporal sequence of gene expression is useful in monitoring the maturation and mineralization process. Matrix mineralization, which does not begin until several days after maturation has started, involves deposition of mineral on and within collagen fibrils deep within the matrix near the cell layer-culture plate interface. The collagen fibril-associated mineral formed by cultured osteoblasts resembles that found in woven bone in vivo and therefore is used frequently as a study reagent.

MC3T3 cells were transfected with antisense oligonucleotides for the first week of the differentiation, according to the manufacturer's specifications (U.S. Patent No. 5,849,902).

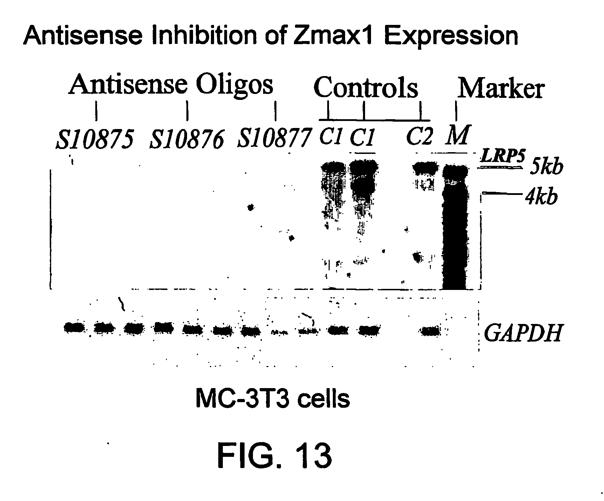

The oligonucleotides designed for Zmax1 are given below:
**10875:** AGUACAGCUUCUUGCCAACCCAGUC
**10876:** UCCUCCAGGUCGAUGGUCAGCCCAU
**10877:** GUCUGAGUCCGAGUUCAAAUCCAGG
Figure 13 shows the results of antisense inhibition of Zmax1 in MC3T3 cells. The three oligonucleotides shown above were transfected into MC3T3 and RNA was isolated according to standard procedures. Northern analysis clearly shows markedly lower steady state levels of the Zmax1 transcript while the control gene GAPDH remained unchanged. Thus, antisense technology using the primers described above allows for the study of the role of Zmax1 expression on bone biology. Similar primers can be used to study Zmax1 expression and its ability to regulate lipid levels in an animal.

The protein encoded by Zmax1 is related to the Low Density Lipoprotein receptor (LDL receptor). *See,* Goldstein et al., Ann. Rev. Cell Biology, 1:1-39 (1985); Brown et al., Science, 232:34-47 (1986). The LDL receptor is responsible for uptake of low density lipoprotein, a lipid-protein aggregate that includes cholesterol. Individuals with a defect in the LDL receptor are deficient in cholesterol removal and tend to develop artherosclerosis. In addition, cells with a defective LDL receptor show increased production of cholesterol, in part because of altered feedback regulation of cholesterol synthetic enzymes and in part because of increased transcription of the genes for these enzymes. In some cell types, cholesterol is a precursor for the formation of steroid hormones.

Thus, the LDL receptor may, directly or indirectly, function as a signal transduction protein and may regulate gene expression. Because Zmax1 is related to the LDL receptor, this protein may also be involved in signaling between cells in a way that affects bone remodeling as well as regulate lipid levels and therefore lipid-mediated diseases.

The glycine 171 amino acid is likely to be important for the function of Zmax1 because this amino acid is also found in the mouse homologue of Zmax1. The closely related LRP6 protein also contains glycine at the corresponding position (Brown et al., Biochemical and Biophysical Research Comm., 248:879-888 (1988)). Amino acids that are important in a protein's structure or function tend to be conserved between species, because natural selection prevents mutations with altered amino acids at important positions from arising.

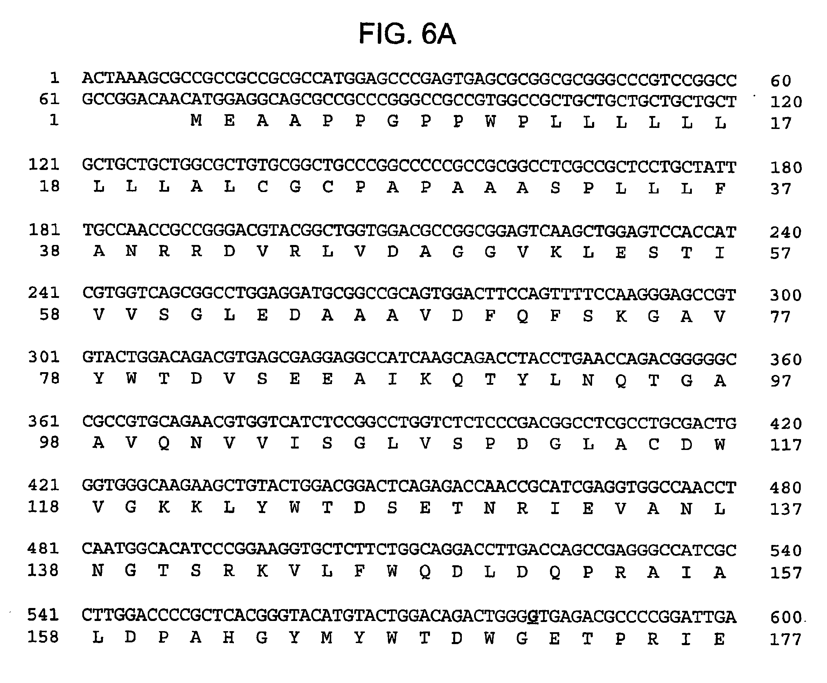

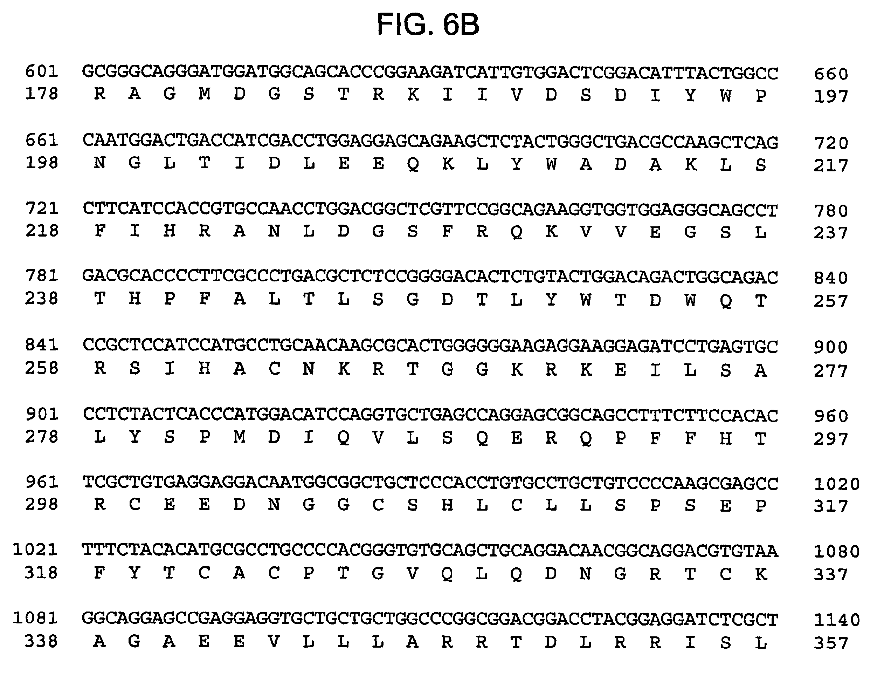

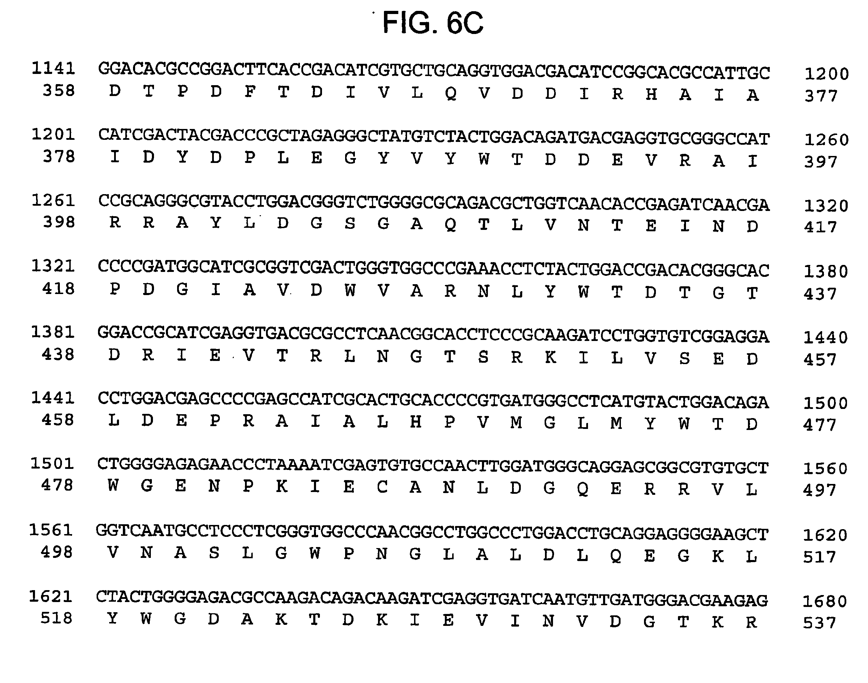

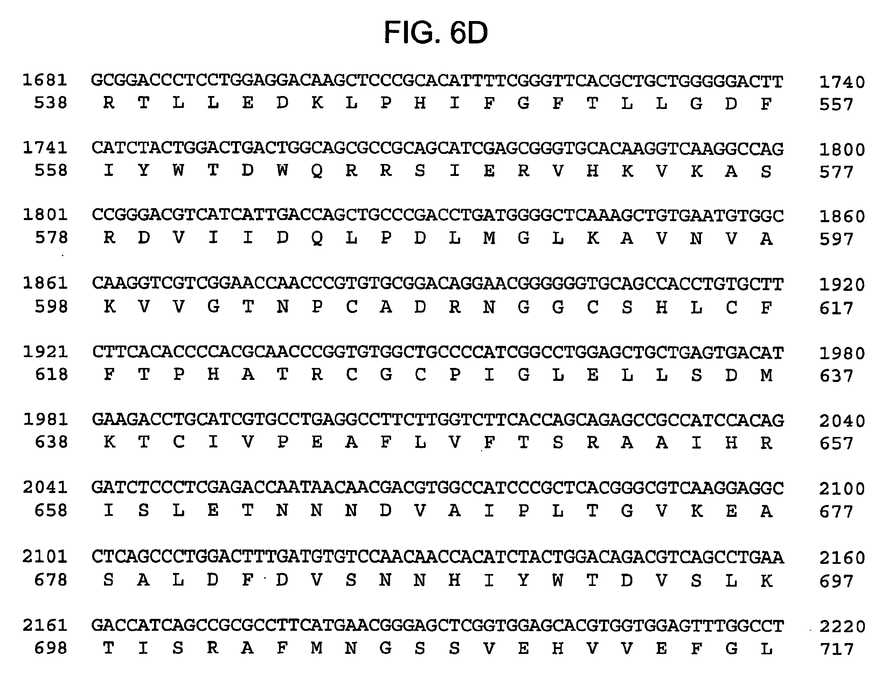

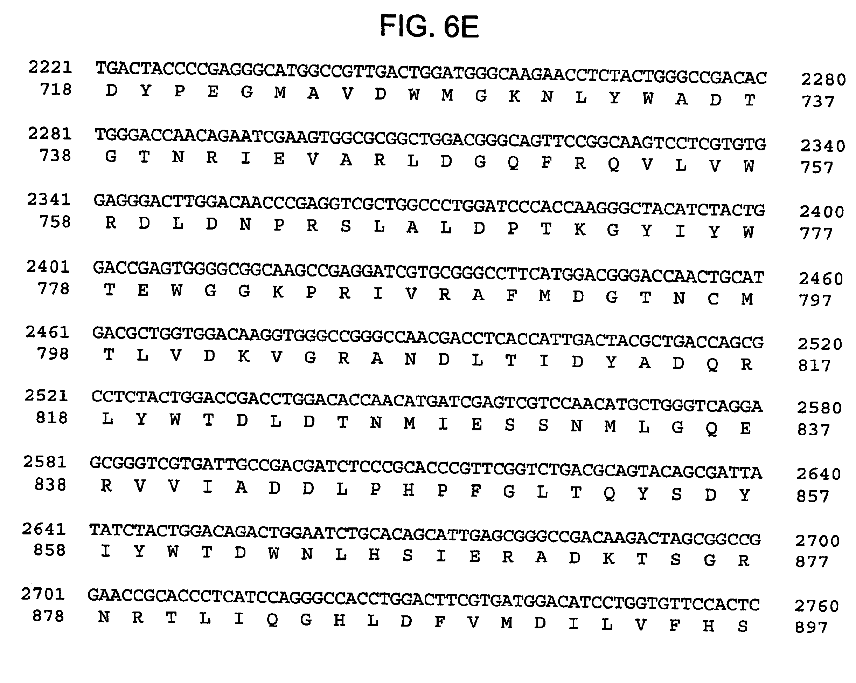

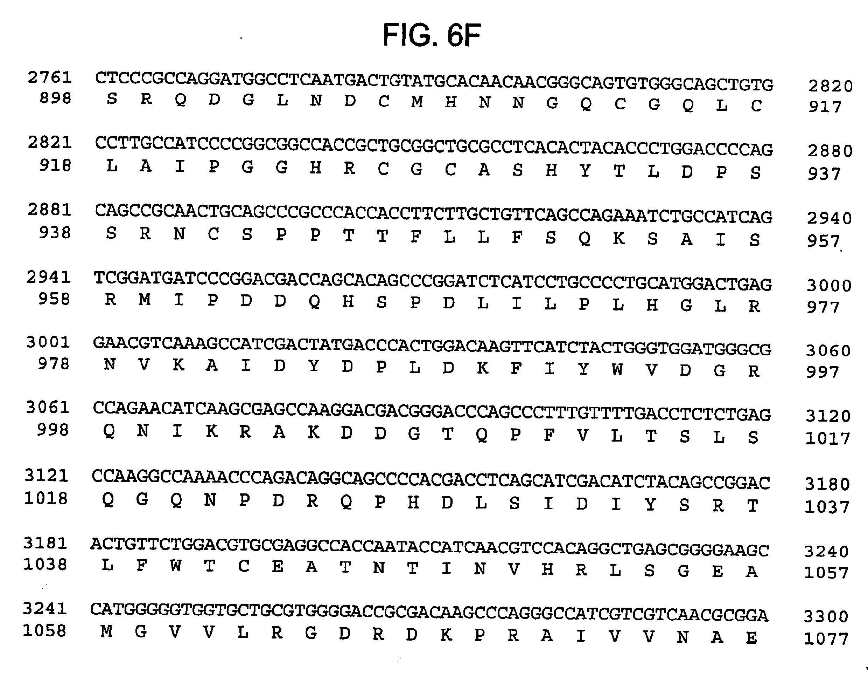

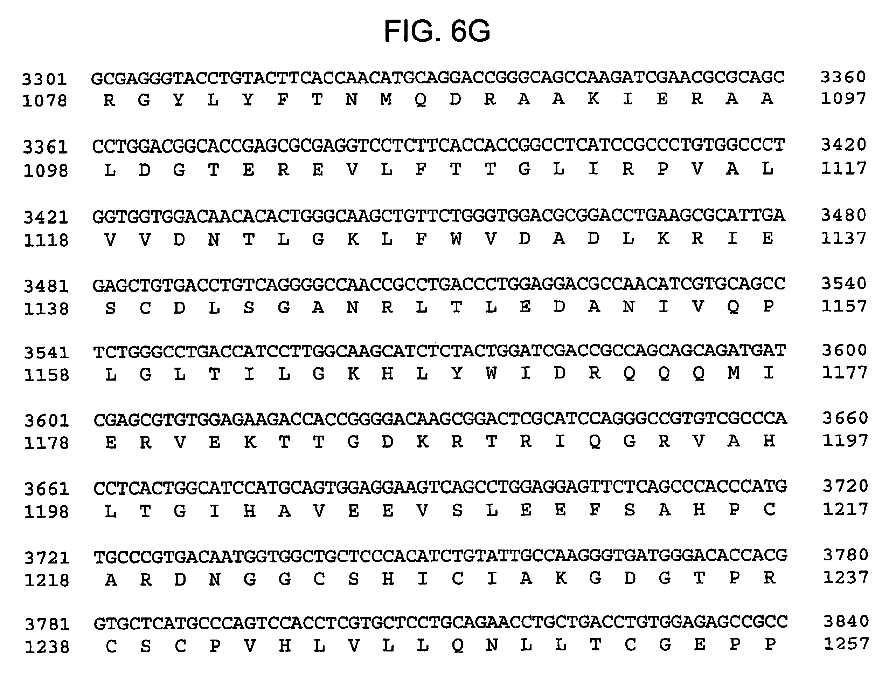

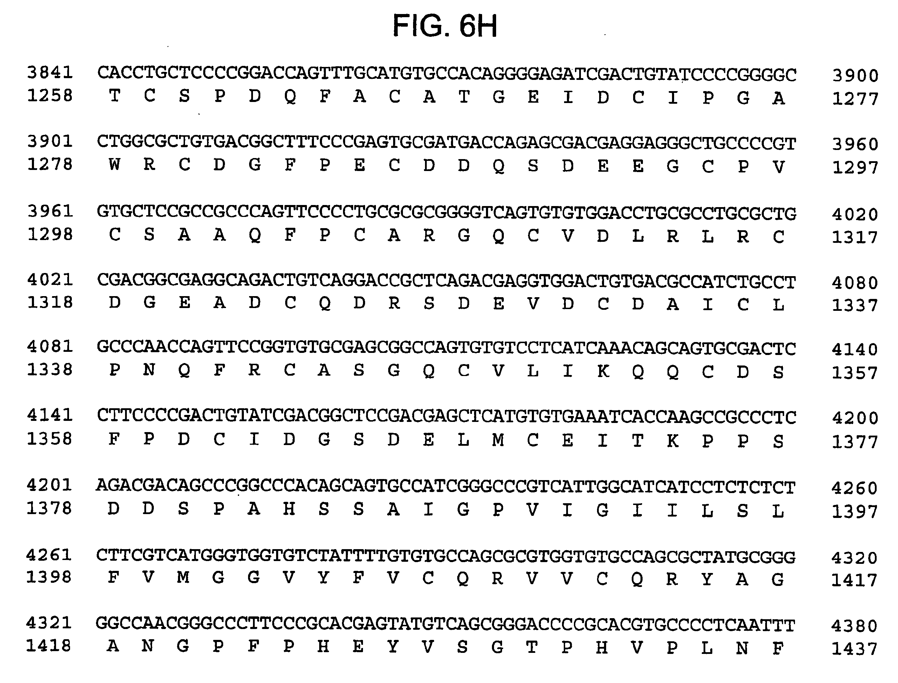

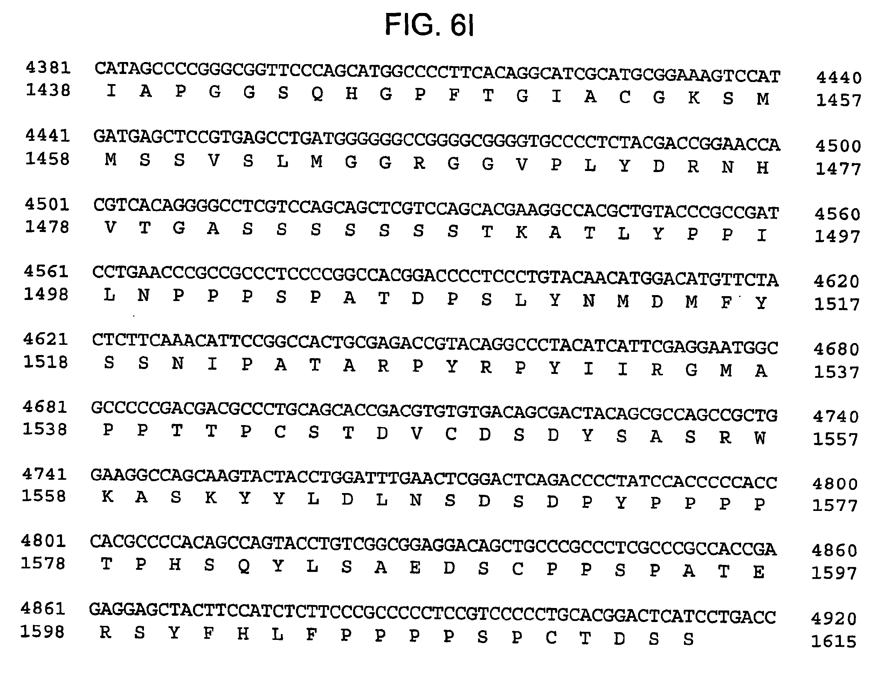

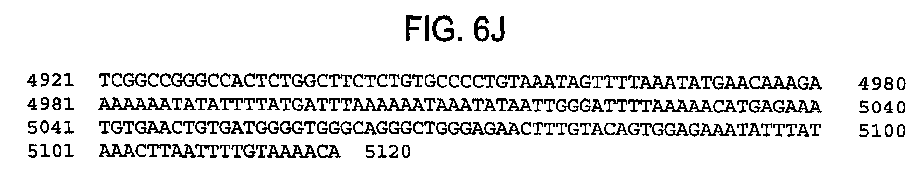

In addition, the extracellular domain of Zmax1 contains four repeats consisting of five YWT motifs followed by an EFG motif. This 5YWT+EGF repeat is likely to form a distinct folded protein domain, as this repeat is also found in the LDL receptor and other LDL receptor-related proteins. The first three 5YWT+EGF repeats are very similar in their structure, while the fourth is highly divergent Glycine 171 occurs in the central YWT motif of the first 5YWT+EGF repeat in Zmax1. The other two similar 5YWT+EGF repeats of Zmax1 also contain glycine at the corresponding position, as does the 5YWT+EGF repeat in the LDL receptor protein. However, only 17.6% of the amino acids are identical among the first three 5YWT+EGF repeats in Zmax1 and the single repeat in the LDL receptor. These observations indicate that glycine 171 is essential to the function of this repeat, and mutation of glycine 171 causes a functional alteration of Zmax1. The cDNA and peptide sequences are shown in **Figs. 6A-6J**. The critical base at nucleotide position 582 is indicated in bold and is underlined.

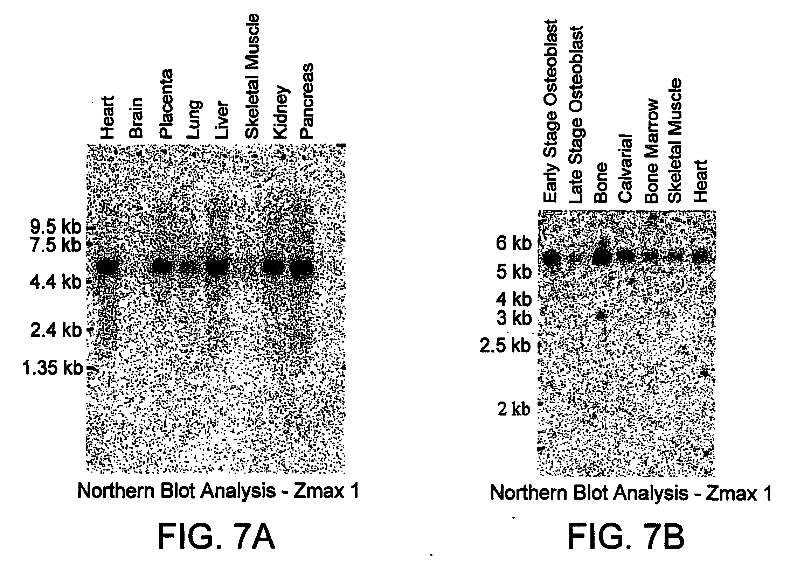

Northern blot analysis (**Figs. 7A-B**) reveals that Zmax1 is expressed in human bone tissue as well as numerous other tissues. A multiple-tissue Northern blot (Clontech, Palo Alto, CA) was probed with exons from Zmax1. As shown in **Fig. 7A**, the 5.5 kb Zmax1 transcript was highly expressed in heart, kidney, lung, liver and pancreas and is expressed at lower levels in skeletal muscle and brain. A second northern blot, shown in **Fig. 7B**, confirmed the transcript size at 5.5 kb, and indicated that Zmax1 is expressed m bone, bone marrow, calvaria and human osteoblastic cell lines.

Taken together, these results indicate that the HBM polymorphism in the *Zmax1* gene is responsible for the HBM phenotype, and that the *Zmax1* gene is important in bone development In addition, because mutation of Zmax1 can alter bone mineralization and development as well as lipid levels, it is likely that molecules that bind to Zmax1 may usefully alter bone development and lipid levels. Such molecules may include, for example, small molecules, proteins, RNA aptamers, peptide aptamers, and the like.

### XV. Preparation of Nucleic Acids, Vectors, Transformations and Host Cells

Large amounts of the nucleic acids of the present invention may be produced by replication in a suitable host cell. Natural or synthetic nucleic acid fragments coding for a desired fragment will be incorporated into recombinant nucleic acid constructs, usually DNA constructs, capable of introduction into and replication in a prokaryotic or eukaryotic cell. Usually the nucleic acid constructs will be suitable for replication in a unicellular host, such as yeast or bacteria, but may also be intended for introduction to (with and without integration within the genome) cultured mammalian or plant or other eukaryotic cell lines. The purification of nucleic acids produced by the methods of the present invention is described, for example, in Sambrook et al., Molecular Cloning. A Laboratory Manual, 2nd Ed. (Cold Spring Harbor Laboratory, Cold Spring Harbor, NY (1989) or Ausubel et al., Current Protocols in Molecular Biology, J. Wiley and Sons, NY (1992).

The nucleic acids of the present invention may also be produced by chemical synthesis, e.g., by the phosphoramidite method described by Beaucage et al., Tetra. Letts., 22:1859-1862 (1981) or the triester method according to Matteucci, et al., J. Am. Chem. Soc., 103:3185 (1981), and may be performed on commercial, automated oligonucleotide synthesizers. A double-stranded fragment may be obtained from the single-stranded product of chemical synthesis either by synthesizing the complementary strand and annealing the strands together under appropriate conditions or by adding the complementary strand using DNA polymerase with an appropriate primer sequence.

Nucleic acid constructs prepared for introduction into a prokaryotic or eukaryotic host may comprise a replication system recognized by the host, including the intended nucleic acid fragment encoding the desired protein, and will preferably also include transcription and translational initiation regulatory sequences operably linked to the protein encoding segment. Expression vectors may include, for example, an origin of replication or autonomously replicating sequence (ARS) and expression control sequences, a promoter, an enhancer and necessary processing information sites, such as ribosome-binding sites, RNA splice sites, polyadenylation sites, transcriptional terminator sequences, and mRNA stabilizing sequences. Secretion signals may also be included where appropriate, whether from a native HBM or Zmax1 protein or from other receptors or from secreted proteins of the same or related species, which allow the protein to cross and/or lodge in cell membranes, and thus attain its functional topology, or be secreted from the cell. Such vectors may be prepared by means of standard recombinant techniques well known in the art and discussed, for example, in Sambrook et al., Molecular Cloning. A Laboratory Manual, 2nd Ed. (Cold Spring Harbor Laboratory, Cold Spring Harbor, NY (1989) or Ausubel et al., Current Protocols in Molecular Biology, J. Wiley and Sons, NY (1992).

An appropriate promoter and other necessary vector sequences will be selected so as to be functional in the host, and may include, when appropriate, those naturally associated with Zmax1 or *HBM* genes. Examples of workable combinations of cell lines and expression vectors are described in Sambrook et al., Molecular Cloning. A Laboratory Manual, 2nd Ed. (Cold Spring Harbor Laboratory, Cold Spring Harbor, NY (1989) or Ausubel et al., Current Protocols in Molecular Biology, J. Wiley and Sons, NY (1992). Many useful vectors are known in the art and may be obtained from such vendors as Stratagene, New England BioLabs, Promega Biotech, and others. Promoters such as the trp, lac and phage promoters, tRNA promoters and glycolytic enzyme promoters may be used in prokaryotic hosts. Useful yeast promoters include promoter regions for metallothionein, 3-phosphoglycerate kinase or other glycolytic enzymes such as enolase or glyceraldehyde-3-phosphate dehydrogenase, enzymes responsible for maltose and galactose utilization, and others. Vectors and promoters suitable for use in yeast expression are further described in EP 73,675A. Appropriate non-native mammalian promoters might include the early and late promoters from SV40 (Fiers et al., Nature, 273:113 (1978)) or promoters derived from murine Moloney leukemia virus, mouse tumor virus, avian sarcoma viruses, adenovirus II, bovine papilloma virus or polyoma. In addition, the construct may be joined to an amplifiable gene (e.g., DHFR) so that multiple copies of the gene may be made. For appropriate enhancer and other expression control sequences, see also Enhancers and Eukaryotic Gene Expression, Cold Spring Harbor Press, Cold Spring Harbor, NY (1983).

While such expression vectors may replicate autonomously, they may also replicate by being inserted into the genome of the host cell, by methods well known in the art.

Expression and cloning vectors will likely contain a selectable marker, a gene encoding a protein necessary for survival or growth of a host cell transformed with the vector. The presence of this gene ensures growth of only those host cells which express the inserts. Typical selection genes encode proteins that a) confer resistance to antibiotics or other toxic substances, e.g. ampicillin, neomycin, methotrexate, etc.; b) complement auxotrophic deficiencies, or c) supply critical nutrients not available from complex media, e.g., the gene encoding D-alanine racemase for Bacilli. The choice of the proper selectable marker will depend on the host cell, and appropriate markers for different hosts are well known in the art.

The vectors containing the nucleic acids of interest can be transcribed *in vitro,* and the resulting RNA introduced into the host cell by well-known methods, e.g., by injection (see, Kubo et al., FEBS Letts. 241:119 (1988)), or the vectors can be introduced directly into host cells by methods well known in the art, which vary depending on the type of cellular host, including electroporation; transfection employing calcium chloride, rubidium chloride, calcium phosphate, DEAE-dextran, or other substances; microprojectile bombardment; lipofection; infection (where the vector is an infectious agent, such as a retroviral genome); and other methods. See generally, Sambrook *et al.,* 1989 and *Ausubel et al.,* 1992. The introduction of the nucleic acids into the host cell by any method known in the art, including those described above, will be referred to herein as "transformation." The cells into which have been introduced nucleic acids described above are meant to also include the progeny of such cells.

Large quantities of the nucleic acids and proteins of the present invention may be prepared by expressing the Zmax1 or HBM nucleic acids or portions thereof in vectors or other expression vehicles in compatible prokaryotic or eukaryotic host cells. The most commonly used prokaryotic hosts are strains of *Escherichia coli,* although other prokaryotes, such as *Bacillus subtilis* or *Pseudomonas* may also be used.

Mammalian or other eukaryotic host cells, such as those of yeast, filamentous fungi, plant, insect, or amphibian or avian species, may also be useful for production of the proteins of the present invention. Propagation of mammalian cells in culture is per se well known. See, Jakoby and Pastan (eds.), Cell Culture. Methods in Enzymology, volume 58, Academic Press, Inc., Harcourt Brace Jovanovich, NY, (1979)). Examples of commonly used mammalian host cell lines are VERO and HeLa cells, Chinese hamster ovary (CHO) cells, and WI38, BHK, and COS cell lines, although it will be appreciated by the skilled practitioner that other cell lines may be appropriate, e.g., to provide higher expression desirable glycosylation patterns, or other features.

Clones are selected by using markers depending on the mode of the vector construction. The marker may be on the same or a different DNA molecule, preferably the same DNA molecule. In prokaryotic hosts, the transformant may be selected, e.g., by resistance to ampicillin, tetracycline or other antibiotics. Production of a particular product based on temperature sensitivity may also serve as an appropriate marker.

Prokaryotic or eukaryotic cells transformed with the nucleic acids described herein will be useful not only for the production of the nucleic acids and proteins of the present invention, but also, for example, in studying the characteristics of Zmax1 or HBM proteins.

Antisense nucleic acid sequences arc useful in preventing or diminishing the expression of Zmax1 or HBM, as will be appreciated by one skilled in the art. For example, nucleic acid vectors containing all or a portion of the Zmax1 or *HBM* gene or other sequences from the Zmax1 or HBM region may be placed under the control of a promoter in an antisense orientation and introduced into a cell. Expression of such an antisense construct within a cell will interfere with Zmax1 or HBM transcription and/or translation and/or replication.

The probes and primers based on the Zmax1 and *HBM* gene sequences disclosed herein are used to identify homologous Zmax1 and *HBM* gene sequences and proteins in other species. These Zmax1 and *HBM* gene sequences and proteins are used in the diagnostic/prosnostic, therapeutic and drug screening methods described herein for the species from which they have been isolated.

### XVI. Protein Expression and Purification

Expression and purification of the HBM protein described above can be performed essentially as outlined below. To facilitate the cloning, expression and purification of membrane and secreted protein from the *HBM* gene, a gene expression system, such as the pET System (Novagen), for cloning and expression of recombinant proteins in *E*. *coli* was selected. Also, a DNA sequence encoding a peptide tag, the His-Tap, was fused to the 3" end of DNA sequences of interest to facilitate purification of the recombinant protein products. The 3' end was selected for fusion to avoid alteration of any 5' terminal signal sequence.

Nucleic acids chosen, for example, from the nucleic acids set forth in SEQ ID NOS: 1,3 and 5-12 for cloning HBM were prepared by polymerase chain reaction (PCR). Synthetic oligonucleotide primers specific for the 5' and 3' ends of the HBM nucleotide sequence were designed and purchased from Life Technologies (Gaithersburg, MD). All forward primers (specific for the 5' end of the sequence) were designed to include.an NcoI cloning site at the 5' terminus. These primers were designed to permit initiation of protein translation at the methionine residue encoded within the NcoI site followed by a valine residue and the protein encoded by the HBM DNA sequence. All reverse primers (specific for the 3' end of the sequence) included an EcoRI site at the 5' terminus to permit cloning of the HBM sequence into the reading frame of the pET-28b. The pET-28b vector provided a sequence encoding an additional 20 carboxyl-tenninal amino acids including six histidine residues (at the C-terminus), which comprised the histidine affinity tag.

Genomic DNA prepared from the *HBM* gene was used as the source of template DNA for PCR amplification (Ausubel et al., Current Protocols in Molecular Biology, John Wiley & Sons (1994)). To amplify a DNA sequence containing the HBM nucleotide sequence, genomic DNA (50 ng) was introduced into a reaction vial containing 2 mM MgCl₂, 1 µM synthetic oligonucleotide primers (forward and reverse primers) complementary to and flanking a defined HBM, 0.2 mM of each of deoxynucleotide triphosphate, dATP, dGTP, dCTP, dTTP and 2.5 units of heat stable DNA polymerase (Amplitaq, Roche Molecular Systems, Inc., Branchburg, NJ) in a final volume of 100 µl.

Upon completion of thermal cycling reactions, each sample of amplified DNA was purified using the Qiaquick Spin PCR purification kit (Qiagen, Gaithersburg, MD). All amplified DNA samples were subjected to digestion with the restriction endonucleases, e.g., NcoI and EcoRI (New England BioLabs, Beverly, MA) (Ausubel et al., Current Protocols in Molecular Biology, John Wiley & Sons, Inc. (1994)). DNA samples were then subjected to electrophoresis on 1.0% NuSeive (FMC BioProducts, Rockland, ME) agarose gels. DNA was visualized by exposure to ethidium bromide and long wave UV irradiation. DNA contained in slices isolated from the agarose gel was purified using the Bio 101 GeneClean Kit protocol (Bio 101, Vista, CA).

The pET-28b vector was prepared for cloning by digestion with restriction endonucleases, e.g., Ncol and EcoRI (New England BioLabs, Beverly, MA) (Ausubel et al., Current Protocols in Molecular Biology, John Wiley & Sons, Inc. (1994)). The pET-28a vector, which encodes the histidine affinity tag that can be fused to the 5' end of an inserted gene, was prepared by digestion with appropriate restriction endonucleases.

Following digestion, DNA inserts were cloned (Ausubel et al., Current Protocols in Molecular Biology, John Wiley & Sons, Inc. (1994)) into the previously digested pET-28b expression vector. Products of the ligation reaction were then used to transform the BL21 strain of *E. coli* (Ausubel et al., Current Protocols in Molecular Biology, John Wiley & Sons, Inc. (1994)) as described below.

Competent bacteria, *E. coli* strain BL21 or *E. coli* strain BL21 (DE3), were transformed with recombinant pET expression plasmids carrying the cloned HBM sequence according to standard methods (Ausubel et al., Current Protocols in Molecular Biology, John Wiley & Sons, Inc. (1994)). Briefly, 1 µl of ligation reaction was mixed with 50 µl of electrocompetent cells and subjected to a high voltage pulse, after which samples were incubated in 0.45 ml SOC medium (0.5% yeast extract, 2.0% tryptone, 10 mM NaCl, 2.5 mM KCl,10 mM MgCl₂, 10 mM MgSO₄ and 20 mM glucose) at 37°C with shaking for 1 hour. Samples were then spread on LB agar plates containing 25 µg/ml kanamycin sulfate for growth overnight. Transformed colonies of BL21 were then picked and analyzed to evaluate cloned inserts, as described below.

Individual BL21 clones transformed with recombinant pET-28b HBM nucleotide sequences were analyzed by PCR amplification of the cloned inserts using the same forward and reverse primers specific for the HBM sequences that were used in the original PCR amplification cloning reactions. Successful amplification verifies the integration of the HBM sequence in the expression vector (Ausubel et al., Current Protocols in Molecular Biology, John Wiley & Sons, Inc. (1994)).

Individual clones of recombinant pET-28b vectors carrying properly cloned HBM nucleotide sequences were picked and incubated in 5 ml of LB broth plus 25 µg/ml kanamycin sulfate overnight The following day plasmid DNA was isolated and purified using the Qiagen plasmid purification protocol (Qiagen Inc., Chatsworth, CA).

The pET vector can be propagated in any *E. coli* K-12 strain, e.g., HMS174, HB101, JM109, DH5 and the like, for purposes of cloning or plasmid preparation. Hosts for expression include E. *coli* strains containing a chromosomal copy of the gene for T7 RNA, polymerase. These hosts were lysogens of bacteriophage DE3, a lambda derivative that carries the lacI gene, the lacUV5 promoter and the gene for T7 RNA polymerase. T7 RNA polymerase was induced by addition of isopropyl-β-D-thiogalactoside (IPTG), and the T7 RNA polymerase transcribes any target plasmid containing a functional T7 promoter, such as pET-28b, carrying its gene of interest. Strains include, for example, BL21(DE3) (Studier et al., Meth. Enzymol., 185:60-89(1990)).

To express the recombinant HBM sequence, 50 ng of plasmid DNA are isolated as described above to transform competent BL21(DE3) bacteria as described above (provided by Novagen as part of the pET expression kit). The lacZ gene (β-galactosidase) is expressed in the pET-System as described for the HBM recombinant constructions. Transformed cells were cultured in SOC medium for 1 hour, and the culture was then plated on LB plates containing 25 µg/ml kanamycin sulfate. The following day, the bacterial colonies were pooled and grown in LB medium containing kanamycin sulfate (25 µg/ml) to an optical density at 600 nM of 0.5 to 1.0 O.D. units, at which point 1 mM IPTG was added to the culture for 3 hours to induce gene expression of the HBM recombinant DNA constructions.

After induction of gene expression with IPTG, bacteria were collected by centrifugation in a Sorvall RC-3B centrifuge at 3500 x g for 15 minutes at 4°C. Pellets were resuspended in 50 ml of cold mM Tris-HCl, pH 8.0,0.1 M NaCl and 0.1 mM EDTA (STE buffer). Cells were then centrifuged at 2000 x g for 20 minutes at 4°C. Wet pellets were weighed and frozen at -80°C until ready for protein purification.

A variety of methodologies known in the art can be used to purity the isolated proteins (Coligan et al., Current Protocols in Protein Science, John Wiley & Sons (1995)). For example, the frozen cells can be thawed, resuspended in buffer and ruptured by several passages through a small volume microfluidizer (Model M-1105, Microfluidics International Corp., Newton, MA). The resultant homogenate is centrifuged to yield a clear supernatant (crude extract) and, following filtration, the crude extract is fractioned over columns. Fractions are monitored by absorbance at OD₂₈₀ nm and peak fractions may be analyzed by SDS-PAGE.

The concentrations of purified protein preparations are quantified spectrophotometrically using absorbance coefficients calculated from amino acid content (Perkins, Eur. J. Biochem., 157:169-180 (1986)). Protein concentrations are also measured by the method of Bradford, Anal. Biochem., 72:248-254 (1976) and Lowry et al., J. Biol. Chem., 193:265-275 (1951) using bovine serum albumin as a standard.

SDS-polyacrylamide gels of various concentrations were purchased from BioRad (Hercules, CA), and stained with Coomassie blue. Molecular weight markers may include rabbit skeletal muscle myosin (200 kDa), *E. coli* β-galactosidase (116 kDa), rabbit muscle phosphorylase B (97.4 kDa), bovine serum albumin (66.2 kDa), ovalbumin (45 kDa), bovine carbonic anyhdrase (31 kDa), soybean trypsin inhibitor (21.5 kDa), egg white lysozyme (14.4 kDa) and bovine aprotinin (6.5 kDa).

Once a sufficient quantity of the desired protein has been obtained, it may be used for various purposes. A typical use is the production of antibodies specific for binding. These antibodies may be either polyclonal or monoclonal, and may be produced by *in vitro* or *in vivo* techniques well known in the art. Monoclonal antibodies to epitopes of any of the peptides identified and isolated as described can be prepared from murine hybridomas (Kohler, Nature, 256:495 (1975)). In summary, a mouse is inoculated with a few micrograms ofHBM protein over a period of two weeks. The mouse is then sacrificed. The cells that produce antibodies are then removed from the mouse's spleen. The spleen cells are then fused with polyethylene glycol with mouse myeloma cells. The successfully fused cells are diluted in a microtiter plate and growth of the culture is continued. The amount of antibody per well is measured by immunoassay methods such as ELISA (Engvall, Meth. Enzymol., 70:419 (1980)). Clones producing antibody can be expanded and further propagated to produce HBM antibodies. Other suitable techniques involve *in vitro* exposure of lymphocytes to the antigenic polypeptides, or alternatively, to selection of libraries of antibodies in phage or similar vectors. See Huse et al., Science, 246:1275-1281 (1989). For additional information on antibody production see Davis et al., Basic Methods in Molecular Biology, Elsevier, NY, Section 21-2 (1989).

Standard protocols for assessing the influence of an agent (e.g., antibody, HBM protein, protein polymorphism or Zmax1 protein or compound) to alter lipid levels in a cell or the physiological levels in a subject are known. For example, see F.W. HEMMING, LIPID ANALYSIS (Bios Scientific Pub.1996) and J. M. ORDOVAS, LIPOPROTEIN PROTOCOLS (Humana Press Inc. 1997). More specifically, cholesterol and triglyceride analysis can be performed using the Olympus AU5000 Cholesterol method. This method of measuring cholesterol combines the use of the enzymes with a modification of the peroxidase-pheol-4-aminoantipyrine system, substituting 2-hydroxy-3,5-dichlorobenzene sulfonic acid (2-OH 3,5 DCBSA) for the phenolic group for the measurement of total cholesterol in the subject serum. The assay is based on a series of coupled enzymatic reactions. Cholesterol esters present in serum are hydrolyzed to free cholesterol and fatty acids by cholesterol esterase. The cholesterol is in turn oxidized by cholesterol oxidase to cholest-4-en-3-one with the simultaneous production of hydrogen peroxidase. The hydrogen peroxidase reacts with 4-aminoautipyrine in the presence of 2-OH-3,5-DCBSA to produce a chromophore that absorbs at 570 nm. The absorbance of the reaction mixture is measured biochromatically at 570/750 nm and is proportional to the cholesterol concentration of the sample.

For serum triglyceride analysis, the Olympus AU5000 triglyceride procedure can also be used. Briefly, it is based on a series of coupled enzymatic reactions. Triglycerides in the serum are hydrolyzed to free fatty acids and glycerol by lipoprotein lipase. Glycerol is phosphorylated enzymatically and then oxidized with glycerol phosphate oxidase. The hydrogen peroxidase reacts with the chromogen 4-amino-antipyrine in the presence of DCB Sulfonic Acid to give a chromophore with absorption which is measured bichromatically at 520/660 nm. The increase in absorbance of the reaction mixture is proportional to the triglyceride concentration of the sample.

### XVII. Methods of Use: Gene Therapy

In recent years, significant technological advances have been made in the area of gene therapy for both genetic and acquired diseases. (Kay et al., Proc. Natl. Acad. Sci. USA, 94:12744-12746 (1997)) Gene therapy can be defined as the deliberate transfer of DNA for therapeutic purposes. Improvement in gene transfer methods has allowed for development of gene therapy protocols for the treatment of diverse types of diseases. Gene therapy has also taken advantage of recent advances in the identification of new therapeutic genes, improvement in both viral and nonviral gene delivery systems, better understanding of gene regulation, and improvement in cell isolation and transplantation.

The experiments below identify the *HBM* gene as a dominant mutation conferring elevated bone mass and that alters lipid levels. The fact that this mutation is dominant indicates that expression of the HBM protein causes elevated bone mass and perhaps changes in lipid levels. Older individuals carrying the *HBM* gene, and, therefore expressing the HBM protein, do not suffer from osteoporosis. These individuals are equivalent to individuals being treated with the HBM protein. These observations are a strong experimental indication that therapeutic treatment with the HBM protein prevents osteoporosis. The bone mass elevating activity of the *HBM* gene is termed "HBM function"

Therefore, HBM function may be supplied to mesenchymal stem cells (Onyia et al., J. Bone Miner. Res., 13:20-30 (1998); Ko et al., Cancer Res., 56:4614-4619 (1996)). Supplying such a function provides protection against osteoporosis. For regulating lipid levels, HBM function can be supplied to liver cells, as well as other cells involved in lipid metabolism and lipid regulation (e.g., muscle cells, lesion cells, lipid laiden foam cells and megakaryoblasts). The *HBM* gene or a part of the gene may be introduced into the cell in a vector such that the gene remains extrachromosomal. In such a situation, the gene will be expressed by the cell from the extrachromosomal location.

Vectors for introduction of genes both for recombination and for extrachromosomal maintenance are known in the art, and any suitable vector may be used. Methods for introducing DNA into cells such as electroporation, calcium phosphate co-precipitation, and viral transduction are known in the art, and the choice of method is within the competence of one skilled in the art (Robbins, Ed., Gene Therapy Protocols, Human Press, NJ (1997)). Cells transformed with the *HBM* gene can be used as model systems to study osteoporosis and drug treatments that promote bone growth as well as to study lipid-mediated diseases.

As generally discussed above, the *HBM* gene or fragment, where applicable, may be used in gene therapy methods in order to increase the amount of the expression products of such genes in mesenchymal stem cells or in other cells. It may be useful also to increase the level of expression of a given HBM protein, or a fragment thereof, even in those cells in which the wild type gene is expressed normally. Gene therapy would be carried out according to generally accepted methods as described by, for example, Friedman, Therapy for Genetic Diseases, Friedman, Ed., Oxford University Press, pages 105-121 (1991).

A virus or plasmid vector containing a copy of the *HBM* gene linked to expression control elements and capable of replicating inside mesenchymal stem cells or liver cells, is prepared. Suitable vectors are known and described, for example, in U.S. Patent No. 5,252,479 and WO 93/07282. The vector is then injected into the patient, either locally into the bone marrow or liver, or systemically (in order to reach any mesenchymal stem cells located at other sites, i.e., in the blood). If the transfected gene is not permanently incorporated into the genome of each of the targeted cells, the treatment may have to be repeated periodically.

Gene transfer systems known in the art may be useful in the practice of the gene therapy methods described herein. These include viral and non-viral transfer methods. A number of viruses have been used as gene transfer vectors, including polyoma, i.e., SV40 (Madzak et al., J. Gen. Virol., 73:1533-1536 (1992)), adenovirus (Berkner, Curr. Top. Microbiol. Immunol, 158:39-61 (1992); Berkner et al., Bio Techniques, 6:616-629 (1988); Gorziglia et al., J. Virol., 66:4407-4412 (1992); Quantin et al., Proc. Natl. Acad. Sci. USA, 89:2581-2584 (1992); Rosenfeld et al., Cell, 68:143-155(1992); Wilkinson et al., Nucl. Acids Res., 20:2233-2239 (1992); Stratford-Perricaudet et al., Hum. Gene Ther., 1:241-256 (1990)), vaccinia virus (Mackett et al., Biotechnology, 24:495-499 (1992)), adeno-associated virus (Muzyczka, Curr. Top. Microbiol. Immunol., 158:91-123 (1992); Ohi et al., Gene, 89:279-282 (1990)), herpes viruses including HSV and EBV (Margolskee, Curr. Top. Microbiol. Immunol., 158:67-90 (1992); Johnson et al., J. Virol., 66:2952-2965 (1992); Fink et al., Hum. Gene Ther., 3:11-19 (1992); Breakfield et al., Mol. Neurobiol., 1:337-371 (1987;) Fresse et al., Biochem. Pharmacol., 40:2189-2199 (1990)), and retroviruses of avian (Brandyopadhyay et al., Mol. Cell Biol., 4:749-754 (1984); Petropouplos et al., J. Virol., 66:3391-3397 (1992)), murine (Miller, Curr. Top. Microbiol. Immunol., 158:1-24 (1992); Miller et al., Mol. Cell Biol., 5:431-437 (1985); Sorge et al., Mol. Cell Biol., 4:1730-1737 (1984); Mann et al., J. Virol., 54:401-407 (1985)), and human origin (Page et al., J. Virol., 64:5370-5276 (1990); Buchschalcher et al., J. Virol., 66:2731-2739 (1992)). Most human gene therapy protocols have been based on disabled murine retroviruses.

Non-viral gene transfer methods known in the art include chemical techniques such as calcium phosphate coprecipitation (Graham et al., Virology, 52:456-467 (1973); Pellicer et al., Science, 209:1414-1422 (1980)), mechanical techniques, for example microinjection (Anderson et al., Proc. Natl. Acad Sci. USA, 77:5399-5403 (1980); Gordon et al., Proc. Natl. Acad. Sci. USA, 77:7380-7384 (1980); Brinster et al., Cell, 27:223-231 (1981); Constantini et al., Nature, 294:92-94 (1981)), membrane fusion-mediated transfer via liposomes (Felgner et al., Proc. Natl. Acad. Sci. USA, 84:7413-7417 (1987); Wang et al., Biochemistry, 28:9508-9514 (1989); Kaneda et al., J. Biol. Chem., 264:12126-12129 (1989); Stewart et al., Hum. Gene Ther., 3:267-275 (1992); Nabel et al., Science, 249:1285-1288 (1990); Lim et al., Circulation, 83:2007-2011 (1992)), and direct DNA uptake and receptor-mediated DNA transfer (Wolff et al., Science, 247:1465-1468 (1990); Wu et al., BioTechniques, 11:474-485 (1991); Zenke et al., Proc. Natl. Acad Sci. USA, 87:3655-3659 (1990); Wu et al., J. Biol. Chem., 264:16985-16987 (1989); Wolff et al., BioTechniques, 11:474-485 (1991); Wagner *et al.,* 1990; Wagner et al., Proc. Natl. Acad. Sci. USA, 88:4255-4259 (1991); Cotten et al., Proc. Natl. Acad. Sci. USA, 87:4033-4037 (1990); Curiel et al., Proc. Natl. Acad. Sci. USA, 88:8850-8854 (1991); Curiel et al., Hum. Gene Ther., 3:147-154 (1991)). Viral-mediated gene transfer can be combined with direct *in vivo* vectors to the mesenchymal stem cells and not into the surrounding cells (Rommo et al., In Vivo, 12(1):59-67 (1998); Gonez et al., Hum. Mol. Genetics, 7(12):1913-9 (1998)). Alternatively, the retroviral vector producer cell line can be injected into the bone marrow (Culver et al., Science, 256:1550-1552 (1992)). Injection of producer cells would then provide a continuous source of vector particles. This technique has been approved for use in humans with inoperable brain tumors.

In an approach which combines biological and physical gene transfer methods, plasmid DNA of any size is combined with a polylysine-conjugated antibody specific to the adenovirus hexon protein, and the resulting complex is bound to an adenovirus vector. The trimolecular complex is then used to infect cells. The adenovirus vector permits efficient binding, internalization, and degradation of the endosome before the coupled DNA is damaged.

Liposome/DNA complexes have been shown to be capable of mediating direct *in vivo* gene transfer. While in standard liposome preparations the gene transfer process is non-specific, localized *in vivo* uptake and expression have been reported in tumor deposits, for example, following direct in *situ* administration (Nabel, Hum. Gene Ther., 3:399-410 (1992)).,

### XVIII. Methods of Use: Transformed Hosts, Development of Pharmaceuticals and Research Tools

Cells and animals that carry the *HBM* gene can be used as model systems to study and test for substances that have potential as therapeutic agents (Onyia et al., J. Bone Miner. Res., 13:20-30 (1998); Broder et al., Bone, 21:225-235 (1997)). The cells are typically cultured mesenchymal stem cells or liver cells. These may be isolated from individuals with somatic or germline *HBM* genes. Alternatively, the cell line can be engineered to carry the *HBM* gene, as described above. After a test substance is applied to the cells, the transformed phenotype of the cell is determined. Any trait of transformed cells can be assessed, including formation of bone matrix in culture (Broder et al., Bone, 21:225-235 (1997)), mechanical properties (Kizer et al., Proc. Natl. Acad Sci. USA, 94:1013-1018 (1997)), and response to application of putative therapeutic agents.

Animals for testing therapeutic agents can be selected after treatment of germline cells or zygotes. Such treatments include insertion of the *Zmax1* gene, as well as insertion of the *HBM* gene and disrupted homologous genes. Alternatively, the inserted *Zmax1* gene(s) and/or *HBM* gene(s) of the animals may be disrupted by insertion or deletion mutation of other genetic alterations using conventional techniques, such as those described by, for example, Capechi, Science, 244:1288 (1989); Valancuis et al., Mol. Cell Biol., 11: 1402 (1991); Hasty et al., Nature, 350:243 (1991); Shinkai et al., Cell, 68:855 (1992); Mombaerts et al., Cell, 68:869 (1992); Philpott et al., Science, 256:1448 (1992); Snouwaert et al., Science, 257:1083 (1992); Donehower et al., Nature, 356:215 (1992). After test substances have been administered to the animals, the growth of bone or modulation of lipids must be assessed. If the test substance enhances the growth of bone or regulates lipid levels, then the test substance is a candidate therapeutic agent. These animal models provide an extremely important vehicle for potential therapeutic products. Preferred models for studying lipid modulation include mice (Smith et al., J. Intern. Med., 242: 99-109 (1997)) and guinea pigs.

Individuals carrying the HBM gene have elevated bone mass and altered lipid levels as discussed in the example below. The HBM gene causes this phenotype by altering the activities, levels, expression patterns, and modification states of other molecules involved in bone development. Using a variety of established techniques, it is possible to identify molecules, preferably proteins or mRNAs, whose activities, levels, expression patterns, and modification states are different between systems containing the Zmax 1 gene and systems containing the *HBM* gene. Such systems can be, for example, cell-free extracts, cells, tissues or living organisms, such as mice or humans. For a mutant form of Zmax1, a complete deletion of Zmax1, mutations lacking the extracellular or intracellular portion of the protein, or any other mutation in the *Zmax1* gene may be used. It is also possible to use expression of antisense *Zmax1* RNA or oligonucleotides to inhibit production of the Zmax1 protein. For a mutant form of HBM, a complete deletion of HBM, mutations lacking the extracellular or intracellular portion of the HBM protein, or any other mutation in the *HBM* gene may be used. It is also possible to use expression of antisense HBM RNA or oligonucleotides to inhibit production of the HBM protein.

Molecules identified by comparison of Zmax1 systems and HBM systems can be used as surrogate markers in pharmaceutical development or in diagnosis of human or animal bone disease. Alternatively, such molecules may be used in treatment of bone disease. *See,* Schena et al., Science, 270:467-470 (1995).

For example, a transgenic mouse carrying the *HBM* gene in the mouse homologue is constructed. A mouse of the genotype HBM/+ is viable, healthy and has elevated bone mass. To identify surrogate markers for elevated bone mass, HBM-/+ (i.e., heterozygous) and isogenic +/+ (i.e., wild-type) mice are sacrificed. Bone tissue mRNA is extracted from each animal, and a "gene chip" corresponding to mRNAs expressed in the +/+ individual is constructed. mRNA from different tissues is isolated from animals of each genotype, reverse-transcribed, fluorescently labeled, and then hybridized to gene fragments affixed to a solid support. The ratio of fluorescent intensity between the two populations is indicative of the relative abundance of the specific mRNAs in the +/+ and HBM/+ animals. Genes encoding mRNAs over- and under-expressed relative to the wild-type control are candidates for genes coordinately regulated by the *HBM* gene. This strategy can be similarly used to study lipid regulation.

Mice also serve as the most common experimental animal model for atherosclerosis research. There are at least three ways of inducing atherosclerosis in mice: (1) diet induced, apoE deficiency-induced and LDL receptor-deficiency induced. The methods for using a mouse model for testing agents which modulate lipid levels *in vivo* can be performed as described in Smith et al., J. Intern. Med. 242: 99-109 (1997).

One standard procedure for identification of new proteins that are part of the same signaling cascade as an already-discovered protein is as follows. Cells are treated with radioactive phosphorous, and the already-discovered protein is manipulated to be more or less active. The phosphorylation state of other proteins in the cell is then monitored by polyacrylamide gel electrophoresis and autoradiography, or similar techniques. Levels of activity of the known protein may be manipulated by many methods, including, for example, comparing wild-type mutant proteins using specific inhibitors such as drugs or antibodies, simply adding or not adding a known extracellular protein, or using antisense inhibition of the expression of the known protein (Tamura et al., Science, 280(5369):1614-7 (1998); Meng, EMB4 J., 17(15):4391-403 (1998); Cooper et al., Cell, 1:263-73 (1982)).

In another example, proteins with different levels of phosphorylation are identified in TE85 osteosarcoma cells expressing either a sense or antisense cDNA for Zmax1. TE85 cells normally express high levels of Zmax1 (Dong et al., Biochem. & Biophys. Res. Comm., 251:784-790 (1998)). Cells containing the sense construct express even higher levels of Zmax1, while cells expressing the antisense construct express lower levels. Cells are grown in the presence of ³²P, harvested, lysed, and the lysates run on SDS polyacrylamide gels to separate proteins, and the gels subjected to autoradiography (Ausubel et al., Current Protocols in Molecular Biology, John Wiley & Sons (1997)). Bands that differ in intensity between the sense and antisense cell lines represent phosphoproteins whose phosphorylation state or absolute level varies in response to levels of Zmax1. As an alternative to the ³²P-labeling, unlabeled proteins may be separated by SDS-PAGE and subjected to immunoblotting, using the commercially available anti-phosphotyrosine antibody as a probe (Thomas et al., Nature, 376(6537):267-71 (1995)). As an alternative to the expression of antisense RNA, transfection with chemically modified antisense oligonucleotides can be used (Woolf et al., Nucleic Acids Res., 18(7):1763-9 (1990)).

Many bone disorders, such as osteoporosis, have a slow onset and a slow response to treatment. It is therefore useful to develop surrogate markers for bone development and mineralization. Such markers can be useful in developing treatments for bone disorders, and for diagnosing patients who may be at risk for later development of bone disorders. Examples of preferred markers are N- and C-terminal telopeptide markers described, for example, in U.S. Patent Nos. 5,455,179, 5,641,837 and 5,652,112. In the area ofHIV disease, CD4 counts and viral load are useful surrogate markers for disease progression (Vlahov et al., JAMA, 279(1):35-40 (1998)). There is a need for analogous surrogate markers in the area of bone disease.

A surrogate marker can be any characteristic that is easily tested and relatively insensitive to non-specific influences. For example, a surrogate marker can be a molecule such as a protein or mRNA in a tissue or in blood serum. Alternatively, a surrogate marker may be a diagnostic sign such as sensitivity to pain, a reflex response or the like.

In yet another example, surrogate markers for elevated bone mass are identified using a pedigree of humans carrying the *HBM* gene. Blood samples are withdrawn from three individuals that carry the *HBM* gene, and from three closely related individuals that do not. Proteins in the serum from these individuals are electrophoresed on a two dimensional gel system, in which one dimension separates proteins by size, and another dimension separates proteins by isoelectric point (Epstein et al., Electrophoresis, 17(11):1655-70 (1996)). Spots corresponding to proteins are identified. A few spots are expected to be present in different amounts or in slightly different positions for the HBM individuals compared to their normal relatives. These spots correspond to proteins that are candidate surrogate markers. The identities of the proteins are determined by microsequencing, and antibodies to the proteins can be produced by standard methods for use in diagnostic testing procedures. Diagnostic assays for HBM proteins or other candidate surrogate markers include using antibodies described in this invention and a reporter molecule to detect HBM in human body fluids, membranes, bones, cells, tissues or extracts thereof. The antibodies can be labeled by joining them covalently or noncovalently with a substance that provides a detectable signal. In many scientific and patent literature, a variety of reporter molecules or labels are described including radionuclides, enzymes, fluorescent, chemi-luminescent or chromogenic agents (U.S. Patent Nos. 3,817,837; 3,850,752; 3,939,350; 3,996,345; 4,277,437; 4,275,149; and 4,366,241).

Using these antibodies, the levels of candidate surrogate markers are measured in normal individuals and in patients suffering from a bone disorder, such as osteoporosis, osteoporosis pseudoglioma, Engelmann's disease, Ribbing's disease, hyperphosphatasemia, Van Buchem's disease, melorheostosis, osteopetrosis, pychodysostosis, sclerosteosis, osteopoikilosis, acromegaly, Paget's disease, fibrous dysplasia, tubular stenosis, osteogenesis imperfecta, hypoparathyroidism, pseudohypoparathyroidism, pseudopseudohypoparathyroidism, primary and secondary hyperparathyroidism and associated syndromes, hypercalciuria, medullary carcinoma of the thyroid gland, osteomalacia and other diseases including lipid-mediated diseases. Techniques for measuring levels of protein in serum in a clinical setting using antibodies are well established. A protein that is consistently present in higher or lower levels in individuals carrying a particular disease or type of disease is a useful surrogate marker.

A surrogate marker can be used in diagnosis of a bone disorder. For example, consider a child that present to a physician with a high frequency of bone fracture. The underlying cause may be child abuse; inappropriate behavior by the child, or a bone disorder. To rapidly test for a bone disorder, the levels of the surrogate marker protein are measured using the antibody described above.

Levels of modification states of surrogate markers can be measured as indicators of the likely effectiveness of a drug that is being developed. It is especially convenient to use surrogate markers in creating treatments for bone disorders, because alterations in bone development or mineralization may require a long time to be observed. For example, a set of bone mRNAs, termed the "HBM-inducible mRNA set" is found to be overexpressed in HBM/+ mice as compared to +/+ mice, as described above. Expression of this set can be used as a surrogate marker. Specifically, if treatment of+/+ mice with a compound results in overexpression of the HBM-inducible mRNA set, then that compound is considered a promising candidate for further development.

Zmax1 or HBM protein or a binding fragment thereof may be used to screen compounds in any of a variety of drug screening techniques.

The Zmax1 or HBM protein or fragment employed in such a test may either be free in solution, affixed to a solid support, or borne on a cell surface. One method of drug screening utilizes eukaryotic or prokaryotic host cells which are stably transformed with recombinant nucleic acids expressing the protein or fragment, preferably in competitive binding assays. Such cells, either in viable or fixed form, can be used for standard binding assays. One may measure, for example, for the formation of complexes between a Zmax1 or HBM protein or fragment and the agent being tested, or examine the degree to which the formation of a complex between a Zmax1 or HBM protein or fragment and a known ligand is interfered with by the agent being tested.

Thus, methods of screening for drugs may comprise contacting such an agent with a Zmax1 or HBM protein, or fragment thereof, and assaying (i) for the presence of a complex between the agent and the Zmax1 or HBM protein or fragment, or (ii) for the presence of a complex between the Zmax1 or HBM protein or fragment and a ligand, by methods well known in the art. In such competitive binding assays the Zmax1 or HBM protein or fragment is typically labeled. Free Zmax1 or HBM protein or fragment is separated from that present in a protein protein complex, and the amount of free (i.e., uncomplexed) label is a measure of the binding of the agent being tested to Zmax1 or HBM or its interference with Zmax1 or HBM: ligand binding, respectively.

Another technique for drug screening provides high throughput screening for compounds having suitable binding affinity to the Zmax1 or HBM proteins and is described in detail in WO 84/03564. Briefly stated, large numbers of different small peptide test compounds are synthesized on a solid substrate, such as plastic pins or some other surface. The peptide test compounds are reacted with Zmax1 or HBM proteins and washed. Bound Zmax1 or HBM protein is then detected by methods well known in the art. Purified Zmax1 or HBM can be coated directly onto plates for use in the aforementioned drug screening techniques. However, non-neutralizing antibodies to the protein can be used to capture antibodies to immobilize the Zmax1 or HBM protein on the solid phase.

Competitive drug screening assays may be used in which neutralizing antibodies capable of specifically binding the Zmax1 or HBM protein compete with a test compound for binding to the Zmax1 or HBM protein or fragments thereof. In this manner, the antibodies can be used to detect the presence of any peptide that shares one or more antigenic determinants of the Zmax1 or HBM protein.

A further technique for drug screening involves the use of host eukaryotic cell lines or cells (such as described above) that have a nonfunctional Zmax1 or *HBM* gene. These host cell lines or cells are defective at the Zmax1 or HBM protein level The host cell lines ,or cells are grown in the presence of drug compound. The rate of growth of the host cells or impact on lipid metabolism is measured to determine if the compound is capable of regulating the growth or lipid metabolism of Zmax1 orHBM defective cells.

The goal of rational drug design is to produce structural analogs of biologically active proteins of interest or of small molecules with which they interact (e.g., agonists, antagonists, inhibitors) in order to fashion drugs which are, for example, more active or stable forms of the protein, or which, e.g., enhance or interfere with the function of a protein *in vivo*. See, e.g., Hodgson, Bio/Technology, 9:19-21 (1991). In one approach, one first determines The three-dimensional structure of a protein of interest (e.g., Zmax1 or HBM protein) or, for example, of the Zmax1- or HBM-receptor or ligand complex, by X-ray crystallography, by computer modeling or most typically, by a combination of approaches. Less often, useful information regarding the structure of a protein may be gained by modeling based on the structure of homologous proteins. An example of rational drug design is the development of HIV protease inhibitors (Erickson et al., Science, 249:527-533 (1990)). In addition, peptides (e.g., Zmax1 or HBM protein) are analyzed by an alanine scan (Wells, Methods in Enzymol., 202:390-411 (1991)). In this technique, an amino acid residue is replaced by Ala, and its effect on the peptide's activity is determined. Each of the amino acid residues of the peptide is analyzed in this manner to determine the important regions of the peptide.

It is also possible to isolate a target-specific antibody, selected by a functional assay, and then to solve its crystal structure. In principle, this approach yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced banks of peptides. Selected peptides would then act as the pharmacore.

Thus, one may design drugs which have, e.g., improved Zmax1 or HBM protein activity or stability or which act as inhibitors, agonists, antagonists, etc. of Zmax1 or HBM protein activity. By virtue of the availability of cloned Zmax1 or HBM sequences, sufficient amounts of the Zmax1 or HBM protein may be made available to perform such analytical studies as X-ray crystallography. In addition, the knowledge of the Zmax1 or HBM protein sequence provided herein will guide those employing computer modeling techniques in place of, or in addition to x-ray crystallography.

### XIX. Methods of Use: Avian and Mammalian Animal Husbandry

The Zmax1 DNA and Zmax1 protein and/or the HBM DNA and HBM protein can be used for vertebrate and preferably human therapeutic agents and for avian and mammalian veterinary agents, including for livestock breeding. Animals contemplated as subjects include livestock (e.g., cattle, pigs, sheep, goats, horses, buffalo, etc.), primates, canines, felines, rodents, birds, as well as reptiles, fish, and amphibians. Birds, including, for example, chickens, roosters, hens, turkeys, ostriches, ducks, pheasants and quails, can benefit from the identification of the gene and pathway for high bone mass. In many examples cited in literature (for example, McCoy et al., Res. Vet. Sci., 60(2):185-186 (1996)), weakened bones due to husbandry conditions cause cage layer fatigue, osteoporosis and high mortality rates. Additional therapeutic agents to treat osteoporosis or other bone disorders in birds can have considerable beneficial effects on avian welfare and the economic conditions of the livestock industry, including, for example, meat and egg production.

### XX. Methods of use: Diagnostic assays using Zmax1-specific oligonucleotides for detection of genetic alterations affecting bone development and lipid regulation.

In cases where an alteration or disease of bone development or lipid metabolism is suspected to involve an alteration of the *Zmax1* gene or the *HBM* gene, specific oligonucleotides may be constructed and used to assess the level of Zmax1 mRNA or HBM mRNA, respectively, in bone tissue or in another tissue that affects bone development.

For example, to test whether a person has the *HBM* gene, which affects bone density and lipid regulation, polymerase chain reaction can be used. Two oligonucleotides are synthesized by standard methods or are obtained from a commercial supplier of custom-made oligonucleotides. The length and base composition are determined by standard criteria using the Oligo 4.0 primer Picking program (Wojchich Rychlik, 1992). One of the oligonucleotides is designed so that it will hybridize only to HBM DNA under the PCR conditions used. The other oligonucleotide is designed to hybridize a segment of Zmax1 genomic DNA, such that amplification of DNA using these oligonucleotide primers produces a conveniently identified DNA fragment. For example, the pair of primers CCAAGTTCTGAGAAGTCC (SEQ ID NO:32) and AATACCTGAAACCA TACCTG (SEQ ID NO:33) will amplify a 530 base pair DNA fragment from a DNA sample when the following conditions are used: step 1 at 95°C for 120 seconds; step 2 at 95°C for 30 seconds; step 3 at 58°C for 30 seconds; step 4 at 72°C for 120 seconds; where steps 2-4 are repeated 35 times. Tissue samples may be obtained from hair follicles, whole blood, or the buccal cavity.

The fragment generated by the above procedure is sequenced by standard techniques. Individuals heterozygous for the *HBM* gene will show an equal amount of G and T at the second position in the codon for glycine 171. Normal or homozygous wild-type individuals will show only G at this position.

Other amplification techniques besides PCR may be used as alternatives, such as ligation-mediated PCR or techniques involving Q-beta replicase (Cahill et al., Clin. Chem., 37(9):1482-5 (1991)). For example, the oligonucleotides AGCTGCTCGTAGCTGTCTCT CCCTGGATCACGGGTACATGTACTGGACAGACTGGGT (SEQ ID NO:34) and TGAGACGCCCCGGATTGAGCGGGCAGGGATAGCTTATTCCCTGTGCCGCATTACG GC (SEQ m NO:35) can be hybridized to a denatured human DNA sample, treated with a DNA ligase, and then subjected to PCR amplification using the primer oligonucleotides AGCTGCTCGTAG CTGTCTCTCCCTGGA (SEQ ID NO:36) and GCCGTAATGCGGCACAGGGAATAAGCT (SEQ ID NO:37). In the first two oligonucleotides, the outer 27 bases are random sequence corresponding to primer binding sites, and the inner 30 bases correspond to sequences in the *Zmax1* gene. The T at the end of the first oligonucleotide corresponds to the *HBM* gene. The first two oligonucleotides are ligated only when hybridized to human DNA carrying the *HBM* gene, which results in the formation of an amplifiable 114 bp DNA fragment.

Products of amplification can be detected by agarose gel electrophoresis, quantitative hybridization, or equivalent techniques for nucleic acid detection known to one skilled in the art of molecular biology (Sambrook et al., Molecular Cloning: A Laboratory Manual, Cold Spring Harbor Laboratory, Cold Spring, NY (1989)).

Other alterations in the *Zmax1* gene or the *HBM* gene may be diagnosed by the same type of amplification-detection procedures, by using oligonucleotides designed to identify those alterations. These procedures can be used in animals as well as humans to identify alterations in Zmax1 or HBM that affect bone development and/or lipid metabolism or levels.

Expression of Zmax1 or HBM in bone tissue may be accomplished by fusing the cDNA of Zmax1 or HBM, respectively, to a bone-specific promoter in the context of a vector for genetically engineering vertebrate cells. DNA constructs are introduced into cells by packaging the DNA into virus capsids, by the use of cationic liposomes, electroporation, or by calcium phosphate transfection. Transfected cells, preferably osteoblasts, may be studied in culture or may be introduced into bone tissue in animals by direct injection into bone or by intravenous injection of osteoblasts, followed by incorporation into bone tissue (Ko et al., Cancer Research, 56(20):4614-9 (1996)). For example, the osteocalcin promoter, which is specifically active in osteoblasts, may be used to direct transcription of the *Zmax1* gene or the *HBM* gene. Any of several vectors and transfection methods may be used, such as retroviral vectors, adenovirus vectors, or vectors that are maintained after transfection using cationic liposomes, or other methods and vectors described herein.

Similarly Zmax1, or HBM can be expressed in liver tissue or in other lipid-metabolism or lipid-regulating cells, such as lipid laden foam cells or lesion cells. This can be accomplished by fusing the cDNA of Zmax1 or HBM respectively to, for example, a liver specific promoter or other suitable promoter in the context of a vector for genetically engineering vertebrate cells. DNA constructs are introduced into cells by packaging the DNA into, for example: virus capsids, by the use of cationic liposomes, electroporation, or calcium phosphate transfection. The transfected cells, preferably liver cells, may be studied in culture or can be introduced into animals by direct injection into the liver or other cell involved in lipid regulation or metabolism. The vectors and transfection methods to be used are similar to those described herein.

Alteration of the level of functional Zmax1 protein or HBM protein affects the level of bone mineralization and lipid levels. By manipulating levels of functional Zmax1 protein or HBM protein, it is possible to affect bone development and to increase or decrease levels of bone mineralization as well as lipid levels. For example, it may be useful to increase bone mineralization in patients with osteoporosis. Alternatively, it may be useful to decrease bone mineralization in patients with osteopetrosis or Paget's disease. Alteration of Zmax1 levels or HBM levels can also be used as a research tool. Specifically, it is possible to identify proteins, mRNA and other molecules whose level or modification status is altered in response to changes in functional levels of Zmax1 or HBM. The pathology and pathogenesis of bone disorders is known and described, for example, in Rubin and Farber (Eds.), Pathology, 2nd Ed., S.B. Lippincott Co., Philadelphia, PA (1994).

Zmax1 or HBM protein levels can be altered to regulate lipid levels in a cell or a subject. The pathology and pathogenesis of atherosclerosis and arteriosclerosis is known and described, for example, in Edwin L. Bierman, "Atherosclerosis and Other Forms of Arteriosclerosis," in Harrison's Principles of Internal Medicine, 1106-1116 (13th Ed., 1994). Modulation of lipid levels may be useful to lower certain levels of lipids (*e.g.*, LDL) in patients with arteriosclerosis and/or atherosclerosis, as well as conditions and diseases affiliated with atherosclerosis and arteriosclerosis, as described by Bierman (1994).

A variety of techniques can be used to alter the levels of functional Zmax1 or HBM. For example, intravenous or intraosseous injection of the extracellular portion of Zmax1 or mutations thereof, or HBM or mutations thereof, will alter the level of Zmax1 activity or HBM activity, respectively, in the body of the treated human, animal or bird. Truncated versions of the Zmax1 protein or HBM protein can also be injected to alter the levels of functional Zmax1 protein or HBM protein, respectively. Certain forms of Zmax1 or HBM enhance the activity of endogenous protein, while other forms are inhibitory.

The HBM protein may be used to treat osteoporosis or arteriosclerosis, or alternatively, the extracellular portion of the HBM protein is used. This HBM protein may be optionally modified by the addition of a moiety that causes the protein to adhere to the surface of cells. The protein is prepared in a pharmaceutically acceptable solution and is administered by injection or another method that achieves acceptable pharmacokinetics and distribution.

In the method described herein, Zmax1 or HBM levels may be increased or decreased by gene therapy techniques. To increase Zmax1 or HBM levels, osteoblasts or another useful cell type are genetically engineered to express high levels of Zmax1 or HBM as described above. Alternatively, to decrease Zmax1 or HBM levels, antisense constructs that specifically reduce the level of translatable Zmax1 or HBM mRNA can be used. In general, a tissue-nonspecific promoter may be used, such as the CMV promoter or another commercially available promoter found in expression vectors (Wu et al., Toxicol. Appl. Pharmacol., 141(1):330-9 (1996)). A Zmax1 cDNA or its antisense may be transcribed by a bone-specific promoter, such as the osteocalcin or another promoter, to achieve specific expression in bone tissue. In this way, if a Zmax1-expressing DNA construct or HBM-expressing construct is introduced into non-bone tissue, it will not be expressed. Similarly, if a liver-specific promoter is used to express the HBM or Zmax1 proteins in liver or other cell involved in lipid regulation or metabolism, the DNA construct with, for example, a liver-specific promoter will not be expressed in other non-liver tissues.

Alternatively, antibodies against Zmax1 or HBM may be used to inhibit its function. Such antibodies are identified herein.

In addition, drugs that inhibit Zmax1 function or HBM function may be used. Such drugs are described herein and optimized according to techniques of medicinal chemistry well known to one skilled in the art of pharmaceutical development

Zmax1 and HBM interact with several proteins, such as ApoE. Molecules that inhibit the interaction between Zmax1 or HBM and ApoE or another binding partner are expected to alter bone development and mineralization. Such inhibitors may be useful as drugs in the treatment of osteoporosis, osteopetrosis, or other diseases of bone mineralization. Such inhibitors may be low molecular weight compounds, proteins or other types of molecules. *See,* Kim et al., J. Biochem. (Tokyo), 124(6):1072-1076 (1998).

Inhibitors of the interaction between Zmax1 or HBM and interacting proteins may be isolated by standard drug-screening techniques. For example, Zmax1 protein, (or a fragment thereof) or HBM protein (or a fragment thereof) can be immobilized on a solid support such as the base of microtiter well. A second protein or protein fragment, such as ApoE is derivatized to aid in detection, for example with fluorescein. Iodine, or biotin, then added to the Zmax1 or HBM in the presence of candidate compounds that may specifically inhibit this protein-protein domain of Zmax1 or HBM, respectively, and thus avoid problems associated with its transmembrane segment Drug screens of this type are well known to one skilled in the art of pharmaceutical development.

Because Zmax1 and HBM are involved in bone development and lipid regulation, proteins that bind to Zmax1 and HBM are also expected to be involved in bone development and lipid regulation. Such binding proteins can be identified by standard methods, such as co-immunoprecipitation, co-fractionation, or the two-hybrid screen (Ausubel et al., Current Protocols in Molecular Biology, John Wiley & Sons (1997)). For example, to identify Zmax1-interacting proteins or HBM-interacting proteins using the two-hybrid system, the extracellular domain of Zmax1 or HBM is fused to LexA and expressed for the yeast vector pEG202 (the "bait") and expressed in the yeast strain EGY48. The yeast strain is transformed with a "prey" library in the appropriate vector, which encodes a galactose-inducible transcription-activation sequence fused to candidate interacting proteins. The techniques for initially selecting and subsequently verifying interacting proteins by this method are well known to one skilled in the art of molecular biology (Ausubel et al, Current Protocols in Molecular Biology, John Wiley & Sons (1997)).

Proteins that interact with HBM, but not Zmax1 may be identified using a variation of the above procedure (Xu et al., Proc. Natl. Acad. Sci. USA, 94(23):12473-8 (Nov. 1997)). This variation of the two-hybrid system uses two baits, and Zmax1 and HBM are each fused to LexA and TetR, respectively. Alternatively, proteins that interact with the HBM but not Zmax1 are also isolated. These procedures are well known to one skilled in the art of molecular biology, and are a simple variation of standard two-hybrid procedures.

As an alternative method of isolating Zmax1 or HBM interacting proteins, a biochemical approach is used. The Zmax1 protein or a fragment thereof, such as the extracellular domain, or the HBM protein or a fragment thereof, such as the extracellular domain, is chemically coupled to Sepharose beads. The Zmaxl- or HBM-coupled beads are poured into a column. An extract of proteins, such as serum proteins, proteins in the supernatant of a bone biopsy, or intracellular proteins from gently lysed TE85 osteoblastic cells, is added to the column. Non-specifically bound proteins are eluted, the column is washed several times with a low-salt buffer, and then tightly binding proteins are eluted with a high-salt buffer. These, are candidate proteins that bind to Zmax1 or HBM, and can be tested for specific binding by standard tests and control experiments. Sepharose beads used for coupling proteins and the methods for performing the coupling are commercially available (Sigma), and the procedures described here are well known to one skilled in the art of protein biochemistry.

As a variation of the above procedure, proteins that are eluted by high salt from the Zmaxl- or HBM-Sepharose column are then added to an HBM-Zmax1-sepharo column. Proteins that flow through without sticking are proteins that bind to Zmax1 but not to HBM. Alternatively, proteins that bind to the HBM protein and not to the Zmax1 protein can be isolated by reversing the order in which the columns are used. Similar columns can be prepared for use in assessing lipid regulation in liver and other tissues and cells involved in lipid regulation and or metabolism.

### XXI. Method of Use: Transformation-Associated Recombination (TAR) Cloning

Essential for the identification of novel allelic variants of Zmax1 is the ability to examine the sequence of both copies of the gene in an individual. To accomplish this, two "hooks," or regions of significant similarity, are identified within the genomic sequence such that they flank the portion of DNA that is to be cloned. Most preferably, the first of these hooks is derived from sequences 5' to the first exon of interest and the second is derived from sequences 3' to the last exon of interest. These two "hooks" are cloned into a bacterial/yeast shuttle vector such as that described by Larionov et al., Proc. Natl. Acad Sci. USA, 94:7384-7387 (1997). Other similar vector systems may also be used. To recover the entire genomic copy of the *Zmax1* gene, the plasmid containing the two "hooks" is linearized with a restriction endonuclease or is produced by another method such as PCR. This linear DNA fragment is introduced into yeast cells along with human genomic DNA. Typically, the yeast *Saccharomyces cerevisiae* is used as a host cell, although chicken host cells can be used as well (Larionov et al., Genet. Eng. (NY). 21:37-55 (1999). During and after the process of transformation, the endogenous host cell converts the linear plasmid to a circle by a recombination event whereby the region of the human genomic DNA homologous to the "hooks" is inserted into the plasmid. This plasmid can be recovered and analyzed by methods well known to one skilled in the art. Obviously, the specificity for this reaction requires the host cell machinery to recognize sequences similar to the "hooks" present in the linear fragment. However, 100% sequence identity is not required, as shown by Kouprina et al., Genomics, 53(1):21-28 (October 1998), where the author describes using degenerate repeated sequences common in the human genome to recover fragments of human DNA from a rodent/human hybrid cell line.

In another example, only one "hook" is required, as described by Larionov et al., Proc. Natl. Acad Sci. USA, 95(8):4469-74 (April 1998). For this type of experiment, termed "radial TAR cloning," the other region of sequence similarity to drive the recombination is derived from a repeated sequence from the genome. In this way, regions of DNA adjacent to the *Zmax1* gene coding region can be recovered and examined for alterations that may affect function.

### XXII. Methods of Use: Genomic Screening

The use of polymorphic genetic markers linked to the *HBM* gene or to Zmax1 is very useful in predicting susceptibility to osteoporosis or other bone diseases. Polymorphic genetic markers linked to the *HBM* gene or the *Zmax1* gene also can be used to predict susceptibility to arteriosclerosis or atherosclerosis and conditions related thereto. Koller et al., Amer. J. Bone Min. Res., 13:1903-1908 (1998) have demonstrated that the use of polymorphic genetic markers is useful for linkage analysis. Similarly, the identification of polymorphic genetic markers within the *HBM* gene will allow the identification of specific allelic variants that are in linkage disequilibrium with other genetic lesions that affect bone development. Using the DNA sequence from the BACs, a dinucleotide CAn repeat was identified and two unique PCR primers that will amplify the genomic DNA containing this repeat were designed, as shown below:
B200E21C16_L: GAGAGGCTATATCCCTGGGC (SEQ ID NO:38)
B200E21C16_R: ACAGCACGTGTTTAAAGGGG (SEQ ID NO:39)
and used in the genetic mapping study.

This method has been used successfully by others skilled in the art (e.g., Sheffield et al., Genet., 4:1837-1844 (1995); LeBlanc-Straceski et al., Genomics, 19:341-9 (1994); Chen et al., Genomics, 25:1-8 (1995)). Use of these reagents with populations or individuals will predict their risk for osteoporosis. Similarly, single nucleotide polymorphisms (SNPs), such as those shown in Table 4 above, can be used as well to predict risk for developing bone diseases or resistance to osteoporosis in the case of the *HBM* gene. It is also contemplated that single nucleotide polymorphisms (SNPs) such as those described above, may be used to predict the risk in a subject for developing arteriosclerosis and atherosclerosis and related conditions.

### XXIII. Methods of Use: Modulators of Tissue Calcification

The calcification of tissues in the human body is well documented. Towler et al., J. Biol. Chem., 273:30427-34 (1998) demonstrated that several proteins known to regulate calcification of the developing skull in a model system are expressed in calcified aorta. The expression of Msx2, a gene transcribed in osteoprogenitor cells, in calcified vascular tissue indicates that genes which are important in bone development are involved in calcification of other tissues. Treatment with HBM protein, agonists or antagonists is likely to ameliorate calcification (such as the vasculature, dentin and bone of the skull visera) due to its demonstrated effect on bone mineral density. In experimental systems where tissue calcification is demonstrated, the over-expression or repression of Zmax1 activity permits the identification of molecules that are directly regulated by the *Zmax1* gene. These genes are potential targets for therapeutics aimed at modulating tissue calcification. For example, an animal, such as the LDLR -/-, mouse is fed a high fat diet and is observed to demonstrate expression of markers of tissue calcification, including Zmax1. These animals are then treated with antibodies to Zmax1 or HBM protein, antisense oligonucleotides directed against Zmax1 or HBM cDNA, or with compounds known to bind the Zmax1 or HBM protein or its binding partner or ligand. RNA or proteins are extracted from the vascular tissue and the relative expression levels of the genes expressed in the tissue are determined by methods well known in the art. Genes that are regulated in the tissue are potential therapeutic targets for pharmaceutical development as modulators of tissue calcification.

The nucleic acids, proteins, peptides, amino acids, small molecules or other pharmaceutically useful compounds described herein that are to be given to an individual may be administered in the form of a composition with a pharmaceutically acceptable carrier, excipient or diluent, which are well known in the art. The individual may be a mammal or a bird, preferably a human, a rat, a mouse or bird. Such compositions may be administered to an individual in a pharmaceutically effective amount The amount administered will vary depending on the condition being treated and the patient being treated. The compositions may be administered alone or in combination with other treatments.

### XXIV. Pharmaceutical Compositions

Pharmaceutical compositions may comprise a lipid mediating agent which modulated HBM and/or Zmax1 activity in combination with a lipoprotein modulating agent (e.g., blofibrate, gemfibrozil, nicotinic acid, cholestyramine, cholestipol, lovastatin, simvastatin, pravastain, probucol, premarin or estradiol.). Liprotein modulating agents can include compounds or compositions which modulate (*e.g.,* up-regulate or down-regulate) LDL, VLDL, HDL or IDL levels.

The lipid mediating agent, which modulates HBM and/or Zmax1 activity, can include proteins, monoclonal antibodies or fragments thereof, chemicals, and mimetics. One contemplated pharmaceutical composition can comprise the monoclonal antibody and a pharmaceutically acceptable carrier. For the purposes of the present invention, a "pharmaceutically acceptable carrier" can be any of the standard carriers well known in the art. For example, suitable carriers can include phosphate buffered saline solutions, emulsions such as oil/water emulsions, and various types of wetting agents. Other carriers can also include sterile solutions, tablets, coated tablets, and capsules. Typically, such carriers can also contain excipients such as starch, milk, sugar, types of clay, gelatin, stearic acid, or salts thereof, magnesium or calcium sterate, talc, vegetable fats or oils, gums, glycerols, or other known excipients. Such carriers can also include flavors and color additives, preservatives, or other ingredients. Compositions comprising such carriers are formulated by well known conventional means. See REMINGTON'S PHARMACEUTICAL SCIENCE (15th ed. 1980).

For diagnostic purposes, the antibodies and recombinant binding proteins can be either labeled or unlabeled. Typically, diagnostic assays entail detecting the formation of a complex through the binding of the monoclonal antibody or recombinant binding protein to a HBM protein or Zmax1 protein. When unlabeled, the antibodies and recombinant binding proteins find use in agglutination assays. In addition, unlabeled antibodies can be used in combination with other labeled antibodies (second antibodies) that are specifically reactive with the monoclonal antibody or recombinant binding protein, such as antibodies specific for immunoglobulin. Alternatively, the monoclonal antibodies and recombinant binding proteins can be directly labeled. A wide variety of labels can be employed, such as radionuclides (e.g., ⁹⁹Tc, ¹¹¹In, ¹²³I and ¹³¹I), fluorescers, enzymes, enzyme substrates, enzyme cofactors, enzyme inhibitors, ligands (particularly haptens), etc. Numerous types of immunoassays are well known in the art.

Commonly, the monoclonal antibodies and recombinant binding proteins of the present invention are used in fluorescent assays, where the subject antibodies or recombinant binding proteins are conjugated to a fluorescent molecule, such as fluorescein isothiocyanate (FITC).

The examples provided below illustrate the invention:

### EXAMPLES

Standard techniques well known in the art or the techniques specifically described below were utilized.

### Example 1

The propositus was referred by her physicians to the Creighton Osteoporosis Center for evaluation of what appeared to be unusually dense bones. She was 18 years old and came to medical attention two years previous because of back pain, which was precipitated by an auto accident in which the car in which she was riding as a passenger was struck from behind. Her only injury was soft tissue injury to her lower back that was manifested by pain and muscle tenderness. There was no evidence of fracture or subluxation on radiographs. The pain lasted for two years, although she was able to attend school full time. By the time she was seen in the Center, the pain was nearly resolved and she was back to her usual activities as a high school student. Physical exam revealed a normal healthy young woman standing 66 inches and weighing 128 pounds. Radiographs of the entire skeleton revealed dense looking bones with thick cortices. All bones of the skeleton were involved. Most importantly, the shapes of all the bones were entirely normal. The spinal BMC was 94.48 grams in L1-4, and the spinal BMD was 1.667 gm/cm² in L1-4. BMD was 5.62 standard deviations (SD) above peak skeletal mass for women. These were measured by DXA using a Hologic 2000~. Her mother was then scanned and a lumbar spinal BMC of 58.05 grams and BMD of 1.500 gm/cm² were found. Her mother's values place her 4.12 SD above peak mass and 4.98 SD above her peers. Her mother was 51 years old, stood 65 inches and weighed 140 pounds. Her mother was in excellent health with no history of musculoskeletal or other symptoms. Her father's lumbar BMC was 75.33 grams and his BMD was 1.118 gm/cm². These values place him 0.25 SD above peak bone mass for males. He was in good health, stood 72 inches tall, and weighed 187 pounds.

These clinical data suggested that the propositus inherited a trait from her mother, which resulted in very high bone mass, but an otherwise normal skeleton, and attention was focused on the maternal kindred. In U.S. Patent No. 5,691,153, twenty- two of these members had measurement of bone mass by DXA. In one case, the maternal grandfather of the propositus, was deceased, however, medical records, antemortem skeletal radiographs and a gall bladder specimen embedded in paraffin for DNA genotyping were obtained. His radiographs showed obvious extreme density of all of the bones available for examination including the femur and the spine, and he was included among the affected members. In this invention, the pedigree has been expanded to include 37 informative individuals. These additions are a significant improvement over the original kinship (Johnson et al., Am. J. Hum. Genet., 60:1326-1332 (1997)) because, among the fourteen individuals added since the original study, two individuals harbor key crossovers. X-linkage is ruled out by the presence of male-to-male transmission from individual 12 to 14 and 15.

### Example 2

The present invention describes DNA sequences derived from two BAC clones from the *HBM* gene region, as evident in Table 6 below, which is an assembly of these clones. Clone b200e21-h (ATCC No. 980812; SEQ ID NOS: 10-11) was deposited at the American Type Culture Collection (ATCC), 10801 University Blvd., Manassas, VA 20110-2209 U.S.A., on December 30, 1997. Clone b527d12-h (ATCC No. 980720; SEQ ID NOS: 5-9) was deposited at the American Type Culture Collection (ATCC), 10801 University Blvd., Manassas, VA 20110-2209 U.S.A., on October 2, 1998. These sequences are unique reagents that can be used by one skilled in the art to identify DNA probes for the *Zmax1* gene, PCR primers to amplify the gene, nucleotide polymorphisms in the *Zmax1* gene, or regulatory elements of the *Zmax1* gene.

**TABLE 6**

| **Contig** | **ATCC No.** | **SEQ ID NO.** | **Length** |
|---|---|---|---|
| b527d12-h_Contig302G | 980720 | 5 | 3096 |
| b527d12-h_contig306G | 980720 | 6 | 26928 |
| b527d12-h_contig307G | 980720 | 7 | 29430 |
| b527d12-h_contig308G | 980720 | 8 | 33769 |
| bS27d12-h_contig309G | 980720 | 9 | 72049 |
| b200e21-h_contigl | 980812 | 10 | 8705 |
| b200e21-h_contig4 | 980812 | 11 | 66933 |

### Example 3

Since Zmax1 has similarity to the LDL receptor family of genes, it may be involved in lipid metabolism. However, others have reported that lipid profile variables did not show significant association with bone mass and could not be used as indicators for bone mineral density (Zabaglia et al., "An exploratory study of association between lipid profile and bone mineral density in menopausal women in a Campinas reference hospital," Cad. Saude Publica 14: 779-86 (1998)). Zmax1 may be normally involved in regulating bone density by depositing calcium during bone remodeling. The HBM mutation may result in increased deposition thus conferring denser bone structure. Interestingly, atherosclerotic plaques contain calcified material and express a variety of genes involved in bone differentiation.

To test whether the HBM gene was involved in lipid regulation, biochemical tests were performed to measure serum level of various lipid containing molecules or precursors in affected and unaffected HBM family members to test whether the HBM mutation in the Zmax1 gene effects lipid metabolism. Table 7 shows the results of testing eight HBM individuals and seven unaffected individuals. Wilcoxon rank-sum tests (non-parametric equivalent of a T-test) were performed to assess whether levels of biochemical markers from affected HBM individuals deviated from unaffected individuals. The data obtained were analyzed separately by gender, as well as by combining values from males and females, when appropriate.

Standard diagnostic protocols were used to determine the concentration (mg/dL) with triglycerides, cholesterol, high density lipoprotein (HDL), low density lipoprotein (LDL), very low density lipoprotein (VLDL), apolipoprotein A-1 (APO A-1), apolipoprotein B (APO B), and lipoprotein a (LIPOa). For such procedures, see for example, F. W. HEMMING, LIPID ANALYSIS (Bios Scientific Pub. 1996) and J. M. ORDOVAS, LIPOPROTEIN PROTOCOLS (Humana Press Inc., 1997). The genotype for apolipoprotein E (APO E) was also reported. There are three common alleles (*e.g.*, E2, E3 and E4). The affected and unaffected HBM family members are heterozygous or homozygous for the alleles.

The results obtained were statistically significant: (1) Triglyceride levels were generally lower in affected individuals than in unaffected individuals, and (2) very low density lipoprotein (VLDL) levels were generally lower in affected individuals than in unaffected individuals. Additionally, the following comparisons approached statistical significance (p=0.06): (1) high density lipoprotein (HDL) levels were higher in affected males than in unaffected males, and (2) the ratio of low density lipoprotein (LDL) to high density lipoprotein (HDL) was generally higher in affected males than in unaffected males.

In Table 7, "ARUP" is ARUP Laboratories, 500 Chipeta Way, Salt Lake City, UT 84108 where one of the studies was performed. "SJH" refers to the second center which performed these studies, Creighton Medical Laboratories, 28th & Burt, Dental-Rm 306, Omaha, NE 68178. APO-A1, APO-B and LIPO-a are reported in mg.dL. Total serum levels also are in mg/dL.

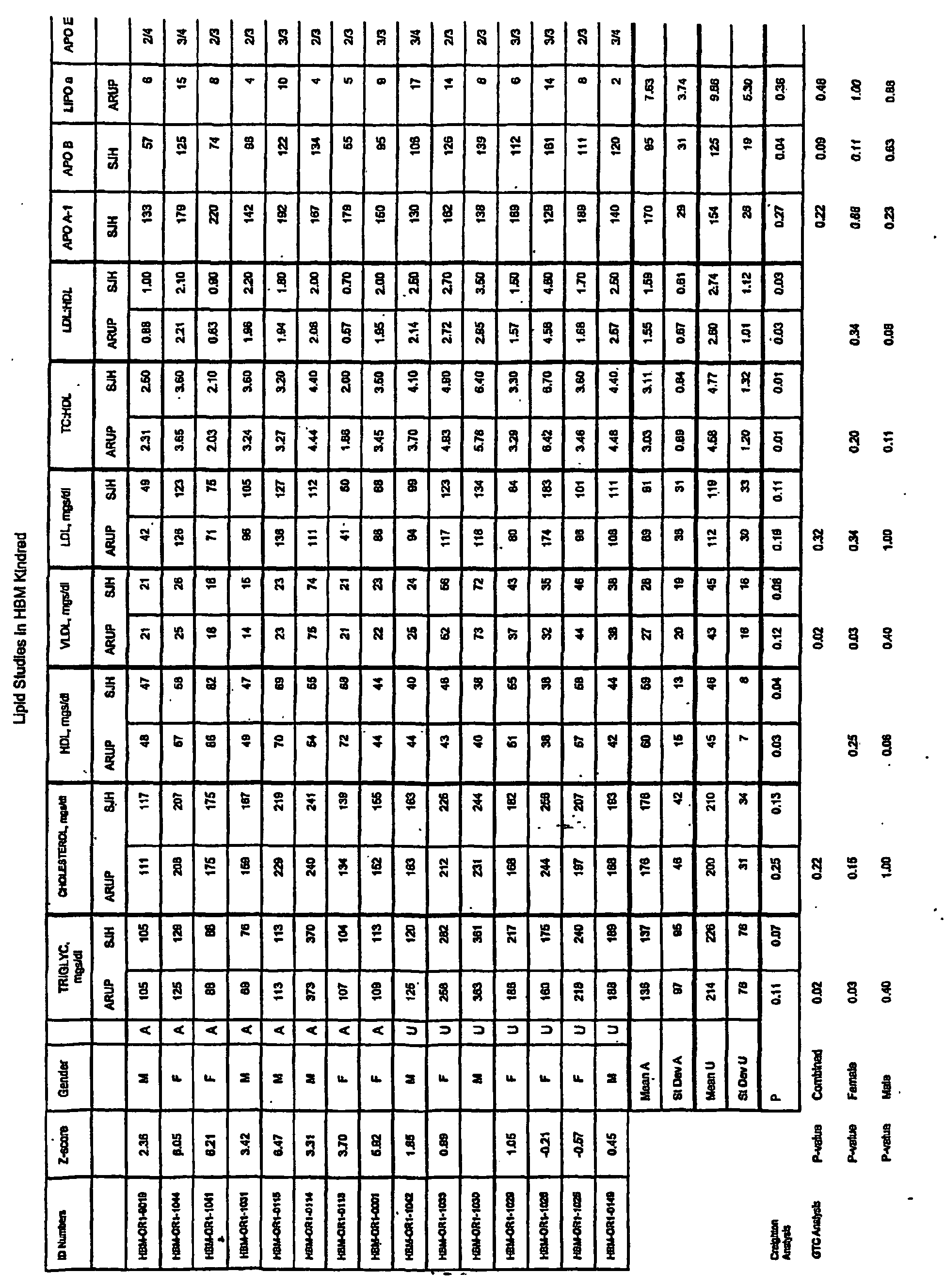

## Claims

1. A method for identifying a molecule involved in lipid regulation comprising:
(A) constructing a first host that contains a gene having the sequence set out in SEQ ID NO: 1 or a polypeptide encoded thereby;
(B) constructing a second host that contains a gene having the sequence set out in SEQ ID NO: 2 or a polypeptide encoded thereby;
(C) analyzing a difference between the first host and the second host; and
(D) identifying a molecule that, when added to the first host, causes the first host to exhibit lipid levels characteristic of the second host.

2. The method of claim 1 wherein said molecule is a protein.

3. The method of claim 2 further comprising producing an antibody to the protein.

4. The method of claim 1, 2 or 3 wherein the lipid levels characteristic of the second host are lower trigylcerides, very low density lipoproteins and low density lipoproteins and/or increased high density lipoproteins.

5. The method of any one of claims 1 to 4, wherein the first and second hosts are eukaryotic or prokaryotic cells.

6. The method of claim 5, wherein the eukaryotic cells are mesenchymal cells or liver cells.

7. The method of any one of claims 1 or 4, wherein the host is a non-human animal.

8. The method of claim 7, wherein the animal is a livestock animal, a primate, a canine, a feline, a rodent, a bird, a reptile, a fish, or an amphibian.

9. A method for identifying a patient who has an increased disposition to arteriosclerosis, an arteriosclerosis associated condition, hypercholesteremia, hypolipidemia or atherosclerosis, which method comprises screening a sample from the patient to determine the presence or absence of a polypeptide having the sequence set out in SEQ ID NO: 3 or a nucleic acid encoding a said polypeptide in the said sample, thereby to determine if the patient has a genetic predisposition to a said condition.

10. The method of claim 9, wherein haplotype analysis is used to determine whether or not said nucleic acid is present in the said sample.

11. The method of claim 9, wherein an antibody is used to determine whether or not the said polypeptide is present in the said sample.

12. A method of expressing the polypeptide having the sequence of SEQ ID NO: 3 in tissue comprising constructing an expression vector comprising a promoter that directs expression in tissue operably linked to SEQ ID NO:1 and the tissue in which the said polypeptide is expressed is a lipid regulating cell or a cell involved in lipid metabolism.

## Patentansprüche

1. Verfahren zum Identifizieren eines Moleküls, das an der Lipidregulation beteiligt ist, umfassend:
(A) das Konstruieren eines ersten Wirts, der ein Gen mit der in SEQ ID NO:1 angegebenen Sequenz oder ein **dadurch** kodiertes Polypeptid enthält;
(B) das Konstruieren eines zweiten Wirts, der ein Gen mit der in SEQ ID NO:2 angegebenen Sequenz oder ein **dadurch** kodiertes Polypeptid enthält;
(C) das Analysieren eines Unterschieds zwischen dem ersten und zweiten Wirt; und
(D) das Identifizieren eines Moleküls, das bei Zugabe zum ersten Wirt bewirkt, dass der erste Wirt Lipidspiegel aufweist, die charakteristisch für den zweiten Wirt sind.

2. Verfahren nach Anspruch 1, wobei es sich bei dem Molekül um ein Protein handelt.

3. Verfahren nach Anspruch 2, ferner umfassend das Erzeugen eines Antikörpers gegen das Protein.

4. Verfahren nach Anspruch 1, 2 oder 3, wobei es sich bei den für den zweiten Wirt charakteristischen Lipidspiegeln um niedrigere Spiegel an Triglyceriden, Lipoproteinen von sehr geringer Dichte und Lipoproteinen von geringer Dichte und/oder um erhöhte Spiegel von Lipoproteinen von hoher Dichte handelt.

5. Verfahren nach einem der Ansprüche 1 bis 4, wobei es sich beim ersten und zweiten Wirt um eukaryontische oder prokaryontische Zellen handelt,

6. Verfahren nach Anspruch 5, wobei es sich bei den eukaryontischen Zellen um mesenchymale Zellen oder Leberzellen handelt,

7. Verfahren nach einem der Ansprüche 1 bis 4, wobei es sich beim Wirt um ein nicht-humanes Tier handelt.

8. Verfahren nach Anspruch 7, wobei es sich beim Tier um Zuchtvieh, einen Primaten, einen Hund, eine Katze, ein Nagetier, einen Vogel, eine Reptilie, einen Fisch oder eine Amphibie handelt,

9. Verfahren zum Identifizieren eines Patienten mit einer erhöhten Disposition in Bezug auf Arteriosklerose, einen mit Arteriosklerose verbundenen Zustand, Hypercholesterinämie, Hypolipidämie oder Atherosklerose handelt, wobei das Verfahren das Screening einer Probe des Patienten zur Bestimmung der Anwesenheit oder Abwesenheit eines Polypeptids mit der in SEQ ID NO:3 angegebenen Sequenz oder einer Nucleinsäure, die für das Polypeptid kodiert, in der Probe umfasst, um **dadurch** festzustellen, ob der Patient eine genetische Prädisposition in bezug auf den genannten Zustand aufweist.

10. Verfahren nach Anspruch 9, wobei eine Haplotyp-Analyse angewandt wird, um zu bestimmen, ob die Nucleinsäure in der Probe vorhanden ist oder nicht.

11. Verfahren nach Anspruch 9, wobei ein Antikörper verwendet wird, um festzustellen, ob in der Probe das Polypeptid vorhanden ist oder nicht.

12. Verfahren zum Exprimieren des Polypeptids mit der Sequenz gemäß SEQ ID NO:3 in Gewebe, umfassend das Konstruieren eines Expressionsvektors, der einen Promotor, der die Expression im Gewebe dirigiert, in funktioneller Verknüpfung mit SEQ ID NO:1 umfasst, wobei es sich bei dem Gewebe, in dem das Polypeptid exprimiert wird, um eine lipidregulierende Zelle oder um eine Zelle, die am Lipidstoffwechsel beteiligt ist, handelt,

## Revendications

1. Procédé permettant d'identifier une molécule impliquée dans la régulation des lipides, lequel procédé comporte :
A) le fait de construire un premier hôte qui contient un gène ayant la séquence présentée en tant que Séquence N° 1 ou un polypeptide codé par ce gène ;
B) le fait de construire un deuxième hôte qui contient un gène ayant la séquence présentée en tant que Séquence N° 2 ou un polypeptide codé par ce gène ;
C) le fait d'analyser une différence entre le premier hôte et le deuxième hôte ;
D) et le fait d'identifier une molécule qui, mise en présence du premier hôte, fait que le premier hôte présente des taux de lipides caractéristiques du deuxième hôte.

2. Procédé conforme à la revendication 1, dans lequel ladite molécule est une protéine.

3. Procédé conforme à la revendication 2, qui comporte en outre le fait de produire un anticorps dirigé contre la protéine.

4. Procédé conforme à la revendication 1, 2 ou 3, dans lequel les taux de lipides caractéristiques du deuxième hôte sont des taux relativement bas de triclycérides, de VLDL (lipoprotéines de très basse densité) et de LDL (lipoprotéines de basse densité) et/ou des taux accrus de HDL (lipoprotéines de haute densité).

5. Procédé conforme à l'une des revendications 1 à 4, dans lequel les premier et deuxième hôtes sont des cellules eucaryotes ou procaryotes.

6. Procédé conforme à la revendication 5, dans lequel les cellules eucaryotes sont des cellules mésenchymateuses ou des celules hépatiques.

7. Procédé conforme à la revendication 1 ou 4, dans lequel l'hôte est un animal non-humain.

8. Procédé conforme à la revendication 7, dans lequel ledit animal est un animal d'élevage, un primate, un canidé, un félidé, un rongeur, un oiseau, un reptile, un poisson ou un amphibien.

9. Procédé permettant d'identifier un patient grandement prédisposé à l'artériosclérose, à un état associé à l'artériosclérose, à l'hypercholestérolémie, à l'hypolipidémie ou à l'athérosclérose, lequel procédé comporte le fait de tester un échantillon provenant du patient pour établir la présence ou l'absence, dans cet échantillon, d'un polypeptide ayant la séquence présentée en tant que Séquence N° 3 ou d'un acide nucléique codant un tel polypeptide, ce qui permet de déterminer si le patient est génétiquement prédisposé audit état.

10. Procédé conforme à la revendication 9, dans lequel on a recours à une analyse d'haplotype pour déterminer si ledit acide nucléique est présent dans ledit échantillon ou non.

11. Procédé conforme à la revendication 9, dans lequel on a recours à un anticorps pour déterminer si ledit polypeptide est présent dans ledit échantillon ou non.

12. Procédé d'expression d'un polypeptide ayant la séquence présentée en tant que Séquence N° 3 dans un tissu, lequel procédé comporte le fait de construire un vecteur d'expression qui comprend un promoteur induisant l'expression tissulaire, opérationnellement lié à la Séquence N° 1, et dans lequel procédé le tissu dans lequel est exprimé ledit polypeptide est une cellule de régulation des lipides ou une cellule impliquée dans le métabolisme des lipides.
